(19) Europäisches Patentamt
European Patent Office
Office européen des brevets

(11) EP 4 458 823 A1

(12) **EUROPEAN PATENT APPLICATION**
published in accordance with Art. 153(4) EPC

(43) Date of publication:
**06.11.2024 Bulletin 2024/45**

(21) Application number: **22915177.4**

(22) Date of filing: **30.12.2022**

(51) International Patent Classification (IPC):
***C07D 405/14** (2006.01)* ***A61K 31/407** (2006.01)*
***A61P 35/00** (2006.01)*

(52) Cooperative Patent Classification (CPC):
**A61K 31/407; A61K 31/506; A61K 31/53; A61P 35/00; C07D 405/14; C07D 471/10; C07D 519/00**

(86) International application number:
**PCT/CN2022/143908**

(87) International publication number:
**WO 2023/125928 (06.07.2023 Gazette 2023/27)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **31.12.2021 CN 202111650341**
**12.08.2022 CN 202210948453**

(71) Applicant: **Hitgen Inc.**
**Chengdu, Sichuan 610200 (CN)**

(72) Inventors:
- **LI, Jin**
  **Chengdu, Sichuan 610200 (CN)**
- **BAI, Xiaoguang**
  **Chengdu, Sichuan 610200 (CN)**
- **ZHAO, Hongchuan**
  **Chengdu, Sichuan 610200 (CN)**
- **DENG, Ning**
  **Chengdu, Sichuan 610200 (CN)**
- **DENG, Jie**
  **Chengdu, Sichuan 610200 (CN)**
- **CHEN, Di**
  **Chengdu, Sichuan 610200 (CN)**
- **QU, Qingxi**
  **Chengdu, Sichuan 610200 (CN)**
- **NONG, Yunhong**
  **Chengdu, Sichuan 610200 (CN)**
- **ZHENG, Zhihui**
  **Chengdu, Sichuan 610200 (CN)**
- **BO, Chongfei**
  **Chengdu, Sichuan 610200 (CN)**
- **YAN, Yunbiao**
  **Chengdu, Sichuan 610200 (CN)**

(74) Representative: **De Clercq & Partners**
**Edgard Gevaertdreef 10a**
**9830 Sint-Martens-Latem (BE)**

# (54) MENIN INHIBITOR AND USE THEREOF

(57) Provided in the present invention is a compound represented by formula (I). The compound has Menin-MLL protein-protein interaction inhibitory activity and cell proliferation inhibitory activity. Also provided in the present invention is a use thereof in the treatment of cancer and other diseases mediated by Menin-MLL interaction.

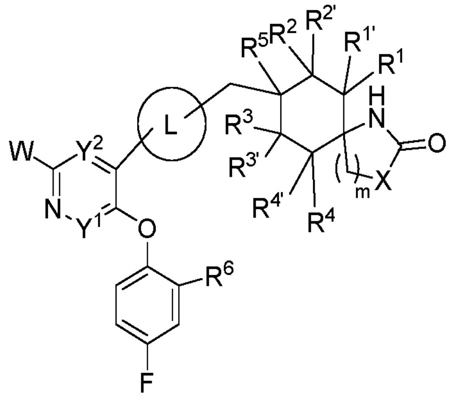

EP 4 458 823 A1

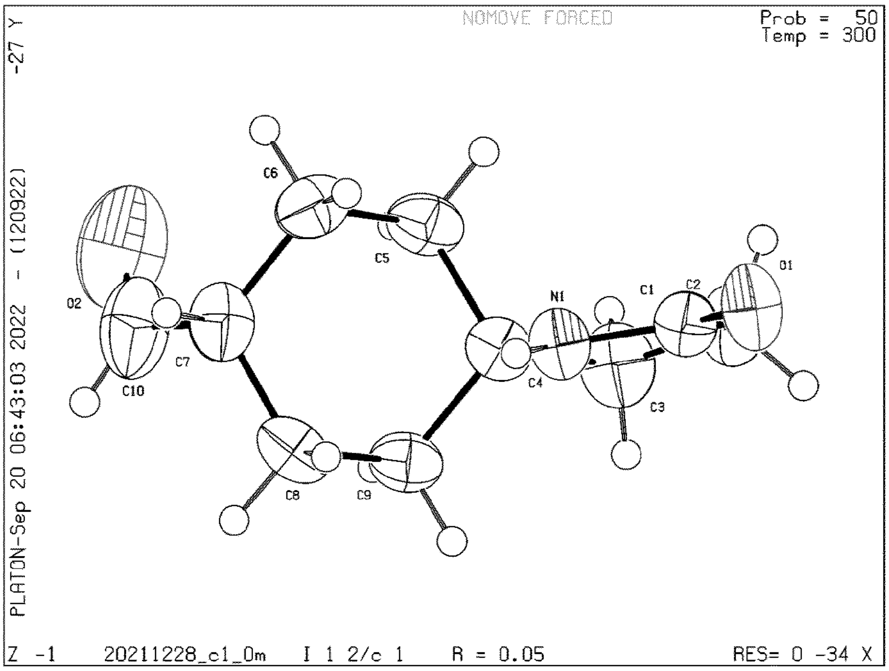
NOMOVE FORCED
Prob = 50
Temp = 300
-27 Y
PLATON-Sep 20 06:43:03 2022 - (120922)
Z -1 20211228_c1_0m I 1 2/c 1 R = 0.05 RES= 0 -34 X

FIG. 1

## Description

## FIELD

The present disclosure relates to a compound with Menin-MLL protein-protein interaction inhibitory activity and cell proliferation inhibitory activity, and use thereof in treating cancer and other diseases mediated by Menin-MLL interaction.

## BACKGROUND

Mixed-lineage leukemia (MI,L) protein is a histone methyltransferase that may mutate clinically and in biologically distinct subtypes of acute leukemia. The MLL gene family includes five members, MLL1-5, and is closely associated with the development, progression, and metastasis of various tumors. The multiple endocrine oncoprotein (Menin protein) is encoded by the multiple endocrine neoplasia type 1 (MEN1) gene, which acts as a tumor suppressor gene in endocrine organs. Menin protein interacts with many other proteins, forming a complex interaction network. Studies have demonstrated that the direct interaction of Menin with MLL1 and MI,L2 proteins is indispensable for the enzymatic activity of the complex histone methylation modification (H3K4), the transcriptional regulation of target genes, and their corresponding functions. Menin interacts with the amide terminus of MLL1 and acts as an oncogenic cofactor that increases the transcription of gene clusters such as HOX and MEIS1. The interaction between Menin and MLL fusion proteins is indispensible for the abnormal activation of a series of gene clusters and the onset of leukemia caused by MLL fusion proteins. Furthermore, Menin, as a nuclear protein widely expressed in tissues, participates in the formation of a multitude of important transcriptional regulatory complexes and exhibits a variety of important biological functions in the body. In addition to its role in the formation of MLL1 and MI,L2 epigenetic regulatory complexes, the Menin protein has been reported to interact with a variety of transcription factors, including JunD, NFKB, and SMAD3, to regulate the activation or inhibition on the transcription of target genes.

The use of small molecules to target the Menin-MI,L interaction represents an attractive strategy for the development of new therapies for MLL leukemia. Moreover, inhibiting the interaction of Menin with wild-type MLL1 and MI,L2 may have therapeutic potential for numerous solid cancers, including liver, brain, colon, and breast cancers.

Therefore, inhibitors of Menin-MLL1 protein-protein interaction can be considered as prospective therapeutic compounds with a broad range of applications in the treatment of tumors. The selective targeting of this interaction interface is conducive to the development of new drugs related thereto.

## SUMMARY

The present disclosure provides a compound represented by Formula I, or a deuterated compound, a stereoisomer or a pharmaceutically acceptable salt thereof:

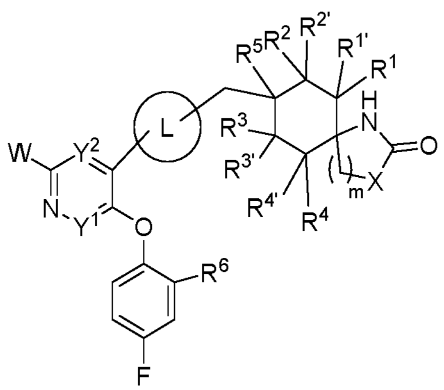

Formula I

wherein,

ring L is

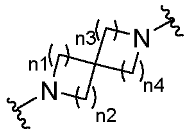;

wherein n1, n2, n3, n4 are independently 1 or 2;

$Y^1$ and $Y^2$ are independently CH or N;

m is selected from the group consisting of 1, 2 and 3;

W is selected from the group consisting of hydrogen, halogen, cyano, nitro, $-C_{1-6}$ alkyl, $-C_{2-6}$ alkenyl, $-C_{2-6}$ alkynyl, halogen-substituted $-C_{1-6}$ alkyl, halogen-substituted $-C_{2-6}$ alkenyl, halogen-substituted $-C_{2-6}$ alkynyl, $-C_{0-4}$ alkylene-$OR^{W1}$, and $-C_{0-4}$ alkylene-$NR^{W1}R^{W2}$;

$R^{W1}$ and $R^{W2}$ are independently selected from the group consisting of hydrogen, $-C_{1-6}$ alkyl, $-C_{2-6}$ alkenyl, $-C_{2-6}$ alkynyl, 3- to 10-membered carbocyclyl and 4- to 10-membered heterocycloalkyl;

X is selected from the group consisting of $CR^aR^b$, $NR^a$, O and S;

$R^a$ and $R^b$ are independently selected from the group consisting of hydrogen, halogen, cyano, nitro, $-C_{1-6}$ alkyl, deuterium-substituted $-C_{1-6}$ alkyl, $-C_{2-6}$ alkenyl, $-C_{2-6}$ alkynyl, halogen-substituted $-C_{1-6}$ alkyl, halogen-substituted $-C_{2-6}$ alkenyl, halogen-substituted $-C_{2-6}$ alkynyl, $-C_{0-4}$ alkylene-$OR^{A1}$, $-C_{0-4}$ alkylene-$NR^{A1}R^{A2}$, $-C_{0-4}$ alkylene-$NR^{A1}C(O)R^{A2}$, $-C_{0-4}$ alkylene-$C(O)NR^{A1}R^{A2}$, $-C_{0-4}$ alkylene-$C(O)R^{A1}$, $-C_{0-4}$ alkylene-$S(O)_2NR^{A1}R^{A2}$, $-C_{0-4}$ alkylene-$NR^{A1}S(O)_2R^{A2}$, $-C_{0-4}$ alkylene-(3- to 10-membered carbocyclyl), $-C_{0-4}$ alkylene-(4- to 10-membered heterocycloalkyl), $-C_{0-4}$ alkylene-(6- to 10-membered aromatic ring) and $-C_{0-4}$ alkylene-(5- to 10-membered aromatic heterocycle); wherein the alkyl, alkenyl, alkynyl, alkylene, carbocyclyl, heterocycloalkyl, aromatic ring, or aromatic heterocycle is further optionally substituted by one, two, three or four independent $R^{B1}$;

alternatively, $R^a$, $R^b$ and the atom connecting therewith together form

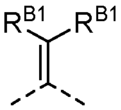, 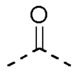,

3- to 10-membered carbocyclyl or 4- to 10-membered heterocycloalkyl; wherein the carbocyclyl or heterocycloalkyl is further optionally substituted by one, two, three or four independent $R^{B1}$;

$R^{A1}$ and $R^{A2}$ are independently selected from the group consisting of hydrogen, $-C_{1-6}$ alkyl, $-C_{2-6}$ alkenyl, $-C_{2-6}$ alkynyl, $-C_{0-4}$ alkylene-$C(O)R^{B1}$, $-C_{0-4}$ alkylene-(3- to 10-membered carbocyclyl), $-C_{0-4}$ alkylene-(4- to 10-membered heterocycloalkyl), $-C_{0-4}$ alkylene-(6- to 10-membered aromatic ring) and $-C_{0-4}$ alkylene-(5- to 10-membered aromatic heterocycle); wherein the alkyl, alkenyl, alkynyl, alkylene, carbocyclyl, heterocycloalkyl, aromatic ring, or aromatic heterocycle is further optionally substituted by one, two, three or four independent $R^{B1}$;

each $R^{B1}$ is independently selected from the group consisting of hydrogen, deuterium, halogen, cyano, nitro, $-C_{1-6}$ alkyl, $-C_{2-6}$ alkenyl, $-C_{2-6}$ alkynyl and $-C_{0-4}$ alkylene-$OR^{C1}$;

$R^{C1}$ is independently selected from the group consisting of hydrogen, $-C_{1-6}$ alkyl, $-C_{2-6}$ alkenyl, $-C_{2-6}$ alkynyl, halogen-substituted $-C_{1-6}$ alkyl, halogen-substituted $-C_{2-6}$ alkenyl and halogen-substituted $-C_{2-6}$ alkynyl;

$R^1$, $R^2$, $R^3$, $R^4$, $R^5$, $R^{1'}$, $R^{2'}$, $R^{3'}$ and $R^{4'}$ are independently selected from the group consisting of hydrogen, halogen, cyano, nitro, $-C_{1-6}$ alkyl, $-C_{2-6}$ alkenyl, $-C_{2-6}$ alkynyl, halogen-substituted $-C_{1-6}$ alkyl, halogen-substituted $-C_{2-6}$ alkenyl, halogen-substituted $-C_{2-6}$ alkynyl, $-C_{0-4}$ alkylene-$OR^{D1}$, $-C_{0-4}$ alkylene-$NR^{D1}R^{D2}$, $-C_{0-4}$ alkylene-(3- to 10-membered carbocyclyl), $-C_{0-4}$ alkylene-(4- to 10-membered heterocycloalkyl), $-C_{0-4}$ alkylene-(6- to 10-membered aromatic ring) and $-C_{0-4}$ alkylene-(5- to 10-membered aromatic heterocycle); wherein the alkyl, alkenyl, alkynyl, alkylene, carbocyclyl, heterocycloalkyl, aromatic ring, or aromatic heterocycle is further optionally substituted by one, two, three or

four independent $R^{D3}$;

alternatively, $R^1$ and $R^{1'}$, $R^2$ and $R^{2'}$, $R^3$ and $R^{3'}$, or $R^4$ and $R^{4'}$ connected to the identical atom are independently interconnected to form 3- to 10-membered carbocyclyl, 4- to 10-membered heterocycloalkyl,

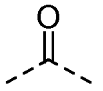 , 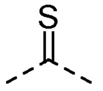 , 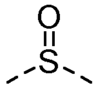 , or 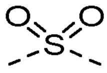 ;

wherein the carbocyclyl or heterocycloalkyl is further optionally substituted by one, two, three or four independent $R^{D3}$;

alternatively, two non-adjacent groups or three groups of $R^1$, $R^2$, $R^3$, $R^4$ and $R^5$ are interconnected, and together with the ring where the atom connecting therewith is located, form 7-to 12-membered bridged cycloalkyl or 7- to 12-membered bridged heterocycloalkyl; wherein the bridged cycloalkyl or bridged heterocycloalkyl is further optionally substituted by one, two, three, or four independent $R^{D3}$;

$R^{D1}$ and $R^{D2}$ are independently selected from the group consisting of hydrogen, $-C_{1-6}$ alkyl, $-C_{2-6}$ alkenyl, $-C_{2-6}$ alkynyl, $-C_{0-4}$ alkylene-(3- to 10-membered carbocyclyl), $-C_{0-4}$ alkylene-(4- to 10-membered heterocycloalkyl), $-C_{0-4}$ alkylene-(6- to 10-membered aromatic ring) and $-C_{0-4}$ alkylene-(5- to 10-membered aromatic heterocycle); wherein the alkyl, alkenyl, alkynyl, alkylene, carbocyclyl, heterocycloalkyl, aromatic ring, or aromatic heterocycle is further optionally substituted by one, two, three or four independent $R^{D4}$;

each $R^{D4}$ is independently selected from the group consisting of hydrogen, $-C_{1-6}$ alkyl, - $C_{2-6}$ alkenyl, $-C_{2-6}$ alkynyl, halogen-substituted $-C_{1-6}$ alkyl, halogen-substituted $-C_{2-6}$ alkenyl and halogen-substituted $-C_{2-6}$ alkynyl;

each $R^{D3}$ is independently selected from the group consisting of hydrogen, halogen, cyano, nitro, oxo, $-C_{1-6}$ alkyl, $-C_{2-6}$ alkenyl, $-C_{2-6}$ alkynyl, halogen-substituted $-C_{1-6}$ alkyl, halogen-substituted $-C_{2-6}$ alkenyl, halogen-substituted $-C_{2-6}$ alkynyl, $-C_{0-4}$ alkylene-$OR^{d1}$, $-C_{0-4}$ alkylene-$OC(O)R^{d1}$, $-C_{0-4}$ alkylene-$SR^{d1}$, $-C_{0-4}$ alkylene-$S(O)_2R^{d1}$, $-C_{0-4}$ alkylene-$S(O)R^{d1}$, $-C_{0-4}$ alkylene$S(O)_2NR^{d1}R^{d2}$, $-C_{0-4}$ alkylene-$S(O)NR^{d1}R^{d2}$, $-C_{0-4}$ alkylene-$S(O)(NH)R^{d1}$, $-C_{0-4}$ alkylene$S(O)(NH)NR^{d1}R^{a2}$, $-C_{0-4}$ alkylene-$C(O)R^{d1}$, $-C_{0-4}$ alkylene-$C(O)OR^{d1}$, $-C_{0-4}$ alkylene-$C(O)NR^{d1}R^{d2}$, $-C_{0-4}$ alkylene-$NR^{d1}R^{d2}$, $-C_{0-4}$ alkylene-$NR^{d1}C(O)R^{d2}$, $-C_{0-4}$ alkylene-$NR^{d1}S(O)_2R^{d2}$, $-C_{0-4}$ alkylene-$NR^{d1}S(O)R^{d2}$, $-C_{0-4}$ alkylene-$P(O)R^{d1}R^{d2}$, $-C_{0-4}$ alkylene-$P(O)(OR^{d1})R^{d2}$, $-C_{0-4}$ alkylene-$P(O)(OR^{d1})(OR^{d2})$, $-C_{0-4}$ alkylene-(3- to 10-membered carbocyclyl), $-C_{0-4}$ alkylene-(4- to 10-membered heterocycloalkyl), $-C_{0-4}$ alkylene-(6- to 10-membered aromatic ring) and $-C_{0-4}$ alkylene-(5- to 10-membered aromatic heterocycle);

$R^{d1}$ and $R^{d2}$ are independently selected from the group consisting of hydrogen, $-C_{1-6}$ alkyl, $-C_{2-6}$ alkenyl, $-C_{2-6}$ alkynyl, halogen-substituted $-C_{1-6}$ alkyl, halogen-substituted $-C_{2-6}$ alkenyl and halogen-substituted $-C_{2-6}$ alkynyl;

$R^6$ is selected from the group consisting of hydrogen, $-C_{1-6}$ alkyl, $-C_{2-6}$ alkenyl, $-C_{2-6}$ alkynyl, $-C_{0-4}$ alkylene-$C(O)R^{E1}$, $-C_{0-4}$ alkylene-$C(O)OR^{E1}$, $-C_{0-4}$ alkylene-$C(O)NR^{E1}R^{E2}$, $-C_{0-4}$ alkylene-$NR^{E1}C(O)R^{E2}$, $-C_{0-4}$ alkylene-$NR^{E1}S(O)_2R^{E2}$, $-C_{0-4}$ alkylene-$NR^{E1}S(O)R^{E2}$, $-C_{0-4}$ alkylene-(5- to 10-membered aromatic ring), $-C_{0-4}$ alkylene-(5- to 10-membered heteroaromatic ring), $-C_{0-4}$ alkylene-(3- to 10-membered carbocyclyl), $-C_{0-4}$ alkylene-(4- to 10-membered heterocycloalkyl), $-C_{0-4}$ alkylene-$S(O)R^{E1}$, $-C_{0-4}$ alkylene-$S(O)_2R^{E1}$, $-C_{0-4}$ alkylene-$S(O)_2NR^{E1}R^{E2}$, $-C_{0-4}$ alkylene-$S(O)(NH)R^{E1}$, $-C_{0-4}$ alkylene-$S(O)(NH)NR^{E1}R^{E2}$, $-C_{0-4}$ alkylene-$OR^{E1}$, $-C_{0-4}$ alkylene-$OC(O)R^{E1}$, $-C_{0-4}$ alkylene-$SR^{E1}$, $-C_{0-4}$ alkylene-$P(O)R^{E1}R^{E2}$, $-C_{0-4}$ alkylene-$P(O)(OR^{E1})R^{E2}$ and $-C_{0-4}$ alkylene-$P(O)(OR^{E1})(OR^{E2})$; wherein the alkyl, alkylene, alkenyl, alkynyl, carbocyclyl, heterocycloalkyl, aryl or heteroaryl is further optionally substituted by one, two, three or four independent $R^{E5}$;

$R^{E1}$ and $R^{E2}$ are each independently selected from the group consisting of hydrogen, $-C_{1-6}$ alkyl, $-C_{0-4}$ alkylene-(3- to 10-membered carbocyclyl), $-C_{0-4}$ alkylene-(4- to 10-membered heterocycloalkyl), $-C_{0-4}$ alkylene-$OR^{E3}$, $-C_{0-4}$ alkylene-(5- to 10-membered aromatic ring), $-C_{0-4}$ alkylene-(5- to 10-membered heteroaromatic ring), $-C_{0-4}$ alkylene-$S(O)R^{E3}$, $-C_{0-4}$ alkylene$S(O)_2R^{E3}$, $-C_{0-4}$ alkylene-$S(O)_2NR^{E3}R^{E4}$, $-C_{0-4}$ alkylene-$S(O)(NH)R^{E3}$, $-C_{0-4}$ alkylene$S(O)(NH)NR^{E3}R^{E4}$, $-C_{0-4}$ alkylene-$OC(O)R^{E3}$ and $-C_{0-4}$ alkylene-$SR^{E3}$; wherein the alkyl, alkylene, carbocyclyl, heterocycloalkyl, aryl or heteroaryl is further optionally substituted by one, two, three or four independent $R^{E5}$;

alternatively, $R^{E1}$ and $R^{E2}$ are interconnected to form 4- to 10-membered heterocycloalkyl or 4- to 10-membered bridged heterocycloalkyl; wherein the heterocycloalkyl or bridged heterocycloalkyl is further optionally substituted

by one, two, three or four independent $R^{E5}$;

$R^{E3}$ and $R^{E4}$ are independently selected from the group consisting of hydrogen, $-C_{1-6}$ alkyl, $-C_{2-6}$ alkenyl, $-C_{2-6}$ alkynyl, halogen-substituted $-C_{1-6}$ alkyl, halogen-substituted $-C_{2-6}$ alkenyl and halogen-substituted $-C_{2-6}$ alkynyl; alternatively, $R^{E3}$, $R^{E4}$ and the nitrogen atom connecting therewith together form 4- to 10-membered heterocycloalkyl or 4- to 10-membered bridged heterocycloalkyl; wherein the heterocycloalkyl or bridged heterocycloalkyl is further optionally substituted by one, two, three or four independent $R^{E5}$;

alternatively, $R^{E3}$ and $R^{E4}$ are interconnected to form 4- to 10-membered heterocycloalkyl or 4- to 10-membered bridged heterocycloalkyl; wherein the heterocycloalkyl or bridged heterocycloalkyl is further optionally substituted by one, two, three or four independent $R^{E5}$;

each $R^{E5}$ is independently selected from the group consisting of hydrogen, halogen, cyano, nitro, oxo, $-C_{1-6}$ alkyl, $-C_{2-6}$ alkenyl, $-C_{2-6}$ alkynyl, halogen-substituted $-C_{1-6}$ alkyl, halogen-substituted $-C_{2-6}$ alkenyl, halogen-substituted $-C_{2-6}$ alkynyl, $-O(C_{1-6}$ alkyl), $-NH_2$, $-C_{0-4}$ alkylene-(3-to 10-membered carbocyclyl), $-C_{0-4}$ alkylene-(4- to 10-membered heterocycloalkyl), $-C_{0-4}$ alkylene-(5- to 10-membered aromatic ring) and $-C_{0-4}$ alkylene-(5- to 10-membered heteroaromatic ring); wherein the carbocyclyl, heterocycloalkyl, aryl or heteroaryl is further optionally substituted by one, two, three or four independent $R^{E6}$; and

$R^{E6}$ is independently selected from the group consisting of hydrogen, halogen, cyano, nitro, $-C_{1-6}$ alkyl, $-C_{2-6}$ alkenyl, $-C_{2-6}$ alkynyl, halogen-substituted $-C_{1-6}$ alkyl, halogen-substituted - $C_{2-6}$ alkenyl and halogen-substituted $-C_{2-6}$ alkynyl.

In some embodiments of the present disclosure, when $R^1$, $R^2$, $R^3$, $R^4$, $R^5$, $R^{1'}$, $R^{2'}$, $R^{3'}$ and $R^{4'}$ are all hydrogen, X is O, and $Y^1$ is CH, m is not 1.

In some embodiments of the present disclosure, preferably, n1 and n2 are 1, n3 and n4 are 2; alternatively, n1 and n2 are 2, n3 and n4 are 1. Preferably, $Y^1$ is N, and $Y^2$ is N; alternatively, $Y^1$ is CH, and $Y^2$ is N. Preferably, m is 1 or 2; more preferably, m is 1.

In some embodiments of the present disclosure, preferably, W is selected from the group consisting of hydrogen, fluorine, chlorine, cyano, methyl, ethyl, n-propyl, isopropyl, monofluoromethyl, difluoromethyl, trifluoromethyl, hydroxy, methoxy, ethoxy, methoxymethyl, amino, methylamino and dimethylamino.

In some embodiments of the present disclosure, preferably, X is selected from the group consisting of $CR^aR^b$, $NR^a$ and O; more preferably, X is $CR^aR^b$ or $NR^a$.

In some embodiments of the present disclosure, when X is $CR^aR^b$, preferably, $R^a$ and $R^b$ are independently selected from the group consisting of hydrogen, fluorine, chlorine, cyano, methyl, ethyl, n-propyl, isopropyl, monodeuteriomethyl, dideuteriomethyl, trideuteriomethyl, monofluoromethyl, difluoromethyl, trifluoromethyl, $-C_0$ alkylene-$OR^{A1}$, $-C_1$ alkylene-$OR^{A1}$, $-C_2$ alkylene-$OR^{A1}$ and $-C_3$ alkylene-$OR^{A1}$; $R^{A1}$ is selected from the group consisting of hydrogen, methyl, ethyl, n-propyl and isopropyl.

In some embodiments of the present disclosure, when X is $CR^aR^b$, preferably, $R^a$ is hydrogen, $R^b$ is selected from the group consisting of hydrogen, fluorine, chlorine, cyano, methyl, ethyl, n-propyl, isopropyl, monodeuteriomethyl, dideuteriomethyl, trideuteriomethyl, monofluoromethyl, difluoromethyl, trifluoromethyl, $-C_0$ alkylene-$OR^{A1}$, $-C_1$ alkylene-$OR^{A1}$, $-C_2$ alkylene-$OR^{A1}$ and $-C_3$ alkylene-$OR^{A1}$; $R^{A1}$ is selected from the group consisting of hydrogen, methyl, ethyl, n-propyl and isopropyl.

In some embodiments of the present disclosure, when X is $CR^aR^b$, preferably, $R^a$ and $R^b$ are the same and are selected from the group consisting of hydrogen, fluorine, chlorine, cyano, methyl, ethyl, n-propyl, isopropyl, monodeuteriomethyl, dideuteriomethyl, trideuteriomethyl, monofluoromethyl, difluoromethyl, trifluoromethyl, $-C_0$ alkylene-$OR^{A1}$, $-C_1$ alkylene-$OR^{A1}$, $-C_2$ alkylene-$OR^{A1}$ and $-C_3$ alkylene-$OR^{A1}$; $R^{A1}$ is selected from the group consisting of hydrogen, methyl, ethyl, n-propyl and isopropyl.

In some embodiments of the present disclosure, when X is $CR^aR^b$, preferably, $R^a$, $R^b$ and the atom connecting therewith together form

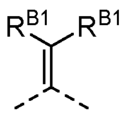 , 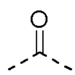 ,

3-membered carbocyclyl, 4-membered carbocyclyl, 5-membered carbocyclyl, 6-membered carbocyclyl, 4-membered heterocycloalkyl, 5-membered heterocycloalkyl or 6-membered heterocycloalkyl; each $R^{B1}$ is independently selected

from the group consisting of hydrogen, methyl, ethyl, n-propyl and isopropyl.

In some embodiments of the present disclosure, when X is $NR^a$, preferably, $R^a$ is selected from the group consisting of hydrogen, methyl, ethyl, n-propyl, isopropyl, monodeuteriomethyl, dideuteriomethyl, trideuteriomethyl, monofluoromethyl, difluoromethyl, trifluoromethyl, $-C_1$ alkylene-$OR^{A1}$, $-C_2$ alkylene-$OR^{A1}$ and $-C_3$ alkylene-$OR^{A1}$; $R^{A1}$ is selected from the group consisting of hydrogen, methyl, ethyl, n-propyl and isopropyl.

In some embodiments of the present disclosure, preferably, $R^1$, $R^2$, $R^3$, $R^4$, $R^5$, $R^{1'}$, $R^{2'}$, $R^{3'}$ and $R^{4'}$ are independently selected from the group consisting of hydrogen, methyl, ethyl, n-propyl, isopropyl, monodeuteriomethyl, dideuteriomethyl, trideuteriomethyl, monofluoromethyl, difluoromethyl and trifluoromethyl.

In some embodiments of the present disclosure, preferably, $R^{1'}$, $R^{2'}$, $R^{3'}$ and $R^{4'}$ are independently selected from the group consisting of hydrogen, methyl, ethyl, n-propyl, isopropyl, monodeuteriomethyl, dideuteriomethyl, trideuteriomethyl, monofluoromethyl, difluoromethyl and trifluoromethyl; two non-adjacent groups of $R^1$, $R^2$, $R^3$, $R^4$ and $R^5$ are interconnected, and together with the ring where the atom connecting therewith is located, form 7-membered bridged cycloalkyl, 8-membered bridged cycloalkyl or 9-membered bridged cycloalkyl; wherein two non-adjacent groups of $R^1$, $R^2$, $R^3$, $R^4$ and $R^5$ are interconnected into a connecting chain selected from the group consisting of $-CH_2-$, $-CH_2CH_2-$ and $-CH_2CH_2CH_2-$. More preferably, $R^2$ and $R^4$ are interconnected, or $R^2$ and $R^3$ are interconnected, or $R^1$ and $R^4$ are interconnected.

In some embodiments of the present disclosure, preferably, $R^6$ is $-C(O)NR^{E1}R^{E2}$, $R^{E1}$ and $R^{E2}$ are independently selected from the group consisting of hydrogen, methyl, ethyl, n-propyl, isopropyl, $-C_4$ alkyl, $-C_5$ alkyl, $-C_6$ alkyl, 3-membered carbocyclyl, 4-membered carbocyclyl, 5-membered carbocyclyl, 6-membered carbocyclyl, $-C_1$ alkylene-(3-membered carbocyclyl), $-C_1$ alkylene-(4-membered carbocyclyl), $-C_1$ alkylene-(5-membered carbocyclyl), and $-C_1$ alkylene-(6-membered carbocyclyl).

In some embodiments of the present disclosure, preferably, $R^6$ is selected from the group consisting of 5-membered heteroaromatic ring, 6-membered heteroaromatic ring, $-C_1$ alkylene-(5-membered heteroaromatic ring) and $-C_1$ alkylene-(6-membered heteroaromatic ring); wherein the heteroaromatic group is further optionally substituted by one, two, three or four independent $R^{E5}$; each $R^{E5}$ is independently selected from the group consisting of hydrogen, methyl, ethyl, n-propyl, isopropyl, 3-membered carbocyclyl, 4-membered carbocyclyl, 5-membered carbocyclyl and 6-membered carbocyclyl.

Further,

X is $CR^aR^b$ or $NR^a$;

$R^a$ and $R^b$ are independently selected from the group consisting of hydrogen, halogen, cyano, nitro, $-C_{1-6}$ alkyl, deuterium-substituted $-C_{1-6}$ alkyl, $-C_{2-6}$ alkenyl, $-C_{2-6}$ alkynyl, halogen-substituted $-C_{1-6}$ alkyl, halogen-substituted $-C_{2-6}$ alkenyl, halogen-substituted $-C_{2-6}$ alkynyl, $-C_{0-2}$ alkylene-$OR^{A1}$, $-C_{0-2}$ alkylene-$NR^{A1}R^{A2}$, $-C_{0-2}$ alkylene-$NR^{A1}C(O)R^{A2}$, $-C_{0-4}$ alkylene-$C(O)NR^{A1}R^{A2}$, $-C_{0-4}$ alkylene-$C(O)R^{A1}$, $-C_{0-4}$ alkylene-$S(O)_2NR^{A1}R^{A2}$, $-C_{0-4}$ alkylene-$NR^{A1}S(O)_2R^{A2}$, $-C_{0-2}$ alkylene-(3- to 10-membered carbocyclyl), $-C_{0-2}$ alkylene-(4- to 10-membered heterocycloalkyl), $-C_{0-2}$ alkylene-(6- to 10-membered aromatic ring) and $-C_{0-2}$ alkylene-(5- to 10-membered aromatic heterocycle); wherein the alkyl, alkenyl, alkynyl, alkylene, carbocyclyl, heterocycloalkyl, aromatic ring or aromatic heterocycle is further optionally substituted by one, two, three or four independent $R^{B1}$;

alternatively, $R^a$, $R^b$ and the atom connecting therewith together form

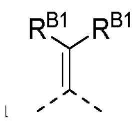 , 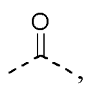 ,

3- to 6-membered carbocyclyl or 4- to 6-membered heterocycloalkyl; wherein the carbocyclyl or heterocycloalkyl is further optionally substituted by one, two, three or four independent $R^{B1}$;

$R^{A1}$ and $R^{A2}$ are independently selected from the group consisting of hydrogen, $-C_{1-6}$ alkyl, $-C_{2-6}$ alkenyl, $-C_{2-6}$ alkynyl, $-C_{1-2}$ alkylene-$C(O)R^{B1}$, $-C_{1-2}$ alkylene-(3- to 10-membered carbocyclyl), $-C_{1-2}$ alkylene-(4- to 10-membered heterocycloalkyl), $-C_{1-2}$ alkylene-(6- to 10-membered aromatic ring) and $-C_{1-2}$ alkylene-(5- to 10-membered aromatic heterocycle); wherein the alkyl, alkenyl, alkynyl, alkylene, carbocyclyl, heterocycloalkyl, aromatic ring or aromatic heterocycle is further optionally substituted by one, two, three or four independent $R^{B1}$;

$Y^1$ and $Y^2$ are independently CH or N;

m is 1 or 2; and

W is selected from the group consisting of hydrogen, methyl, trifluoromethyl, methoxy and methylamino.

Further,

X is $NR^a$; and
$R^a$ is selected from the group consisting of hydrogen, methyl, ethyl, isopropyl, cyclopropyl,

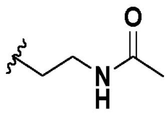, 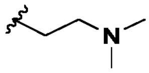, 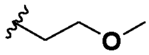, 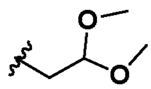, 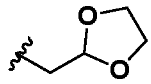, 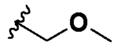, 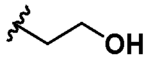, 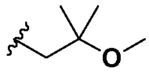 and 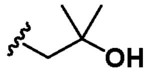.

Further,

X is $CR^aR^b$; and
$R^a$ and $R^b$ are independently selected from the group consisting of hydrogen, fluorine, methyl,

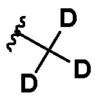,

ethyl, isopropyl,

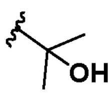, 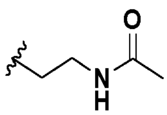, 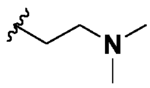, 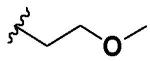, 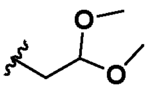, 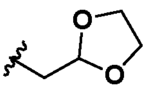, 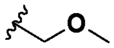, 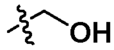, 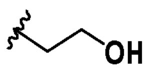, 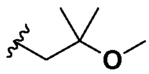 and 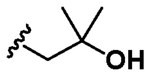;

alternatively, $R^a$, $R^b$ and the atom connecting therewith together form

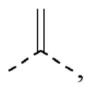, 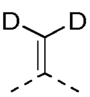,

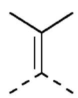, 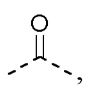, 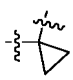, 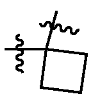, 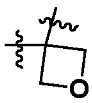, 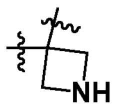, 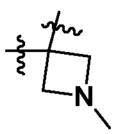, 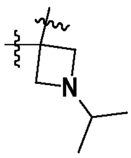, 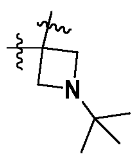, 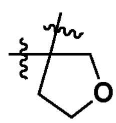

or

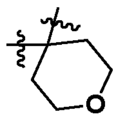.

In some embodiments of the present disclosure, $R^a$ is hydrogen and $R^b$ is selected from the group consisting of hydrogen, fluorine, methyl,

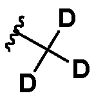,

ethyl, isopropyl,

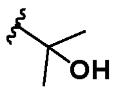,

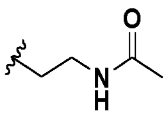, 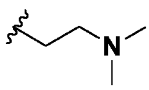, 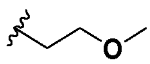, 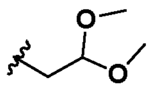, 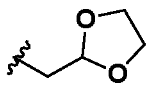, 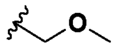, 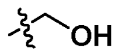,

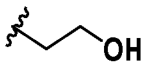, 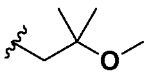 and 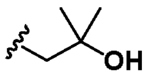.

In some embodiments of the present disclosure, $R^a$ and $R^b$ are the same and are selected from the group consisting of hydrogen, fluorine, methyl,

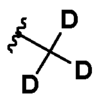,

ethyl, isopropyl,

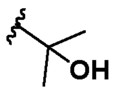,

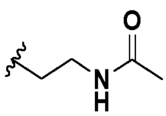, 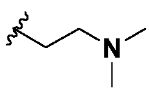, 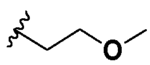, 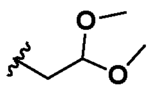, 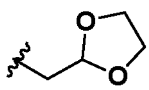, 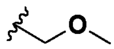, 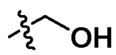,

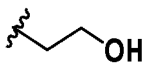, 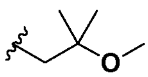 and 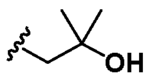.

In some embodiments of the present disclosure, further, the ring L is selected from the group consisting of

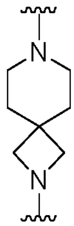, 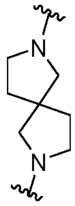, 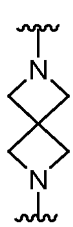, 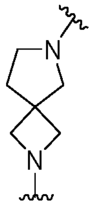, 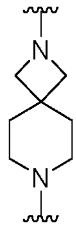

and

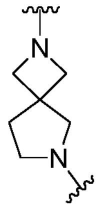.

In some embodiments of the present disclosure, further, the ring L is selected from the group consisting of

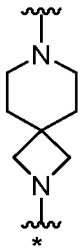, 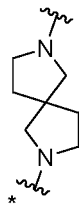, 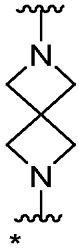, 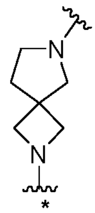,

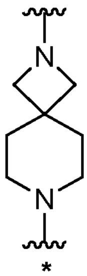 and 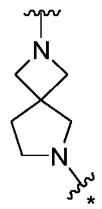;

wherein the end marked with * is connected to the aromatic ring, and the other end is connected to methylene.

In some embodiments of the present disclosure, further,

$R^1$, $R^2$, $R^3$, $R^4$, $R^5$, $R^{1'}$, $R^{2'}$, $R^{3'}$ and $R^{4'}$ are each independently selected from the group consisting of hydrogen, halogen, cyano, nitro, $-C_{1-6}$ alkyl, $-C_{2-6}$ alkenyl, $-C_{2-6}$ alkynyl, halogen-substituted $-C_{1-6}$ alkyl, halogen-substituted $-C_{2-6}$ alkenyl and halogen-substituted $-C_{2-6}$ alkynyl. Preferably, $R^1$, $R^2$, $R^3$, $R^4$, $R^5$, $R^{1'}$, $R^{2'}$, $R^{3'}$ and $R^{4'}$ are all hydrogen.

In some embodiments of the present disclosure, further,

$R^{1'}$, $R^{2'}$, $R^{3'}$ and $R^{4'}$ are independently selected from the group consisting of hydrogen, halogen, cyano, nitro, oxo, $-C_{1-6}$ alkyl, $-C_{2-6}$ alkenyl, $-C_{2-6}$ alkynyl, halogen-substituted $-C_{1-6}$ alkyl, halogen-substituted $-C_{2-6}$ alkenyl, halogen-substituted $-C_{2-6}$ alkynyl, $-C_{0-4}$ alkylene-$OR^{D1}$ and $-C_{0-4}$ alkylene-$NR^{D1}R^{D2}$;

$R^{D1}$ and $R^{D2}$ are independently selected from the group consisting of hydrogen, $-C_{1-6}$ alkyl, $-C_{2-6}$ alkenyl and $-C_{2-6}$ alkynyl;

two non-adjacent groups of $R^1$, $R^2$, $R^3$, $R^4$ and $R^5$ are interconnected, and together with the ring where the atom connecting therewith is located, form 7- to 12-membered bridged cycloalkyl or 7- to 12-membered bridged heterocycloalkyl;

wherein two non-adjacent groups of $R^1$, $R^2$, $R^3$, $R^4$ and $R^5$ are interconnected into a connecting chain selected from the group consisting of -O-, $-(CR^{D3}R^{D3})_q-$, $-(CR^{D3}R^{D3})_n-O-(CR^{D3}R^{D3})_n-$, $-(CR^{D3}R^{D3})_n-S-(CR^{D3}R^{D3})_n-$, $-(CR^{D3}R^{D3})_n-N(R^{D3})-(CR^{D3}R^{D3})_n-$, $-O-(CR^{D3}R^{D3})_n-O-$, - $O-(CR^{D3}R^{D3})_n-S-$, $-O-(CR^{D3}R^{D3})_n-N(R^{D3})-$, $-S-(CR^{D3}R^{D3})_n-O-$, $-S-(CR^{D3}R^{D3})_n-S-$, $-S-(CR^{D3}R^{D3})_n-N(R^{D3})-$, $-N(R^{D3})-(CR^{D3}R^{D3})_n-N(R^{D3})-$, $-N(R^{D3})-(CR^{D3}R^{D3})_n-O-$ and $-N(R^{D3})-(CR^{D3}R^{D3})_n-S-$;
each n is independently selected from the group consisting of 0, 1, 2 and 3;
each q is independently selected from the group consisting of 1, 2 and 3; and
each $R^{D3}$ is independently selected from the group consisting of hydrogen, halogen, cyano, nitro, oxo, $-C_{1-6}$ alkyl, $-C_{2-6}$ alkenyl, $-C_{2-6}$ alkynyl, halogen-substituted $-C_{1-6}$ alkyl, halogen-substituted $-C_{2-6}$ alkenyl and halogen-substituted $-C_{2-6}$ alkynyl; alternatively, two $R^{D3}$ together form

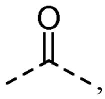, 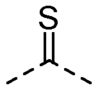, 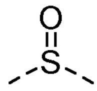 or 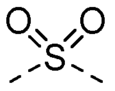.

Further specifically,

two groups of $R^1$, $R^2$, $R^3$, $R^4$ and $R^5$ are interconnected, and together with the ring where the atom connecting therewith is located, form

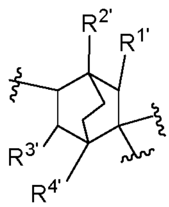, 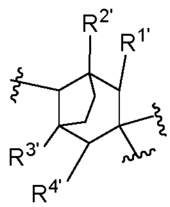, 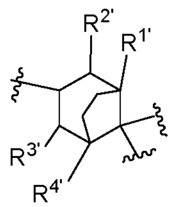,

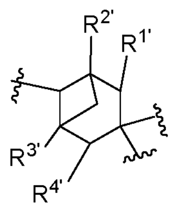 or 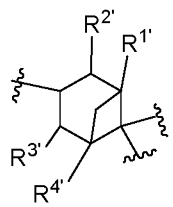;

wherein, $R^{1'}$, $R^{2'}$, $R^{3'}$ and $R^{4'}$ are independently hydrogen or $-C_{1-6}$ alkyl.

In some embodiments of the present disclosure, further,

$R^{1'}$, $R^{2'}$, $R^{3'}$ and $R^{4'}$ are independently selected from the group consisting of hydrogen, halogen, cyano, nitro, oxo, $-C_{1-6}$ alkyl, $-C_{2-6}$ alkenyl, $-C_{2-6}$ alkynyl, halogen-substituted $-C_{1-6}$ alkyl, halogen-substituted $-C_{2-6}$ alkenyl, halogen-substituted $-C_{2-6}$ alkynyl, $-C_{0-4}$ alkylene-$OR^{D1}$ and $-C_{0-4}$ alkylene-$NR^{D1}R^{D2}$;
$R^{D1}$ and $R^{D2}$ are independently selected from the group consisting of hydrogen, $-C_{1-6}$ alkyl, $-C_{2-6}$ alkenyl and $-C_{2-6}$ alkynyl;
three groups of $R^1$, $R^2$, $R^3$, $R^4$ and $R^5$ are interconnected, and together with the ring where the atom connecting therewith is located, form 7- to 12-membered bridged cycloalkyl or 7- to 12-membered bridged heterocycloalkyl;
wherein two groups of $R^1$, $R^2$, $R^3$, $R^4$ and $R^5$ are interconnected into a connecting chain selected from the group consisting of -O-, $-(CR^{D3}R^{D3})_q-$, $-(CR^{D3}R^{D3})_n-O-(CR^{D3}R^{D3})_n-$, - $(CR^{D3}R^{D3})_n-S-(CR^{D3}R^{D3})_n-$, $-(CR^{D3}R^{D3})_n-N(R^{D3})-(CR^{D3}R^{D3})_n-$, $-O-(CR^{D3}R^{D3})_n-O-$, $-O-(CR^{D3}R^{D3})_n-S-$, $-O-(CR^{D3}R^{D3})_n-N(R^{D3})-$, $-S-(CR^{D3}R^{D3})_n-O-$, $-S-(CR^{D3}R^{D3})_n-S-$, $-S-(CR^{D3}R^{D3})_n-N(R^{D3})-$, $-N(R^{D3})-(CR^{D3}R^{D3})_n-N(R^{D3})-$, $-N(R^{D3})-(CR^{D3}R^{D3})_n-O-$ and $-N(R^{D3})-(CR^{D3}R^{D3})_n-S-$; and the other group is connected to the carbon atom or nitrogen atom on the connecting chain formed by the two groups;
each n is independently selected from the group consisting of 0, 1, 2 and 3;
each q is independently selected from the group consisting of 1, 2 and 3; and
each $R^{D3}$ is independently selected from the group consisting of hydrogen, halogen, cyano, nitro, oxo, $-C_{1-6}$ alkyl, $-C_{2-6}$ alkenyl, $-C_{2-6}$ alkynyl, halogen-substituted $-C_{1-6}$ alkyl, halogen-substituted $-C_{2-6}$ alkenyl and halogen-substi-

tuted -$C_{2-6}$ alkynyl; alternatively, two $R^{D3}$ together form

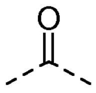, 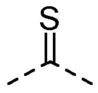, 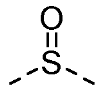 or 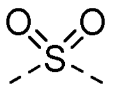.

Further specifically,
$R^1$, $R^4$ and $R^5$ are interconnected, and together with the ring where the atom connecting therewith is located, form

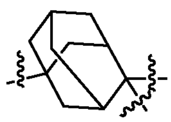.

In some embodiments of the present disclosure, further,
$R^6$ is selected from the group consisting of -C(O)N$R^{E1}R^{E2}$, -N$R^{E1}$C(O)$R^{E2}$, - N$R^{E1}$S$(O)_2R^{E2}$, -N$R^{E1}$S(O)$R^{E2}$, -5- to 10-membered aromatic ring, -5- to 10-membered heteroaromatic ring, -3- to 10-membered carbocyclyl, -4- to 10-membered heterocycloalkyl, - S(O)$R^{E1}$, -S$(O)_2R^{E1}$, -S$(O)_2$N$R^{E1}R^{E2}$, -S(O)(NH)$R^{E1}$, -S(O)(NH)N$R^{E1}R^{E2}$, -O$R^{E1}$, -OC(O)$R^{E1}$ and -S$R^{E1}$; wherein the alkyl, alkylene, alkenyl, alkynyl, carbocyclyl, heterocycloalkyl, aryl or heteroaryl is further optionally substituted by one, two, three or four independent $R^{E5}$.

Preferably, $R^6$ is selected from the group consisting of -C(O)N$R^{E1}R^{E2}$, -N$R^{E1}$C(O)$R^{E2}$, - N$R^{E1}$S$(O)_2R^{E2}$, -N$R^{E1}$S(O)$R^{E2}$, 6-membered aromatic ring, -5-membered heteroaromatic ring, -6-membered heteroaromatic ring, 3-membered carbocyclyl, 4-membered carbocyclyl, 5-membered carbocyclyl, 6-membered carbocyclyl, -4-membered heterocycloalkyl, -5-membered heterocycloalkyl, -6-membered heterocycloalkyl, -S(O)$R^{E1}$, -S$(O)_2R^{E1}$, -S$(O)_2$N$R^{E1}R^{E2}$, -S(O)(NH)$R^{E1}$, -S(O)(NH)N$R^{E1}R^{E2}$, -O$R^{E1}$, -OC(O)$R^{E1}$ and -S$R^{E1}$; wherein the carbocyclyl, heterocycloalkyl, aryl or heteroaryl is further optionally substituted by one, two, three or four independent $R^{E5}$.

Preferably, $R^{E1}$ and $R^{E2}$ are independently selected from the group consisting of hydrogen, methyl, ethyl, n-propyl, isopropyl, monodeuteriomethyl, dideuteriomethyl, trideuteriomethyl, monofluoromethyl, difluoromethyl, trifluoromethyl, -$C_4$ alkyl, -$C_5$ alkyl, -$C_6$ alkyl, 3-membered carbocyclyl, 4-membered carbocyclyl, 5-membered carbocyclyl, 6-membered carbocyclyl, -$C_1$ alkylene-(3-membered carbocyclyl), -$C_1$ alkylene-(4-membered carbocyclyl), -$C_1$ alkylene-(5-membered carbocyclyl) and -$C_1$ alkylene-(6-membered carbocyclyl).

Further specifically, $R^6$ is selected from the group consisting of

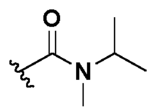, 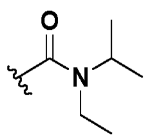,

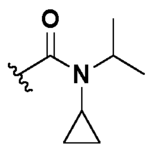, 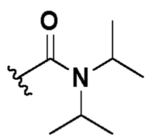, 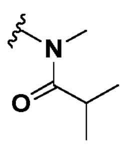, 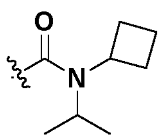, 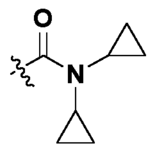, 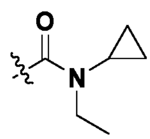, 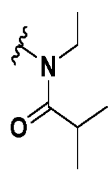,

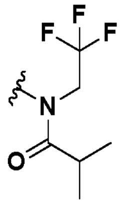, 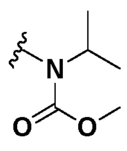, 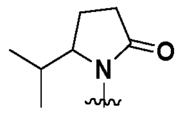, 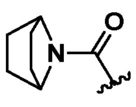, 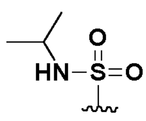, 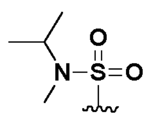, 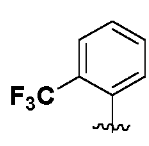,

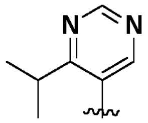, 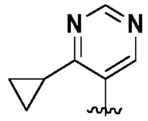, 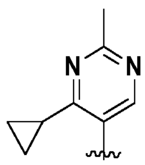, 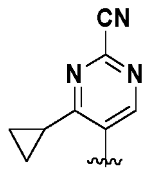, 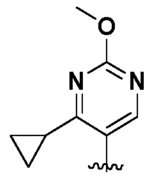, 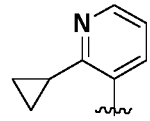, 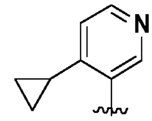,

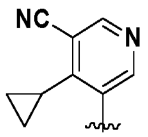, 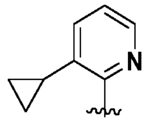, 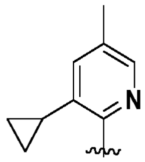, 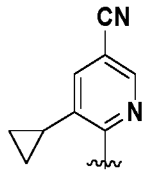, 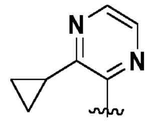, 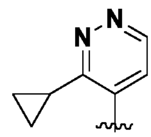, 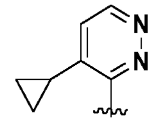,

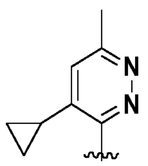, 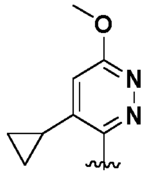, 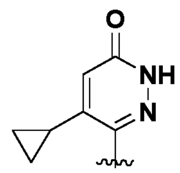, 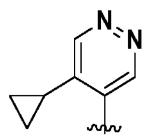, 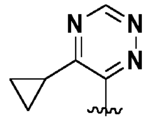, 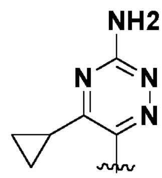,

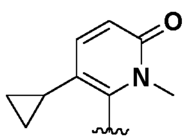, 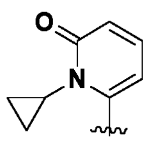, 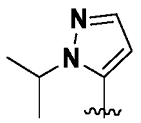, 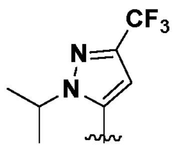, 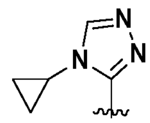, 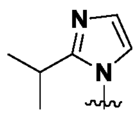,

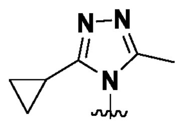, 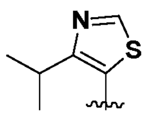, 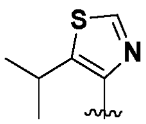, 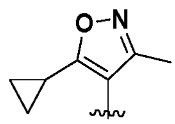 and 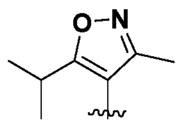.

In some embodiments of the present disclosure, the general formula shown in Formula I may be as follows:

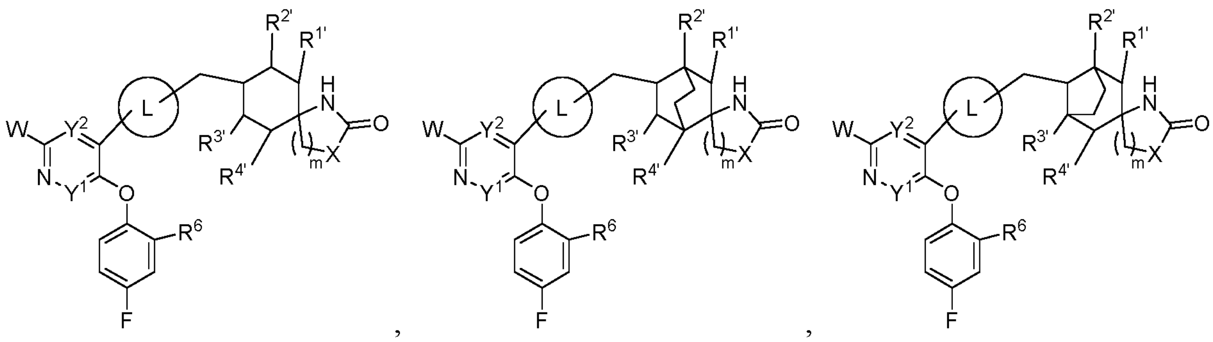

, , ,

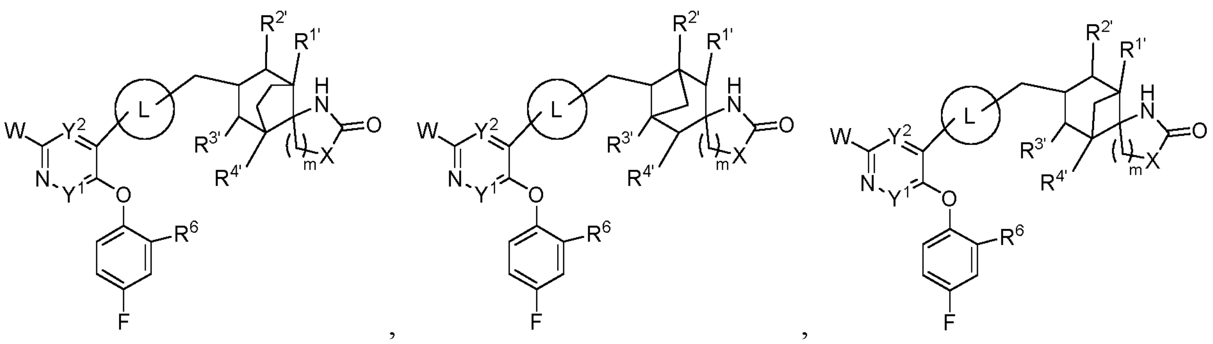

, , ,

or

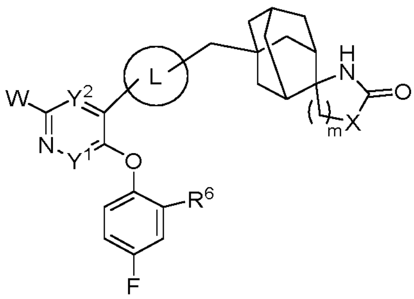

;

wherein, the substituents are defined as above.

In some embodiments of the present disclosure, the general formula shown in Formula I may be as follows:

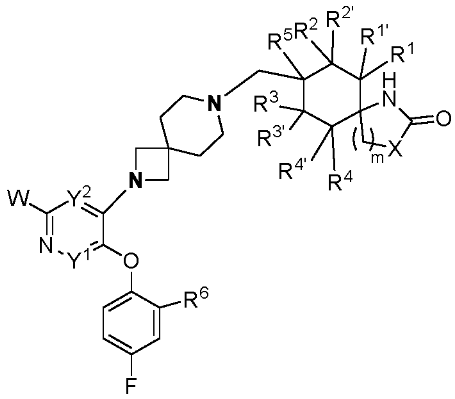

;

wherein, the substituents are defined as above.

In some embodiments of the present disclosure, further, the general formula shown in Formula I may be as follows:

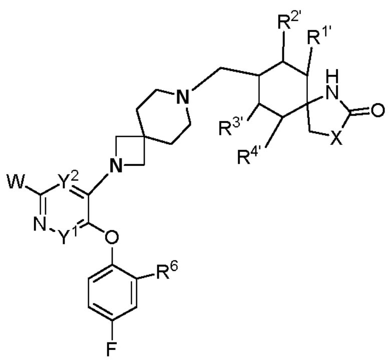

,

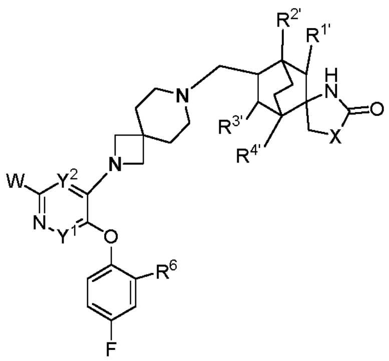

,

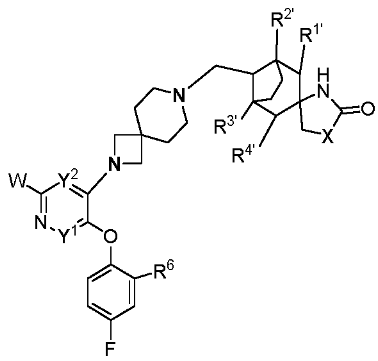

,

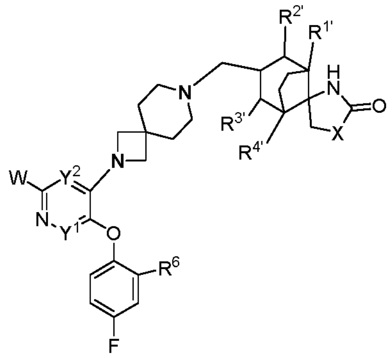

,

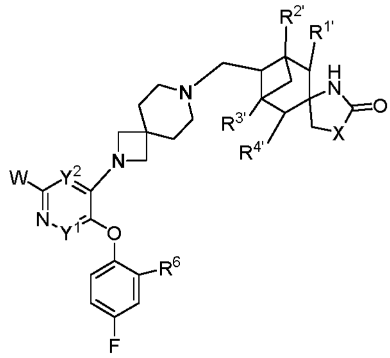

,

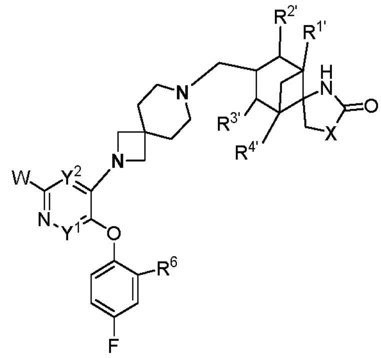

,

or

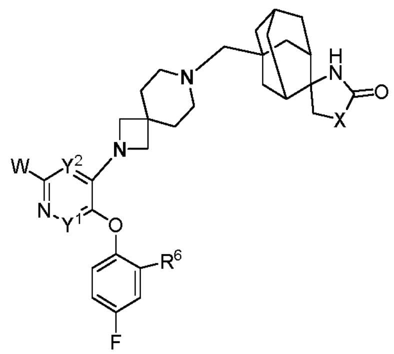

;

wherein, the substituents are defined as above.

In some embodiments of the present disclosure, the compound is represented by Formula II:

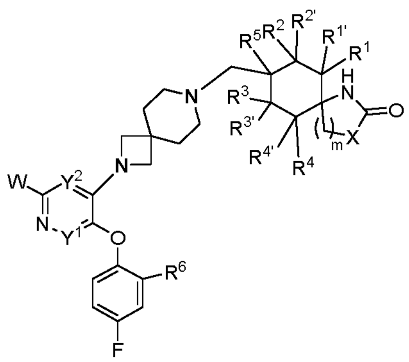

Formula II

wherein, $Y^1$, $Y^2$, W, X, $R^6$, m, $R^1$, $R^2$, $R^3$, $R^4$, $R^5$, $R^{1'}$, $R^{2'}$, $R^{3'}$ and $R^{4'}$ are as defined above.

In some embodiments of the present disclosure, the compound is represented by Formula IIa or Formula IIb:

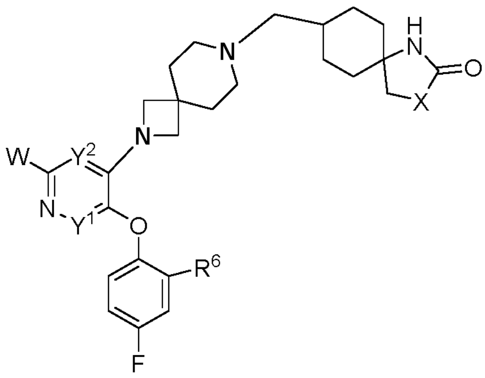
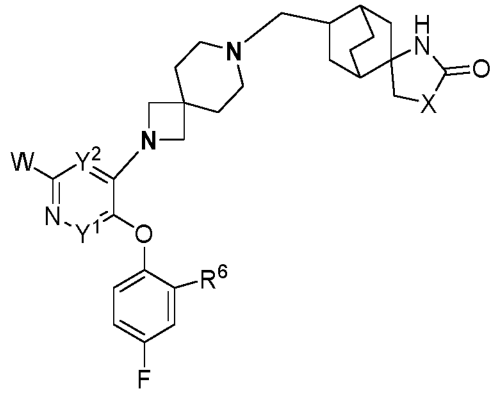

Formula IIa Formula IIb

wherein, $Y^1$, $Y^2$, W, X and $R^6$ are as defined above.

In some embodiments of the present disclosure, the compound is represented by Formula IIIa or Formula IIIb:

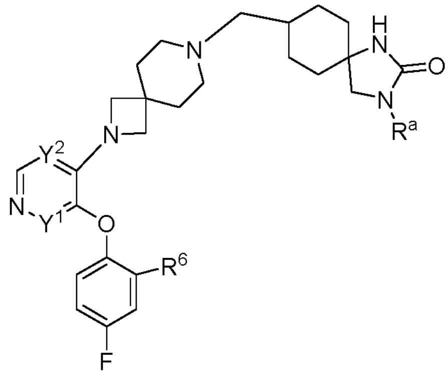
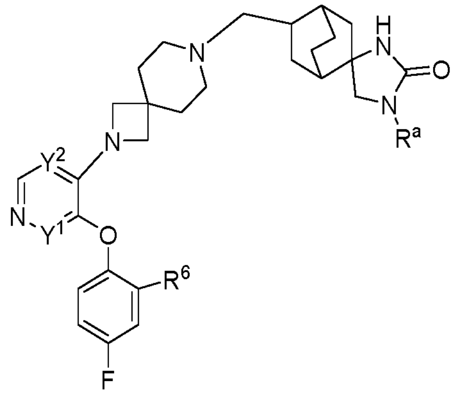

Formula IIIa Formula IIIb

wherein,

$Y^1$ and $Y^2$ are independently CH or N; preferably, $Y^1$ is N and $Y^2$ is N; alternatively, $Y^1$ is CH and $Y^2$ is N;

$R^a$ is selected from the group consisting of hydrogen, methyl, ethyl, isopropyl, cyclopropyl,

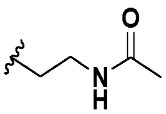, 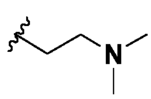, 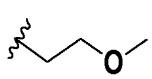, 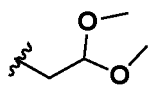, 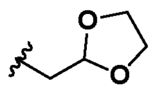, 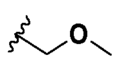,

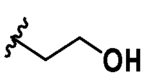, 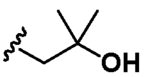 and 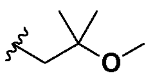;

$R^6$ is selected from the group consisting of

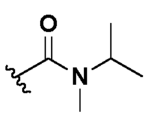, 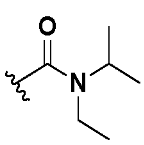, 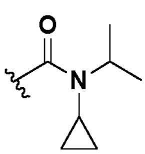,

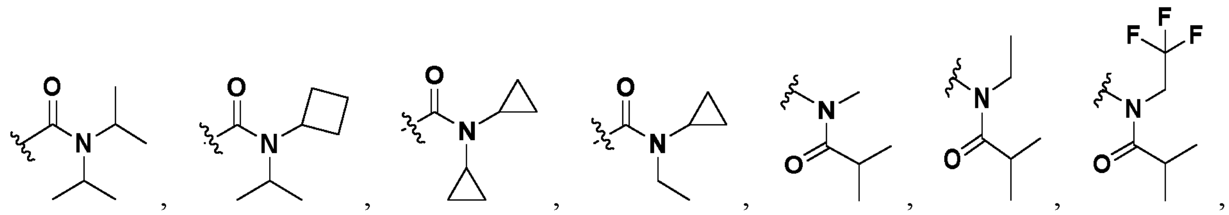
, , , , , , ,

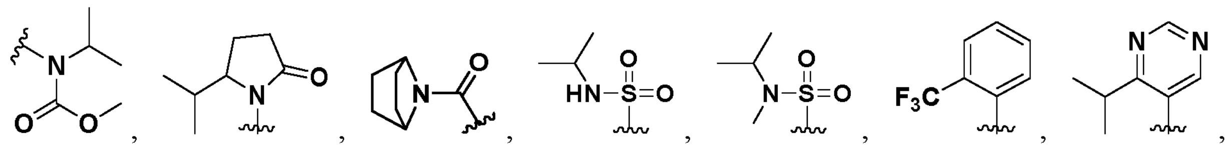
, , , , , , ,

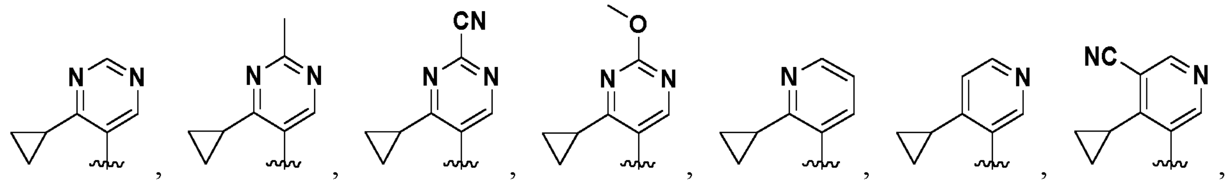
, , , , , , ,

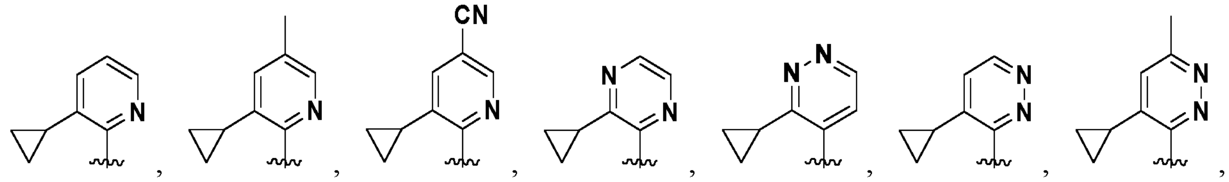
, , , , , , ,

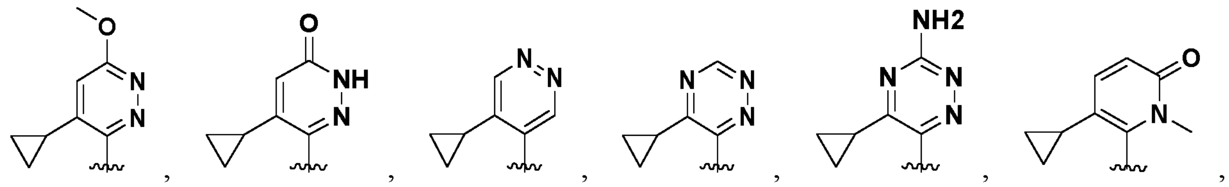
, , , , , ,

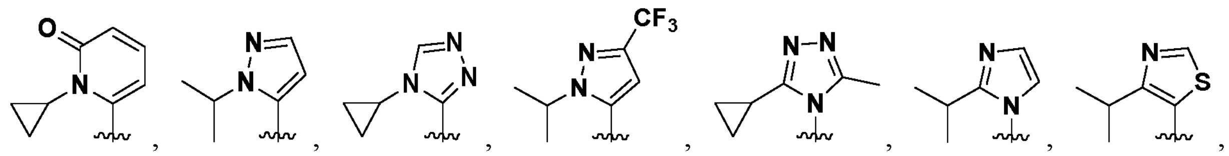
, , , , , , ,

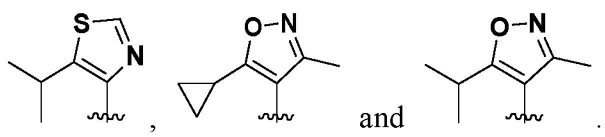
, and .

In some embodiments of the present disclosure, the compound is represented by Formula IVa or Formula IVb:

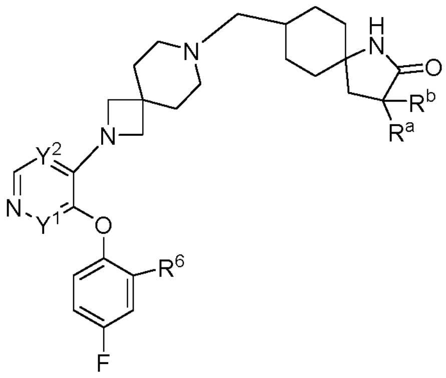 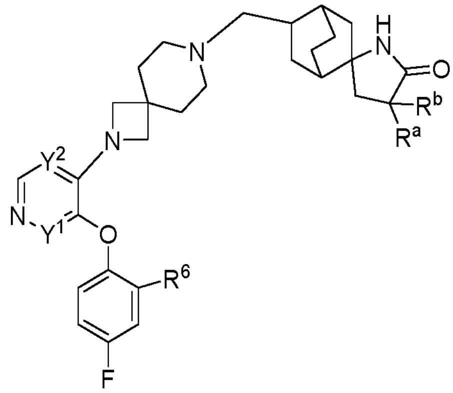

Formula IVa Formula IVb

wherein,

$Y^1$ and $Y^2$ are independently CH or N; preferably, $Y^1$ is N and $Y^2$ is N; alternatively, $Y^1$ is CH and $Y^2$ is N;
$R^a$ and $R^b$ are independently selected from the group consisting of hydrogen, fluorine, methyl,

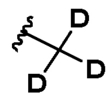,

ethyl, isopropyl,

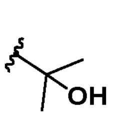, 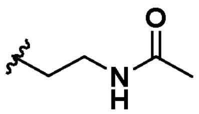, 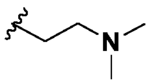, 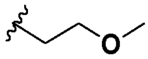,

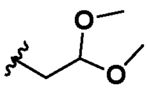, 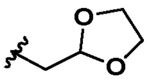, 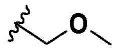, 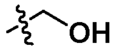, 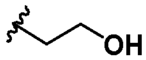, 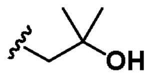 and 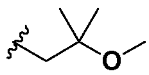;

alternatively, $R^a$, $R^b$ and the atom connecting therewith together form

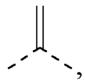, 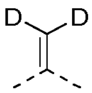,

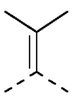, 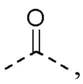, 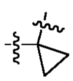, 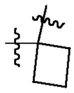, 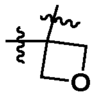, 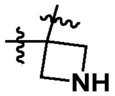, 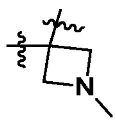, 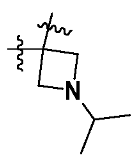, 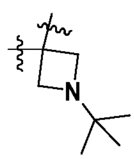, 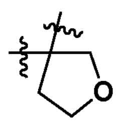

or

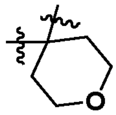;

preferably, $R^a$ is hydrogen, and $R^b$ is independently selected from the group consisting of hydrogen, fluorine, methyl,

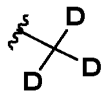,

ethyl, isopropyl,

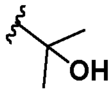, 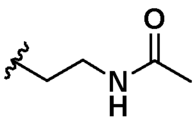, 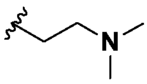,

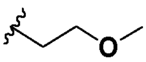, 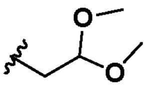, 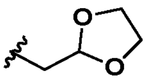, 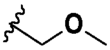, 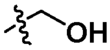, 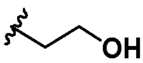, 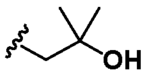

and

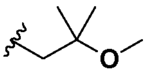;

alternatively, $R^a$ and $R^b$ are the same and are selected from the group consisting of hydrogen, fluorine, methyl,

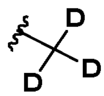,

ethyl, isopropyl,

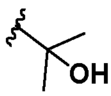, 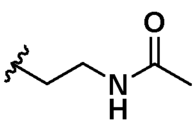, 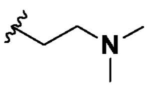,

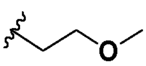, 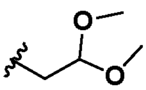, 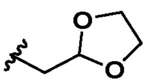, 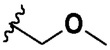, 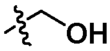, 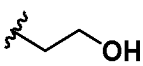, 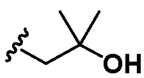

and

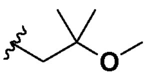;

and
$R^6$ is selected from the group consisting of

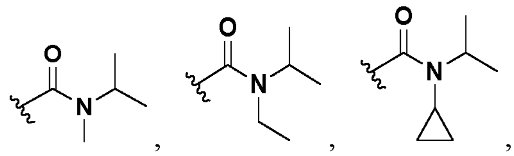
, , ,

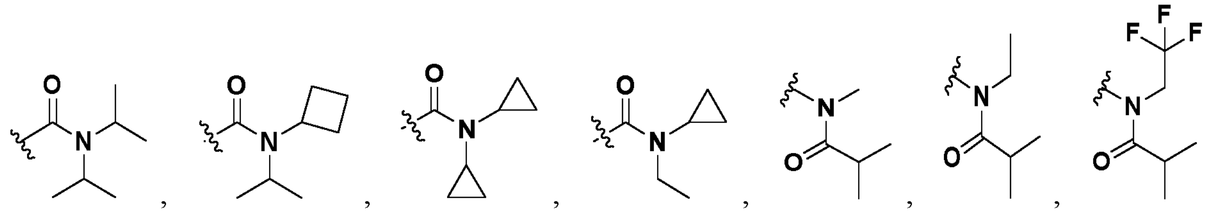
, , , , , , ,

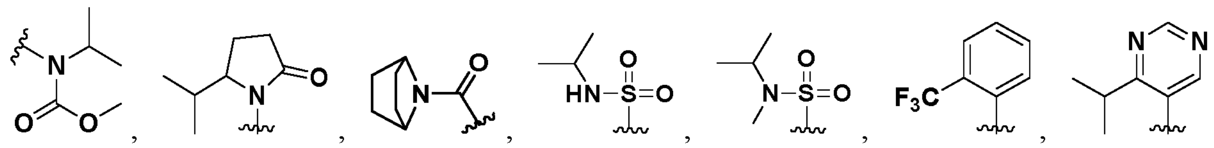
, , , , , , ,

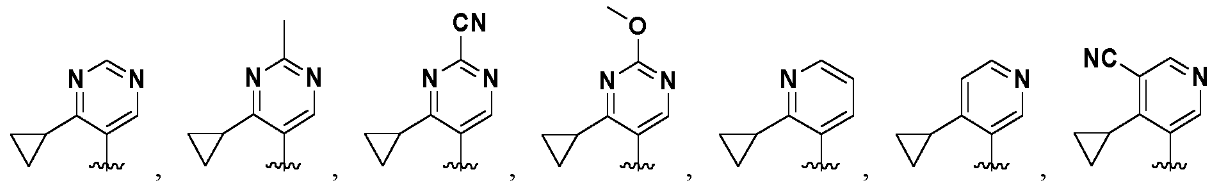
, , , , , , ,

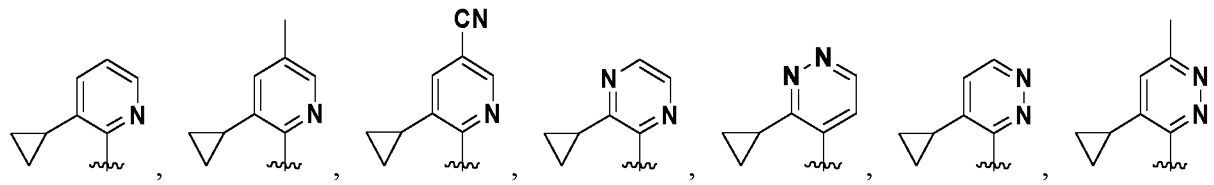
, , , , , , ,

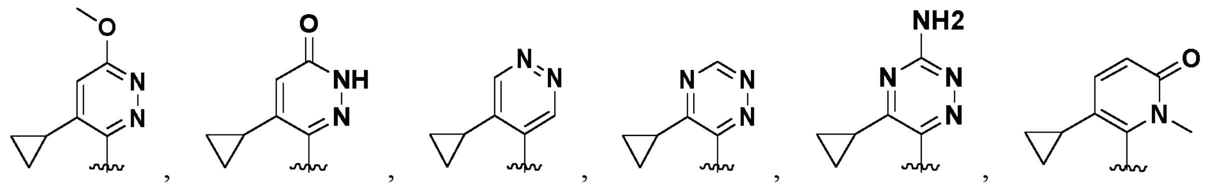
, , , , , ,

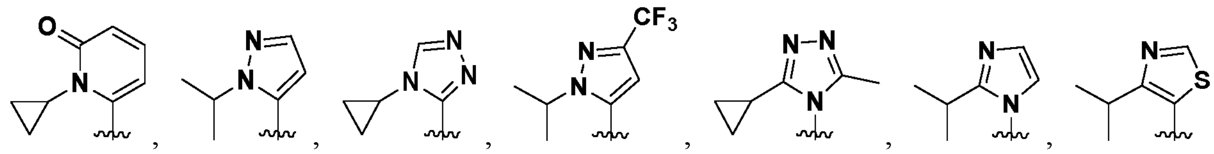
, , , , , , ,

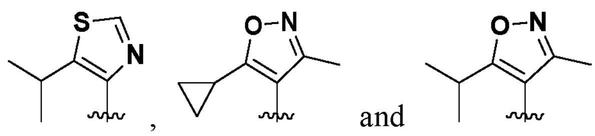
, and .

In some embodiments of the present disclosure, the compound is represented by formula Va:

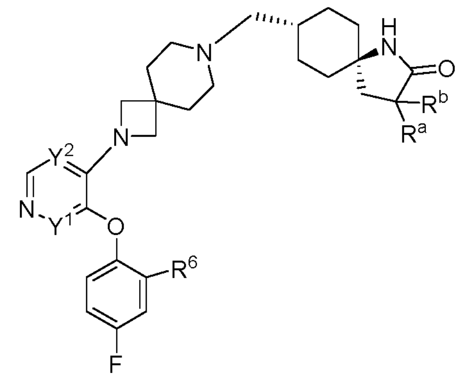

Formula Va

wherein,

$Y^1$ and $Y^2$ are independently CH or N; preferably, $Y^1$ is N and $Y^2$ is N; alternatively, $Y^1$ is CH and $Y^2$ is N;
$R^a$ and $R^b$ are independently selected from the group consisting of hydrogen, fluorine, methyl,

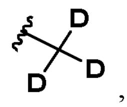,

ethyl, isopropyl,

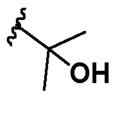, 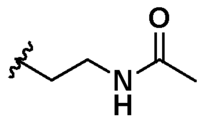, 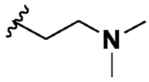, 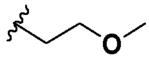,

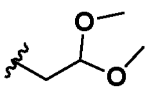, 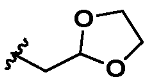, 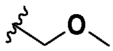, 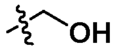, 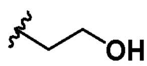, 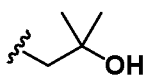 and 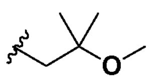;

alternatively, $R^a$, $R^b$ and the atom connecting therewith together form

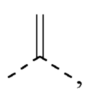, 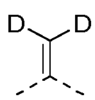,

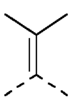, 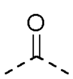, 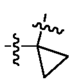, 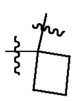, 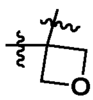, 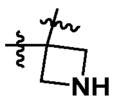, 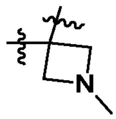, 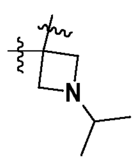, 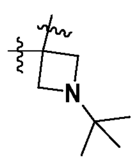, 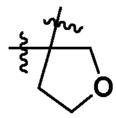

or

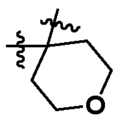;

and
$R^6$ is selected from the group consisting of

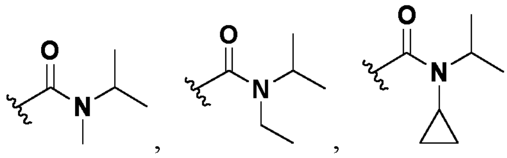, , ,

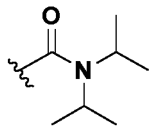, 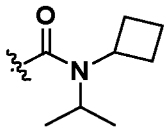, 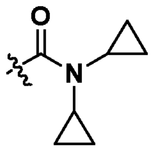, 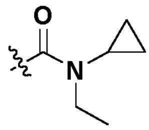, 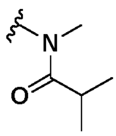, 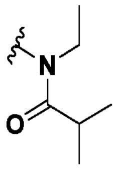, 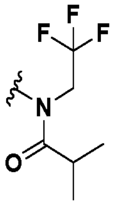,

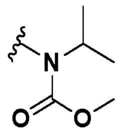, 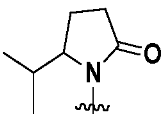, 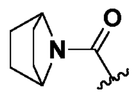, 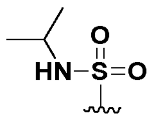, 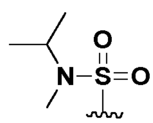, 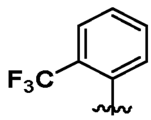, 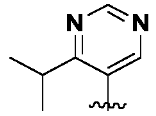,

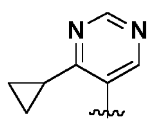, 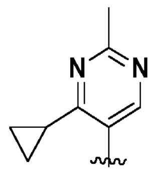, 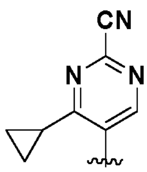, 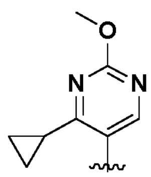, 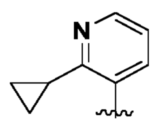, 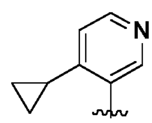, 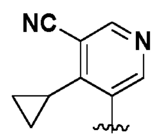,

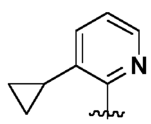, 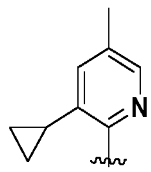, 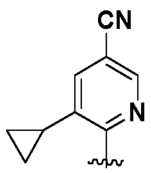, 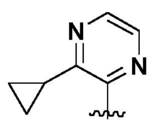, 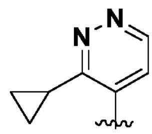, 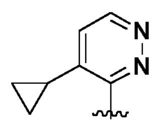, 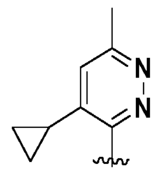,

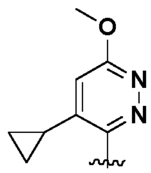, 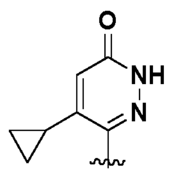, 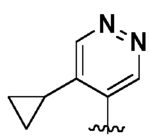, 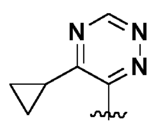, 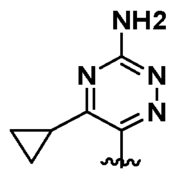, 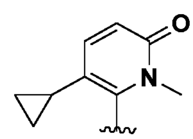,

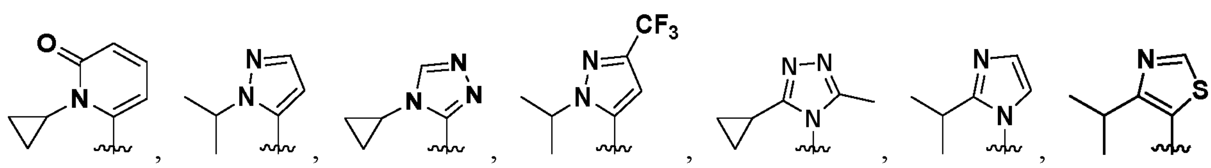, , , , , , ,

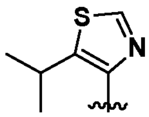, 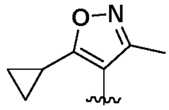 and 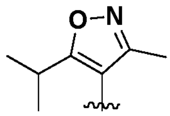.

In some specific embodiments of the present disclosure, the compound is specifically selected from the group consisting of:

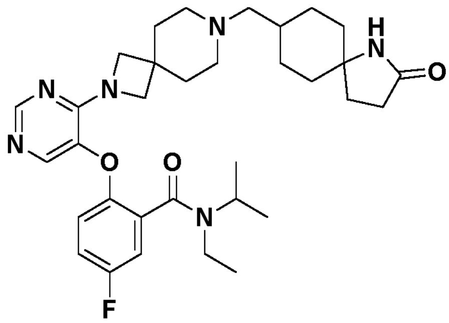,

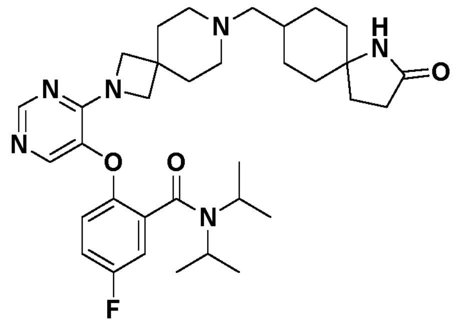,

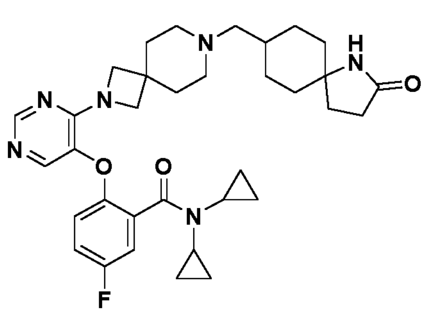,

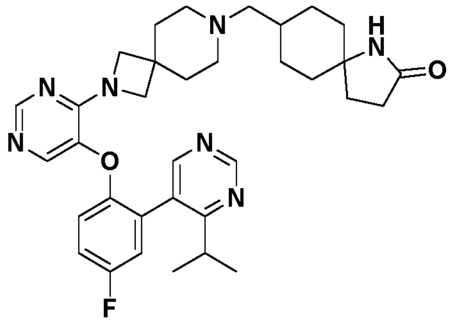,

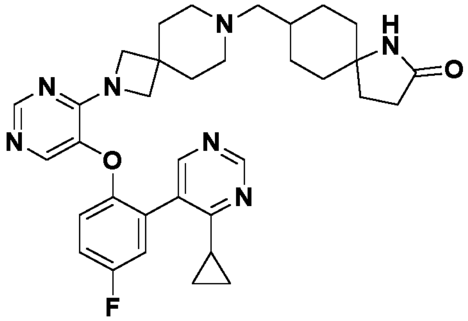,

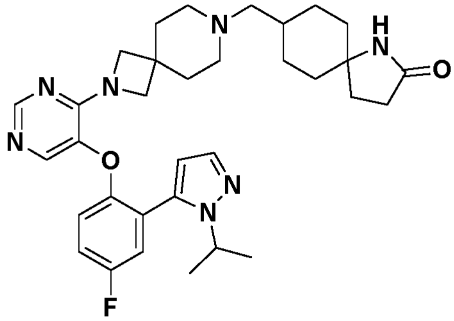,

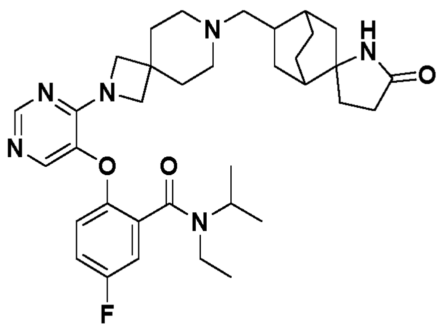,

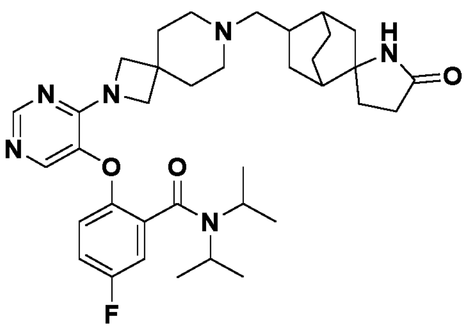,

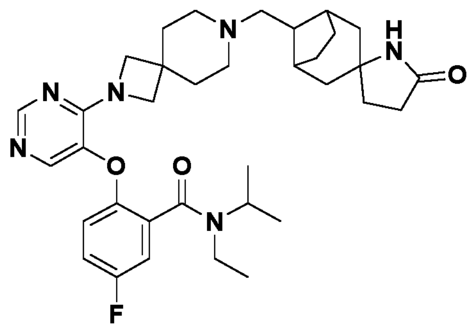
,

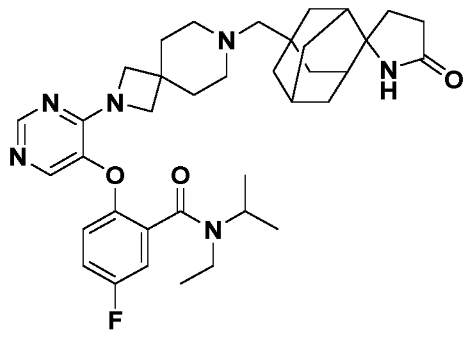
,

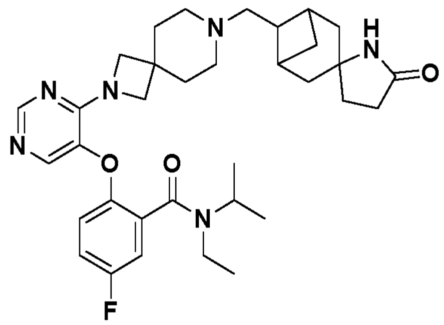
,

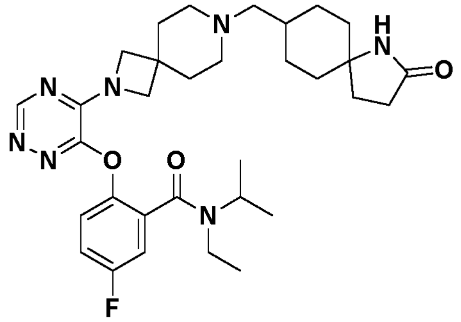
,

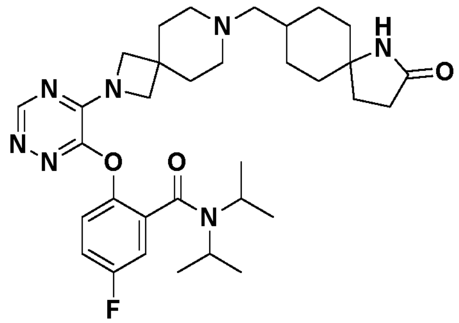
,

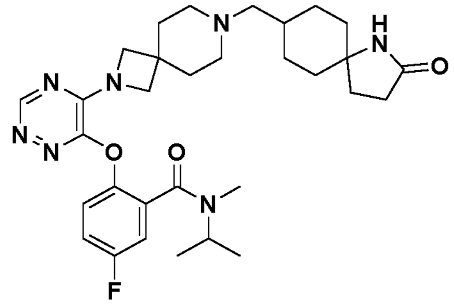
,

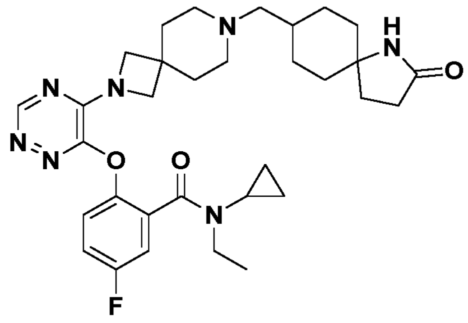
,

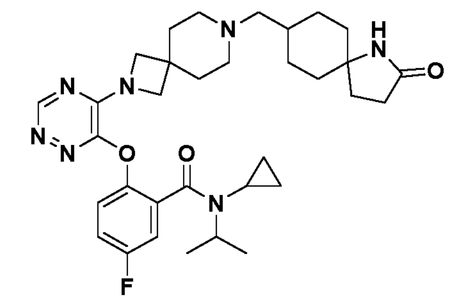
,

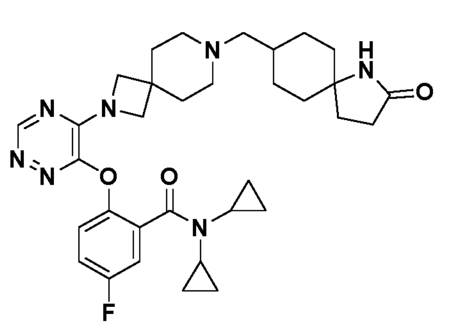
,

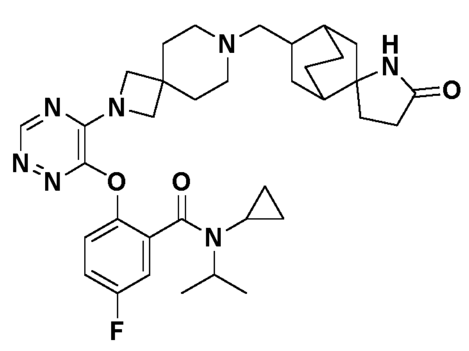
,

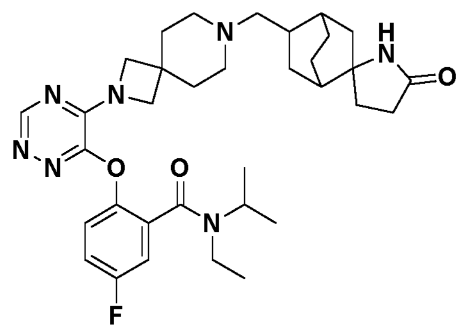
,

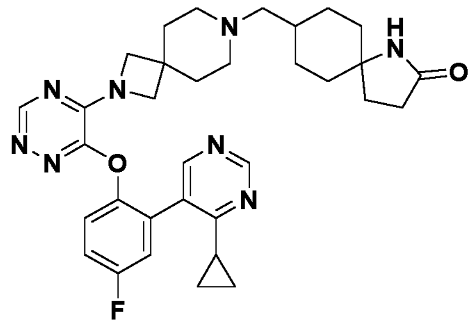
,

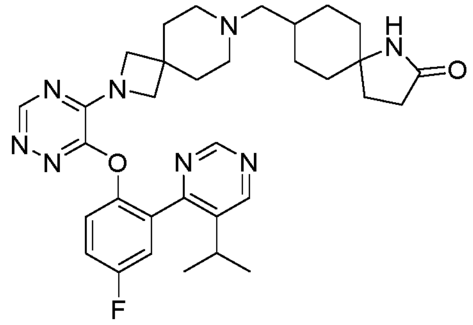
,

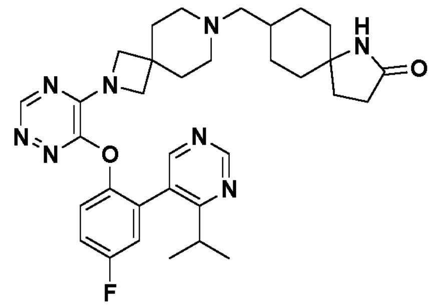
,

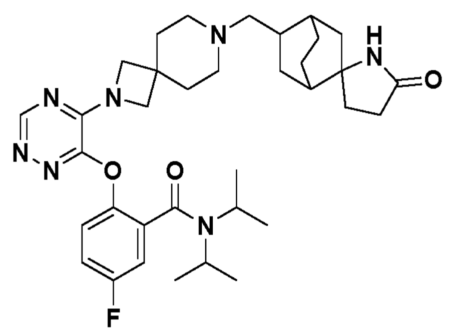
,

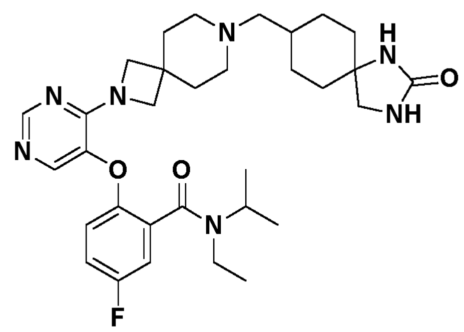
,

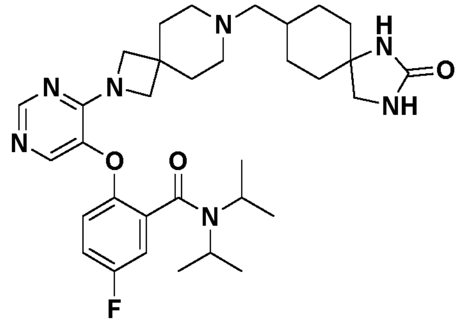

,

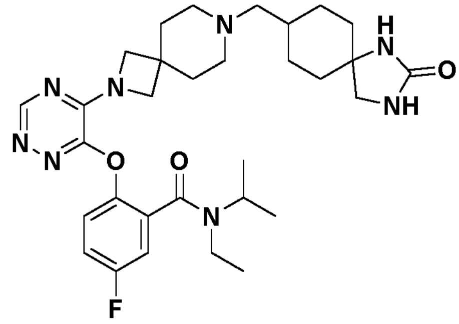

,

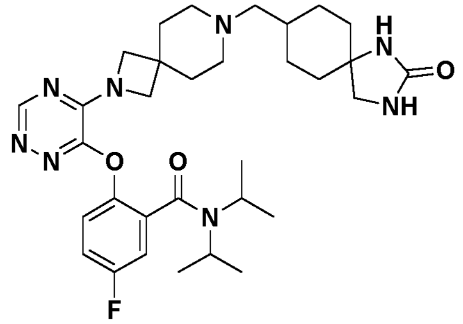

,

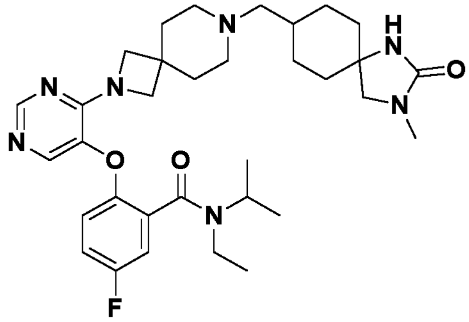

,

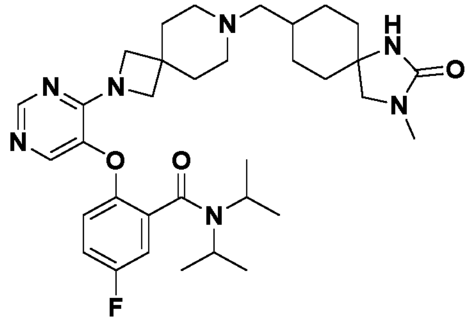

,

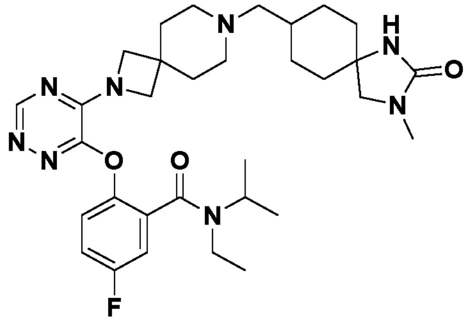

,

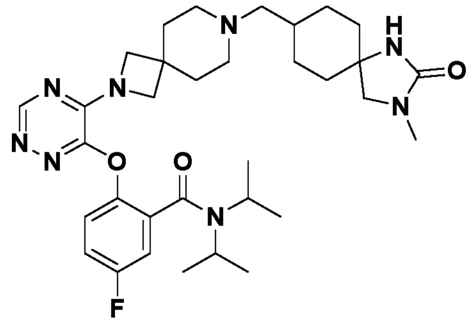

,

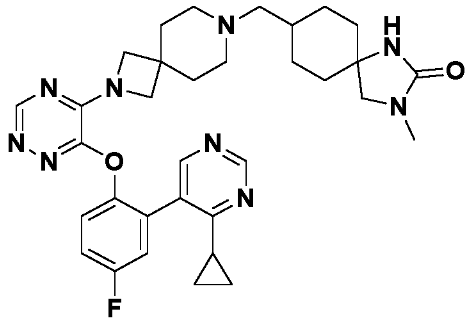

,

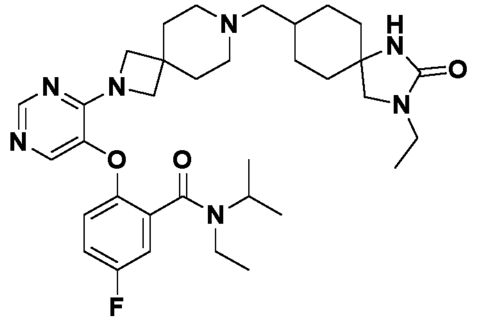

,

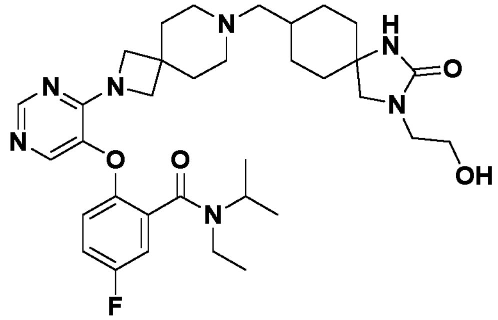

,

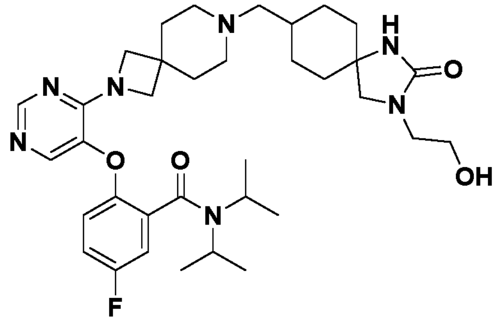

,

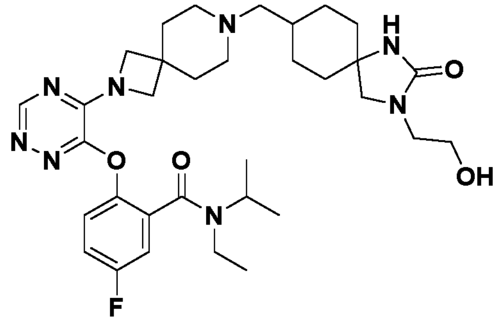

,

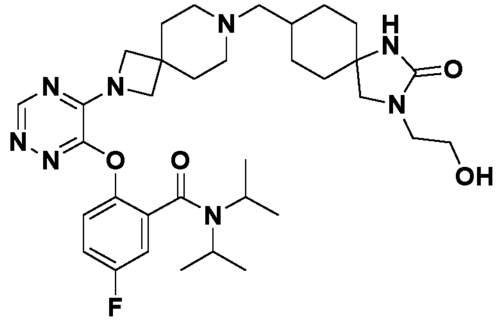

,

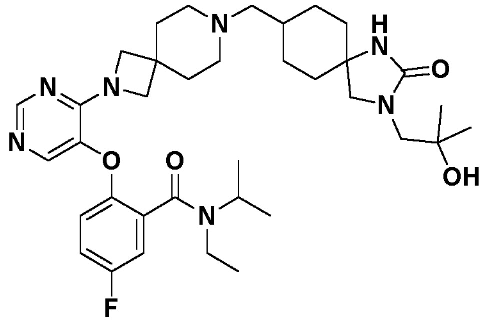

,

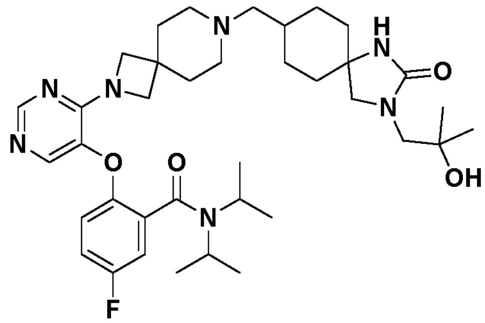

,

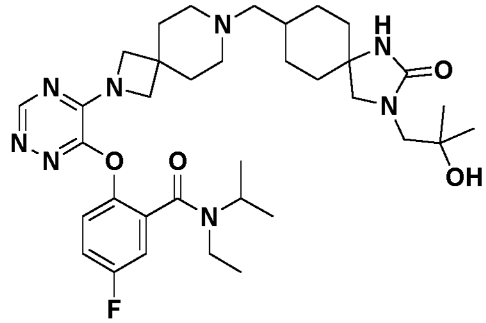

,

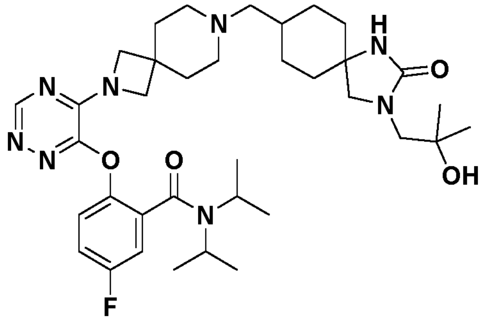

,

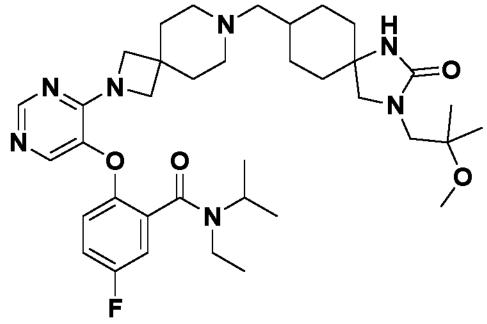

,

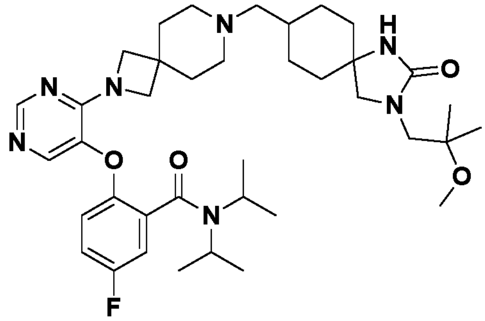

,

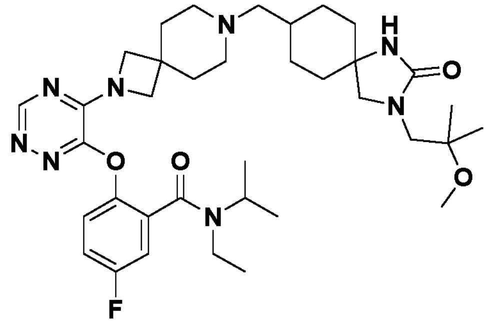

,

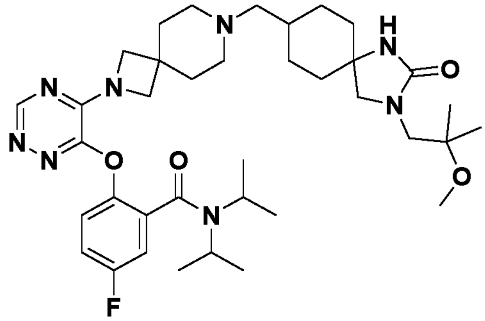

,

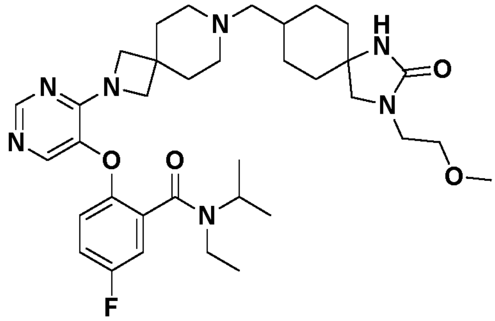

,

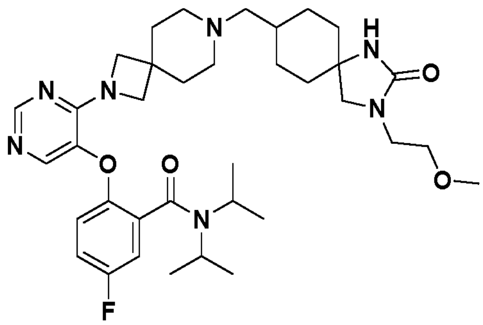

,

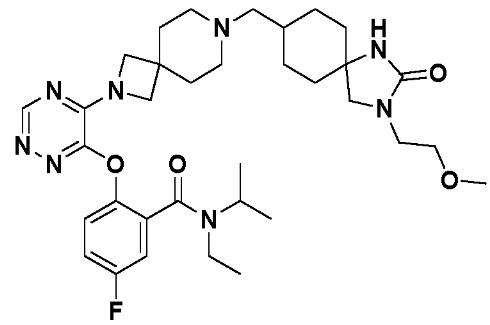

,

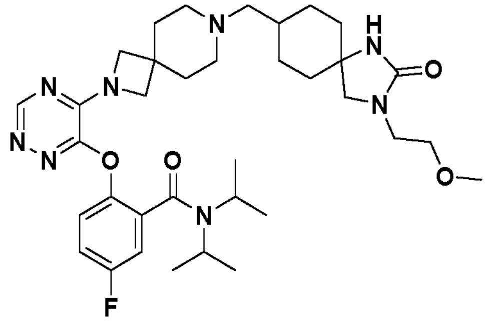,

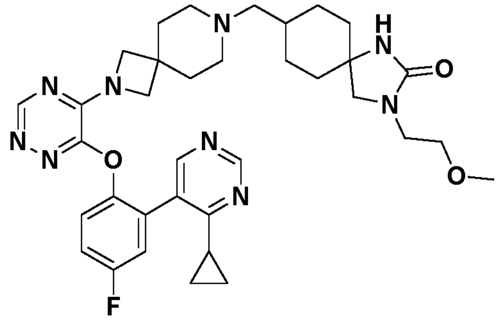,

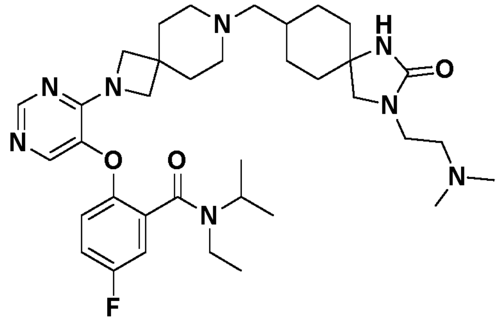,

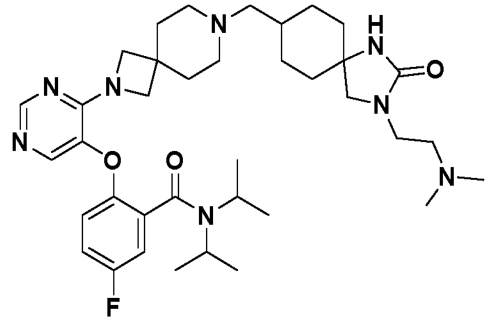,

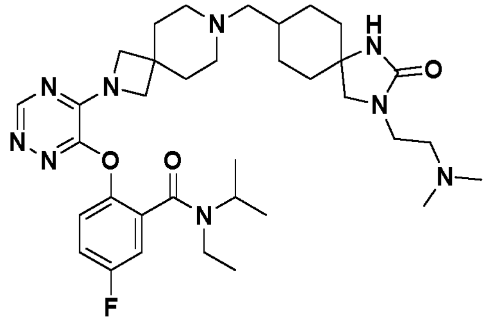,

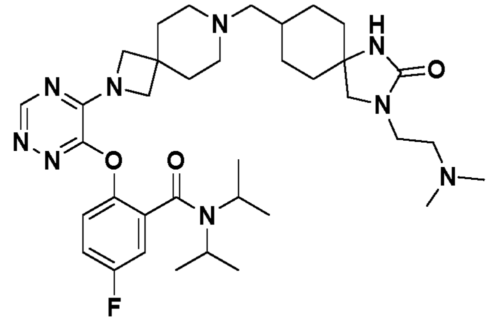,

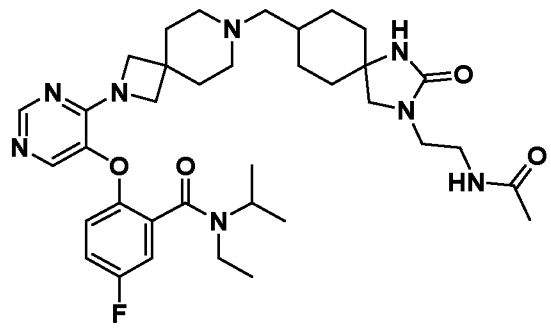,

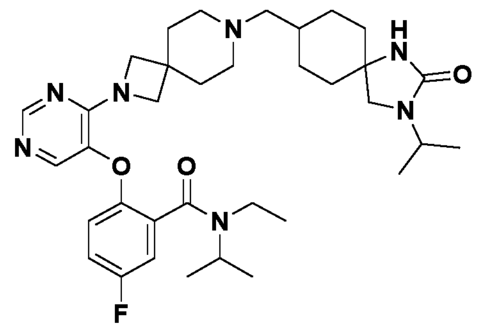,

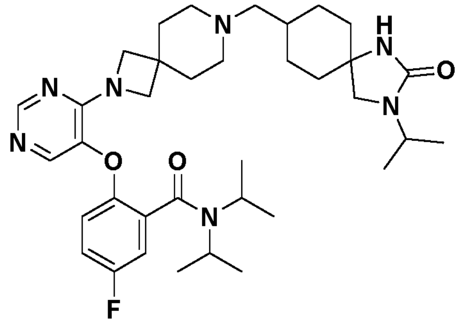

,

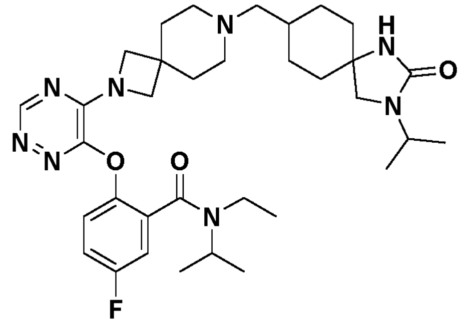

,

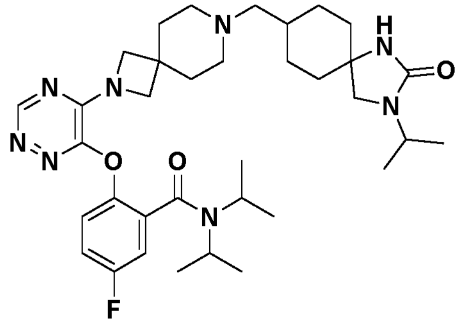

,

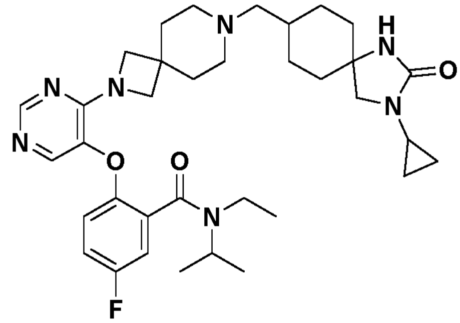

,

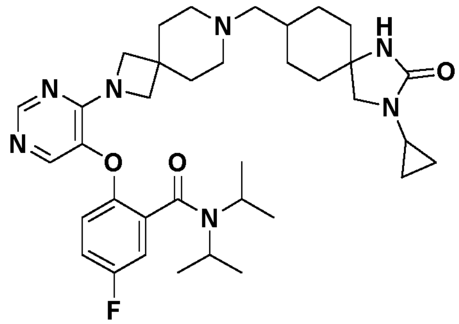

,

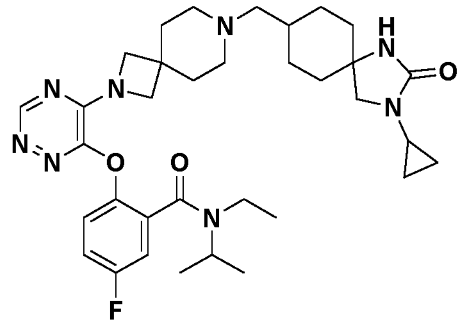

,

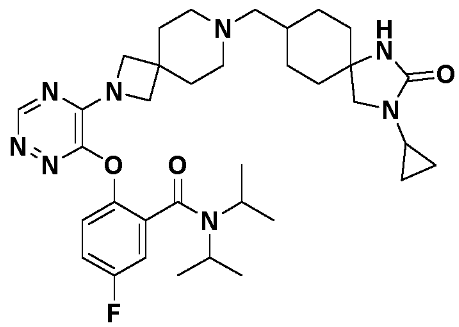

,

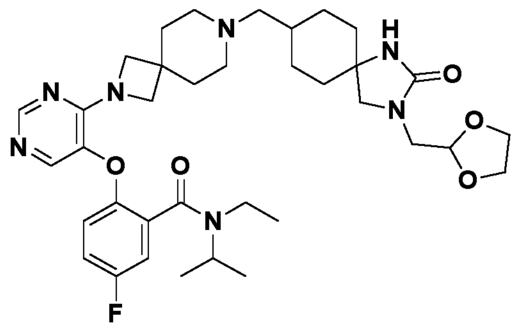

,

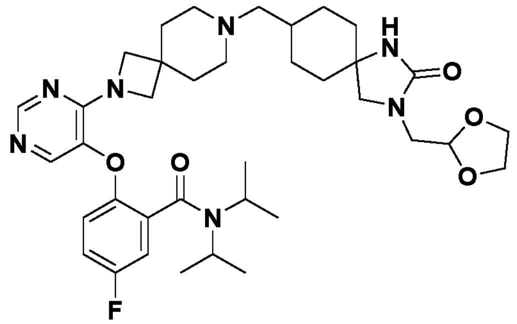,
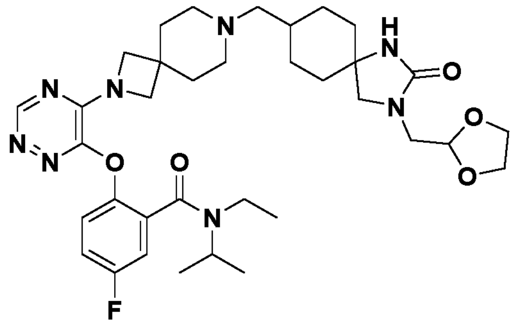,

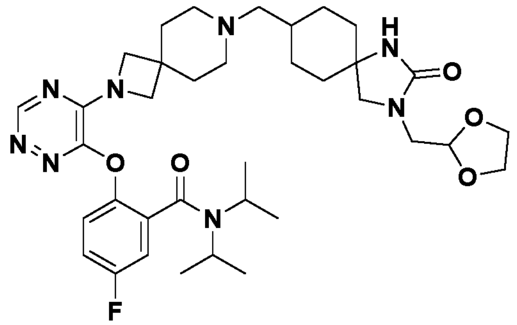,
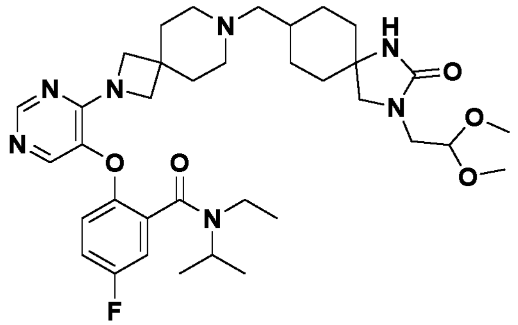,

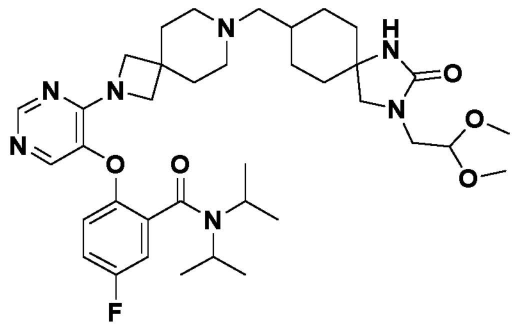,
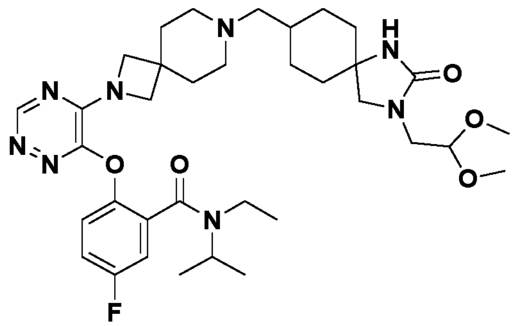,

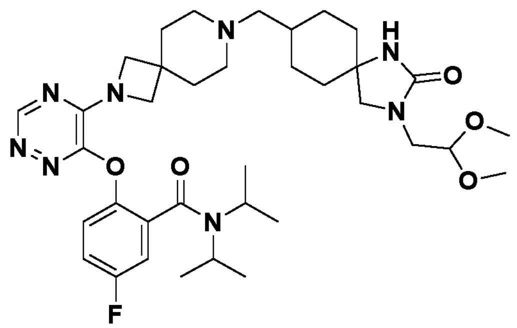,
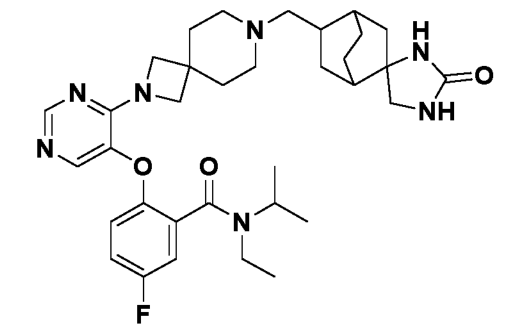,

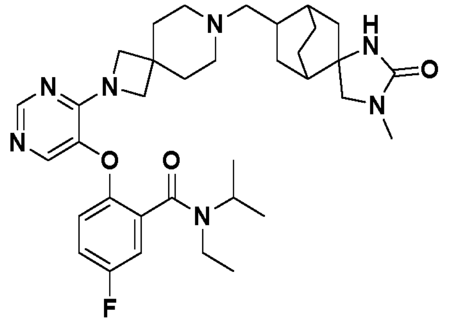
,
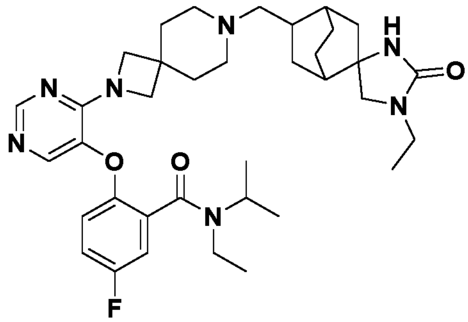
,
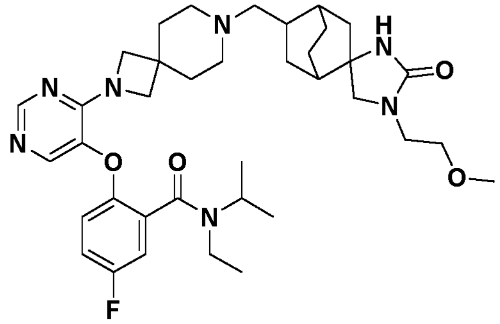
,
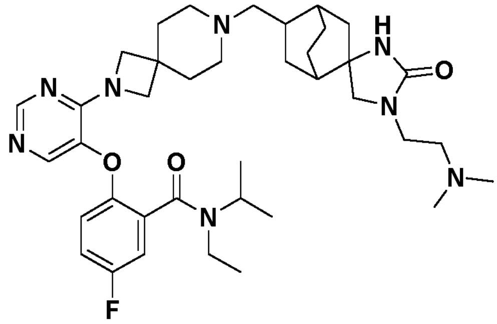
,
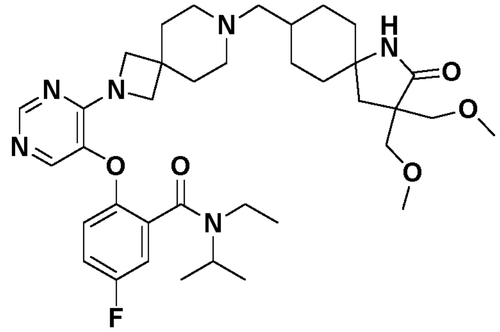
,
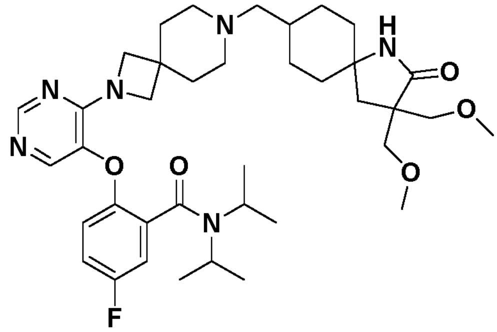
,
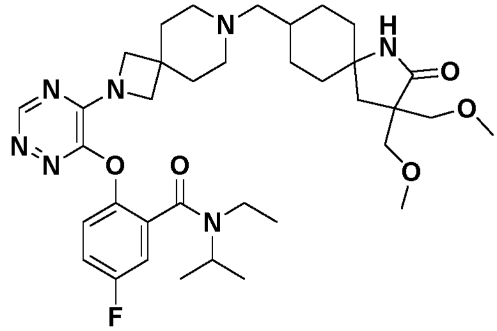
,
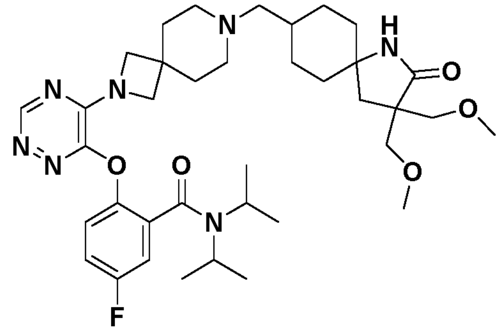
,

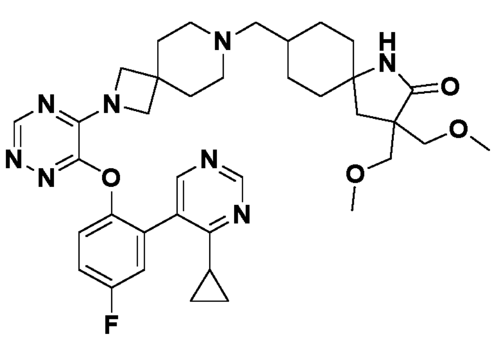

,

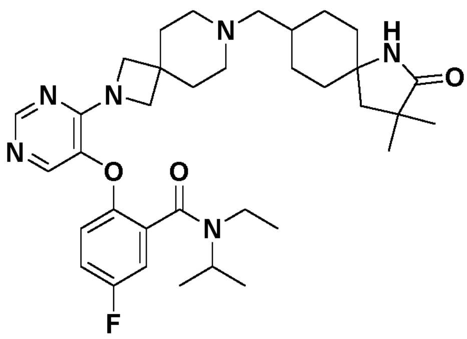

,

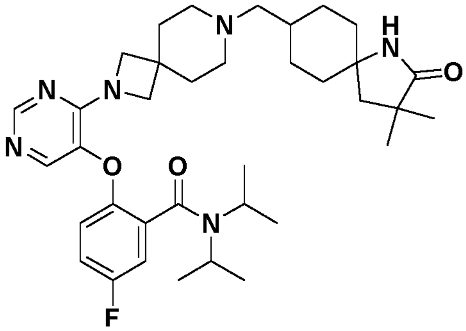

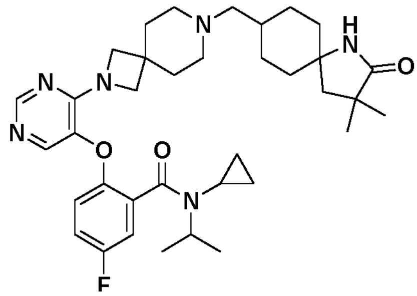

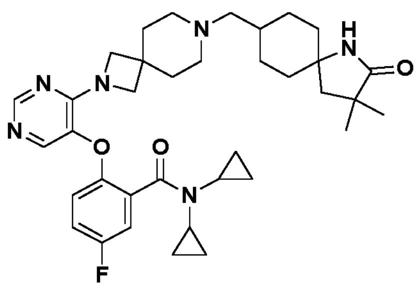

,

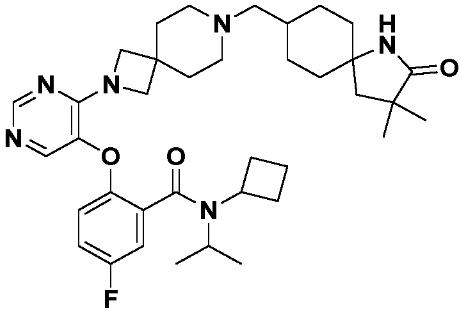

,

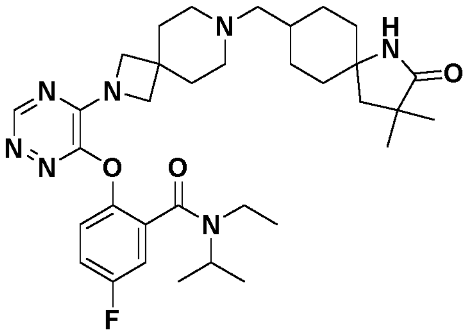

,

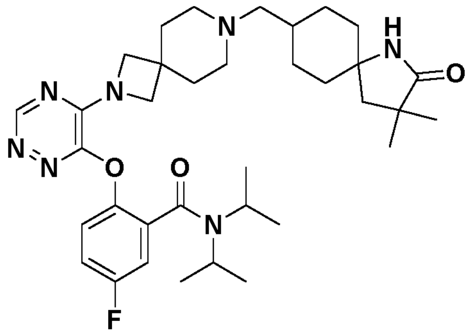

,

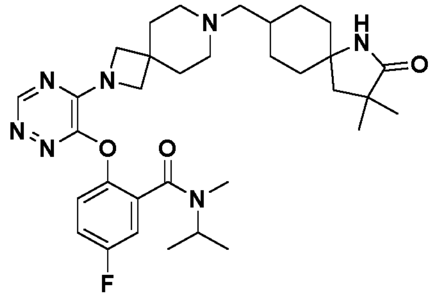
,

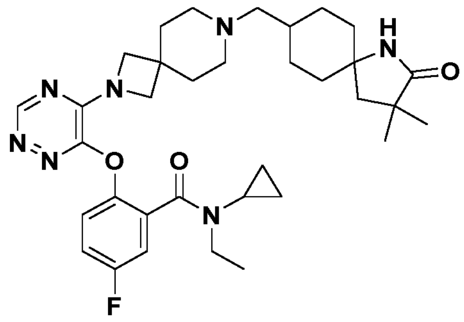
,

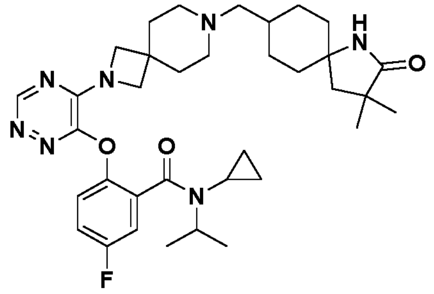
,

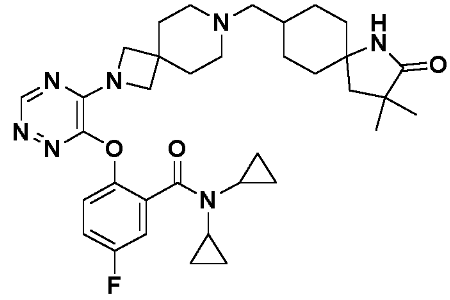
,

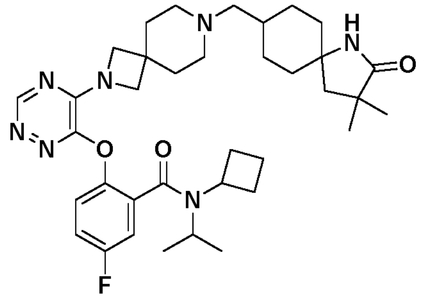
,

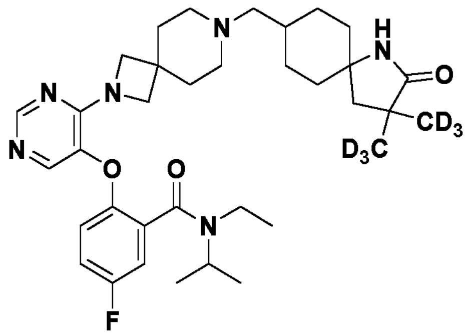
,

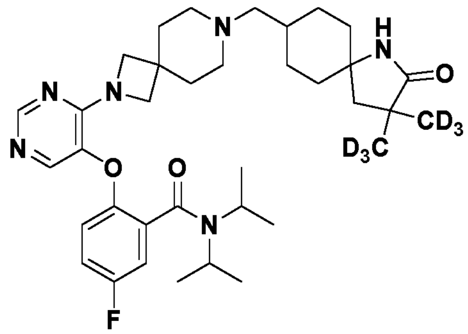
,

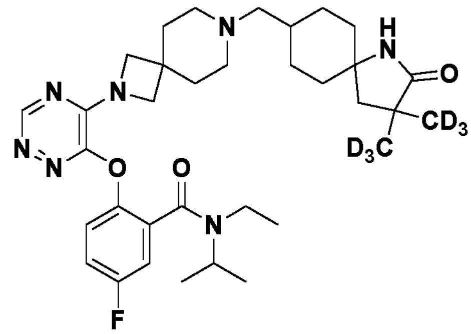
,

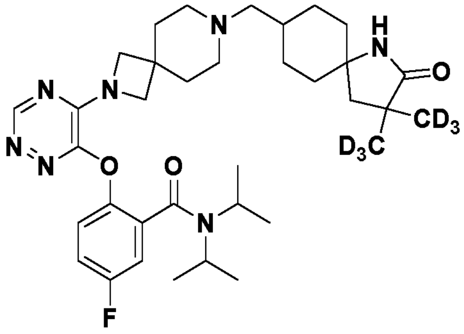

,

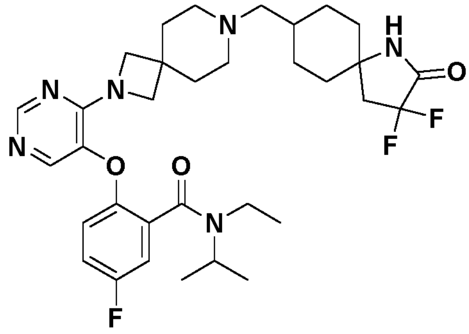

,

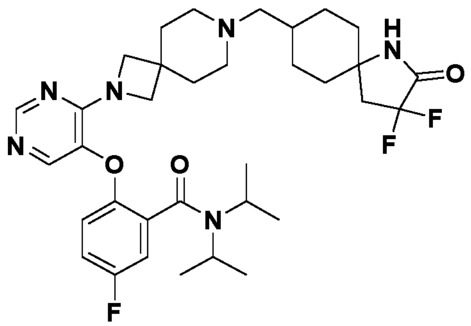

,

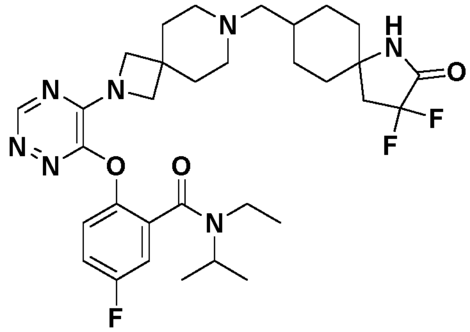

,

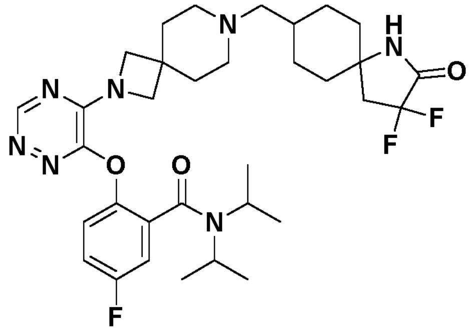

,

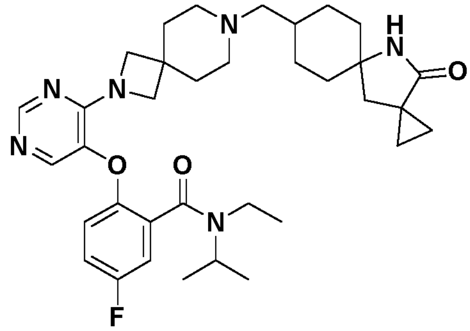

,

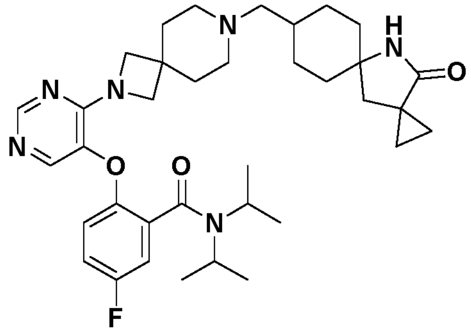

,

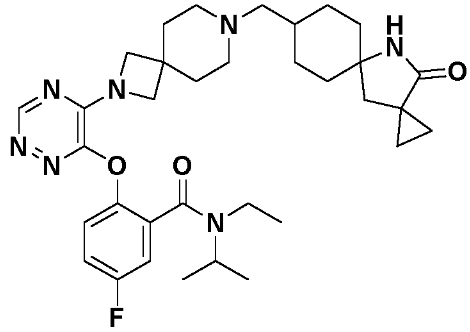

,

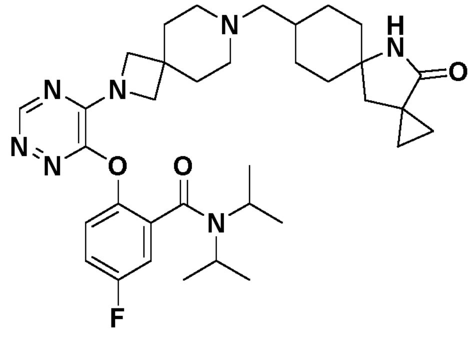

,

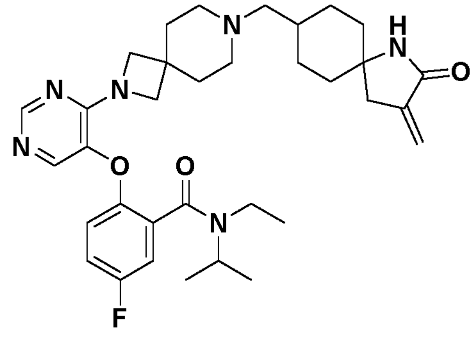

,

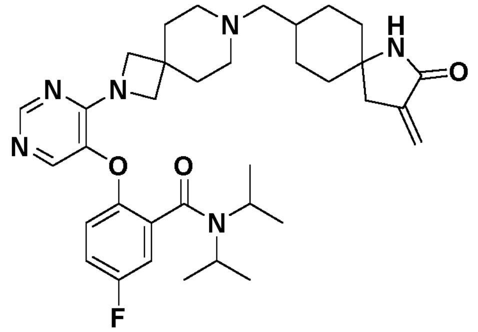

,

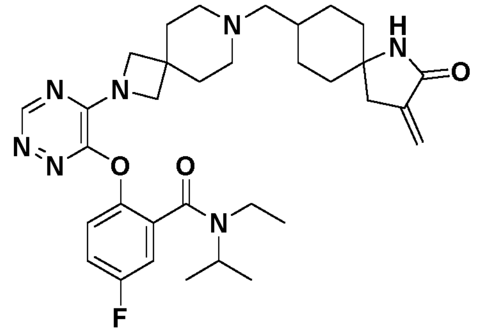

,

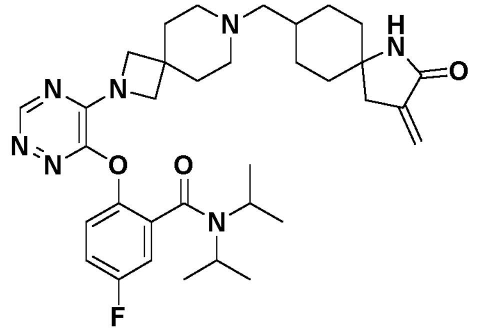

,

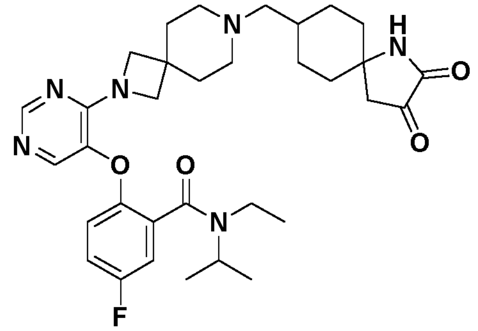

,

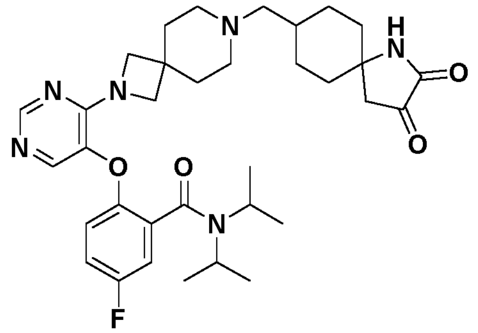

,

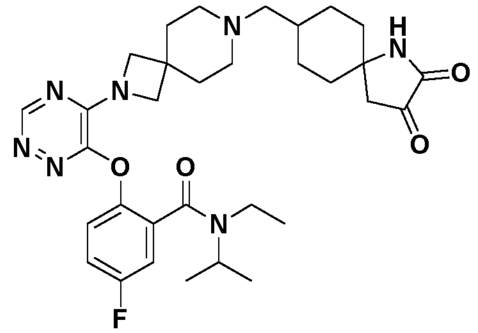

,

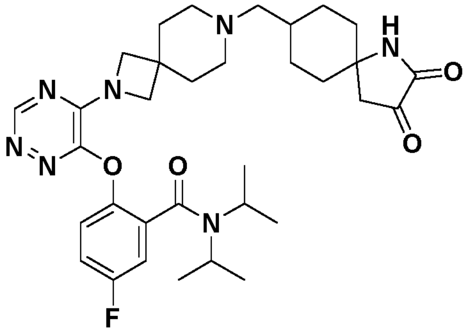

,

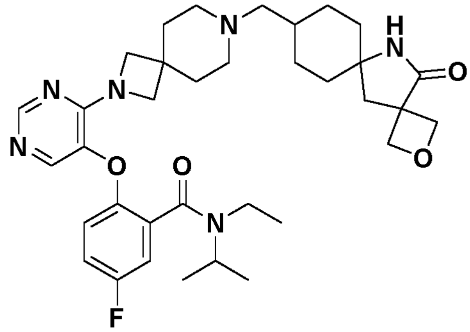

,

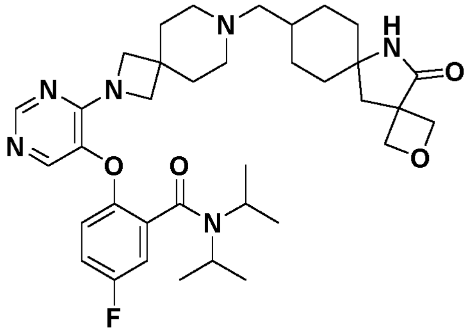

,

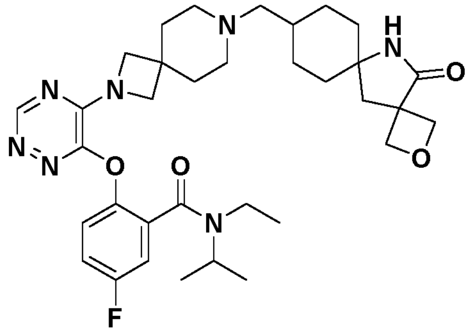

,

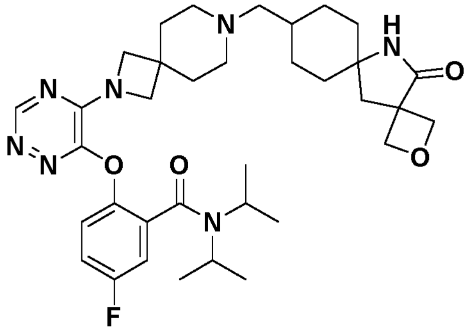

,

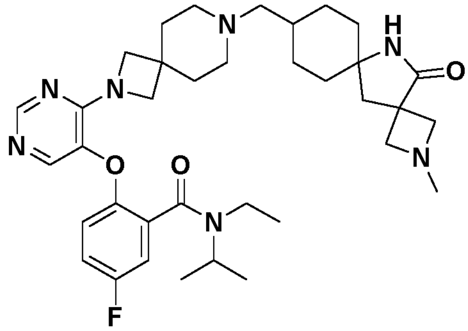

,

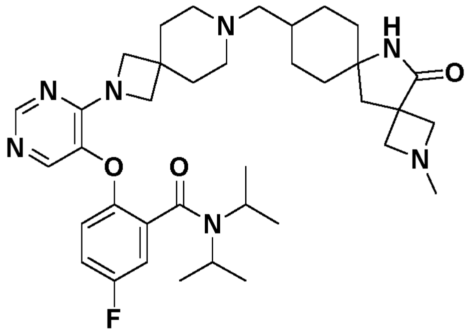

,

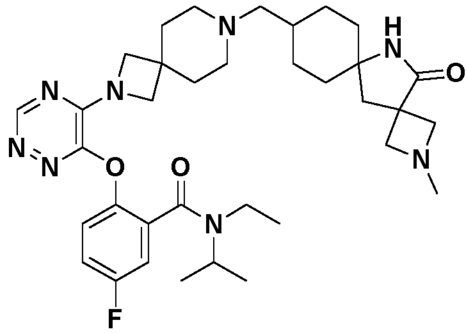

,

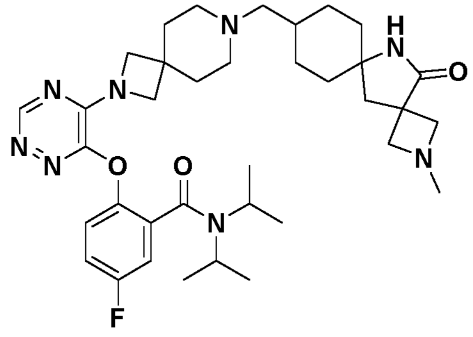

,

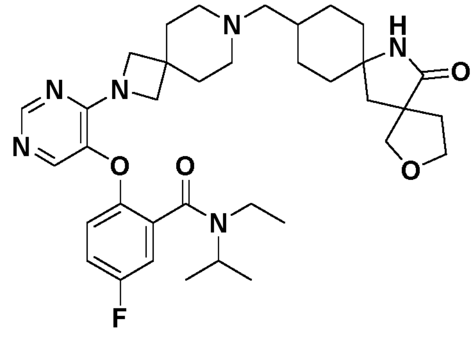

,

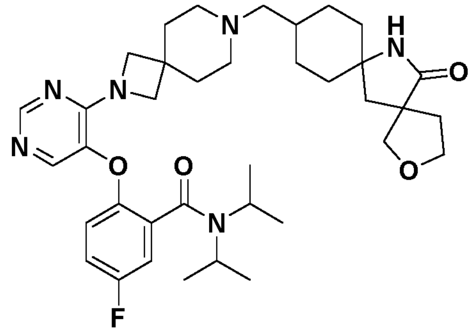

,

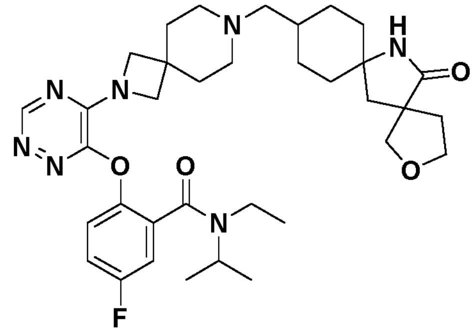

,

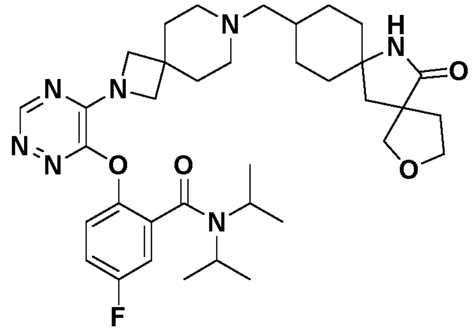

,

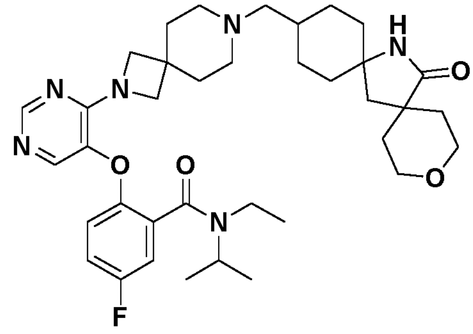

,

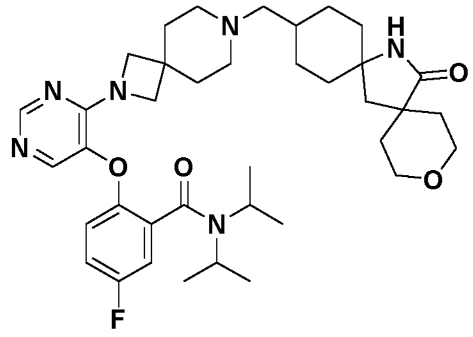

,

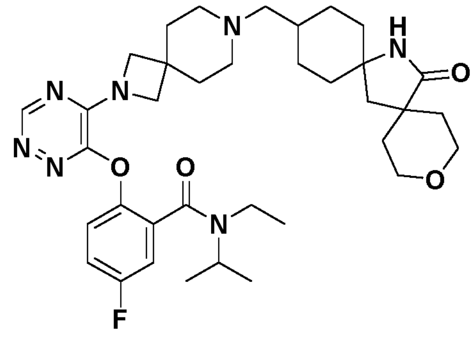

,

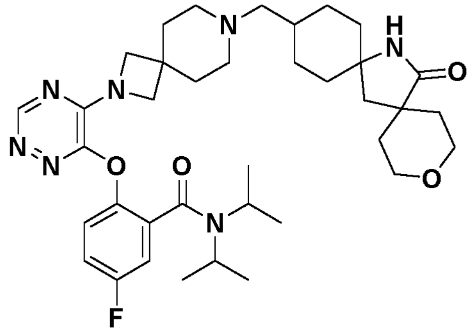

,

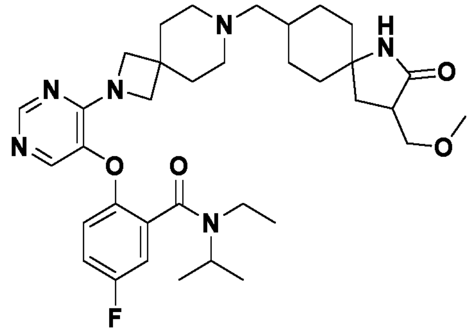

,

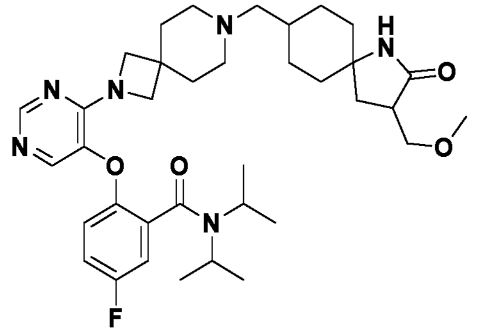

,

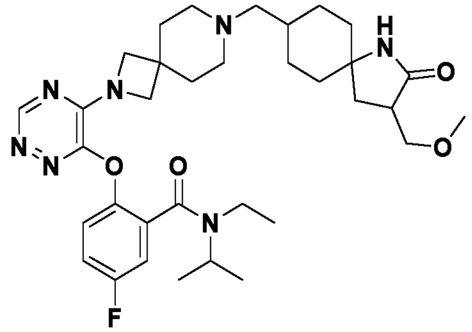

,

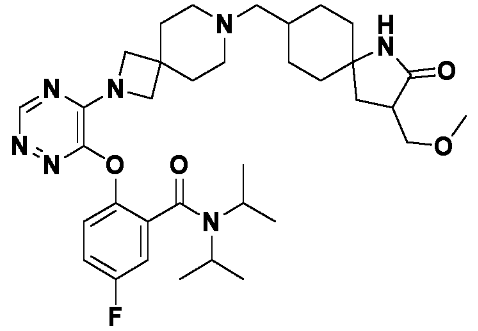

,

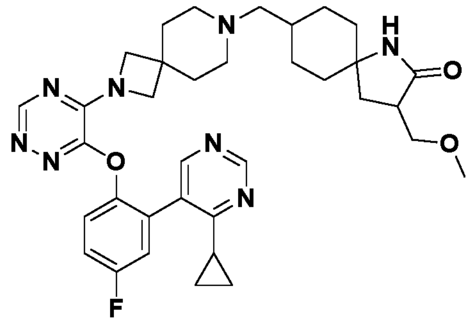

,

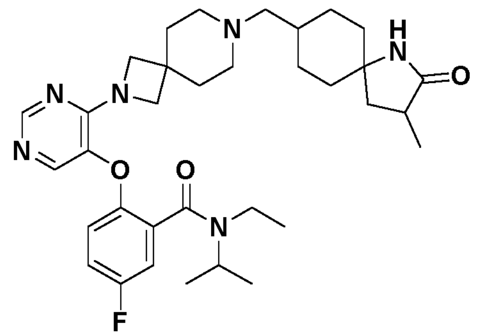

,

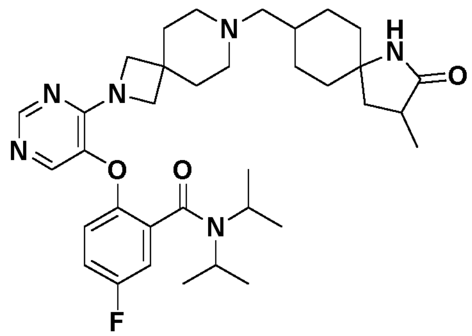

,

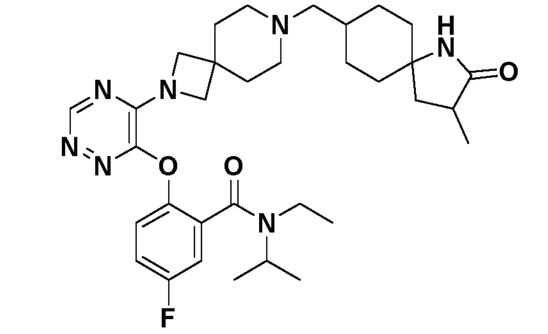

,

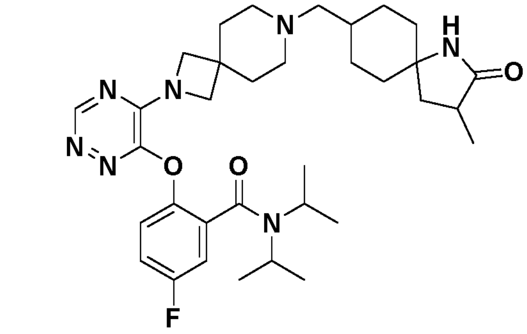

,

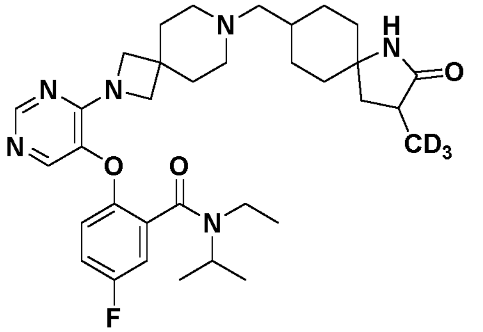

,

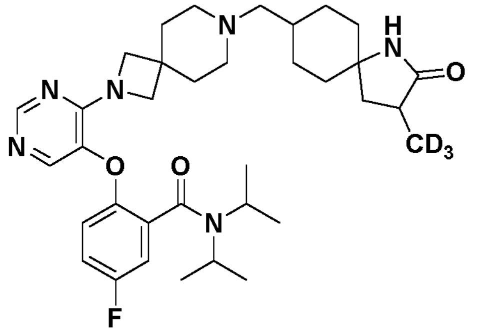

,

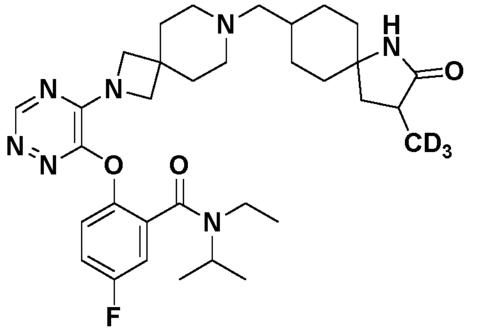

,

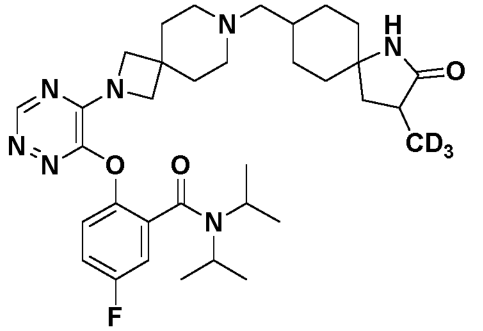

,

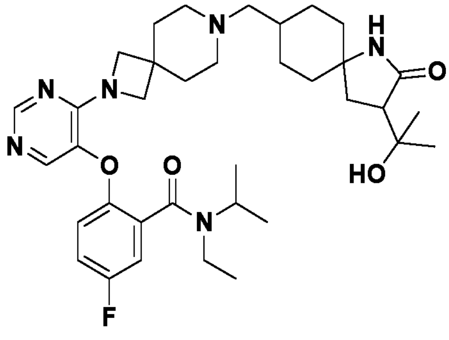

,

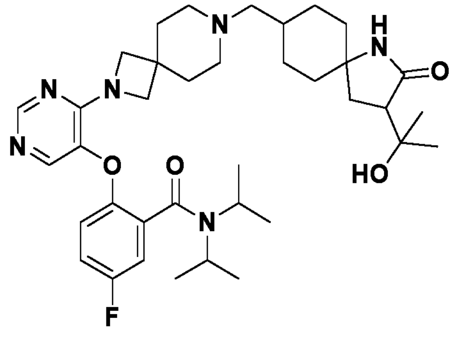

,

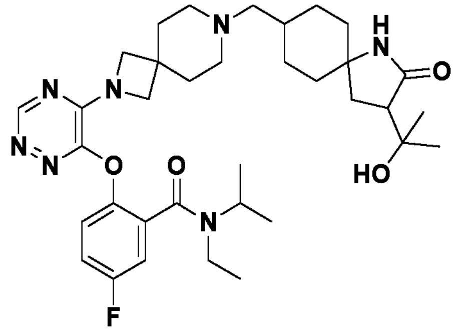

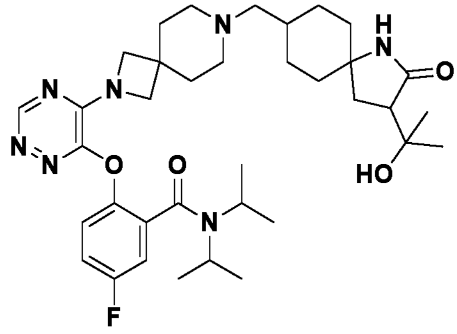

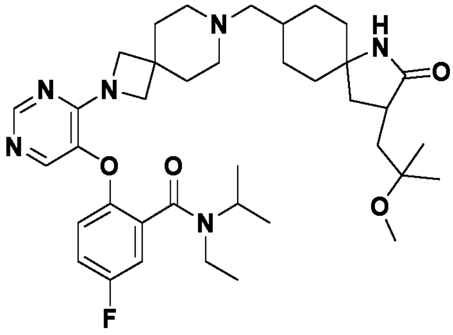

,

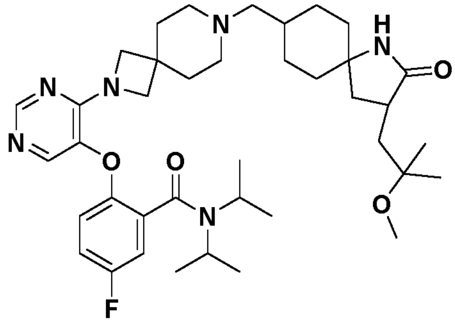

,

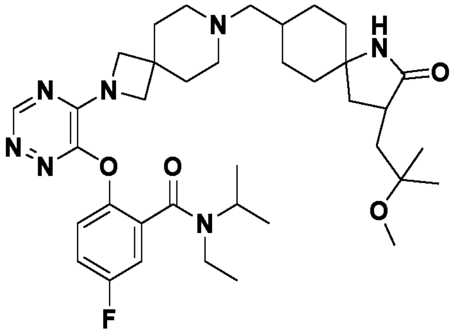

,

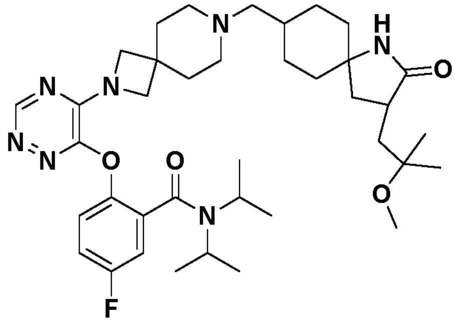

,

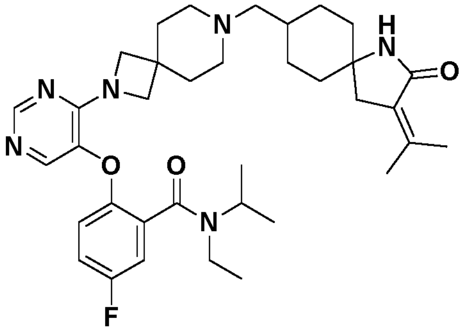

,

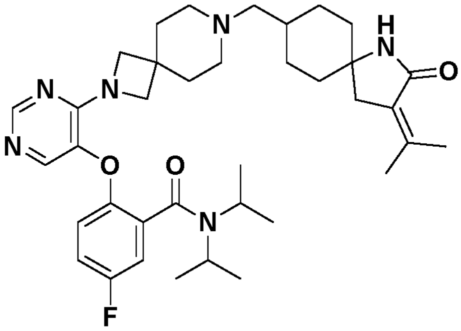

,

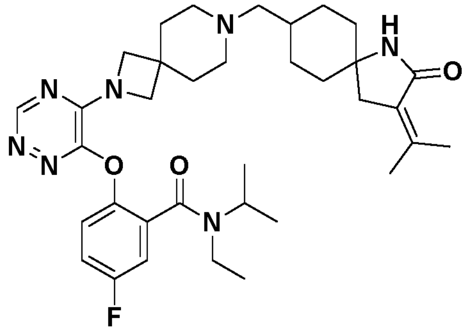

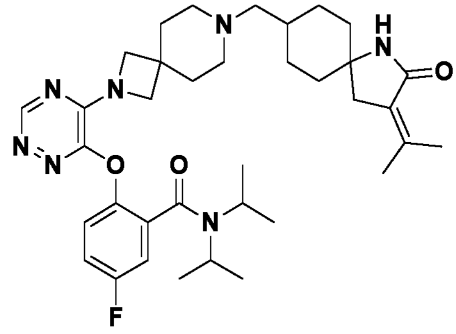

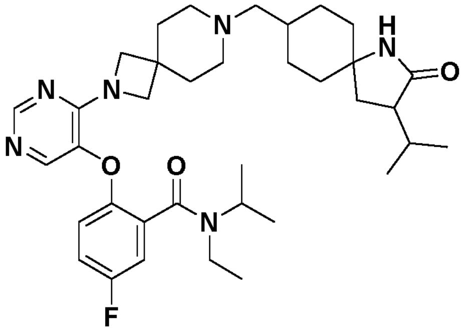

,

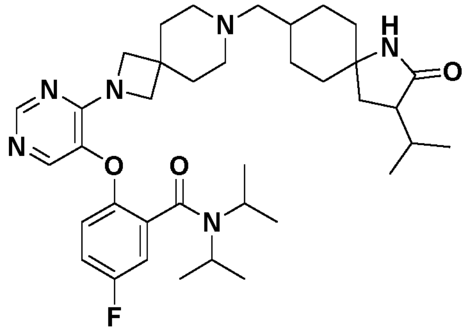

,

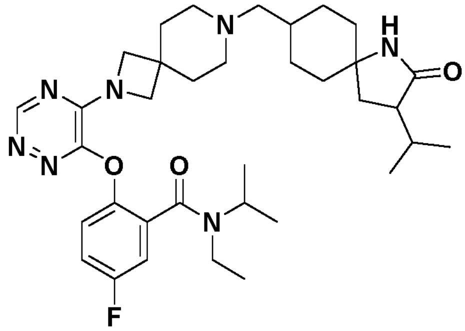

,

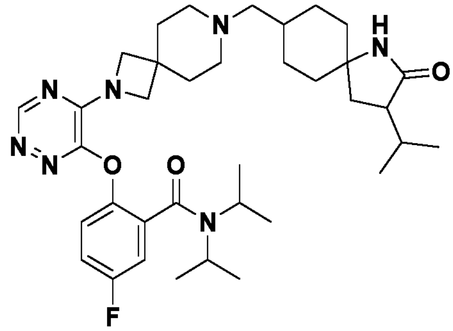

,

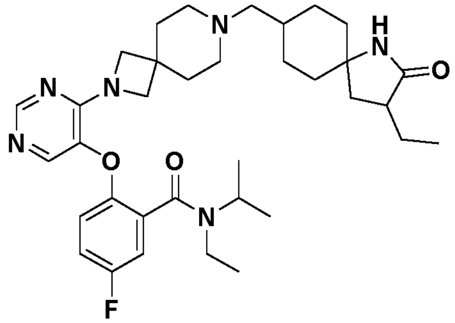

,

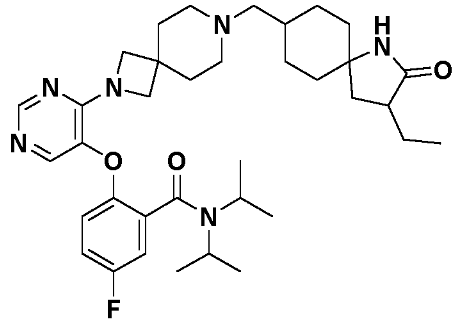

,

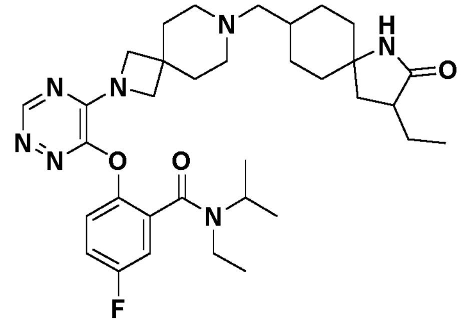

,

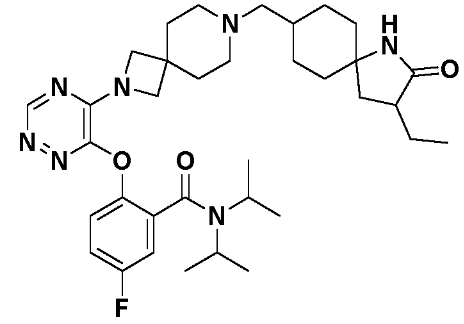

,

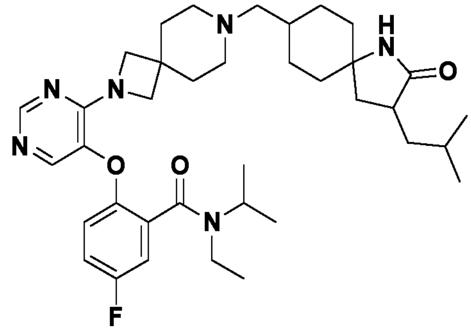

,

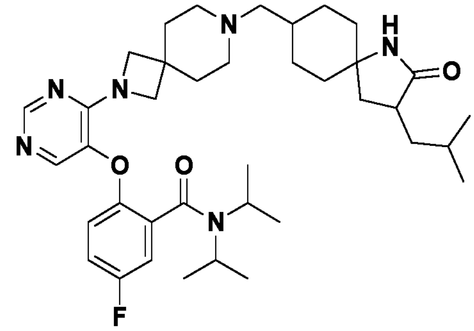

,

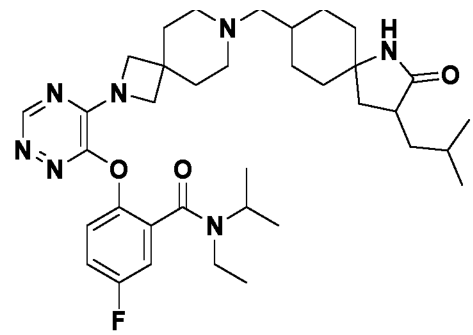

,

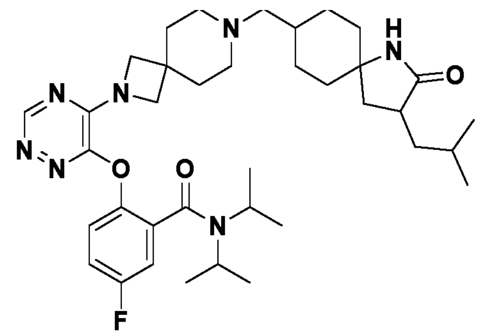

,

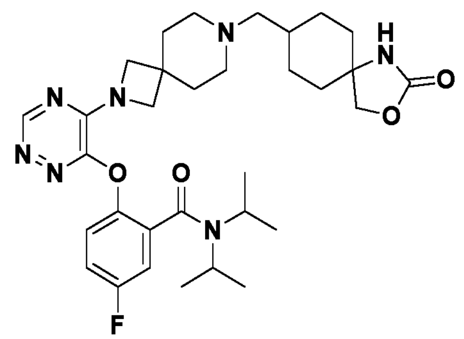

,

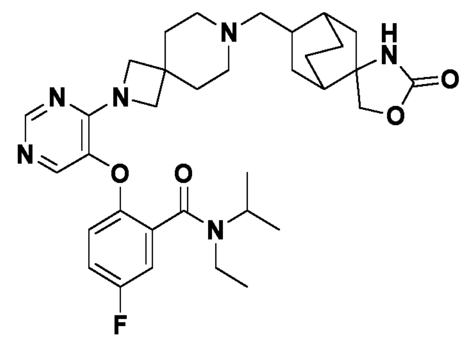

,

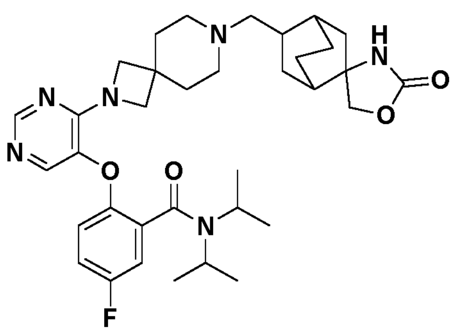

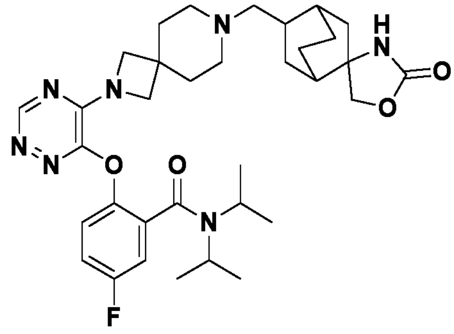

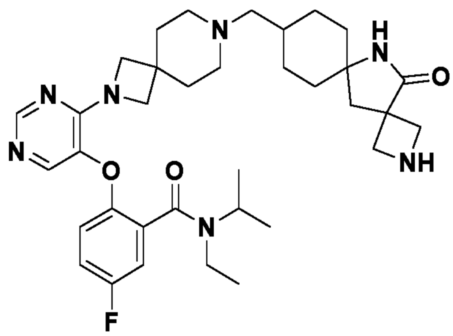

,

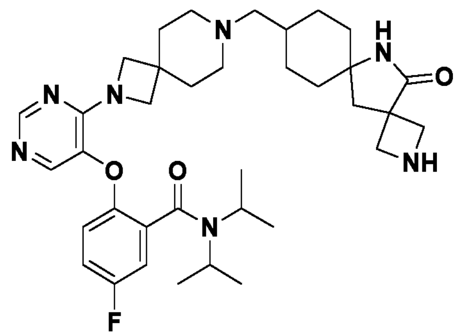

,

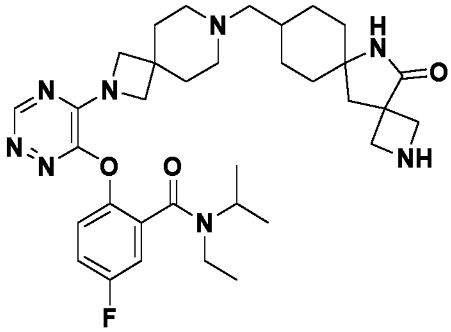

,

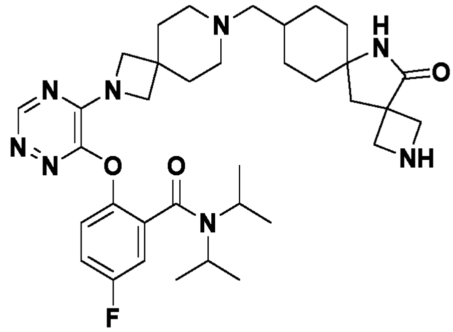

,

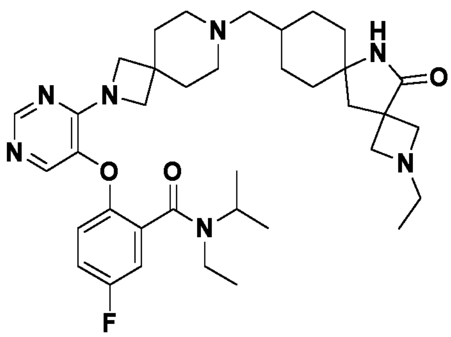

,

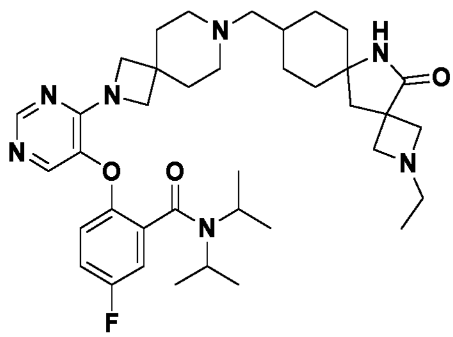

,

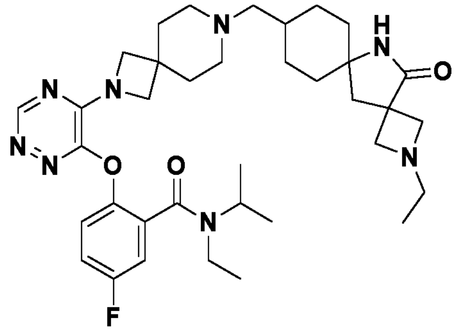

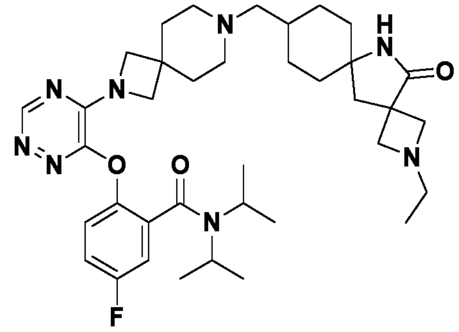

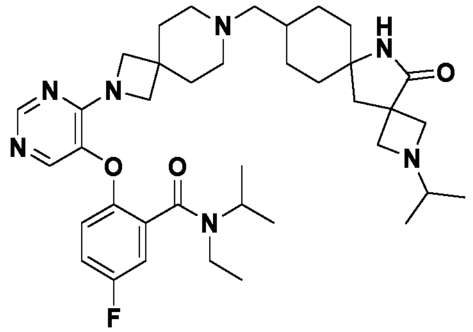

,

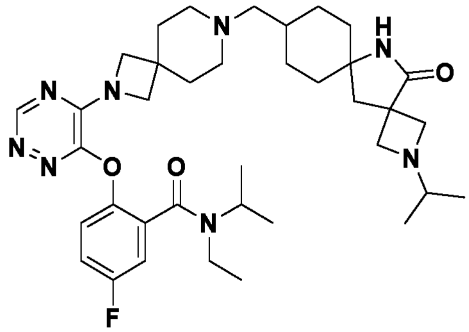

,

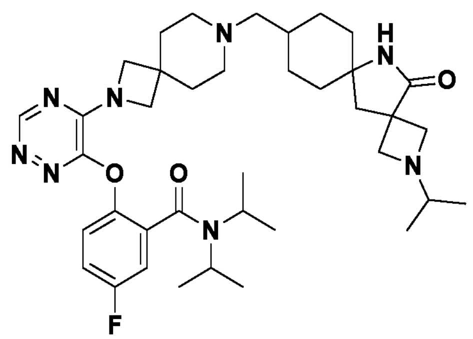

,

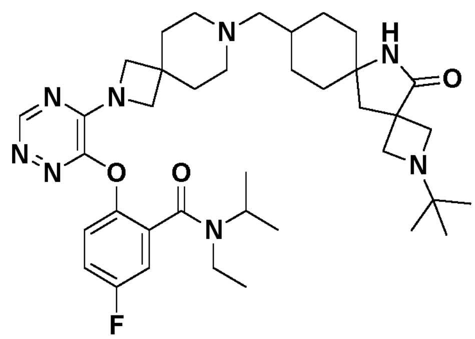

,

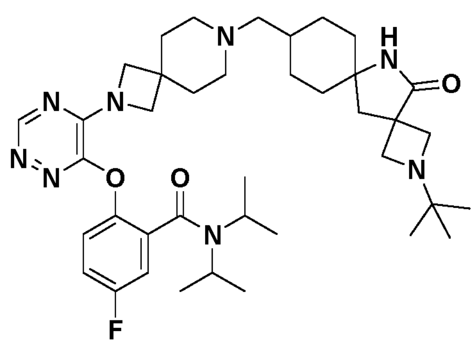

, and

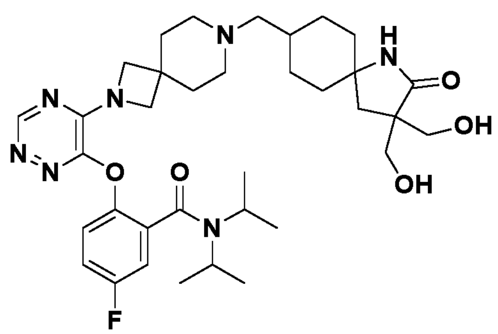

.

In another specific embodiments of the present disclosure, the compound is specifically selected from the group consisting of:

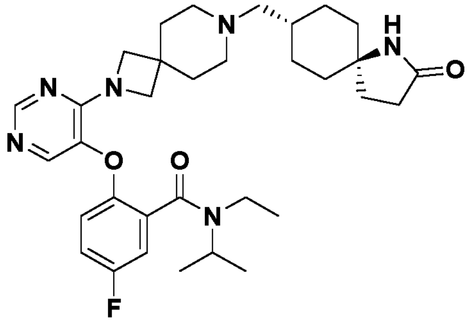

,

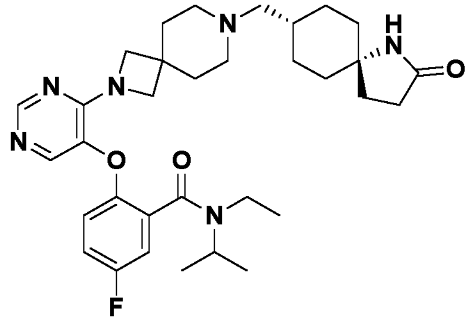

,

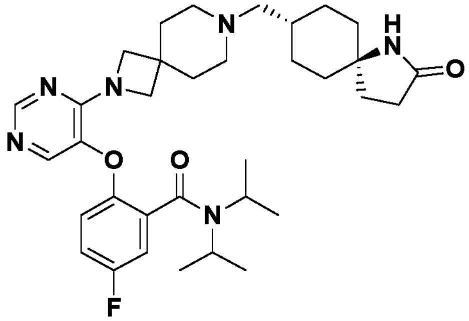

,

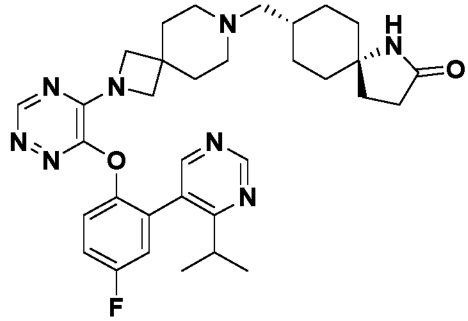

,

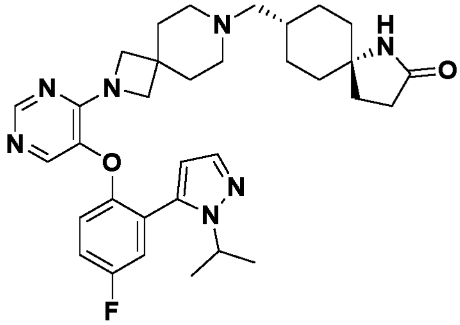

,

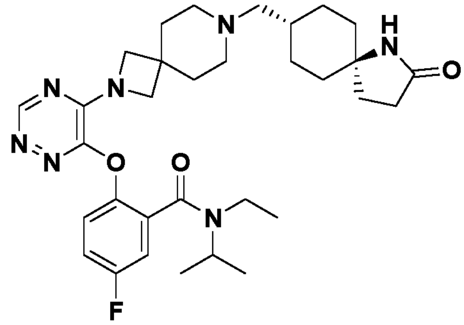

,

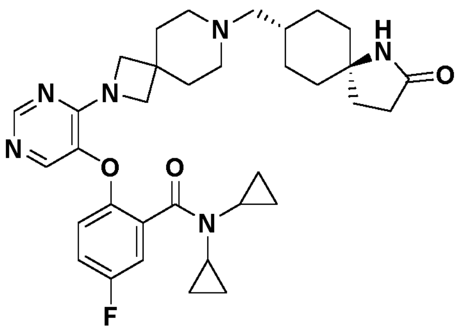

,

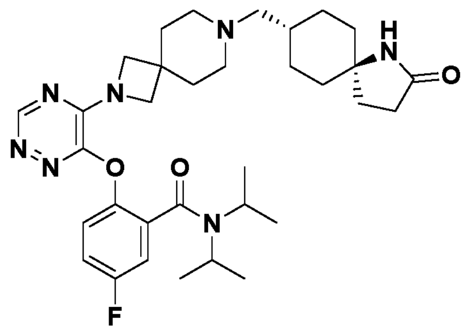

,

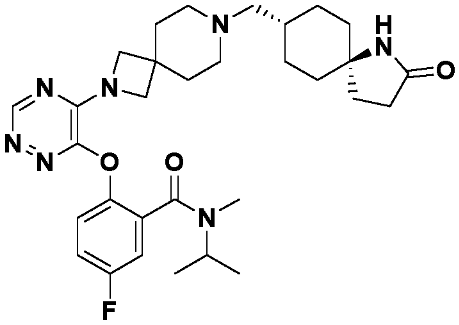
,
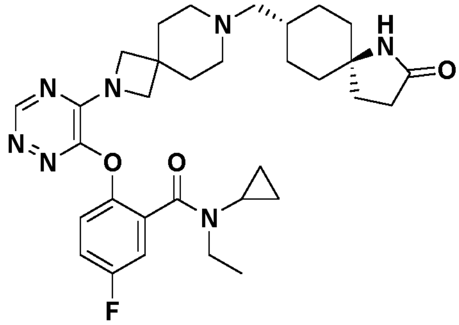
,
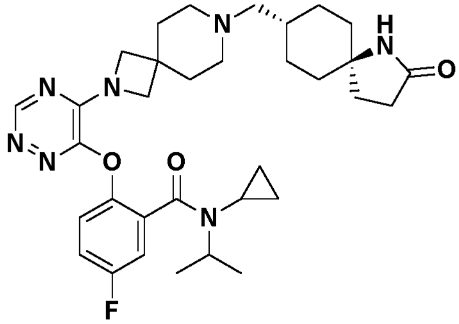
,
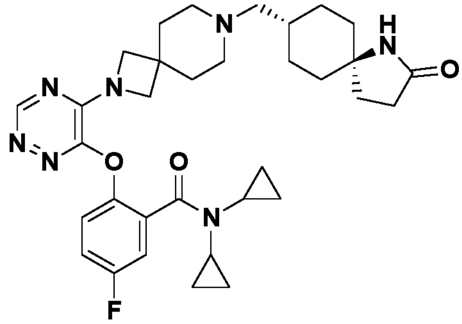
,
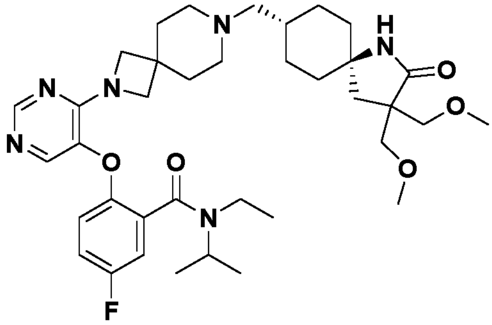
.
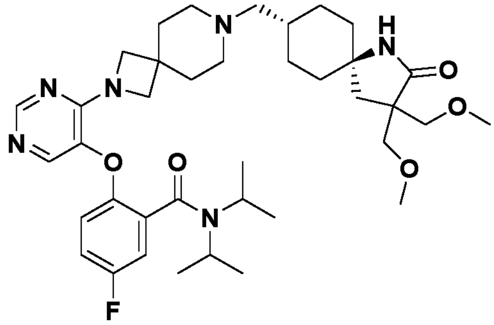

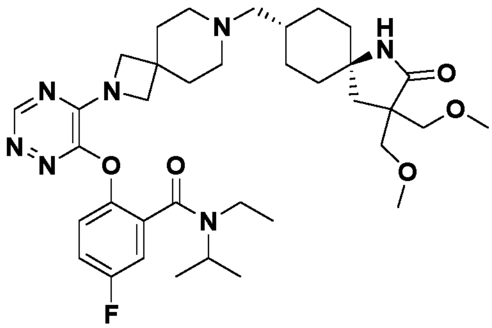
,
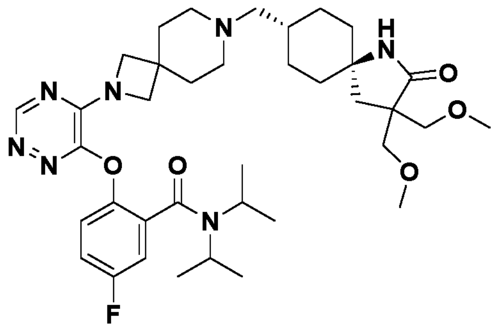
,

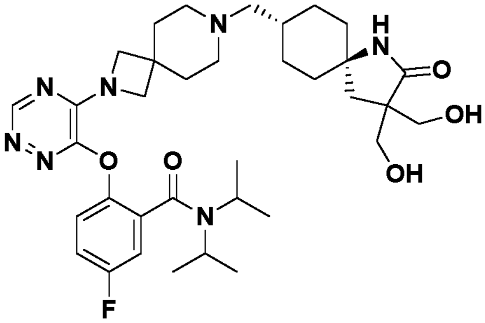
,

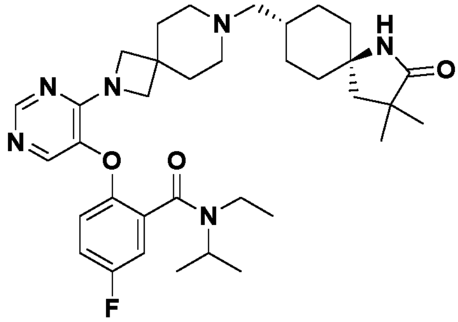
,

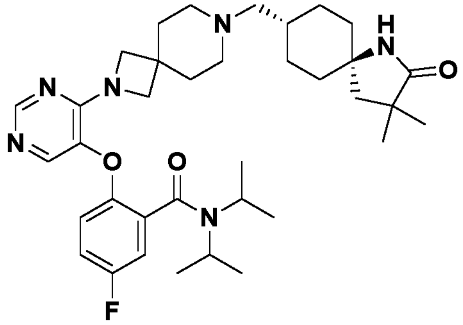
,

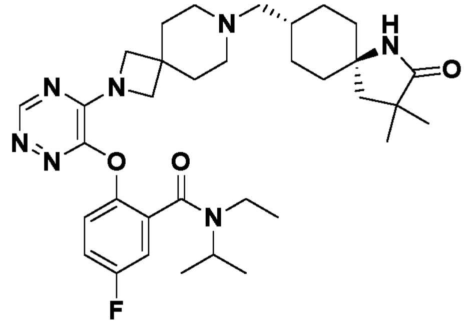
,

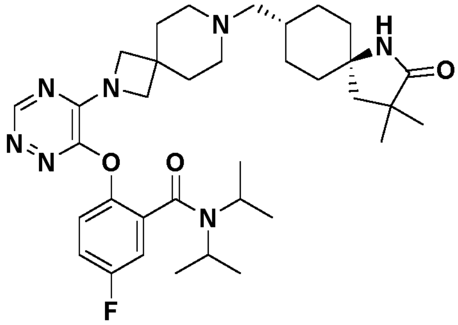
,

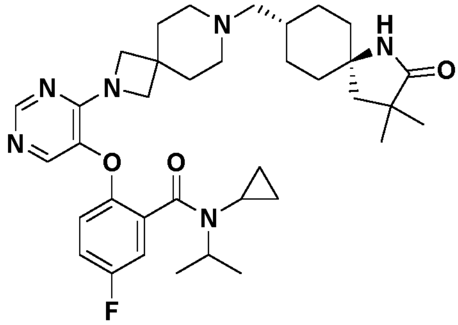
,

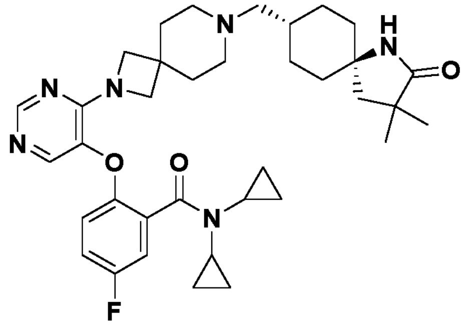
,

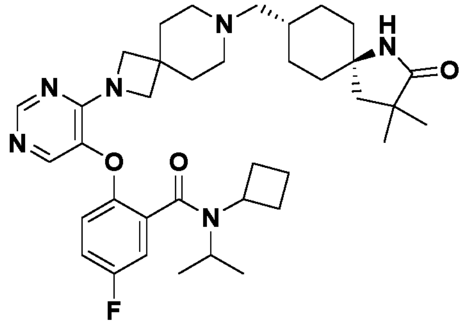
,

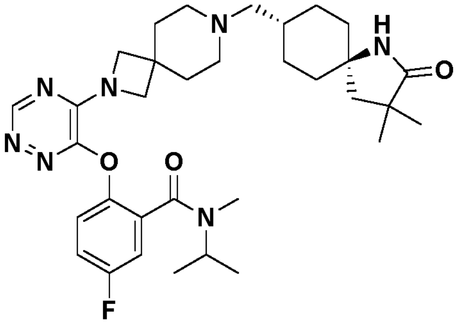

,

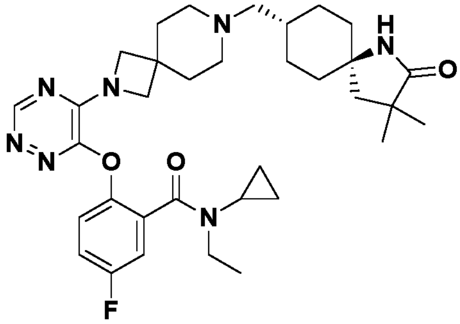

,

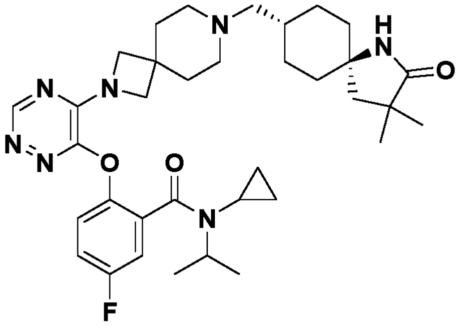

,

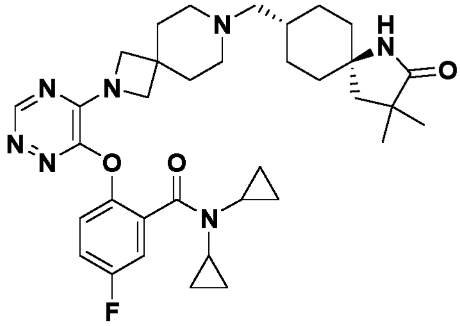

,

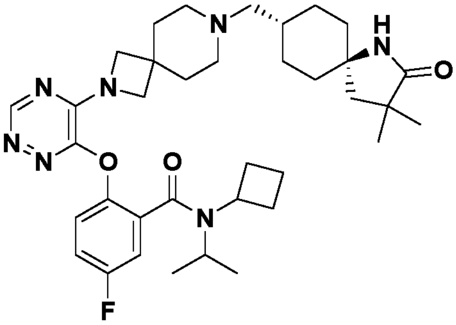

,

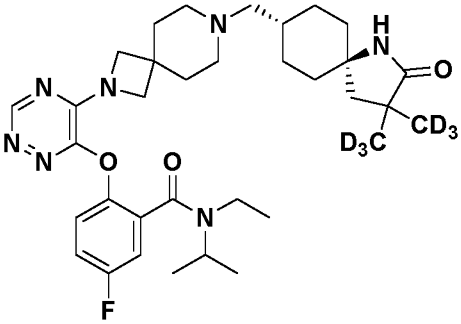

,

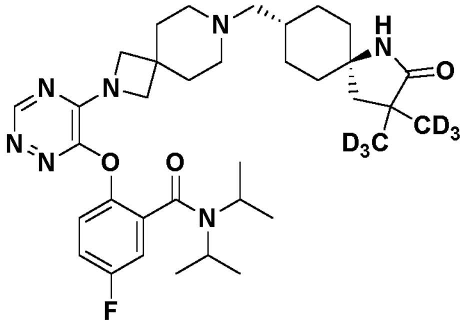

,

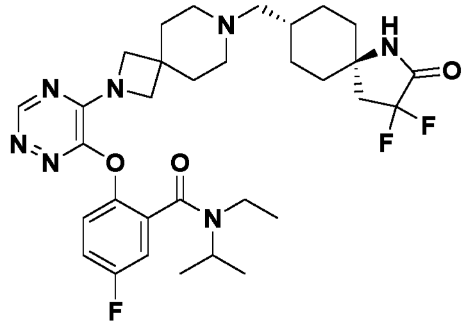

,

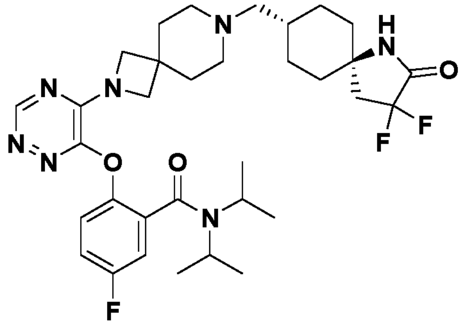

,

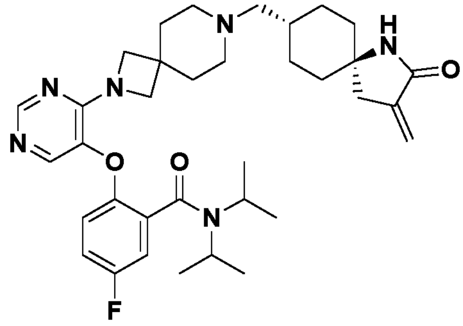

,

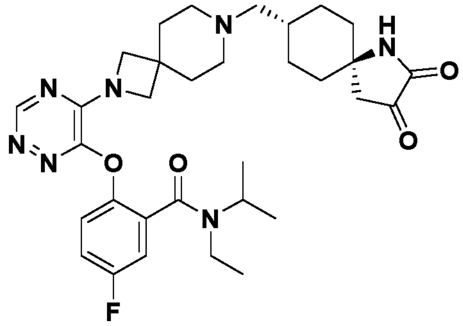

,

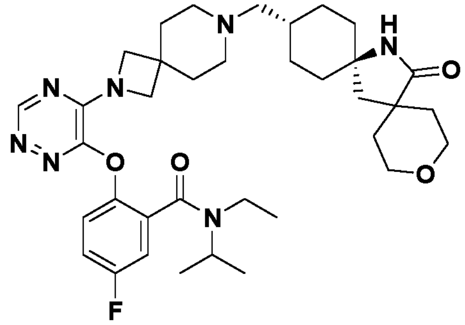

,

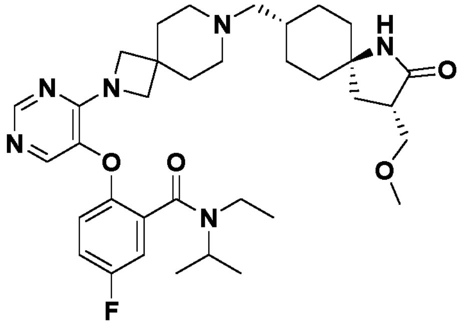

,

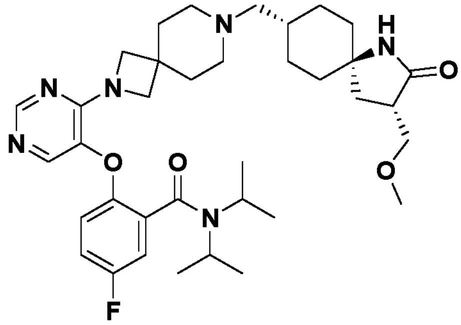

,

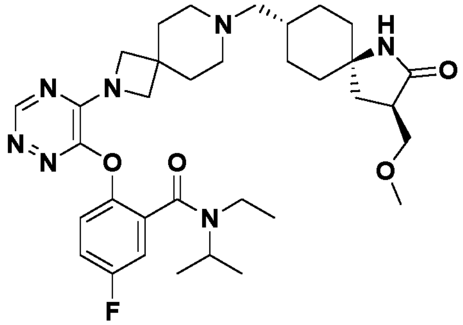

,

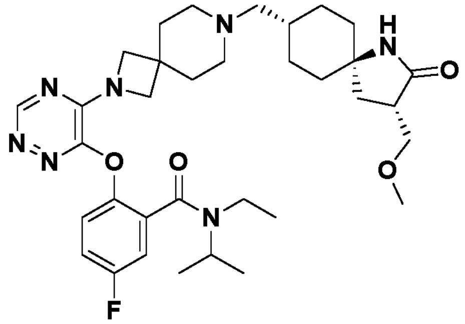

,

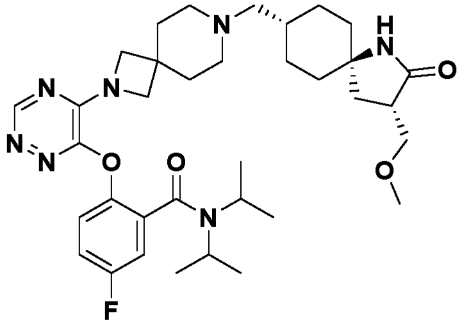

,

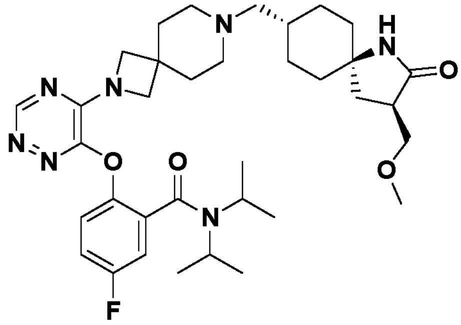

,

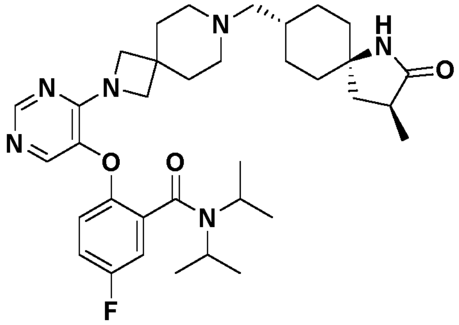

,

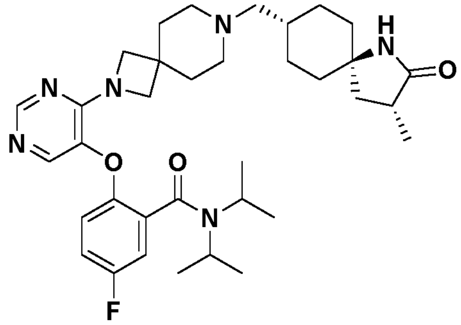

,

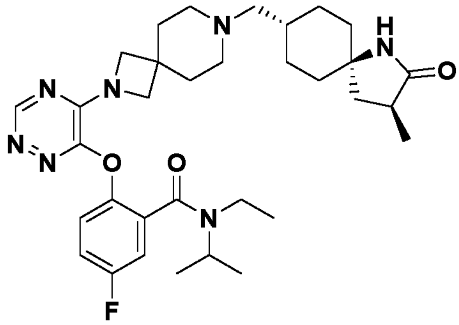

,

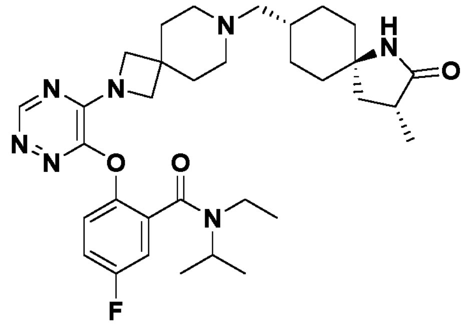

,

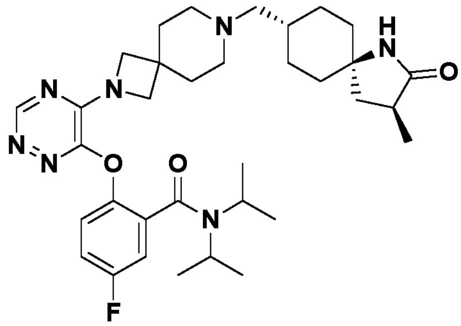

,

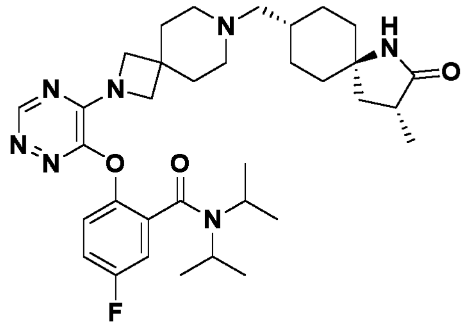

,

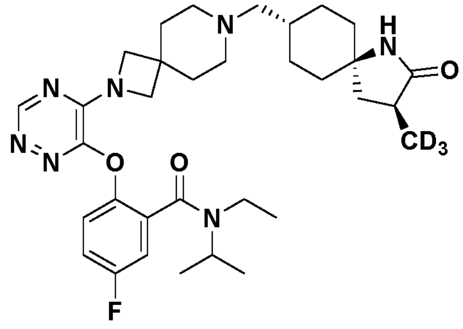

,

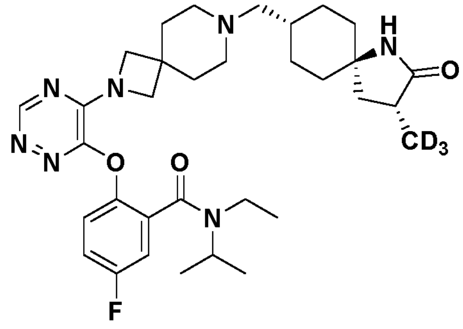

,

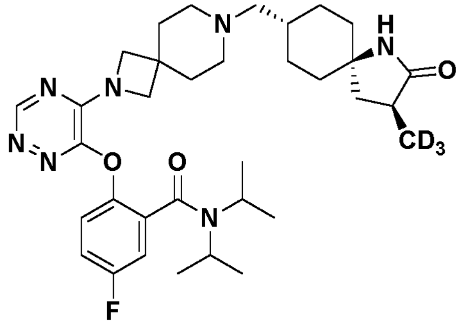

,

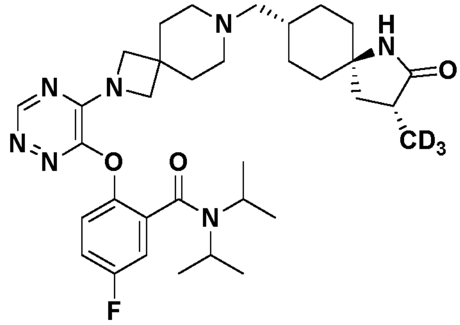

,

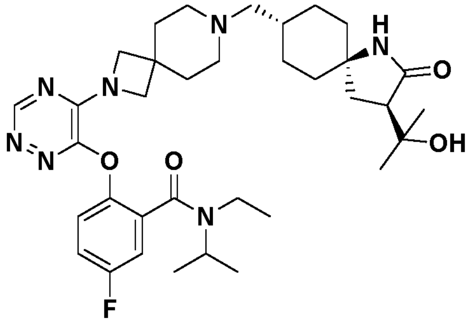

,

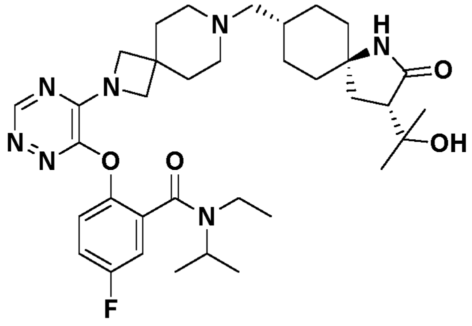

,

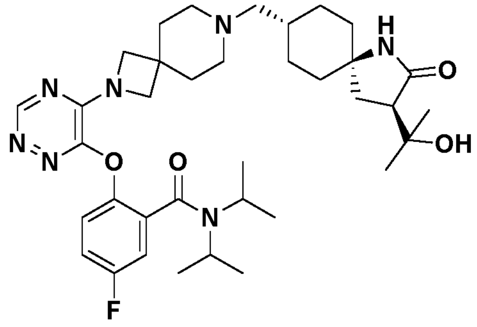

,

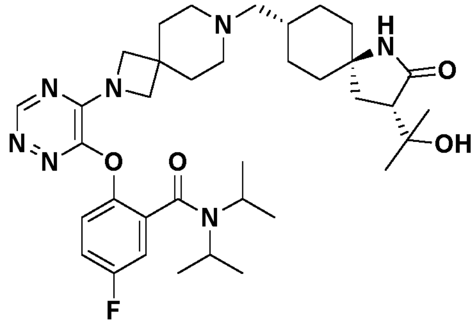

,

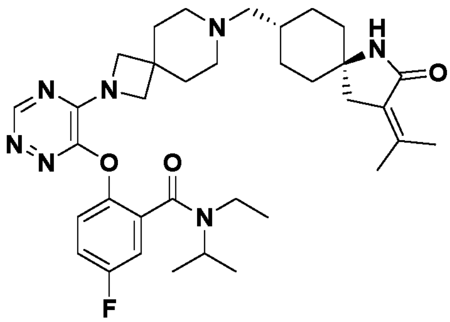

,

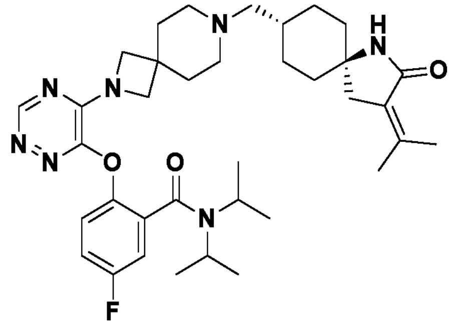

,

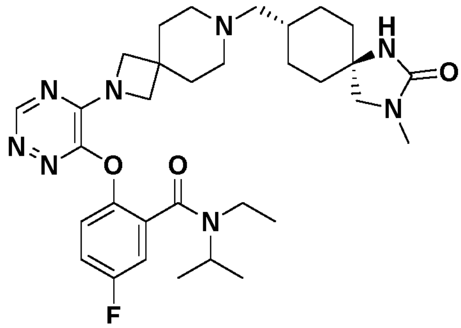

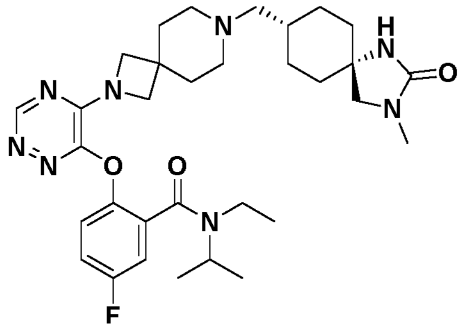

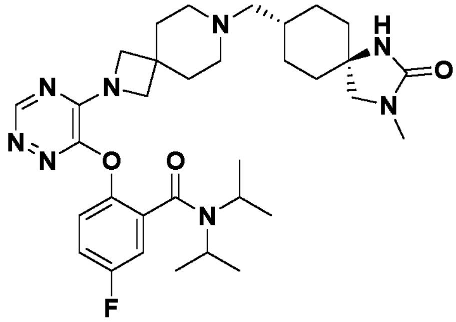

,

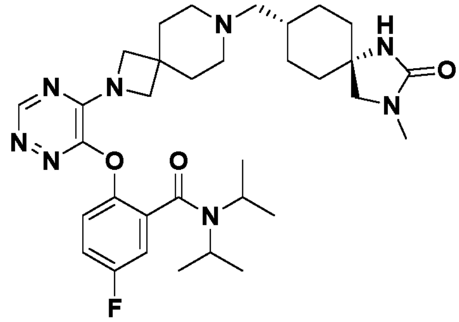

,

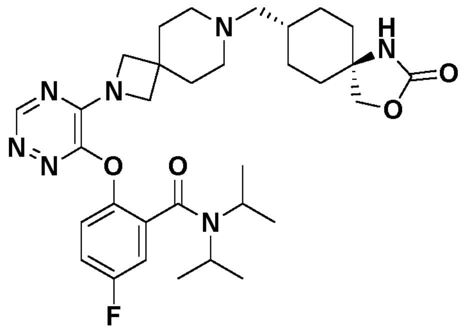

, and

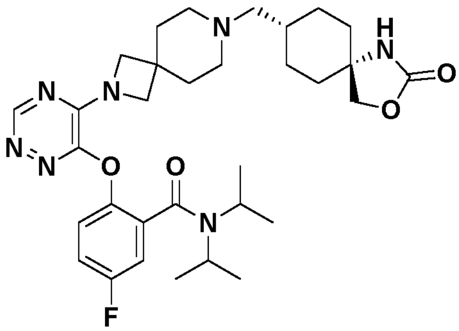

.

The present disclosure further provides use of the above-mentioned compound, or the deuterated compound, stereoisomer thereof or pharmaceutically acceptable salt thereof in the manufacture of a medicament for treating a disease associated with abnormal Menin activity.

The present disclosure further provides use of the above-mentioned compound, or the deuterated compound, stereoisomer thereof or pharmaceutically acceptable salt thereof in the manufacture of a medicament for treating cancer.

The present disclosure further provides a pharmaceutical composition, comprising a preparation prepared from any of the above-mentioned compounds, or the deuterated compound, stereoisomer thereof or pharmaceutically acceptable salt thereof.

The pharmaceutical composition further comprises a pharmaceutically acceptable carrier, excipient or vehicle.

The compound of the present disclosure has excellent inhibitory activity against Menin-MLL protein-protein interaction and cell proliferation, and has excellent performance in terms of safety, bioavailability and animal efficacy.

The diseases related to Menin-MLL interaction or Menin-MI,L fusion protein interaction as defined in the present disclosure include one or more of cancer or malignant tumors, diabetes and other Menin-related diseases. "Cancer" or "malignant tumor" are used as synonymous terms and refer to any of a number of diseases that are characterized by uncontrolled, abnormal proliferation of cells, the ability of affected cells to spread locally or through the bloodstream and lymphatic system to other parts of the body (i.e., metastasize) as well as any of a number of characteristic structural and/or molecular features. "Cancer cell" refers to a cell that undergoes an early, intermediate or advanced stage of multi-step tumor progression. The "cancer" or "malignant tumor" refers to leukemia, lymphoma, sarcoma, lung cancer, esophageal cancer, gastric cancer, liver cancer, brain tumor, myeloma, pancreatic cancer, breast cancer, colon cancer, prostate cancer, bladder cancer, multiple myeloma, brain tumor, or multiple endocrine cancer.

In particular embodiments, the compound of the present disclosure is used to treat leukemia associated with MLL rearrangement, acute lymphocytic leukemia associated with MLL rearrangement, acute lymphoblastic leukemia associated with MLL rearrangement, acute lymphoid leukemia associated with MLL rearrangement, acute myeloid leukemia associated with MLL rearrangement, or acute myeloblastic leukemia associated with MLL rearrangement. As used herein, "MI,L rearrangement" means rearrangement of the *MLL* gene. In certain embodiments, diseases or conditions treatable with the compound of the present disclosure include insulin resistance, prediabetes, diabetes (such as type II diabetes or type I diabetes), risk of diabetes, and hyperglycemia.

Combination therapies: The present disclosure also relates to combination therapies for treating the diseases or disorders described herein. In certain embodiments, combination therapies include administering at least one compound of the present disclosure in combination with one or more other pharmaceutically active agents for treating cancer or other diseases mediated by Menin/MLL. The pharmaceutically active agent can be combined with the compound of the present disclosure in a single dosage form, or can be administered simultaneously or sequentially as separate dosage forms. The compound of the present disclosure can also be used in combination with immunotherapies, including but not limited to cell-based therapies, antibody therapies, and cytokine therapies, for treating the diseases or disorders disclosed herein.

The compounds and derivatives provided in the present disclosure can be named according to the IUPAC (International Union of Pure and Applied Chemistry) or CAS (Chemical Abstracts Service, Columbus, OH) nomenclature system.

Definitions of terms used in the present disclosure: Unless otherwise stated, the initial definitions provided for groups or terms herein apply to the groups or terms throughout the specification; for terms that are not specifically defined herein, the meaning that a person skilled in the art can give them should be given based on the disclosure and context.

"Substitution" means that the hydrogen atom in the molecule is replaced by other different atoms or groups; or the lone pair of electrons of an atom in the molecule is replaced by other atoms or groups, for example, the lone pair of electrons on the S atom can be replaced by an O atom to form

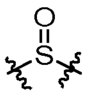
or
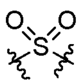
.

"Further optionally substituted", "further substituted" or "optionally substituted " means that "substitution" may but need not occur, and the expression includes situations where substitution occurs or does not occur.

The minimum and maximum carbon atom number of the hydrocarbon group is indicated by a prefix. For example, the prefix $C_{a-b}$ alkyl indicates any alkyl group containing "a" to "b" carbon atoms. Thus, for example, $C_{1-6}$ alkyl refers to an alkyl group containing 1 to 6 carbon atoms.

"Alkyl" refers to a saturated hydrocarbon chain with a specified number of member atoms. Alkyl groups can be linear or branched. Representative branched alkyl groups have one, two or three branches. Alkyl groups may be optionally substituted with one or more substituents as defined herein. Alkyl groups include methyl, ethyl, propyl (n-propyl and isopropyl), butyl (n-butyl, isobutyl and tert-butyl), pentyl (n-pentyl, isopentyl and neopentyl) and hexyl. Alkyl groups may also be part of other groups, such as -O($C_{1-6}$ alkyl).

"Alkylene" refers to a divalent saturated aliphatic hydrocarbon radical having a specified number of member atoms. $C_{a-b}$ alkylene refers to an alkylene group having a to b carbon atoms. Alkylene groups include branched and linear hydrocarbon groups. For example, the term "propylene" can be exemplified by the following structure:

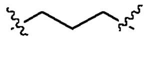
.

Similarly, the term "dimethylbutylene" can be exemplified, for example, by any of the following structures:

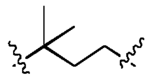 or 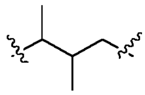 .

The -$C_{0-4}$ alkylene of the present disclosure can be $C_0$ alkylene, $C_1$ alkylene (such as - $CH_2$-), $C_2$ alkylene (such as -$CH_2CH_2$-), $C_3$ alkylene or $C_4$ alkylene; $C_0$ alkylene means that the group here does not exist and is connected in the form of a chemical bond, and A-Co alkylene-B means A-B, that is, the A group and the B group are directly connected by a chemical bond. For example, -$C_0$ alkylene-(3-membered cycloalkyl) refers to cyclopropyl.

"Unsaturation" described herein refers to the presence of carbon-carbon double bond, carbon-carbon triple bond, carbon-oxygen double bond, carbon-sulfur double bond, carbon-nitrogen triple bond, etc. in the groups or molecules.

"Alkenyl" refers to a linear or branched hydrocarbon group having at least one site of vinyl unsaturation (>C=C<). For example, $C_{a-b}$ alkenyl refers to an alkenyl group having a to b carbon atoms and is intended to include, for example, ethenyl, propenyl, isopropenyl, 1,3-butadienyl, and the like.

"Alkynyl" refers to a linear monovalent hydrocarbon group or a branched monovalent hydrocarbon group containing at least one triple bond. The term "alkynyl" is also intended to include those hydrocarbon groups having one triple bond and one double bond. For example, $C_{2-6}$ alkynyl is intended to include ethynyl, propynyl, and the like.

"Carbocyclyl" described herein refers to a saturated or non-aromatic partially saturated cyclic group with a single ring or multiple rings (fused, bridged, or spiro) having multiple carbon atoms and no ring heteroatoms. The term "carbocyclyl" includes cycloalkenyl groups, such as cyclohexenyl. Examples of monocarbocyclyl groups include, for example, cyclopropyl, cyclobutyl, cyclohexyl, cyclopentyl, cyclooctyl, cyclopentenyl and cyclohexenyl. Examples of carbocyclyl groups of fused carbocyclyl systems include dicyclohexyl, dicyclopentyl, dicyclooctyl, etc., and two such dicycloalkyl polycyclic structures are exemplified and named below:

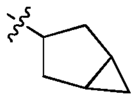

dicyclohexyl and

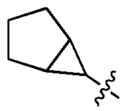

dicyclohexyl. Examples of carbocyclyl groups of bridged carbocyclyl systems include

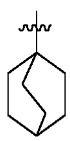 , 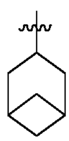 ,

adamantyl, etc. Examples of carbocyclyl groups of spiral carbocyclyl systems include

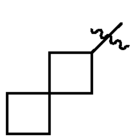 , 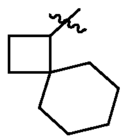 ,

etc. The term "carbocyclyl" also includes the case where an aromatic ring is fused with a non-aromatic ring to form a partially saturated cyclic group, and the attachment point can be located at a non-aromatic carbon atom or an aromatic carbon atom, examples of which include 1,2,3,4-tetrahydronaphthalen-5-yl and 5,6,7,8-tetrahydronaphthalen-5-yl.

The "bridged ring" or "bridged cycloalkyl" described herein refers to a saturated or non-aromatic partially saturated cyclic group formed by bridging multiple rings having multiple carbon atoms and no ring heteroatoms. Examples of this term include, but are not limited to,

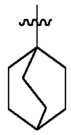, 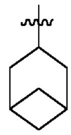,

adamantyl, etc.

The "heterocycloalkyl" described herein refers to a saturated ring or a non-aromatic partially saturated ring with a single ring or multiple rings (fused, bridged, spiro) containing at least one heteroatom; wherein the heteroatom refers to a nitrogen atom, an oxygen atom, a sulfur atom, etc. It usually refers to a monovalent saturated or partially unsaturated monocyclic or polycyclic ring system with multiple ring atoms, which contains 1, 2 and 3 ring heteroatoms selected from the group consisting of N, O and S, and the remaining ring atoms are carbon. Examples of heterocycloalkyl groups of monoheterocycloalkyl systems include oxetanyl, azetidinyl, pyrrolidinyl, 2-oxo-pyrrolidin-3-yl, tetrahydrofuranyl, tetrahydrothienyl, pyrazolidinyl, imidazolidinyl, thiazolidinyl, piperidinyl, tetrahydropyranyl, tetrahydrothiopyranyl, piperazinyl, morpholinyl, thiomorpholinyl, 1,1-dioxo-thiomorpholin-4-yl, azepanyl, diazepanyl, homopiperazinyl, oxaazepanyl, etc. Examples of heterocycloalkyl groups of fused heterocycloalkyl systems include 8-aza-bicyclo[3.2.1]octyl, quinuclidine, 8-oxa -3-aza-bicyclo[3.2.1]octyl, 9-aza-bicyclo[3.3.1]nonyl, etc. Examples of heterocycloalkyl groups of bridged heterocycloalkyl systems include

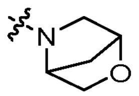, 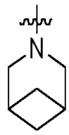, 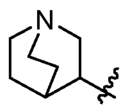,

etc. Examples of heterocycloalkyl groups of spiral heterocycloalkyl systems include

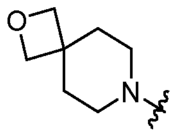, 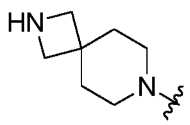,

etc. Examples of partially saturated heterocycloalkyl groups include dihydrofuranyl, imidazolinyl, tetrahydropyridyl, dihydropyranyl, etc. The term "heterocycloalkyl" also includes the case where an aromatic ring containing at least one heteroatom is fused to a non-aromatic ring to form a partially saturated cyclic group, and the attachment site can be located at a non-aromatic carbon atom, an aromatic carbon atom or a heteroatom, and examples include

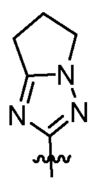, 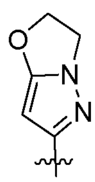,

and

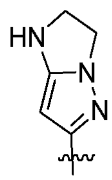.

The "bridged heterocycle" or "bridged heterocycloalkyl" described herein refers to a saturated ring or a non-aromatic partially saturated ring formed by bridging multiple rings containing at least one heteroatom; wherein the heteroatom refers to a nitrogen atom, an oxygen atom, a sulfur atom, etc. Examples of this term include, but are not limited to,

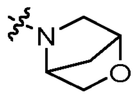, 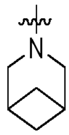,

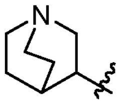,

etc.

The "aromatic ring" described herein refers to an aromatic hydrocarbon group having multiple carbon atoms. Aryl is generally a monocyclic, bicyclic or tricyclic aromatic group having multiple carbon atoms. In addition, the term "aryl" used herein refers to an aromatic substituent that may be a single aromatic ring or multiple aromatic rings fused together. Non-limiting examples include phenyl, naphthyl or tetrahydronaphthyl.

The "aromatic heterocycle" described herein refers to an aromatic unsaturated ring containing at least one heteroatom, wherein the heteroatom refers to a nitrogen atom, an oxygen atom, a sulfur atom, etc; and generally refers to aromatic monocyclic or bicyclic hydrocarbons that contain multiple ring atoms, one or more of which are heteroatoms selected from the group consisting of O, N, and S, preferably one to three heteroatoms. Heterocyclic aryl represents, for example, pyridyl, indolyl, quinoxalinyl, quinolyl, isoquinolinyl, benzothienyl, benzofuranyl, benzothienyl, benzopyranyl, benzothiopyranyl, furyl, pyrrolyl, thiazolyl, oxazolyl, isoxazolyl, triazolyl, tetrazolyl, pyrazolyl, imidazolyl, thienyl, oxadiazolyl, benzimidazolyl, benzothiazolyl, or benzoxazolyl.

The "halogen" described herein refers to fluorine, chlorine, bromine or iodine.

The "halogen-substituted alkyl" described herein refers to an alkyl group in which one or more hydrogen atoms are replaced by halogen; for example, halogen-substituted $C_{1-4}$ alkyl refers to an alkyl group containing 1 to 4 carbon atoms in which hydrogen atoms are replaced by one or more halogen atoms; examples include monofluoromethyl, difluoromethyl, and trifluoromethyl.

The "deuterium-substituted alkyl " described herein refers to an alkyl group in which one or more hydrogen atoms are replaced by deuterium; for example, deuterium-substituted $C_{1-4}$ alkyl refers to an alkyl group containing 1 to 4 carbon atoms in which a hydrogen atom is replaced by one or more deuterium atoms; examples include monodeuteriomethyl, dideuteriomethyl and trideuteriomethyl.

The "-OR", "-NRR" and the like described herein means that the R group is connected to the oxygen atom or the nitrogen atom by a single bond.

The oxygen atom in "-C(O)R", "-S(O)$_2$R " and the like described herein is connected to the carbon atom or the sulfur atom by a double bond, and the R group is connected to the oxygen atom or the sulfur atom by a single bond. For another example, "-S(O)(NH)R" means that the oxygen atom and the nitrogen atom are connected to the sulfur atom by a double bond, and the R group is connected to the sulfur atom by a single bond.

The "oxo" described herein refers to =O, that is, an oxygen atom replaces two hydrogen atoms or lone pairs of electrons through a double bond.

In the description of the groups of the present disclosure,

“- - -“

and

“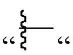“

are used to describe the position of the group substitution. For example,

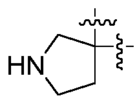

means that the tetrahydropyrrole ring forms a spiro ring with other rings in the structure through the position of

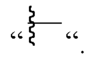

The "deuterated compound" described herein refers to a molecule or group in which one or more hydrogen atoms are replaced by deuterium atoms, wherein the proportion of deuterium atoms is greater than the abundance of deuterium in nature.

The "stereoisomer" described herein refers to a compound composed of the same atoms, bonded by the same chemical bonds, but with different three-dimensional structures. The stereoisomers of the present disclosure cover each single stereoisomer and its compounds, including but not limited to enantiomers and diastereomers. The compounds of the present disclosure or their pharmaceutically acceptable salts may contain one or more chiral carbon atoms, and thus may produce enantiomers, diastereomers and other stereoisomeric forms. Each chiral carbon atom can be defined as (R)- or (S)- based on stereochemistry. The present disclosure is intended to include all possible isomers, as well as their racemates and optically pure forms. The preparation of the compounds of the present disclosure can select racemates, diastereomers or enantiomers as raw materials or intermediates. Optically active isomers can be prepared using chiral synthons or chiral reagents, or separated using conventional techniques, such as crystallization and chiral chromatography.

When the compounds of the present disclosure contain olefinic double bonds, unless otherwise specified, the compounds of the present disclosure include cis-trans isomers of E-form and Z-form. All tautomeric forms of the compounds of the present disclosure are also included within the scope of the present disclosure.

The term "pharmaceutically acceptable" means that a carrier, vehicle, diluent, excipient, and/or formed salt is generally chemically or physically compatible with the other ingredients that constitute a pharmaceutical dosage form and physiologically compatible with the receptor.

The terms "salts" and "pharmaceutically acceptable salts" refer to acidic and/or basic salts of the above compounds or their stereoisomers, formed with inorganic and/or organic acids and bases, and also include zwitterionic salts (inner salts) as well as quaternary ammonium salts such as alkylammonium salts. These salts can be obtained directly during the final separation and purification of the compounds. They can also be obtained by mixing the above compounds, or their stereoisomers, with a certain amount of acid or base appropriately (e.g., equivalent amounts). These salts may be precipitated in the solution and collected by filtration, or recovered after evaporation of the solvent, or obtained by freeze-drying after reaction in an aqueous medium.

In certain embodiments, one or more compounds of the present disclosure may be used in combination with each other. It is also possible to select the compounds of the present disclosure and any other active agent for use in combination to prepare a drug or pharmaceutical composition for regulating cell function or treating a disease. If a group of compounds is used, these compounds may be administered to a subject simultaneously, separately or sequentially.

Obviously, according to the above contents of the present disclosure, in accordance with common technical knowledge and customary means in the art, other various forms of modification, replacement or change may be made without departing from the above basic technical ideas of the present disclosure.

## BRIEF DESCRIPTION OF DRAWINGS

FIG. 1 shows single-crystal X-ray diffraction results of intermediate **A1-a**.

FIG. 2 shows single-crystal X-ray diffraction results of intermediate **A1-b**.

FIG. 3 shows single-crystal X-ray diffraction results of intermediate **7-5a.**

FIG. 4 shows single-crystal X-ray diffraction results of intermediate **7-5a2.**

FIG. 5 shows single-crystal X-ray diffraction results of Example **19.**

## DETAILED DESCRIPTION

The above contents of the present disclosure are further described in detail below through specific embodiments in the form of examples. However, this should not be understood as the scope of the above subject matter of the present disclosure being limited to the following examples. All technologies realized based on the above contents of the present disclosure belong to the scope of the present disclosure.

The known starting materials of the present disclosure can be synthesized by methods known in the art, or can be purchased from companies such as Energy Chemical, Chengdu Chron Chemicals, Accela ChemBio, and J&K Scientific.

The reagents described in the examples are abbreviated as follows: UHP: urea peroxide; DBU: 1,8-diazabicycloundec-7-ene; TCCA: trichloroisocyanuric acid; KOAc: potassium acetate; $Na_2HPO_4$: disodium hydrogen phosphate; TFAA: trifluoroacetic anhydride; DIPEA: N,N-diisopropylethylamine; n-BuLi: n-butyllithium; $NH_2OH$•HCl: hydroxylamine hydrochloride; $NiCl_2$•$6H_2O$: nickel chloride hexahydrate; $NaBH_4$: sodium borohydride; $NaBH_3CN$: sodium cyanoborohydride; NFSI: N-fluorobisbenzenesulfonamide; TEA: triethylamine; $PySO_3$: pyridine sulfur trioxide; HATU: 2-(7-azobenzotriazole)-N,N,N',N'-tetramethyluronium hexafluorophosphate; DMF: N,N-dimethylformamide; DCM: dichloromethane; DCE: 1,2-dichloroethane; TFA: trifluoroacetic acid; MeCN: acetonitrile; EtOH: ethanol; MeOH: methanol; NMP: N-methylpyrrolidone.

Unless otherwise specified in the examples, the reaction is carried out in a nitrogen atmosphere. Unless otherwise specified in the examples, the solution refers to an aqueous solution. Unless otherwise specified in the examples, the reaction temperature is room temperature. Room temperature is the most suitable reaction temperature, which is 20°C-30°C. Unless otherwise specified in the examples, M is mole per liter.

The structures of the compounds were determined by nuclear magnetic resonance (NMR) and mass spectrometry (MS). NMR shifts (δ) are given in units of 10-6 (ppm). NMR measurements were performed using (Bruker Avance III 400MHz and Bruker Avance NEO 600 MHz) NMR spectrometers, and the solvents used were deuterated dimethyl sulfoxide (DMSO-$d_6$), deuterated chloroform ($CDCl_3$) and deuterated methanol ($CD_3OD$), and the internal standard was tetramethylsilane (TMS). LC-MS measurements were performed using Shimadzu's liquid chromatography-mass spectrometry (Shimadzu LC-MS 2020 (ESI)). HPLC measurements were performed using Shimadzu's high pressure liquid chromatograph (Shimadzu LC-20A). MPLC (medium pressure liquid chromatography) was performed using a Gilson GX-281 reverse phase preparative chromatograph. The silica gel plate used for thin layer chromatography was Yantai Huanghai HSGF254 or Qingdao GF254 silica gel plate, and the specifications of thin layer chromatography used for separating and purifying products were 0.4 mm-0.5 mm. For column chromatography, Yantai Huanghai silica gel 200-300 mesh silica gel was generally used as a carrier. The instrument used for supercritical fluid chromatography (SFC) analysis and preparation was SHIMADZU SFC-30A.

### Synthesis of intermediate 1-3

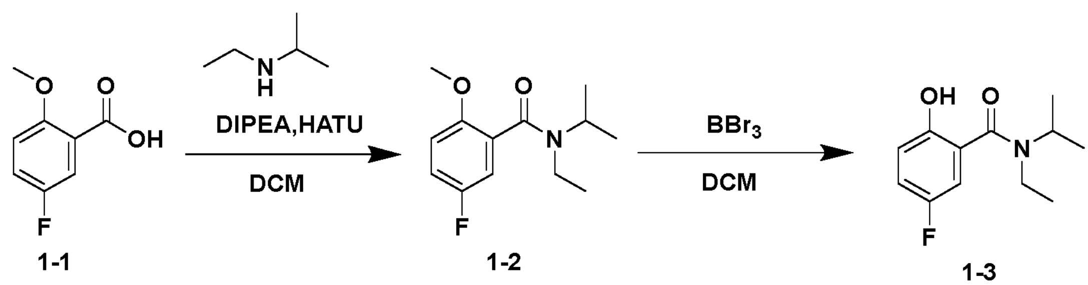


### Step 1: Synthesis of 1-2

To a solution of 5-fluoro-2-methoxybenzoic acid (45 g, 264.49 mmol) and ethylisopropylamine (23.21 g, 317.39 mmol) in DCM (880 mL), HATU (120.61 g, 317.39 mmol) and DIPEA (102.55 g, 793.48 mmol, 138.21 mL) were added at 0°C. The reaction mixture was slowly warmed to room temperature and stirred for 17 h. After the reaction was completed, the reaction mixture was diluted with water, extracted three times with EA, and the organic phases were combined. The crude product obtained by concentration was separated and purified by silica gel column to obtain intermediate **1-2** (60 g, 250.75 mmol, yield 94.8%). MS m/z = 240 $[M+H]^+$.

**Step 2: Synthesis of 1-3**

To a solution of **1-2** (63 g, 263.28 mmol) in DCM (200.00 mL), $BBr_3$ (131.64 g, 526.57 mmol) was added dropwise at -70°C. After the addition was completed, the reaction mixture was slowly warmed to room temperature and stirred for 17 h. After the reaction was completed, the mixture was cooled to -70°C, quenched with MeOH, and extracted with EA. The organic phases were combined, washed with water and saturated brine, dried over anhydrous sodium sulfate and concentrated to obtain a crude product **1-3** (49 g, 217.53 mmol, yield 82.6%), which was used directly in the next step without further purification. MS m/z = 226 $[M+H]^+$.

**Synthesis of intermediate 1-5**

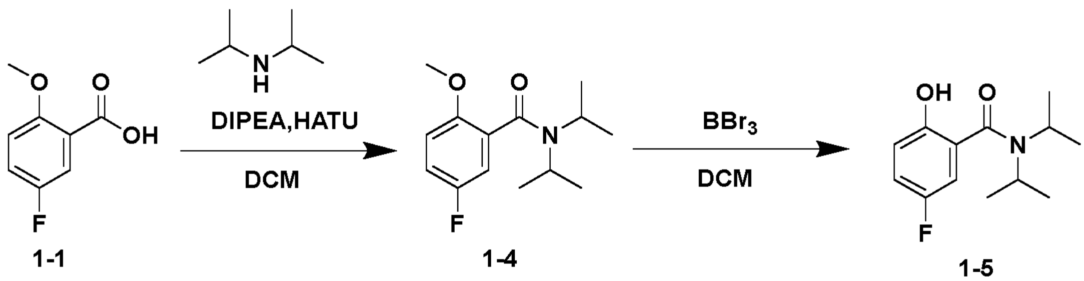


Referring to the synthetic route of intermediate **1-3,** diisopropylamine was used instead of ethylisopropylamine to obtain intermediate **1-5.** MS m/z = 240 $[M+H]^+$.

**Synthesis of intermediate 1-7**

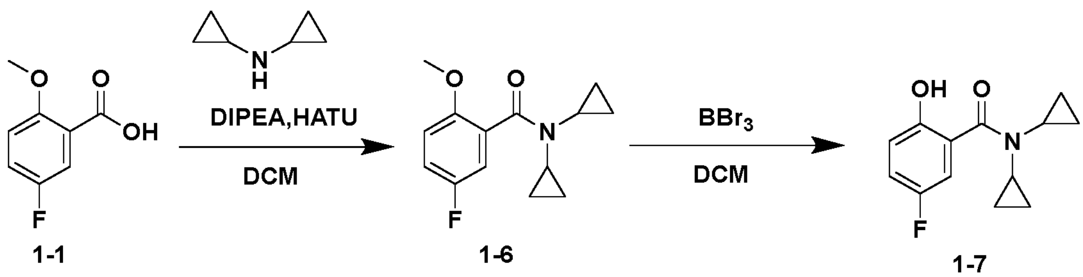


Referring to the synthetic route of intermediate **1-3,** dicyclopropylamine was used instead of ethylisopropylamine to obtain intermediate **1-7.** MS m/z = 236 $[M+H]^+$.

**Synthesis of intermediate 1-11**

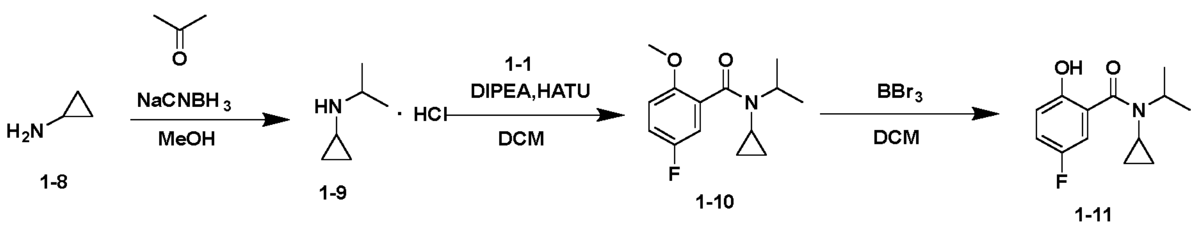


**Step 1: Synthesis of 1-9**

At room temperature, **1-8** (3 g, 52.54 mmol) was dissolved in anhydrous methanol (40 mL), then added with 4

N HCl/MeOH (46 mL), acetone (7.63 g, 131.37 mmol) and $NaBH_3CN$ (4.97 g, 78.81 mmol), and stirred at room temperature for 2 h. After the reaction was completed, the reaction mixture was concentrated directly. The concentrated crude product was dissolved in 10% NaOH aqueous solution and extracted three times with MTBE. The organic phases were combined, acidified with HCl/MeOH, and concentrated to obtain a crude product (6.5 g, 47.92 mmol, yield 91.2%), which was directly used in the next step. MS m/z = 100 $[M+H]^+$.

**Step 2-Step 3: Synthesis of 1-11**

Referring to the synthetic route of intermediate **1-3, 1-9** was used instead of ethylisopropylamine to obtain target intermediate **1-11.** MS m/z = 238 $[M+H]^+$.

**Synthesis of Intermediate 1-15**

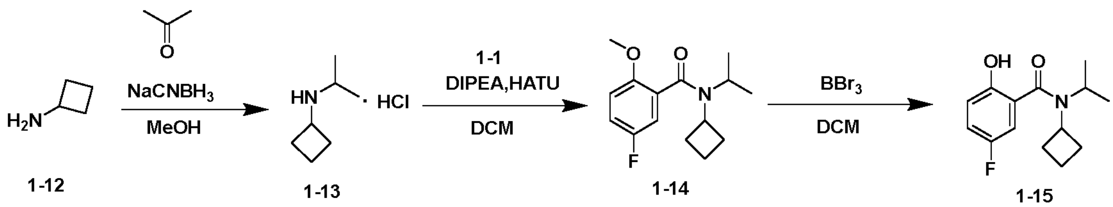


1-12 1-13 1-14 1-15

**Step 1: Synthesis of 1-13**

At room temperature, **1-12** (3.56 g, 50.06 mmol) was dissolved in anhydrous methanol (40 mL), then added with acetone (7.27 g, 125.14 mmol) and $NaBH_3CN$ (4.73 g, 75.08 mmol), and stirred at room temperature for 2 h. After the reaction was completed, the mixture was concentrated directly. The concentrated crude product was dissolved in 10% NaOH aqueous solution and extracted three times with MTBE. The organic phases were combined, acidified with HCl/MeOH, and concentrated to obtain a crude product (6.5 g, 43.43 mmol, yield 86.7%), which was directly used in the next step. MS m/z = 114 $[M+H]^+$.

**Step 2-Step 3: Synthesis of 1-15**

Referring to the synthetic route of intermediate **1-3, 1-13** was used instead of ethylisopropylamine to obtain target intermediate **1-15.** MS m/z = 252 $[M+H]^+$.

**Synthesis of Intermediate 1-17**

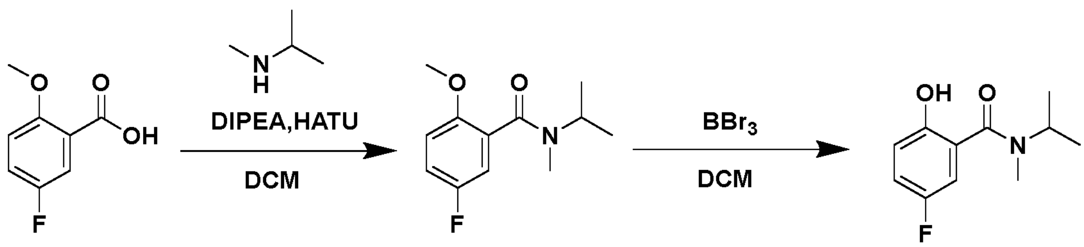


1-1 1-16 1-17

Referring to the synthetic route of intermediate **1-3,** isopropylmethylamine was used instead of ethylisopropylamine to obtain intermediate **1-17.** MS m/z = 212 $[M+H]^+$.

**Synthesis of Intermediate 1-22**

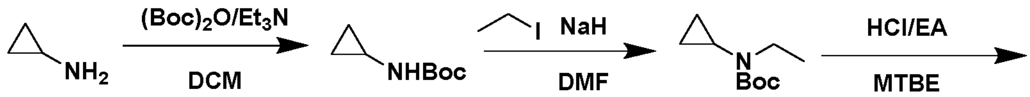


1-8 1-18 1-19

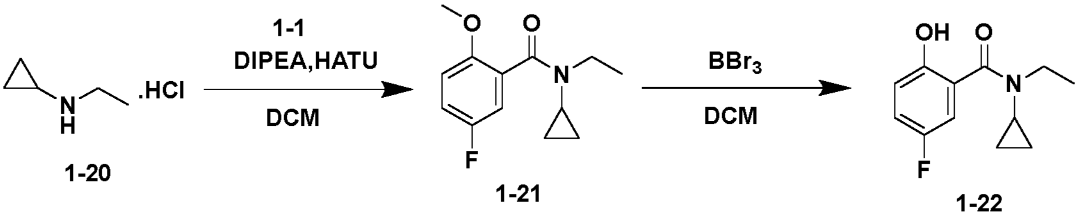


1-20 1-21 1-22

**Step 1: Synthesis of 1-18**

Under ice bath, **1-8** (3.0 g, 52.54 mmol) was dissolved in DCM (50 mL), then added with $(Boc)_2O$ (12.04 g, 55.17 mmol) and triethylamine (6.37 g, 63.05 mmol), slowly warmed to room temperature and stirred overnight. After the reaction was completed, the mixture was diluted with water and extracted three times with DCM. The organic phases were combined, washed with saturated brine, dried over anhydrous sodium sulfate and concentrated to obtain a crude **1-18** was directly used in the next step without further purification. MS m/z = 158 $[M+H]^+$.

**Step 2: Synthesis of 1-19**

At 0°C, **1-18** (8.33 g, 52.99 mmol) was dissolved in anhydrous DMF, and NaH (1.34 g, 55.64 mmol) was added under nitrogen protection. The reaction was carried out at 0°C for 30 min. Ethyl iodide (9.9 g, 58.29 mmol) was slowly added dropwise. After the addition was completed, the mixture was warmed to room temperature and stirred. After the reaction monitored by LCMS was completed, the mixture was diluted with water and extracted three times with EA. The organic phases were combined, washed with saturated brine, dried over anhydrous sodium sulfate and concentrated to obtain a crude product, which was purified by silica gel column to obtain **1-19** (5.6 g, 30.23 mmol, yield 57%). MS m/z = 186 $[M+H]^+$.

**Step 3: Synthesis of 1-20**

**1-19** (5.6 g, 30.23 mmol) was dissolved in MTBE, slowly added with HCl/EA, stirred at room temperature until solid no longer precipitated, and subjected to suction filtration. The filter cake was washed with a small amount of EA to obtain intermediate **1-20** (2.74 g, 22.53 mmol, yield 74.52%). MS m/z = 86 $[M+H]^+$.

**Step 4-Step 5: Synthesis of 1-22**

Referring to the synthetic route of intermediate **1-3, 1-20** was used instead of ethylisopropylamine to obtain target intermediate **1-22.** MS m/z = 224 $[M+H]^+$.

**Synthesis of Intermediate 1-25**

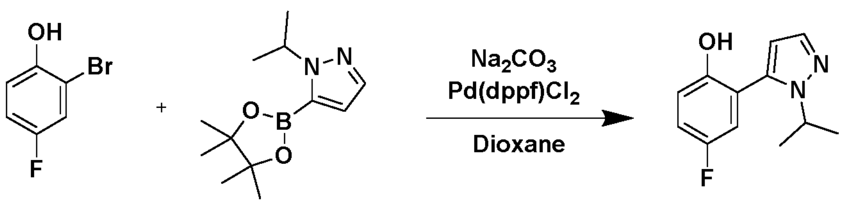


1-23 1-24 1-25

**1-23** (680 mg, 3.56 mmol) was dissolved in a mixed solvent of dioxane (15 mL) and water (3 mL). **1-24** (1.01

g, 4.27 mmol), $Na_2CO_3$ (754.77 mg, 7.12 mmol), and Pd(dppf)$Cl_2$ (130.13 mg, 0.178 mmol) were added in sequence, and stirred at 90°C overnight. After the reaction monitored by LCMS was completed, the mixture was diluted with water and extracted three times with EA. The organic phases were combined, washed with saturated brine, dried over anhydrous sodium sulfate and concentrated to obtain a crude product, which was purified by silica gel column to obtain **1-25** (700 mg, 3.18 mmol, yield 89%). MS m/z = 221 [M+H]$^+$.

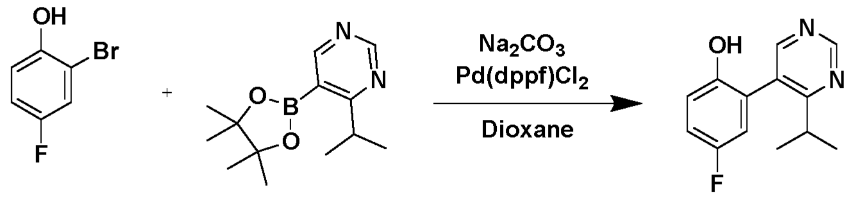


1-23 1-26 1-27

Referring to the synthesis method of **1-25, 1-26** was used instead of **1-24** to obtain **1-27** (232 mg, 1.0 mmol, yield 80%). MS m/z = 233 [M+H]$^+$.

**Synthesis of Intermediates M1-M8**

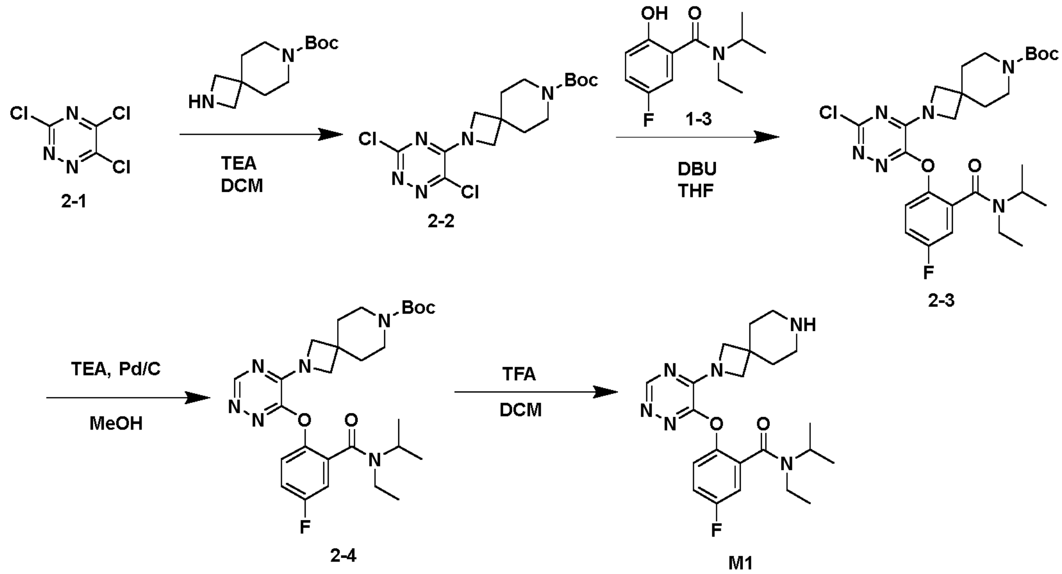


2-1 2-2 1-3 2-3 2-4 M1

**Step 1: Synthesis of 2-2**

At 0°C, **2-1** (4.9 g, 26.57 mmol) and TEA (5.38 g, 53.14 mmol) were dissolved in DCM, and a solution of tert-butyl 2,7-diazaspiro[3.5]nonane-7-carboxylate (4.81 g, 21.26 mmol) in DCM was slowly added dropwise. The mixture was stirred at this temperature for 2 h. After the reaction was completed, the mixture was diluted with water and extracted three times with DCM. The organic phases were combined, washed with saturated brine, dried over anhydrous sodium sulfate and concentrated to obtain a crude product, which was purified by silica gel column (PE/EA= 5:1-1:1, v/v) to obtain **2-2** (6.5 g, 17.37 mmol, yield 65.37%). MS m/z = 374 [M+H]$^+$.

**Step 2: Synthesis of 2-3**

At 0°C, **2-2** (1.0 g, 2.67 mmol) and DBU (488.13 mg, 3.21 mmol) were dissolved in THF, then added with **1-3** (601 mg, 2.67 mmol), warmed to room temperature and stirred overnight. After the reaction was completed, the mixture was diluted with water and extracted three times with DCM. The organic phases were combined, washed with saturated brine, dried over anhydrous sodium sulfate and concentrated to obtain a crude product, which was purified by silica gel column (PE/EA= 10:1-5:1, v/v) to obtain **2-3** (1.04 g, 1.84 mmol, yield 68.9%). MS m/z = 563 [M+H]$^+$.

**Step 3: Synthesis of 2-4**

At room temperature, **2-3** (1.04 g, 1.84 mmol) was dissolved in anhydrous methanol. Pd/C (250 mg, 0.6 mmol) and triethylamine (558 mg, 5.52 mmol) were added. The reaction was carried out at room temperature after replacing with hydrogen. After the reaction was completed, the mixture was filtered. The filtrate was concentrated and purified by silica gel column (PE/EA = 5:1-1:1, v/v) to obtain **2-4** (625 mg, 1.18 mmol, yield 64.13%). MS m/z = 529 $[M+H]^+$.

**Step 4: Synthesis of M1**

At 0°C, **2-4** (40 g, 75.67 mmol) was dissolved in DCM (70 mL), and TFA (30 mL) was slowly added dropwise. The mixture was warmed to room temperature and stirred for 0.5 h. After the reaction was completed, the mixture was concentrated, adjusted to a weak alkaline state with a $NaHCO_3$ aqueous solution, extracted with DCM and concentrated to obtain a crude product M1, which was directly used in the next reaction. MS m/z = 429 $[M+H]^+$.

**Synthesis of Intermediate M2**

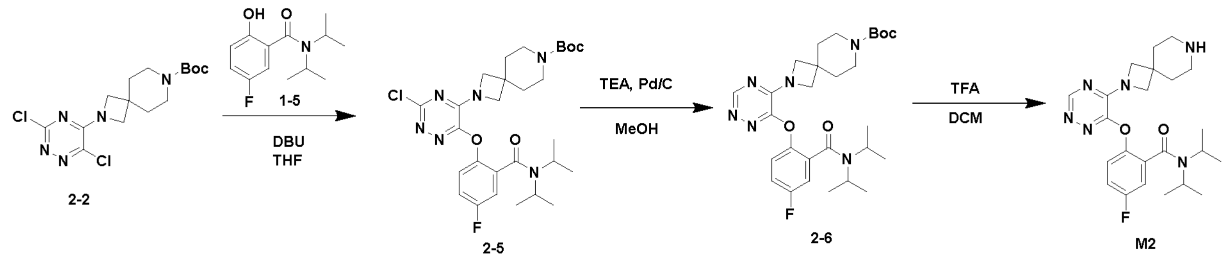


**Step 1: Synthesis of 2-5**

At 0°C, M1 intermediate **2-2** (1.0 g, 2.67 mmol) and DBU (488.13 mg, 3.21 mmol) were dissolved in THF. After adding **1-5** (601 mg, 2.67 mmol), the reaction mixture was warmed to room temperature and stirred overnight. After the reaction was completed, the mixture was diluted with water and extracted three times with DCM. The organic phases were combined, washed with saturated brine, dried over anhydrous sodium sulfate and concentrated to obtain a crude product, which was purified by silica gel column (PE/EA= 10:1-5:1, v/v) to obtain **2-5** (1.0 g, 1.73 mmol, yield 64.9%). MS m/z = 577 $[M+H]^+$.

**Step 2: Synthesis of 2-6**

At room temperature, **2-5** (1.0 g, 1.73 mmol) was dissolved in anhydrous methanol. Pd/C (250 mg, 0.6 mmol) and triethylamine (558 mg, 5.52 mmol) were added. The reaction was carried out at room temperature after replacing with hydrogen. h. After the reaction was completed, the reaction mixture was filtered. The filtrate was concentrated and purified by silica gel column (PE/EA= 5:1-1:1, v/v) to obtain **2-6** (720 mg, 1.33 mmol, yield 76.79%). MS m/z = 543 $[M+H]^+$.

**Step 3: Synthesis of M2**

At 0°C, **2-6** (4.0 g, 7.38 mmol) was dissolved in DCM (7 mL), and TFA (3 mL) was slowly added dropwise. The mixture was warmed to room temperature and stirred for 0.5 h. After the reaction was completed, the mixture was concentrated, adjusted to a weak alkaline state with a $NaHCO_3$ aqueous solution, extracted with DCM and concentrated to obtain a crude product **M2,** which was directly used in the next reaction. MS m/z = 443 $[M+H]^+$.

Referring to the synthesis method of intermediate **M1**, **1-7, 1-11, 1-15, 1-17, 1-22,** and **1-27** were used instead of **1-3,** respectively, to obtain the corresponding intermediates **M3-M8.**

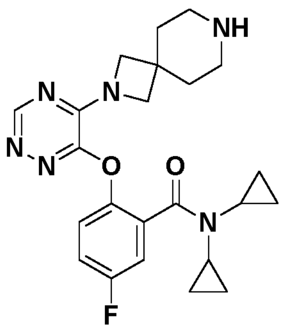
M3

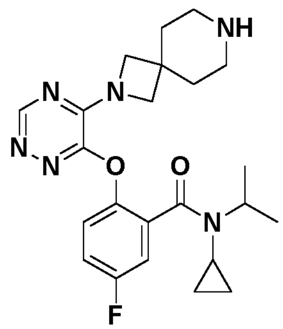
M4

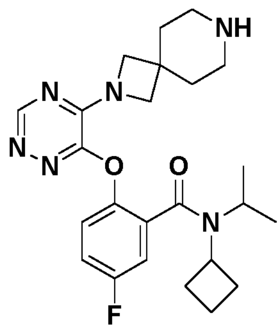
M5

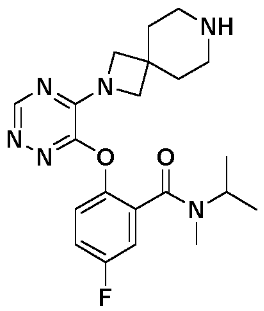
M6

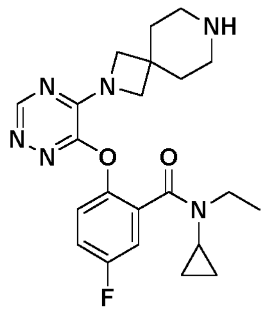
M7

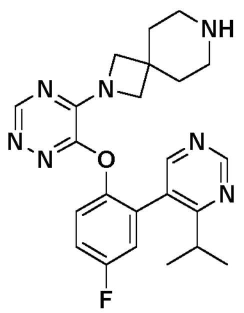
M8

**Synthesis of Intermediates N1-N6**

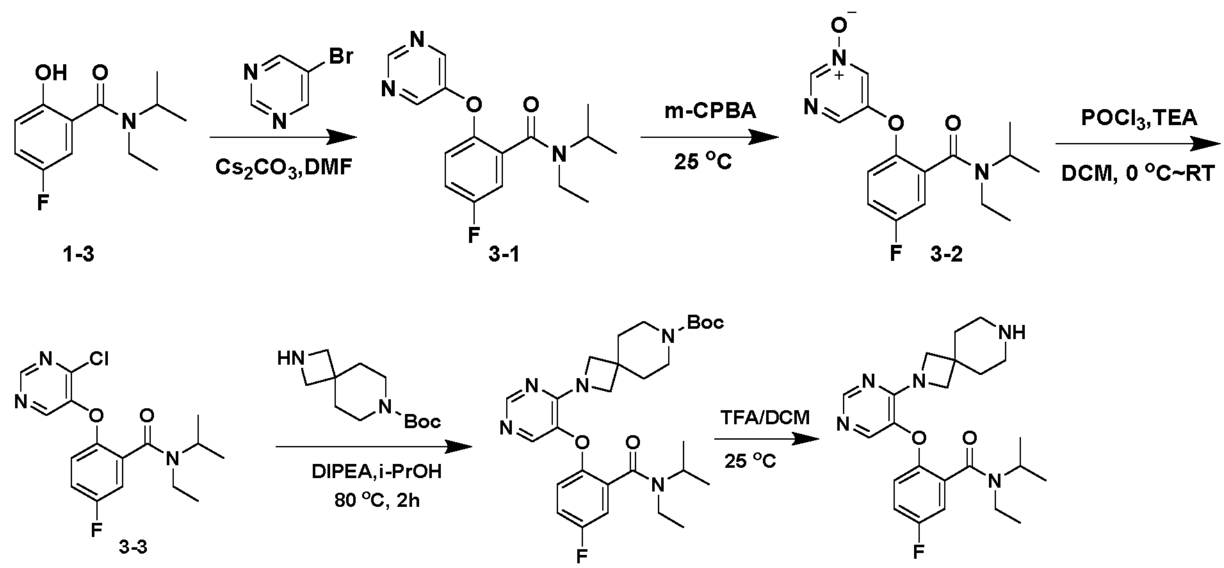

1-3 3-1 3-2 3-3

3-4 N1

**Step 1: Synthesis of 3-1**

At room temperature, **1-3** (10 g, 44.39 mmol) and 5-bromopyrimidine (21.17 g, 133.18 mmol) were dissolved in DMF, added with cesium carbonate (43.39 g, 133.18 mmol), heated to 130°C and stirred overnight. After the reaction was completed, the reaction mixture was diluted with water and extracted three times with EA. The organic phases were combined, washed with saturated brine, dried over anhydrous sodium sulfate and concentrated to obtain a crude product, which was purified by silica gel column (PE/EA= 4: 1, v/v) to obtain **3-1** (11 g, 36.26 mmol, yield 81.68%). MS m/z = 304

[M+H]+.

**Step 2: Synthesis of 3-2**

**3-1** (8.0 g, 26.37 mmol) was dissolved in DCM, and m-CPBA (13.61 g, 79.12 mmol) was slowly added at 0°C. The mixture was warmed to room temperature and stirred for 24 h. After the reaction was completed, the mixture was cooled to 0°C, quenched with saturated aqueous sodium thiosulfate solution, and extracted three times with DCM. The organic phases were combined, washed with saturated brine, dried over anhydrous sodium sulfate and concentrated to obtain a crude product, which was purified by silica gel column to obtain 3-2 (5.6 g, 17.54 mmol, yield 66.51%). MS m/z = 320 [M+H]+.

**Step 3: Synthesis of 3-3**

Triethylamine (3.55 g, 35.07 mmol) was dissolved in DCM, and $POCl_3$ (4.03 g, 26.31 mmol) was slowly added dropwise at 0°C. Then a solution of **3-2** (5.6 g, 17.54 mmol) in DCM was added dropwise to the reaction solution. After the addition was completed, the reaction solution was warmed to room temperature and stirred overnight. After the reaction was completed, the reaction solution was slowly poured into ice water, stirred for half an hour, and then extracted three times with DCM. The organic phases were combined, washed with saturated brine, dried over anhydrous sodium sulfate and concentrated to obtain a crude product, which was purified with silica gel column to obtain **3-3** (1.7 g, 5.03 mmol, yield 28.67%). MS m/z = 338 [M+H]+.

**Step 4: Synthesis of 3-4**

**3-3** (1.0 g, 2.96 mmol) was dissolved in isopropanol, and tert-butyl 2,7-diazaspiro[3.5]nonan-7-carboxylate (871 mg, 3.85 mmol) and DIPEA (1.15 g, 8.88 mmol) were added at room temperature. The mixture was heated to 80° C and stirred for 2 h. After the reaction was completed, the mixture was diluted with water and extracted three times with EA. The organic phases were combined, washed with saturated brine, dried over anhydrous sodium sulfate and concentrated to obtain a crude product, which was purified by silica gel column (PE/EA= 2:1, v/v) to obtain **3-4** (1.54 g, 2.92 mmol, yield 98.6%). MS m/z = 528 [M+H]+.

**Step 5: Synthesis of N1**

At 0°C, **3-4** (1.54 g, 2.92 mmol) was dissolved in DCM (20 mL), and TFA (10 mL) was slowly added dropwise. The mixture was warmed to room temperature and stirred for 0.5 h. After the reaction was completed, the mixture was concentrated, adjusted to a weak alkaline state with a $NaHCO_3$ aqueous solution, extracted with DCM and concentrated to obtain a crude product, which was directly used in the next reaction. MS m/z = 428 [M+H]+.

Referring to the synthesis method of intermediate **N1, 1-5, 1-7, 1-11, 1-15,** and **1-25** were used instead of **1-3** to obtain the corresponding intermediates **N2-N6.**

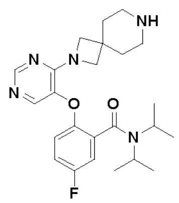
N2

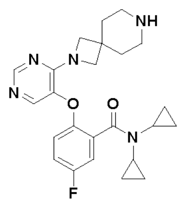
N3

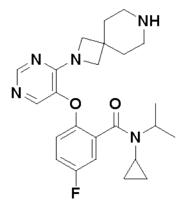
N4

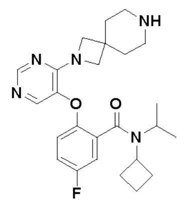
N5

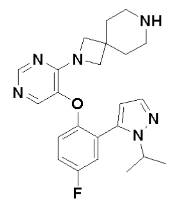
N6

**Synthesis of Intermediates A1-a and A1-b**

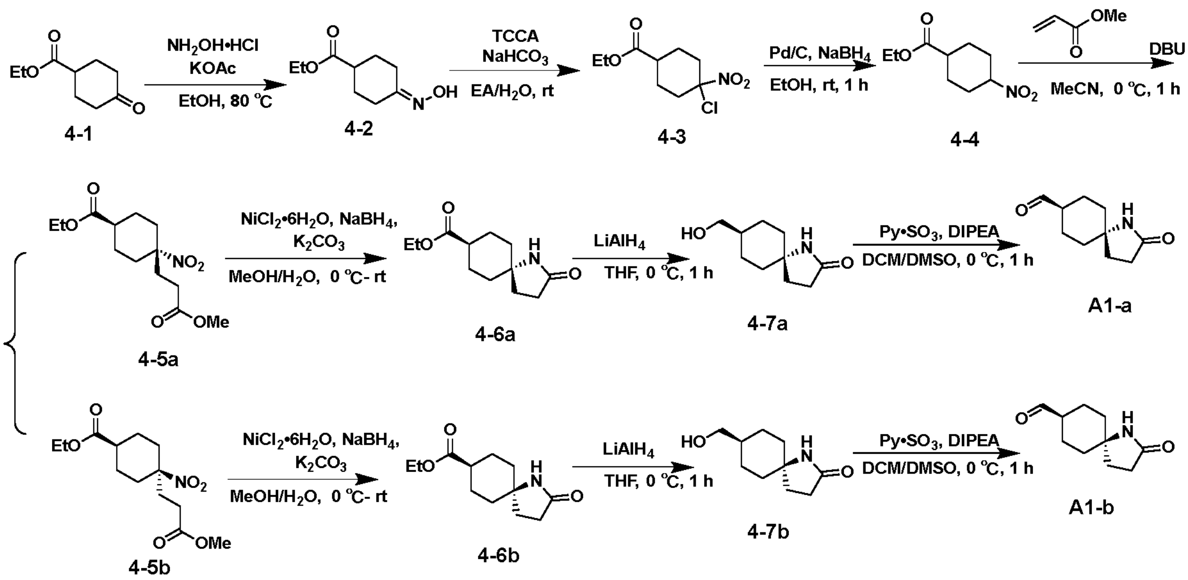


**Step 1: Synthesis of 4-2**

At room temperature, hydroxylamine hydrochloride (3.34 g, 48.06 mmol) and KOAc (4.72 g, 48.08 mmol) were added to a solution of ethyl p-cyclohexanonecarboxylate (6.8 g, 40 mmol) in EtOH (80 mL). The reaction mixture was heated to 80°C and stirred for 4 h. After the reaction was completed, the organic phase was removed by rotary evaporation to obtain a crude product, which was diluted with water and extracted with ethyl acetate. The organic phases were combined and concentrated under reduced pressure to obtain a crude product, which was purified by silica gel column (PE/EA= 5:1, v/v) to obtain **4-2** (4.08 g, 22.02 mmol, yield 55.05%). MS m/z = 186 $[M+H]^+$.

**Step 2: Synthesis of 4-3**

**4-2** (372 mg, 2.01 mmol) and sodium bicarbonate (4.22 g, 50.21 mmol) were dissolved in ethyl acetate (60 mL) and water (60 mL), respectively. TCCA(2.33 g, 10.04 mmol) was added in portions while mixing the above two phases. The organic phase turned blue. After stirring at room temperature for 9 h, the organic phase changed from blue to colorless. After the reaction was completed, the reaction solution was placed in a separatory funnel and extracted three times with EA. The organic phases were combined, washed with saturated brine, dried over anhydrous sodium sulfate and concentrated to obtain a crude product, which was purified by silica gel column (PE/EA= 10:1, v/v) to obtain **4-3** (320 mg, 1.36 mmol, yield 67.66%). MS m/z = 236 $[M+H]^+$.

**Step 3: Synthesis of 4-4**

At -20°C, $NaBH_4$ (4.05 g, 106.93 mmol) was added in portions to a solution of **4-3** (18 g, 76.38 mmol) and Pd/C (1.27 g, 10.49 mmol) in EtOH (200 mL). The reaction was completed when the addition was completed. After the reaction was completed, the mixture was filtered through diatomaceous earth. The filtrate was concentrated, dissolved in EA, washed with water followed by saturated NaCl, dried over anhydrous $Na_2SO_4$, filtered and dried to obtain a concentrated crude product, which was purified by silica gel column (PE/EA= 10:1, v/v) to obtain **4-4** (8.4 g, 41.75 mmol, yield 54.67%). MS m/z = 202 $[M+H]^+$.

**Step 4: Synthesis of 4-5a and 4-5b**

At 0°C, DBU (1.97 g, 12.94 mmol) was added dropwise to a solution of **4-4** (2.17 g, 10.78 mmol) in MeCN (30 mL), and methyl acrylate (1.11 g, 12.94 mmol) was then added dropwise thereto to perform a reaction at 0°C for 1 h. After the reaction was completed, the mixture was added with water, extracted with EA, washed with water followed by saturated NaCl, dried over anhydrous $Na_2SO_4$, filtered, dried, separated and purified by silica gel column chromatography (PE/EA= 40/1→10/1) to obtain products **4-5a** (1.035 g, 3.6 mmol, yield 33.39%, obtained by PE/EA = 30/1, MS m/z = 288 $[M+H]^+$) and **4-5b** (1.424 g, 4.96 mmol, yield 46%, obtained by PE/EA = 10/1, MS m/z = 288 $[M+H]^+$). TLC developing solvent PE/EA = 5/1: **4-5a,** $R_f$ = 0.5; **4-5b,** $R_f$ = 0.3.

**Step 5: Synthesis of 4-6a and 4-6b**

At -10°C, $NaBH_4$ (217.26 mg, 5.74 mmol) was added in portions to **4-5a** (330 mg, 1.15 mmol) and $NiCl_2$•$6H_2O$ (272.88 mg, 1.15 mmol) in MeOH (3 mL), and the mixture was reacted at -10°C for 2 h. Then, an aqueous solution (1 mL) of $K_2CO_3$ (634.94 mg, 4.59 mmol) was added dropwise, and the mixture was reacted at 0°C for 2 h. After the reaction was completed, the mixture was filtered through diatomaceous earth. The filtrate was adjusted to neutral or acidic with 1N HCl, concentrated, and extracted with EA. The organic layers were combined, washed with water followed by saturated NaCl, dried over anhydrous $Na_2SO_4$, filtered and dried, and separated and purified by silica gel column chromatography (DCM/MeOH=40:1) to obtain the product **4-6a** (197 mg, 0.874 mmol, yield 7.6%). MS m/z = 226 $[M+H]^+$. The synthesis method of **4-6b** was the same as above.

**Step 6: Synthesis of 4-7a**

Under ice bath, $LiAlH_4$ (120.88 mg, 3.19 mmol) was added to a solution of **4-6a** (598 mg, 2.65 mmol) in THF (10 mL). The mixture was stirred 0°C for 1 h, quenched with water (100 μL), then added with aq. NaOH (15% wt, 100 μL) and $H_2O$ (300 μL), stirred at room temperature for 10 min, and filtered. The filtrate was concentrated to obtain the crude product **4-7a** (457 mg, 2.49 mmol, yield 93.95%), which was used directly in the next step without further purification. MS m/z = 184 $[M+H]^+$. The synthesis method of **4-7b** was the same as above.

**Step 7: Synthesis of A1-a**

To a solution of **4-7a** (457 mg, 2.49 mmol) in DCM (12 mL)/DMSO (4 mL), DIPEA (1.29 g, 9.98 mmol, 1.74 mL) was added, and then a suspension of Py•$SO_3$ (1.57 g, 9.98 mmol) in DMSO (4 mL) was added dropwise. The reaction mixture was stirred at room temperature for 10 min, then cooled to 0°C, and added with DCM (10 mL)/1N aq HCl (10 mL). The separated aqueous phase was extracted with EA. The combined organic phases were washed with 1 N HCl followed by saturated brine and dried over anhydrous sodium sulfate. The crude product was concentrated under reduced pressure, separated and purified by silica gel column to obtain **A1-a** (279 mg, 1.54 mmol, yield 61.73%), $^1H$ NMR (400 MHz, Chloroform-*d*) δ 9.68 (s, 1H), 7.11 (s, 1H), 2.40 (t, *J* = 8.1 Hz, 2H), 2.35 - 2.24 (m, 1H), 2.00 - 1.87 (m, 2H), 1.78 - 1.70 (m, 2H), 1.68 - 1.53 (m, 4H). MS m/z = 184 $[M+H]^+$. The synthesis method of **A1-b** was the same as above.

**Synthesis of Intermediates B1-a and B1-b**

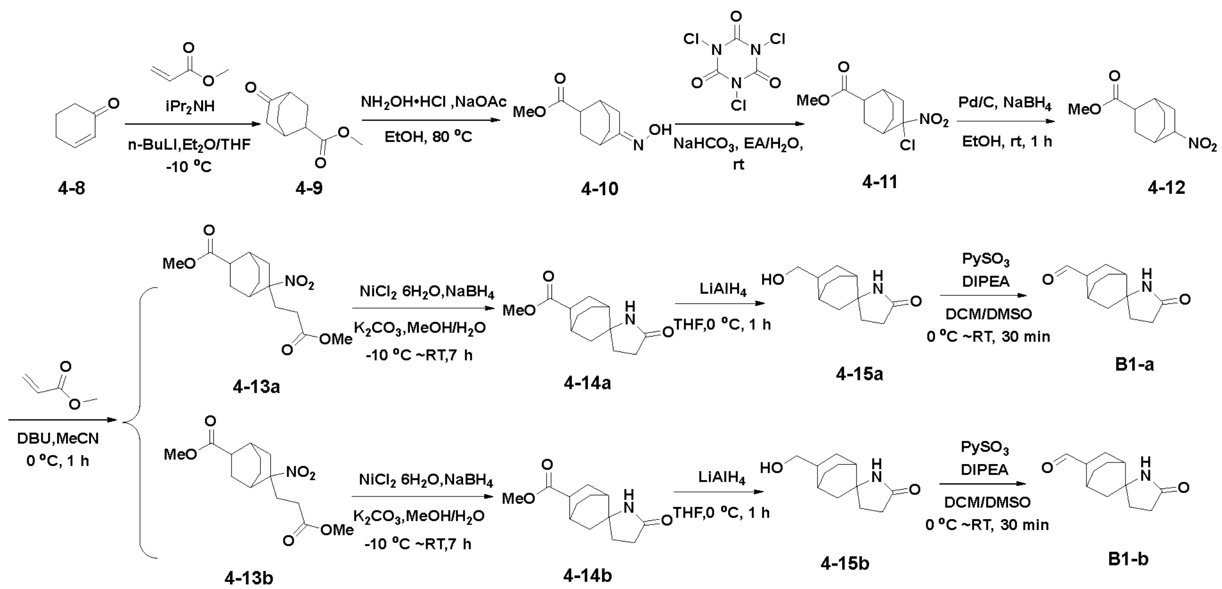


4-8, 4-9, 4-10, 4-11, 4-12, 4-13a, 4-14a, 4-15a, B1-a, 4-13b, 4-14b, 4-15b, B1-b

**Step 1: Synthesis of 4-9**

At -78°C, n-BuLi (11 mL, 27.5 mmol, 2.5 M in hexane) was added dropwise to a solution of diisopropylamine

(2.78 g, 27.46 mmol) in diethyl ether (20 mL). The mixture was gradually warmed to -11°C, and a solution of 2-cyclohexene-1-one (2.4 g, 24.97 mmol) in diethyl ether (20 mL) was added dropwise to the mixture. The temperature of the reaction solution was kept between -11°C and -3°C during the addition process. The reaction solution was stirred for 25 min, and then a solution of methyl acrylate (2.15 g, 24.97 mmol) in THF (20 mL) was added dropwise. Afterwards, the mixture was stirred at -10°C for 1 h. After the reaction was completed, the reaction solution was poured into a saturated ammonium chloride solution (200 mL) and stirred for 15 min. The mixed solution was extracted with EA, and the combined organic phase was dried over anhydrous sodium sulfate and concentrated to obtain a crude product which was separated and purified by a silica gel column (PE/EA=5:1, v/v) to obtain **4-9** (2.1 g, 11.52 mmol, yield 46.16%).

**Step 2: Synthesis of 4-10**

At room temperature, hydroxylamine hydrochloride (2.21 g, 31.8 mmol) and NaOAc (2.61 g, 31.8 mmol) were added to a solution of **4-9** (4.83 g, 26.5 mmol) in EtOH (25 mL). The reaction mixture was heated to 80°C and stirred for 4 h. After the reaction was completed, the organic phase was removed by rotary evaporation to obtain a crude product, which was diluted with water and extracted with ethyl acetate. The combined organic phases were concentrated under reduced pressure to obtain a crude product, which was separated and purified by silica gel column to obtain **4-10** (4.45 g, 22.47 mmol, yield 84.7%). MS m/z = 198 $[M+H]^+$.

**Step 3: Synthesis of 4-11**

Trichloroisocyanuric acid (26.2 g, 112.81 mmol) was added dropwise to a turbid solution of **4-10** (4.45 g, 22.56 mmol) and $NaHCO_3$ (37.6 g, 564 mmol) in EA (500 mL)/$H_2O$ (500 mL). After stirring at room temperature for 20 min, the reaction solution turned blue and stirred continuously for 9 h until the organic phase turned into a colorless solution. After the reaction was completed, the mixed solution was extracted with EA. The organic phases were combined, washed with water followed by saturated brine, dried over anhydrous sodium sulfate, filtered and concentrated to obtain a crude product, which was separated and purified by silica gel column to obtain **4-11** (4.48 g, 18.13 mmol, 80.36% yield).

**Step 4: Synthesis of 4-12**

At 0°C, $NaBH_4$ (950 mg, 24.92 mmol) was added in portions to a solution of **4-11** (4.4 g, 17.8 mmol) and Pd/C (300 mg) in EtOH (80 mL), and the mixture was stirred at room temperature for 1 h. After the reaction was completed, Pd/C was filtered off, and the filtrate was concentrated and purified by silica gel column to obtain **4-12** (3.04 g, 14.27 mmol, 80% yield).

**Step 5: Synthesis of 4-13a and 4-13b**

At 0°C, DBU (2.56 mL, 17.11 mmol) was added to a solution of **4-12** (3.04 g, 14.26 mmol) in MeCN (30 mL), and then methyl acrylate (1.54 mL, 17.11 mmol) was added dropwise. The reaction mixture was stirred at 0°C for 1 h. After the reaction was completed, water was added to quench the reaction, and EA was added for extraction. The combined organic phase was washed with saturated brine, dried over anhydrous sodium sulfate, filtered and concentrated to obtain a crude product, which was separated and purified by silica gel column (PE/EA=10:1, v/v) to obtain **4-13a** (1.66 g, 5.55 mmol, yield 38.92%) and **4-13b** (1.55 g, 5.18 mmol, yield 36.32%). TLC developing agent PE/EA = 5/1: **4-13a,** $R_f$ = 0.40; **4-13b,** $R_f$ = 0.35).

Referring to the method of steps 5 to 7 in the synthetic route of **A1-a**, intermediates **B1-a** and **B1-b** can be obtained, wherein **B1-a** and **B1-b** were not further resolved but directly used in the next step. MS m/z = 208 $[M+H]^+$.

**Synthesis of Intermediates 5-4a and 5-4b**

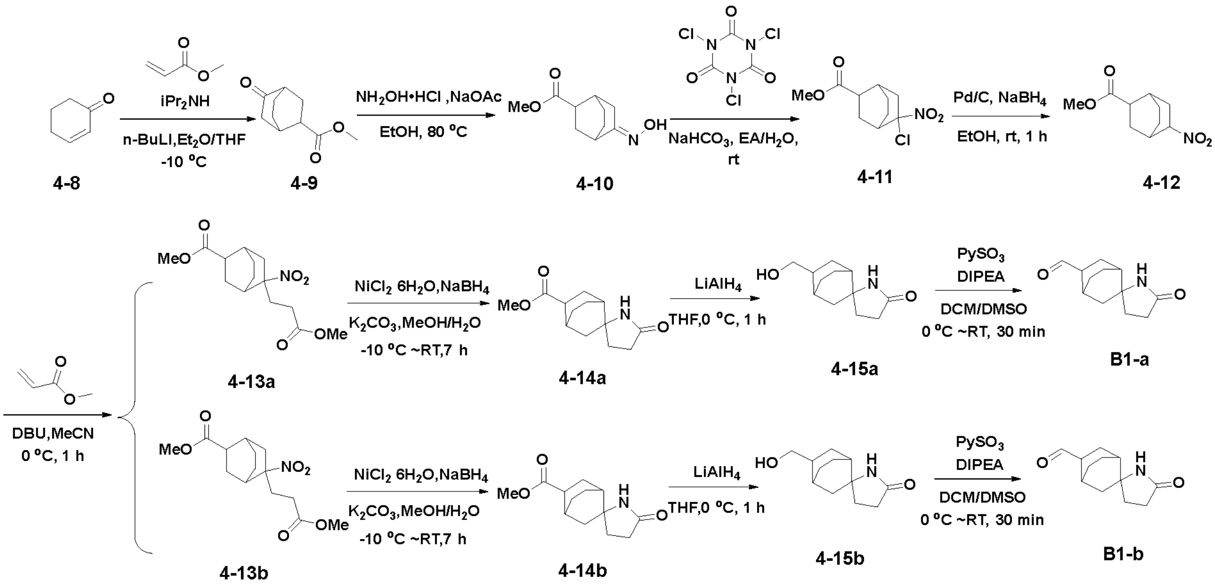


4-8 4-9 4-10 4-11 4-12

4-13a 4-14a 4-15a B1-a

4-13b 4-14b 4-15b B1-b

### Step 1: Synthesis of 5-2

**5-1** (hydrochloride, 193 g, 1.0 mol) was placed in a 5 L beaker, to which 400 mL of a mixed solvent (DCM/IPA = 3/1) was added, and potassium carbonate (1.10 g, 0.8 mol) aqueous solution was slowly added under ice bath, and the temperature was controlled below 20°C. The mixed solvent was repeatedly extracted 3-4 times, and the organic phases were combined and concentrated to obtain a crude product for direct reaction in the next step.

The crude product was dissolved in DCE (2.5 L), and cooled to 10°C. m-CPBA (6.88 g, 4 mol) was added in portions, and the temperature was controlled below 35°C. After the addition was completed, the reaction solution was heated to reflux and stirred for 3 h. After the reaction was completed, the reaction solution was cooled to below 10°C, stirred for 20 min and filtered. The reaction bottle was rinsed with the filtrate, and the filter cake was rinsed with an appropriate amount of DCE and dried. The filtrate was carefully quenched with $Na_2SO_3$ aqueous solution and detected with starch potassium iodide test paper. The filtrate was extracted three times with DCM, concentrated, and purified by silica gel column chromatography (PE/EA= 80/1 → 30/1) to obtain product **5-2** (138 g, 0.737 mol, yield 73.7%). MS m/z = 188 $[M+H]^+$.

### Step 2: Synthesis of 5-3a and 5-3b

Referring to the synthesis method of 4-5a and 4-5b, the target intermediate **5-3a** (PE/EA = 30/1, MS m/z = 274 $[M+H]^+$) and **5-3b** (PE/EA = 20/1, MS m/z = 274 $[M+H]^+$) were obtained by silica gel column purification. TLC developing agent PE/EA = 5/1: **5-3a**, $R_f$ = 0.3; **5-3b,** $R_f$ = 0.2.

### Step 3: Synthesis of 5-4a and 5-4b

Referring to the synthesis method of **4-6a, 5-3a** and **5-3b** were used as raw materials to obtain **5-4a** (MS m/z = 212 $[M+H]^+$) and **5-4b** (MS m/z = 212 $[M+H]^+$).

### Synthesis of Intermediate C1

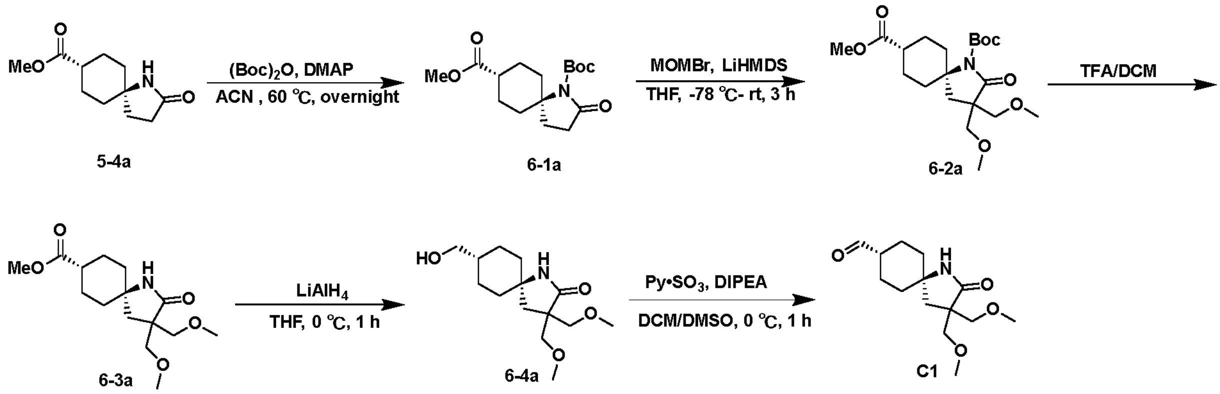

5-4a 6-1a 6-2a

6-3a 6-4a C1

**Step 1: Synthesis of 6-1a**

**5-4a** (2.5 g, 118.34 mmol), $(Boc)_2O$ (103.31 g, 473.36 mmol) and DMAP (5.78 g, 47.34 mmol) were dissolved in acetonitrile (250 mL) and reacted at 60°C overnight under nitrogen protection. After the reaction was completed, the mixture was directly concentrated to obtain a crude product, which was purified by silica gel column (PE/EA= 5:1-3:1, v/v) to obtain **6-1a** (34.28 g, 110.09 mmol, yield 93.02%). MS m/z = 312 $[M+H]^+$.

**Step 2: Synthesis of 6-2a**

**6-1a** (100 mg, 0.307 mmol) was dissolved in THF (20 mL), cooled to -78°C, added with LiHMDS (0.614 mmol, 2.0 eq) dropwise under nitrogen protection, and stirred at this temperature for 1 h. Then the mixture was slowly added with a solution of MOMBr (96 mg, 0.768 mmol) in THF dropwise, heated to room temperature, and stirred for 2 h. After the reaction was completed, saturated ammonium chloride was added to quench the reaction, and the mixture was extracted three times with DCM. The organic phases were combined, washed with saturated brine, dried over anhydrous sodium sulfate and concentrated to obtain a crude product, which was purified by MPLC to obtain **6-2a** (99 mg, 0.239 mmol, yield 77.85%). MS m/z = 400 $[M+H]^+$.

**Step 3: Synthesis of 6-3a**

**6-2a** (244 mg, 0.59 mmol) was dissolved in anhydrous DCM (7 mL), added with TFA (3 mL) dropwise, and stirred at room temperature for 1 h. After the reaction was completed, the mixture was concentrated to obtain a crude product (114 mg, 0.363 mmol) which was directly used for the next step. MS m/z = 300 $[M+H]^+$.

**Step 4: Synthesis of 6-4a**

**6-3a** (114 mg, 0.363 mmol) was dissolved in THF (10 mL), added with $LiAlH_4$ (16.57 mg, 0.436 mmol) at 0°C, and stirred at this temperature for 1 h. After the reaction was completed, sodium sulfate decahydrate was added in portions under ice bath until no bubbles were generated in the system. After filtration, the filtrate was concentrated to obtain a crude product, which was directly used for the next step. MS m/z = 272 $[M+H]^+$.

**Step 5: Synthesis of C1**

**6-4a** (100 mg, 0.368 mmol) was dissolved in a mixed solvent of DCM/DMSO = 6 mL/2 mL and cooled to 0 °C, and DIPEA (190.51 mg, 1.47 mmol) and sulfur trioxide pyridine (234.62 mg, 1.47 mmol) in DMSO (2 mL) were added dropwise in sequence. The mixture was warmed to room temperature and stirred for 30 min. After the reaction was completed, the system was moved to an ice bath, quenched with 1 N HCl, diluted with water, and extracted three times with EA. The organic phases were combined, washed with saturated brine, dried over anhydrous sodium sulfate and concentrated to obtain a crude product, which was purified by silica gel column to obtain **C1** (75 mg, 0.278 mmol, yield 75.54%). MS m/z = 270 $[M+H]^+$.

**Synthesis of Intermediate C2**

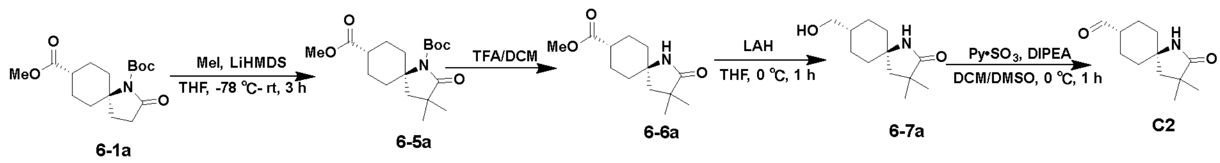


**Step 1: Synthesis of 6-5a**

**6-1a** (2.8 g, 8.7 mmol) was dissolved in THF, cooled to -78°C, added with LiHMDS (15.96 ml, 2.4 eq) dropwise under nitrogen protection, stirred at this temperature for 1 h, then slowly added with a solution of iodomethane (2.93 g, 20.65 mmol) in THF dropwise, heated to room temperature and stirred for 2 h. After the reaction was completed, saturated ammonium chloride was added to quench the reaction, and the mixture was extracted three times with DCM. The organic phases were combined, washed with saturated brine, dried over anhydrous sodium sulfate and concentrated to obtain a crude product, which was purified by silica gel column (PE/EA= 5:1, v/v) to obtain **6-5a** (2.4 g, 6.85 mmol, yield 78.73%). $^1$H NMR (400 MHz, Chloroform-*d*) δ 9.67 (s, 1H), 6.51 (s, 1H), 2.32 - 2.22 (m, 1H), 2.00 - 1.90 (m, 2H), 1.85 (s, 2H), 1.79 - 1.66 (m, 2H), 1.63 - 1.50 (m, 4H), 1.23 (s, 6H). MS m/z = 340 $[M+H]^+$.

**Step 2-Step 4: Synthesis of C2**

Referring to the synthesis method of **C1,** intermediate **C2** was obtained. MS m/z = 210 $[M+H]^+$.

**Synthesis of Intermediate C3**

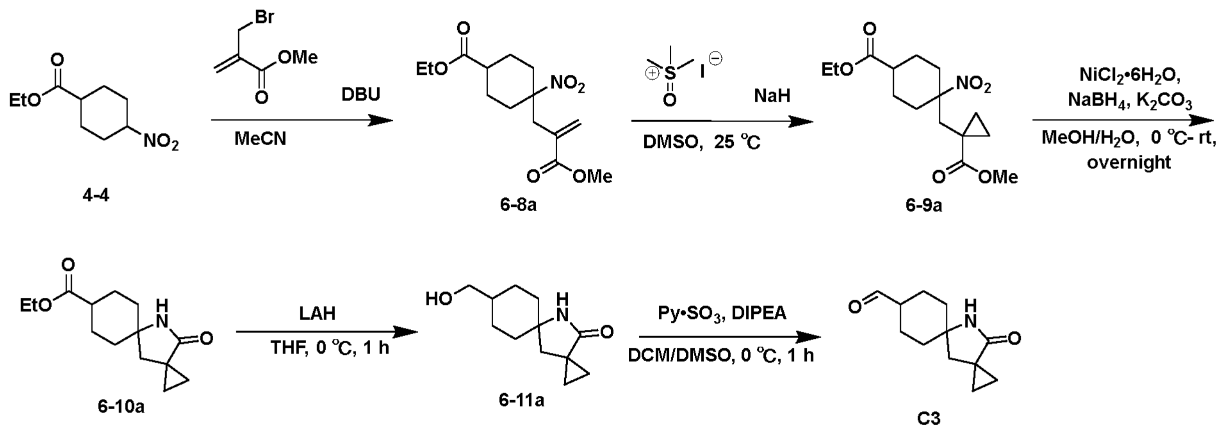


**Step 1: Synthesis of 6-8a**

**4-4** (100 mg, 0.496 mmol) was dissolved in acetonitrile, added with DBU (150 mg, 0.596 mmol) and methyl 2-(bromomethyl)acrylate (106 mg, 0.596 mmol) at 0°C, warmed to room temperature, and stirred for 2 h. After the reaction was completed, the mixture was diluted with water and extracted three times with EA. The organic phases were combined, washed with saturated brine, dried over anhydrous sodium sulfate and concentrated to obtain a crude product, which was purified by silica gel column (PE/EA= 5: 1, v/v) to obtain **6-8a** (52 mg, 0.173 mmol, yield 34.8%, containing two isomers). MS m/z = 300 $[M+H]^+$.

**Step 2: Synthesis of 6-9a**

Trimethylsulfoxide iodide (19.12 g, 86.86 mmol) was dissolved in DMSO, added with NaH (2.08 g, 86.86 mmol) at 0°C, slowly warmed to room temperature, stirred for 1 h and then added with **6-8a** (20 g, 66.82 mmol) to perform a reaction at room temperature for 17 h. After the reaction was completed, saturated ammonium chloride was added to quench the reaction and the mixture was extracted three times with EA. The organic phases were combined, washed with saturated brine, dried over anhydrous sodium sulfate and concentrated to obtain a crude product, which was purified by silica gel column (PE/EA= 4:1, v/v) to obtain **6-9a** (8.5 g, 27.13 mmol, yield 40.6%, containing two isomers). MS m/z

= 314 [M+H]+.

**Step 3-Step 5: Synthesis of C3**

Referring to the synthesis method of **C1,** intermediate **C3** (containing two isomers) was obtained. MS m/z = 208 [M+H]+.

**Synthesis of Intermediate C4**

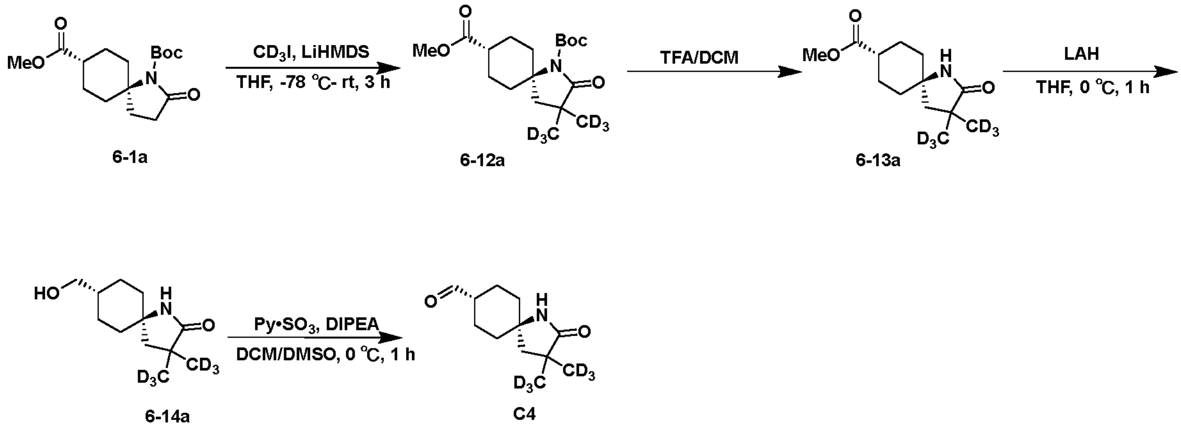


6-1a 6-12a 6-13a 6-14a C4

Referring to the synthesis method of C1, deuterated iodomethane was used instead of iodomethane to obtain intermediate **C4.** MS m/z = 216 [M+H]+.

**Synthesis of Intermediate C5**

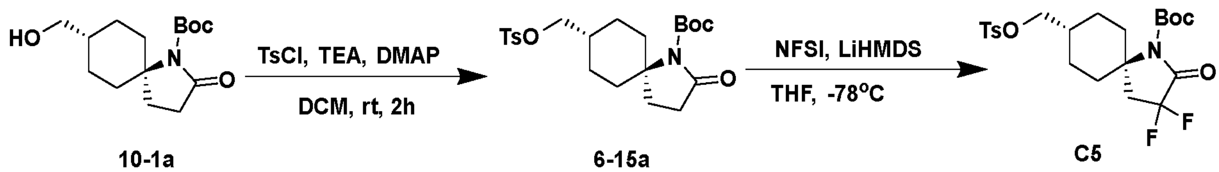


10-1a 6-15a C5

**Step 1: Synthesis of 6-15a**

**10-1a** (1.7 g, 6.0 mmol) was dissolved in DCM (40 mL), and TEA (2.43 g, 24 mmol), TsCl (465.45 mg, 6.6 mmol) and DMAP (73.29 mg, 0.60 mmol) were added in sequence. The mixture was stirred at 0°C for 2 h under nitrogen protection. After the reaction was completed, the mixture was diluted with water and extracted three times with EA. The organic phases were combined, washed with saturated brine, dried over anhydrous sodium sulfate and concentrated to obtain a crude product, which was purified by silica gel column (PE/EA= 3:1, v/v) to obtain **6-15a** (2.1 g, 4.8 mmol, yield 80%). MS m/z = 438 [M+H]+.

**Step 2: Synthesis of C5**

**6-15a** (200 mg, 0.45 mmol) was dissolved in anhydrous THF (3 mL), cooled to -78°C, slowly added with LiHMDS (1 mL, 1.28 mmol) under nitrogen protection, stirred at this temperature for 1 h, then slowly added with NFSI (248.28 mg, 1.28 mmol) in THF (3 mL), and stirred at room temperature for 4 h. The mixture was cooled again to -78°C, slowly added with LiHMDS (1 mL, 1.28 mmol), stirred for 1 h, then slowly added with NFSI (248.28 mg, 1.28 mmol) in THF (3 mL), and stirred at room temperature for 16 h. After the reaction was completed, saturated ammonium chloride was added to quench the reaction and the mixture was extracted three times with EA. The organic phases were combined, washed with saturated brine, dried over anhydrous sodium sulfate and concentrated to obtain a crude product, which

was purified by MPLC to obtain **C5** (90 mg, 0.19 mmol, yield 42.2%). MS m/z = 474 $[M+H]^+$.

**Synthesis of Intermediate C6**

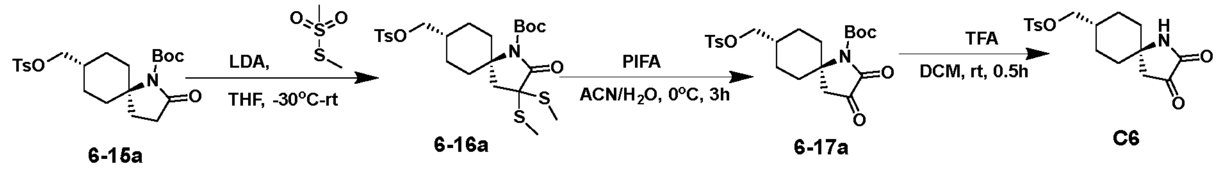


**Step 1: Synthesis of 6-16a**

**6-15a** (500 mg, 1.14 mmol) was dissolved in anhydrous THF (10 mL), cooled to -30°C, and LDA (183.61 mg, 1.71 mmol) and methyl methylthiosulfonate (216.3 mg, 1.17 mmol) were slowly added dropwise under nitrogen protection. The mixture was stirred at room temperature for 20 h. After the reaction was completed, saturated ammonium chloride solution was added to quench the reaction and the mixture was extracted three times with EA. The organic phases were combined, washed with saturated brine, dried over anhydrous sodium sulfate and concentrated to obtain a crude product, which was purified by silica gel column (PE/EA= 5: 1, v/v) to obtain **6-16a** (303 mg, 0.57 mmol, yield 50%). MS m/z = 530 $[M+H]^+$.

**Step 2: Synthesis of 6-17a**

**6-16a** (303 mg, 0.57 mmol) was dissolved in a mixed solvent of acetonitrile and water (5 mL: 0.5 mL), cooled to 0°C, added with PIFA (757.93 mg, 1.76 mmol) under nitrogen protection, and then stirred at the same temperature for 3 h. After the reaction was completed, saturated sodium bicarbonate solution was added to quench the reaction and the mixture was extracted three times with EA. The organic phases were combined, washed with saturated brine, dried over anhydrous sodium sulfate and concentrated to obtain a crude product, which was purified by silica gel column (DCM/MeOH = 100:1, v/v) to obtain **6-17a** (170 mg, 0.37 mmol, yield 64.91%). MS m/z = 452 $[M+H]^+$.

**Step 3: Synthesis of C6**

At 0°C, **6-17a** (170 mg, 0.376 mmol) was dissolved in DCM (2 mL), slowly added with TFA (1 mL) dropwise, warmed to room temperature, and stirred for 0.5 h. After the reaction was completed, the mixture was concentrated to obtain a crude product which was used directly in the next step without further purification. MS m/z = 352 $[M+H]^+$.

**Synthesis of Intermediate C7**

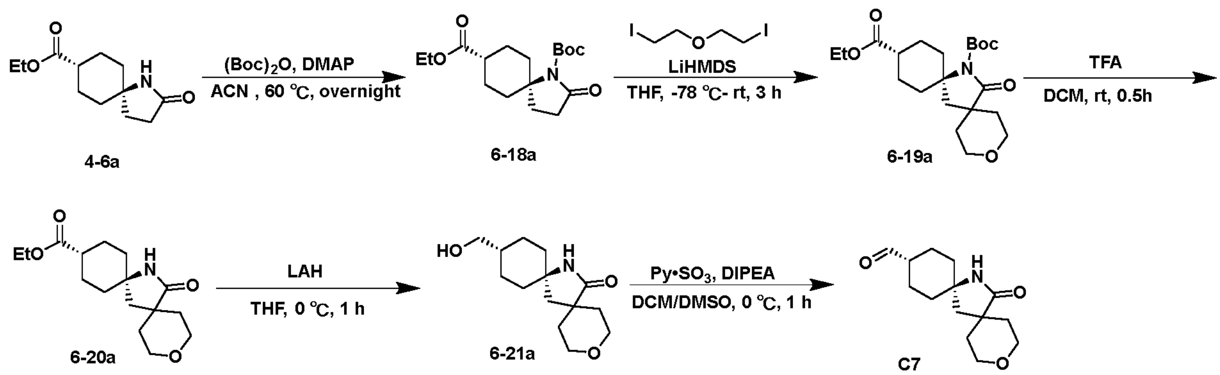


**Step 1: Synthesis of 6-18a**

**4-6a** (12.5 g, 55.5 mmol), $(Boc)_2O$ (51.6 g, 237 mmol) and DMAP (2.89 g, 23.7 mmol) were dissolved in

acetonitrile (250 mL) and reacted at 60°C overnight under nitrogen protection. After the reaction was completed, the mixture was directly concentrated to obtain a crude product, which was purified by silica gel column (PE/EA= 5:1-3: 1, v/v) to obtain **6-18a** (17 g, 52 mmol, yield 93.69%). MS m/z = 326 $[M+H]^+$.

**Step 2: Synthesis of 6-19a**

**6-18a** (500 mg, 1.54 mmol) was dissolved in THF (1.5 mL), cooled to -78°C, added with LiHMDS (3 ml, 3.84 mmol) dropwise under nitrogen protection, and stirred at this temperature for 1 h. Then a solution of 2-iodoethyl ether (751 mg, 2.3 mmol) in THF (1.5 mL) was slowly added dropwise, warmed to room temperature and stirred for 2 h. After the reaction was completed, saturated ammonium chloride was added to quench the reaction and the mixture was extracted three times with DCM. The organic phases were combined, washed with saturated brine, dried over anhydrous sodium sulfate and concentrated to obtain a crude product, which was purified by MPLC to obtain **6-19a** (62 mg, 0.156 mmol, yield 10.12%). MS m/z = 396 $[M+H]^+$.

**Step 3-Step 5: Synthesis of C7**

Referring to the synthesis method of **C1,** the intermediate **C7** (45 mg, 0.179 mmol) was obtained by the same experimental operation. MS m/z = 252 $[M+H]^+$.

**Synthesis of Intermediates D1-a1 and D1-a2**

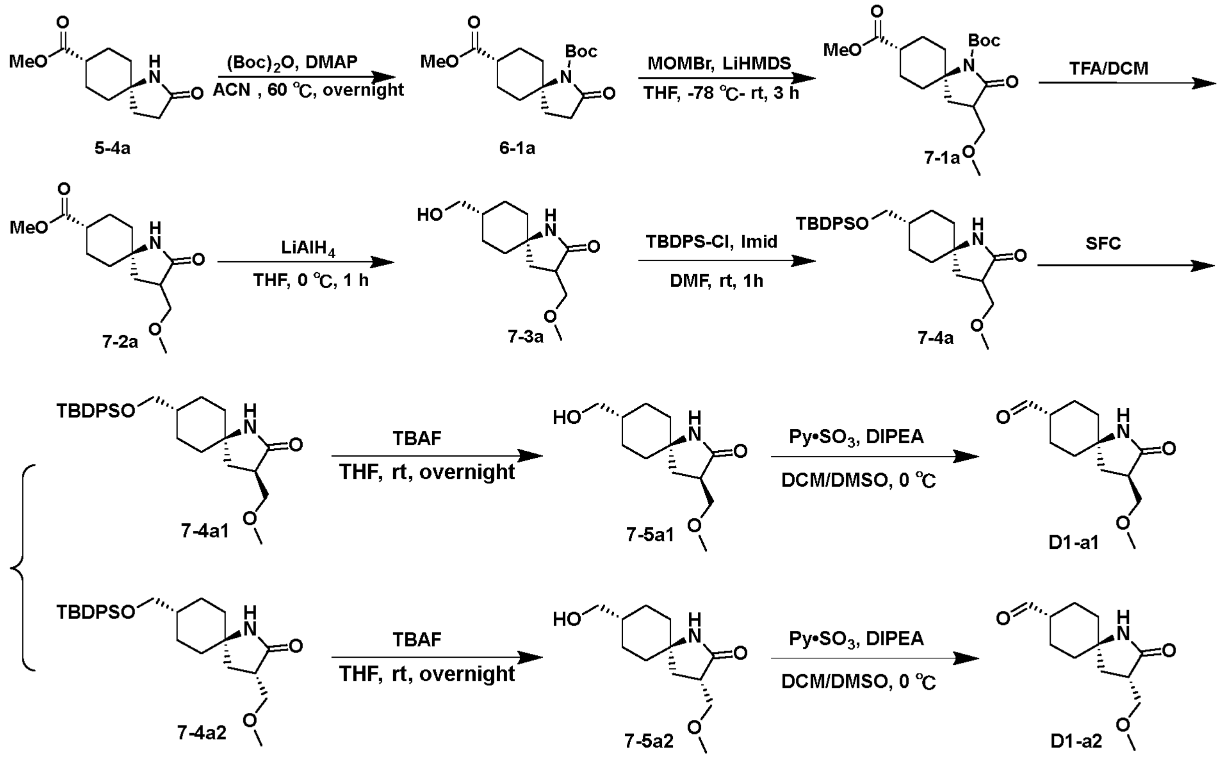


**Step 1: Synthesis of 7-1a**

**6-1a** (34.28 g, 110.09 mmol) was dissolved in THF, cooled to -78°C, added with LiHMDS (93 ml, 1.1 eq) dropwise under nitrogen protection, and stirred at this temperature for 1 h. Then a solution of MOMBr (17.89 g, 143.12 mmol) in THF was slowly added, warmed to room temperature and stirred for 2 h. After the reaction was completed, saturated ammonium chloride was added to quench the reaction and the mixture was extracted three times with DCM. The organic phases were combined, washed with saturated brine, dried over anhydrous sodium sulfate and concentrated to obtain a crude product, which was purified by MPLC to obtain **7-1a** (25.6 g, 72.03 mmol, yield 65.43%). MS m/z = 356 $[M+H]^+$.

**Step 2: Synthesis of 7-2a**

**7-1a** (25.6 g, 72.03 mmol) was dissolved in anhydrous DCM (250 mL), added with TFA (40 mL) dropwise, and stirred at room temperature for 1 h. After the reaction was completed, the concentrated crude product was directly used in the next step. MS m/z = 256 $[M+H]^+$.

**Step 3: Synthesis of 7-3a**

**7-2a** (18.34 g, 71.83 mmol) was dissolved in THF (400 mL), added with $LiAlH_4$ (3.27 g, 86.20 mmol) at 0°C, and stirred at this temperature for 1 h. After the reaction was completed, sodium sulfate decahydrate was added in portions under ice bath until no bubbles were generated in the system. After filtration, the filtrate was concentrated to obtain the crude product, which was directly used in the next step. MS m/z = 228 $[M+H]^+$.

**Step 4: Synthesis of 7-4a**

**7-3a** (16.16 g, 71.10 mmol) was dissolved in anhydrous DMF (160 mL), added with imidazole (7.26 g, 106.64 mmol) and TBDPS-Cl (29.31 g, 106.64 mmol), and stirred at room temperature for 1 h. After the reaction was completed, the mixture was diluted with water and extracted three times with EA. The organic phases were combined, washed with saturated brine, dried over anhydrous sodium sulfate and concentrated to obtain a crude product, which was purified by silica gel column (PE/EA= 1:1, v/v) to obtain **7-4a** (33 g, 70.86 mmol, yield 99.7%). MS m/z = 466 $[M+H]^+$.

**Step 5: Synthesis of 7-4a1 and 7-4a2**

**7-4a** was resolved by SFC into isomers **7-4a1** (SFC peak retention time: 2.986 min) and **7-4a2** (SFC peak retention time: 4.269 min).

SFC method of intermediates **7-4a1** and **7-4a2:** chiral column model: CHIRALPAK AS; specification: 3um, 150mm*3mm; mobile phase A-$CO_2$, mobile phase B-ethanol, A/B=70/30: flow rate: 1 mL/min; column temperature: 40°C.

**Step 6: Synthesis of 7-5a1 and 7-5a2**

**7-4a1** (654 mg, 1.40 mmol) was dissolved in THF (10 mL), added with TBAF (1.47 g, 5.62 mmol), and stirred at room temperature overnight. After the reaction was completed, the mixture was diluted with water and extracted three times with EA. The organic phases were combined, washed with saturated brine, dried over anhydrous sodium sulfate and concentrated to obtain a crude product, which was purified with a silica gel column (PE/EA= 1:1, v/v) to obtain **7-5a1** (300 mg, 1.32 mmol, yield 94.28%). MS m/z = 228 $[M+H]^+$. (The synthesis method of **7-5a2** was the same as above)

**Step 7: Synthesis of D1-a1 and D1-a2**

**7-5a1** (300 mg, 1.32 mmol) was dissolved in a mixed solvent of DCM/DMSO = 24 mL/4 mL, cooled to 0°C, added with solutions of DIPEA (682.31 mg, 5.28 mmol) and sulfur trioxide pyridine (840.27 mg, 5.28 mmol) in DMSO (2 mL) dropwise sequemtially, warmed to room temperature and stirred for 30 min. After the reaction was completed, the system was moved to an ice bath, quenched with 1 N HCl, diluted with water, and extracted three times with EA. The organic phases were combined, washed with saturated brine, dried over anhydrous sodium sulfate and concentrated to obtain a crude product, which was purified by silica gel column to obtain **D1-a1** (130 mg, 1.02 mmol, yield 77.27%) of single configuration. $^1H$ NMR (400 MHz, Chloroform-*d*) $\delta$ 9.67 (s, 1H), 7.01 (s, 1H), 3.59 (d, *J* = 5.0 Hz, 2H), 3.36 (s, 3H), 2.76 (tt, *J* = 9.5, 4.9 Hz, 1H), 2.33 - 2.21 (m, 1H), 2.16 (dd, *J* = 13.0, 9.5 Hz, 1H), 2.00 -1.90 (m, 2H), 1.85 -1.69 (m, 3H), 1.67 - 1.48 (m, 4H). MS m/z = 226 $[M+H]^+$. The synthesis method of another single configuration **D1-a2** was the same as above.

**Synthesis of Intermediates D2-a1 and D2-a2**

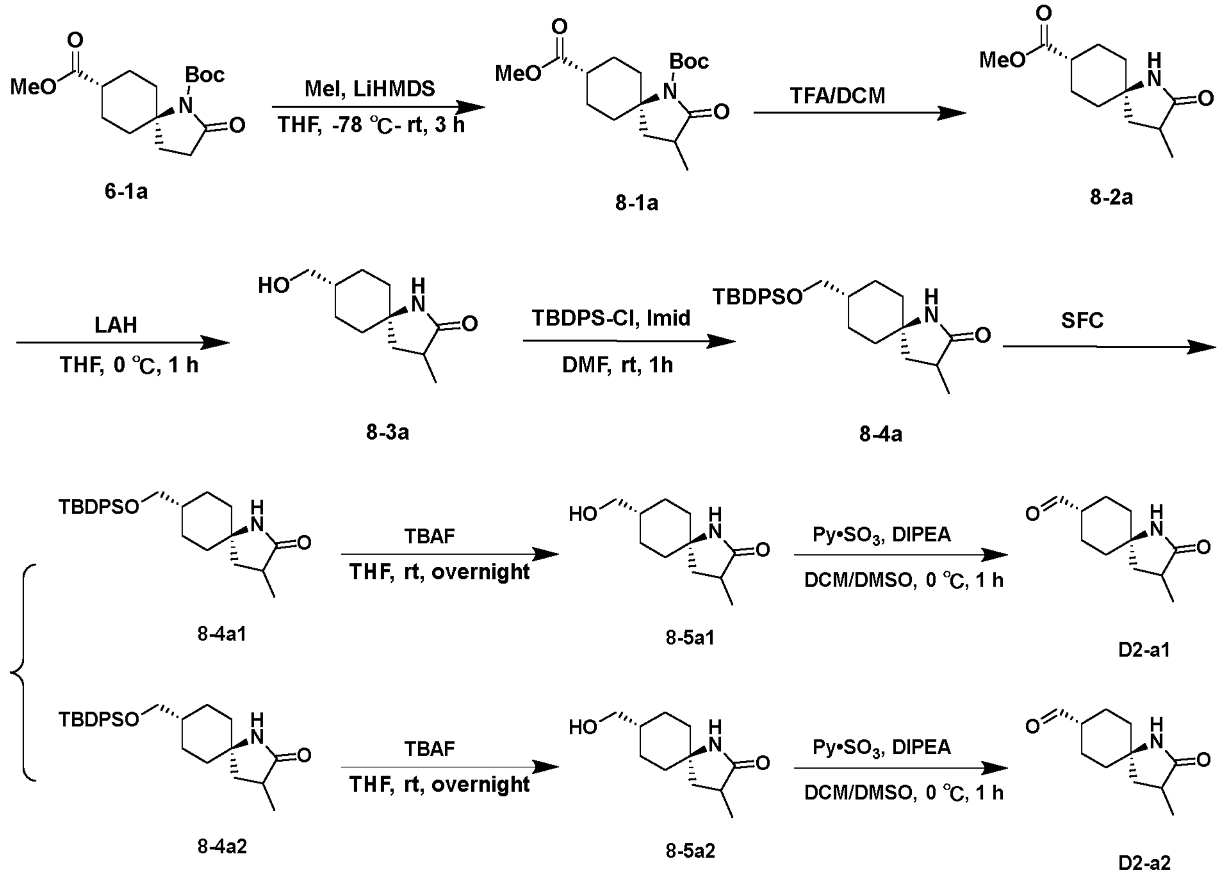


6-1a 8-1a 8-2a 8-3a 8-4a 8-4a1 8-5a1 D2-a1 8-4a2 8-5a2 D2-a2

Referring to the synthesis methods of **D1-a1** and **D1-a2,** iodomethane was used instead of MOMBr to obtain intermediates **D2-a1** (MS m/z = 196 $[M+H]^+$) and **D2-a2** (MS m/z = 196 $[M+H]^+$) of single configuration, respectively.

SFC method of intermediates **8-4a1** and **8-4a2:** chiral column model: CHIRAL ART Cellulose-SC; specification: 3um, 150mm*3mm; mobile phase A-$CO_2$, mobile phase B-isopropanol, A/B=70/30: flow rate: 1 mL/min; column temperature: 40°C; SFC peak times of **8-4a1** and **8-4a2** were 3.809 min and 4.479 min respectively.

**Synthesis of Intermediates D3-a1 and D3-a2**

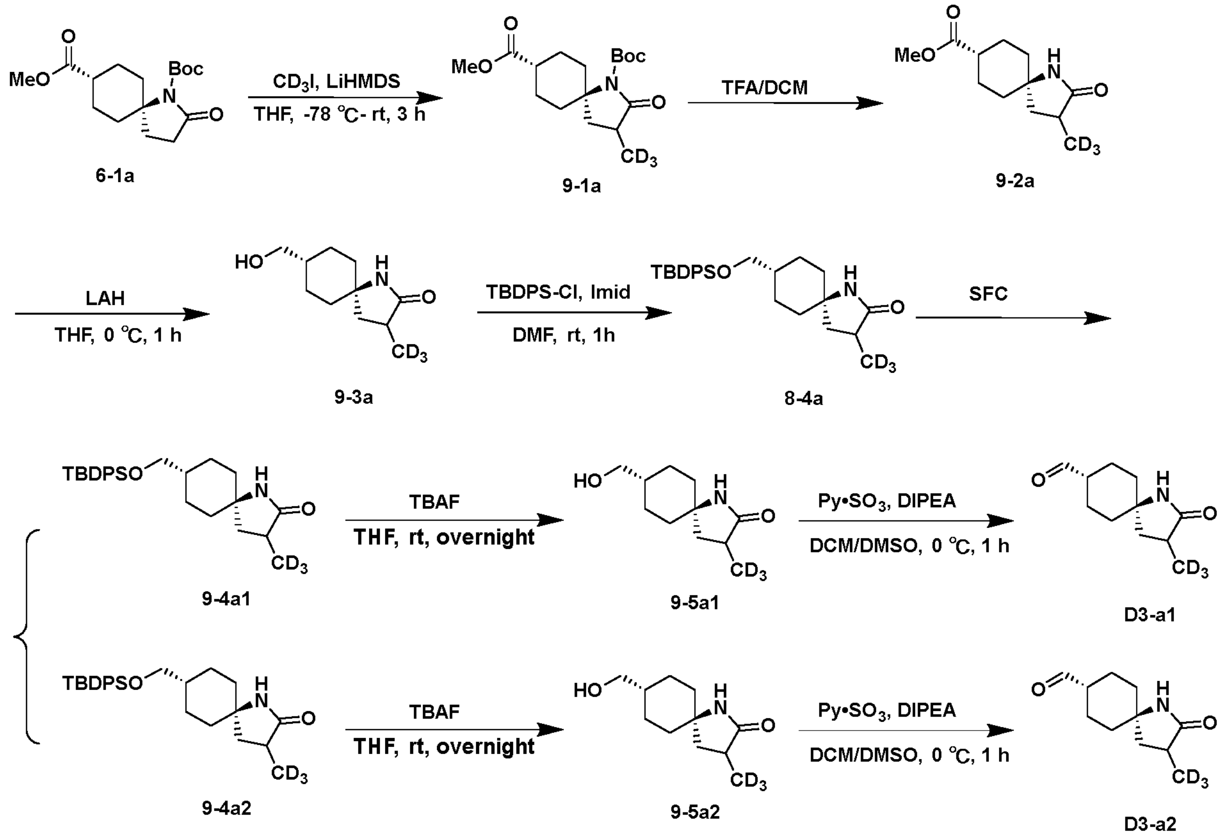


6-1a, 9-1a, 9-2a, 9-3a, 8-4a, 9-4a1, 9-5a1, D3-a1, 9-4a2, 9-5a2, D3-a2

Referring to the synthesis methods of D1-a1 and D1-a2, deuterated iodomethane was used instead of MOMBr to obtain intermediates **D3-a1** (MS m/z = 199 $[M+H]^+$) and **D3-a2** (MS m/z = 199 $[M+H]^+$) of single configuration, respectively.

SFC method of intermediates **9-4a1** and **9-4a2:** chiral column model: CHIRAL ART Cellulose-SC; specification: 3um, 150mm*3mm; mobile phase A-$CO_2$, mobile phase B-isopropanol, A/B=65/35: flow rate: 1 mL/min; column temperature: 40°C; SFC peak times of 9-**4a1** and **9-4a2**were 2.936 min and 3.386 min respectively.

**Synthesis of Intermediates D4-a and D5-a**

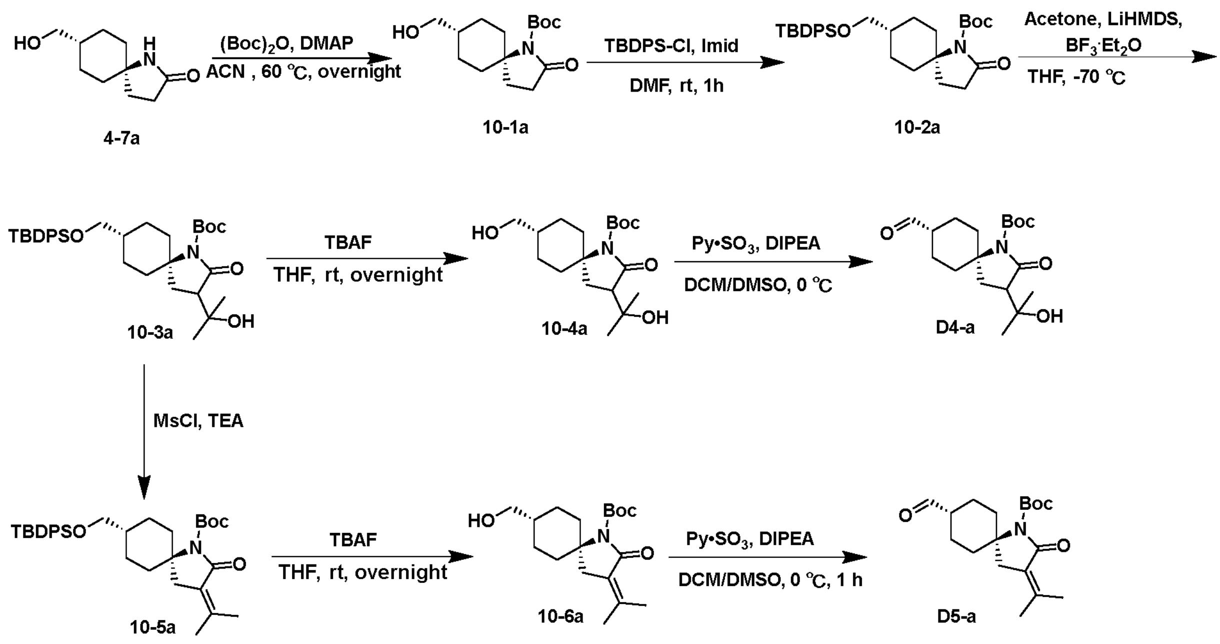


**Step 1: Synthesis of 10-1a**

**4-7a** (1.83 g, 10 mmol), (Boc)$_2$O (8.72 g, 40 mmol) and DMAP (0.48 mg, 4 mmol) were dissolved in acetonitrile (20 mL) and reacted at 60°C overnight under nitrogen protection. After the reaction was completed, the mixture was directly concentrated to obtain a crude product which was purified by silica gel column (PE/EA= 5:1-3:1, v/v) to obtain **10-1a** (2.9 g, 9.3 mmol, yield 93%). MS m/z = 284 $[M+H]^+$.

**Step 2: Synthesis of 10-2a**

**10-1a** (2.9 g, 9.3 mmol) was dissolved in anhydrous DMF (10 mL), added with imidazole (0.95 g, 13.88 mmol) and TBDPS-Cl (3.81 g, 13.88 mmol), and stirred at room temperature for 1 h. After the reaction was completed, the mixture was diluted with water and extracted three times with EA. The organic phases were combined, washed with saturated brine, dried over anhydrous sodium sulfate and concentrated to obtain a crude product, which was purified by silica gel column (PE/EA= 1:1, v/v) to obtain **10-2a** (4.83 g, 9.26 mmol, yield 99.57%). MS m/z = 522 $[M+H]^+$.

**Step 3: Synthesis of 10-3a**

**10-2a** (916 mg, 1.76 mmol) was dissolved in anhydrous THF (10 mL), slowly added with LiHMDS (1.93 mmol, 1.1 eq) dropwise at -70°C, and stirred at this temperature for 1 h. Subsequently, the mixrture was added with acetone (112.16 mg, 1.93 mmol) and boron trifluoride etherate (274.22 mg, 1.93 mmol) dropwise at -70°C, warmed to room temperature, and stirred for 2.5 h. After the reaction was completed, saturated ammonium chloride was added to quench the reaction and the mixture was extracted three times with EA. The organic phases were combined, washed with saturated brine, dried over anhydrous sodium sulfate and concentrated to obtain a crude product, which was purified by MPLC to obtain **10-3a** (883 mg, 1.52 mmol, yield 86.36%). MS m/z = 580 $[M+H]^+$.

**Step 4: Synthesis of 10-4a**

**10-3a** (883 mg, 1.52 mmol) was dissolved in THF (10 mL), added with TBAF (1.59 g, 6.08 mmol) and stirred at room temperature overnight. After the reaction was completed, the mixture was diluted with water and extracted three times with EA. The organic phases were combined, washed with saturated brine, dried over anhydrous sodium sulfate and concentrated to obtain a crude product, which was purified by silica gel column (PE/EA= 1:1, v/v) to obtain **10-4a** (488 mg, 1.43 mmol, yield 94.08%). MS m/z = 342 $[M+H]^+$.

**Step 4: Synthesis of D4-a**

**10-4a** (488 mg, 1.43 mmol) was dissolved in a mixed solvent of DCM/DMSO = 24 mL/4 mL, cooled to 0°C,

added dropwise with solutions of DIPEA (784.65 mg, 6.07 mmol) and sulfur trioxide pyridine (966.31 mg, 6.07 mmol) in DMSO (2 mL) sequentially, warmed to room temperature and stirred for 30 min. After the reaction was completed, the system was moved to an ice bath, quenched with 1 N HCl, diluted with water, and extracted three times with EA. The organic phases were combined, washed with saturated brine, dried over anhydrous sodium sulfate and concentrated to obtain a crude product, which was purified by silica gel column to obtain **D4-a** (397.88 mg, 1.17 mmol, yield 81.8%, containing 2 isomers, which were not resolved and used directly in the next step). MS m/z = 340 $[M+H]^+$.

**Step 5: Synthesis of 10-5a**

**10-3a** (499 mg, 0.86 mmol) was dissolved in DCM (8 mL), added with TEA (870.82 mg, 8.61 mmol) and MsCl (98.58 mg, 0.86 mmol) dropwise at 0°C, warmed to room temperature and stirred for 2 days. After the reaction was completed, the mixture was diluted with water and extracted three times with EA. The organic phases were combined, washed with saturated brine, dried over anhydrous sodium sulfate and concentrated to obtain a crude product, which was purified by silica gel column to obtain **10-5a** (263 mg, 0.47 mmol, yield 54.7%). MS m/z = 562 $[M+H]^+$.

**Step 6-Step 7: Synthesis of D5-a**

Referring to the synthesis method of **D4-a,** intermediate **D5-a** was obtained by TBDPS deprotection and oxidation. MS m/z = 322 $[M+H]^+$.

**Synthesis of Intermediates E2-a and E2-b**

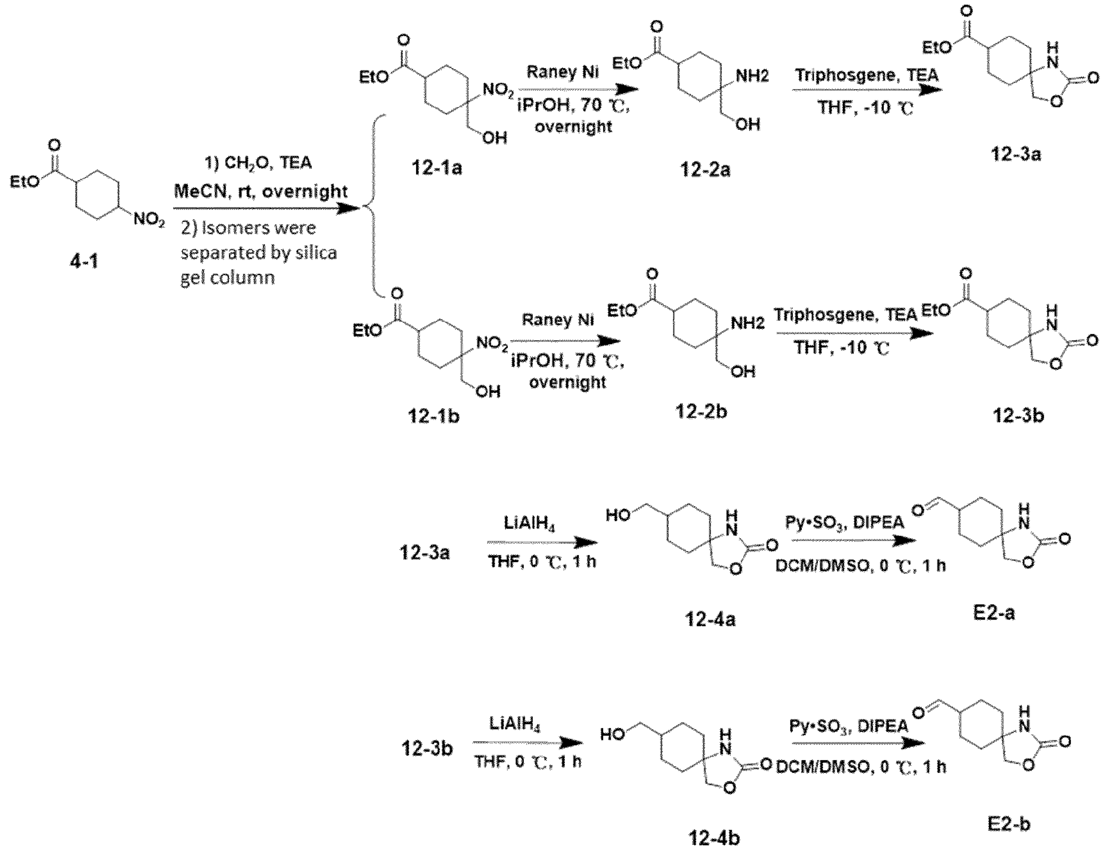


**Step 1: Synthesis of 12-1a**

**4-1** (1.65 g, 8.20 mmol) was dissolved in acetonitrile (7.37 mL), added with TEA (912.74 mg, 9.02 mmol) and formaldehyde aqueous solution (492.43 mg, 16.2 mmol) dropwise at 0°C, warmed to room temperature and stirred overnight. After the reaction was completed, the mixture was directly concentrated to obtain a crude product which was

purified by silica gel column (PE/EA= 4:1, v/v) to obtain stereoisomer **12-1a** (764 mg, 3.3 mmol, obtained by PE/EA=4/1, MS m/z = 232 $[M+H]^+$) and another stereoisomer **12-1b** (1.09 g, 4.7 mmol, yield 57.3%, obtained by PE/EA=2/1, MS m/z = 232 $[M+H]^+$). TLC developing agent PE/EA = 1/1: **12-1a,** $R_f$ = 0.3; **12-1b,** $R_f$ = 0.2.

**Step 2: Synthesis of 12-2a**

**12-1a** (746 mg, 3.63 mmol) was dissolved in isopropanol (20 mL), added with Raney Ni (70 mg, 3.23 mmol), replaced with hydrogen, heated to 70 °C and stirred overnight. After the reaction was completed, the mixture was filtered. The filtrate was concentrated to obtain the crude product (640 mg, 3.18 mmol, yield 87.6%), which was directly used for the next step without purification. MS m/z = 202 $[M+H]^+$.

**Step 3: Synthesis of 12-3a**

**12-2a** (640 mg, 3.18 mmol) was dissolved in THF (25 mL), added with TEA (386.13 mg, 3.82) dropwise, cooled to -10°C, and slowly added with a THF solution of triphosgene (350 mg, 1.18) dropwise. The reaction solution was stirred at this temperature for 30 min, then moved to room temperature and stirred for 2 h. After the reaction was completed, saturated sodium bicarbonate and ethyl acetate were added for extraction for three times. The organic phases were combined, washed with saturated brine, dried over anhydrous sodium sulfate and concentrated to obtain a crude product, which was purified by silica gel column (PE/EA= 1:1, v/v) to obtain **12-3a** (380 mg, 1.67 mmol, yield 52.5%). MS m/z = 228 $[M+H]^+$.

**Step 4: Synthesis of 12-4a**

**12-3a** (380 mg, 1.67 mmol) was dissolved in THF (10 mL), added with $LiAlH_4$ (76.15 mg, 2.01 mmol) at 0°C, and stirred at this temperature for 1 h. After the reaction was completed, sodium sulfate decahydrate was added in portions under ice bath until no bubbles were generated in the system. After filtration, the filtrate was concentrated to obtain a crude product (304 mg, 1.64 mmol, yield 98.2%), which was directly used for the next step. MS m/z = 186 $[M+H]^+$.

**Step 5: Synthesis of E2-a**

**12-4a** (304 mg, 1.64 mmol) was dissolved in a mixed solvent of DCM/DMSO = 12 mL/2 mL, cooled to 0 °C, added with solutions of DIPEA (848.48 mg, 6.57 mmol) and sulfur trioxide pyridine (1.04 g, 6.57 mmol) in DMSO (2 mL) dropwise sequentially, warmed to room temperature and stirred for 30 min. After the reaction was completed, the system was moved to an ice bath, quenched with 1 N HCl, diluted with water, and extracted three times with EA. The organic phases were combined, washed with saturated brine, dried over anhydrous sodium sulfate and concentrated to obtain a crude product, which was purified by silica gel column to obtain **E2-a** (single configuration, 167 mg, 0.91 mmol, yield 55.5%). $^1$H NMR (400 MHz, Chloroform-*d*) $\delta$ 9.67 (s, 1H), 6.42 (s, 1H), 4.11 (s, 2H), 2.40 - 2.30 (m, 1H), 2.03 - 1.94 (m, 2H), 1.87 - 1.79 (m, 2H), 1.73 - 1.56 (m, 4H). MS m/z = 184 $[M+H]^+$.

Referring to the synthesis method of **E2-a,** another intermediate **E2-b** of single configuration can be obtained by the same experimental operation. MS m/z = 184 $[M+H]^+$.

**Synthesis of Intermediates E3-a and E3-b**

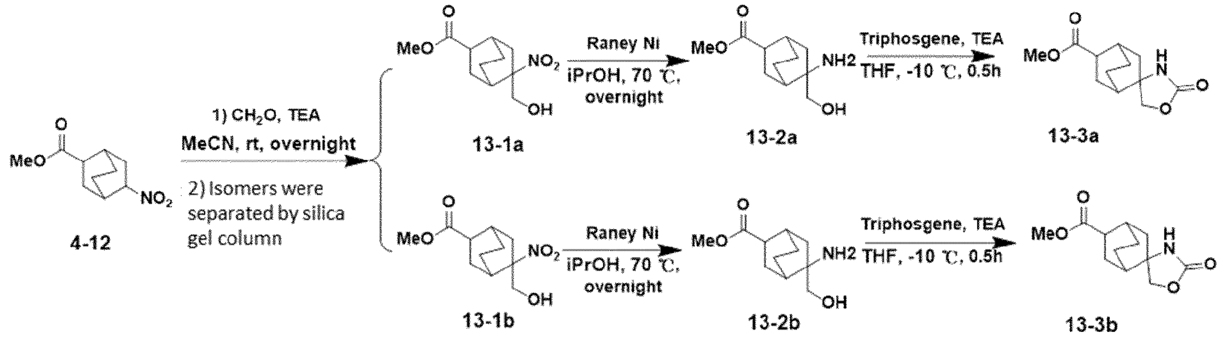

4-12
13-1a
13-2a
13-3a
13-1b
13-2b
13-3b

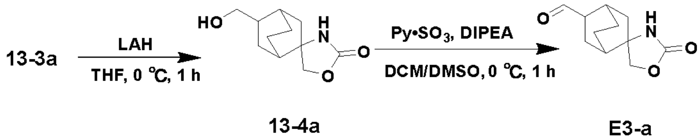

13-3a
13-4a
E3-a

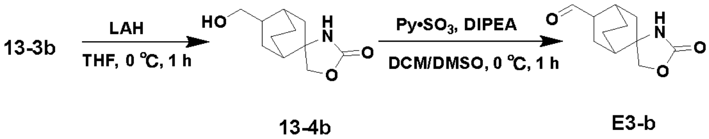

13-3b
13-4b
E3-b

Referring to the synthesis method of **E2-a,** intermediates **E3-a** (obtained by PE/EA = 5/1, MS m/z = 210 $[M+H]^+$, used directly in the next step without resolution) and **E3-b** (obtained by PE/EA = 3/1, MS m/z = 210 $[M+H]^+$, used directly in the next step without resolution) were obtained by the same experimental operation. TLC developing solvent PE/EA = 5/1: **E3-a,** $R_f$ = 0.4; **E3-b,** $R_f$ = 0.3.

## Synthesis of Intermediates E4-a and E4-b

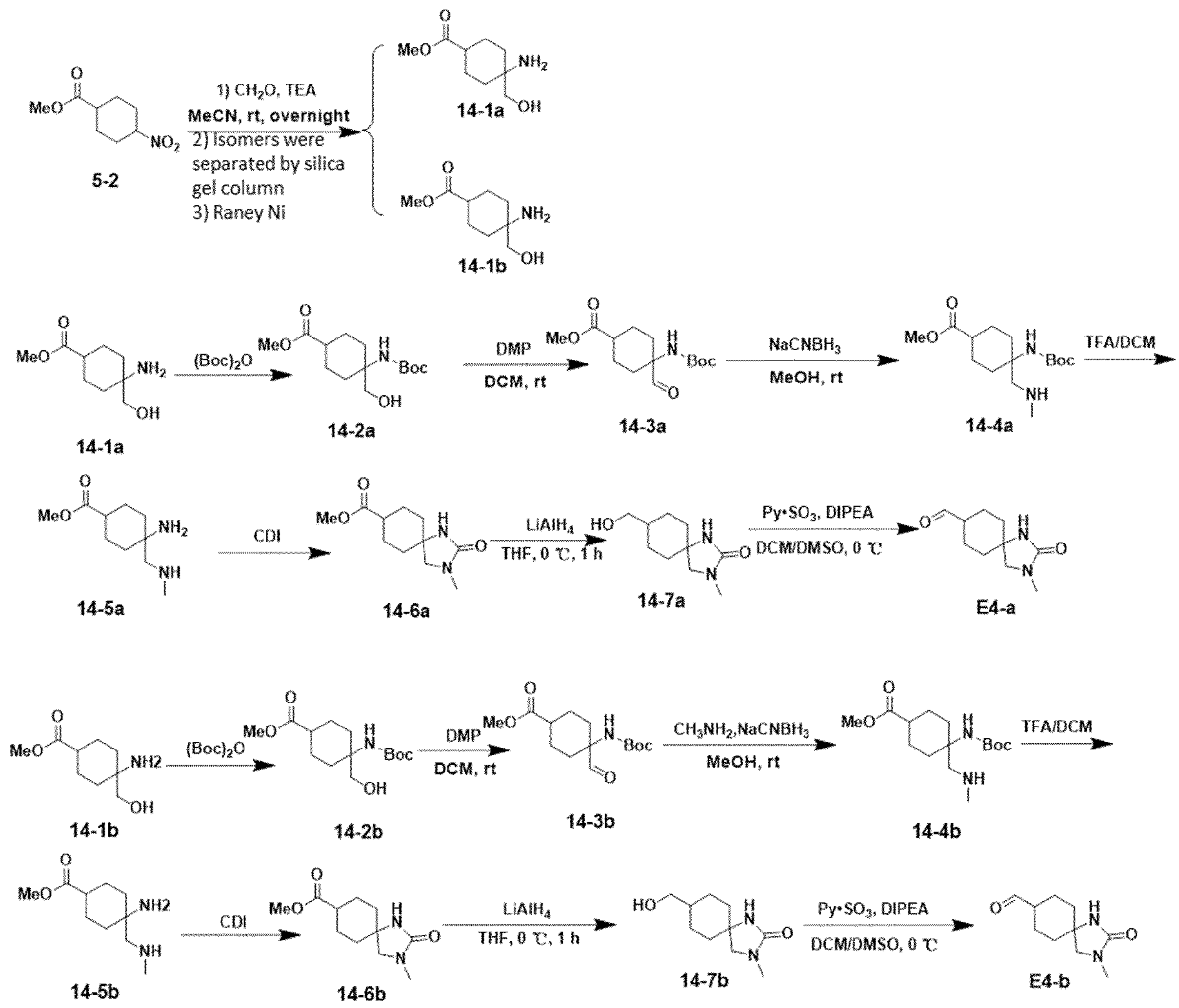


5-2, 14-1a, 14-1b, 14-2a, 14-3a, 14-4a, 14-5a, 14-6a, 14-7a, E4-a, 14-2b, 14-3b, 14-4b, 14-5b, 14-6b, 14-7b, E4-b

**Step 1: Synthesis of 14-2a**

**14-1a** (referring to the synthesis of **12-2a,** obtained from raw material **5-2,** 720 mg, 3.85 mmol, the isomer with less polarity during isomer resolution), $(Boc)_2O$ (1.01 g, 4.61 mmol) and TEA (778 mg, 7.67 mmol) were dissolved in acetonitrile (20 mL) and reacted at 60°C overnight under nitrogen protection. After the reaction was completed, the mixture was directly concentrated to obtain a crude product which was purified by silica gel column (PE/EA= 5:1-3:1, v/v) to obtain **14-2a** (650 mg, 2.26 mmol, yield 58.7%). MS m/z = 288 $[M+H]^+$.

**Step 2: Synthesis of 14-3a**

**14-2a** (650 mg, 2.26 mmol) was dissolved in DCM (15 mL), cooled to 0°C, added with Dess-Martin oxidant (1.92 g, 4.52 mmol) in portions under nitrogen protection, warmed to room temperature and stirred for 2 h. After the reaction was completed, water was added to quench the solution. After filtration, the filtrate was extracted three times with DCM. The organic phases were combined, washed with saturated brine, dried over anhydrous sodium sulfate and concentrated to obtain a crude product, which was purified by silica gel column (PE/EA= 10:1-8:1, v/v) to obtain **14-3a** (537 mg, 1.88 mmol, yield 83.18%). MS m/z = 286 $[M+H]^+$.

**Step 3: Synthesis of 14-4a**

**14-3a** (537 mg, 1.88 mmol) and methylamine hydrochloride (190.60 mg, 2.82 mmol) were dissolved in anhydrous methanol (10 mL), and then TEA was added to adjust the pH to about 8. The reaction mixture was stirred at room temperature for 0.5 h. Then, acetic acid was added to adjust the pH to 5, and sodium cyanoborohydride (237.13 mg, 3.76 mmol) was added and stirred at room temperature for 1.5 h. After the reaction was completed, saturated sodium bicarbonate solution was added to quench the solution, and ethyl acetate was added for extraction for three times. The

organic phases were combined, washed with saturated brine, dried over anhydrous sodium sulfate and concentrated to obtain a crude product, which was purified by silica gel column (DCM/MeOH = 50:1, v/v) to obtain **14-4a** (397 mg, 1.32 mmol, yield 70.21%). MS m/z = 301 $[M+H]^+$.

**Step 4: Synthesis of 14-5a**

At 0°C, **14-4a** (397 mg, 1.32 mmol) was dissolved in DCM (4 mL), slowly added with TFA (2 mL) dropwise, warmed to room temperature and stirred for 0.5 h. After the reaction was completed, the mixture was concentrated to obtain a crude product which was used directly in the next step without further purification. MS m/z = 201 $[M+H]^+$.

**Step 5: Synthesis of 14-6a**

The crude product obtained in the previous step was dissolved in THF (10 mL), cooled to 0°C, added with CDI (285.37 mg, 1.98 mmol) under nitrogen protection, then warmed to room temperature and stirred for 1 h. After the reaction was completed, saturated sodium bicarbonate solution was added to quench the solution, and ethyl acetate was added for extraction for three times. The organic phases were combined, washed with saturated brine, dried over anhydrous sodium sulfate and concentrated to obtain a crude product, which was purified by silica gel column (DCM/MeOH = 60:1, v/v) to obtain **14-6a** (190 mg, 0.84 mmol, yield 63.6%). MS m/z = 227 $[M+H]^+$.

**Step 6: Synthesis of 14-7a**

**14-6a** (190 mg, 0.84 mmol) was dissolved in THF (5 mL), added with $LiAH_4$ (41.43 mg, 1.09 mmol) at 0°C, and stirred at this temperature for 1 h. After the reaction was completed, sodium sulfate decahydrate was added in portions under ice bath until no bubbles were generated in the system. After filtration, the filtrate was concentrated to obtain a crude product (120 mg, 0.6 mmol), which was directly used for the next step. MS m/z = 199 $[M+H]^+$.

**Step 7: Synthesis of E4-a**

**14-7a** (120 mg, 0.6 mmol) was dissolved in a mixed solvent of DCM/DMSO = 4 mL/0.5 mL, cooled to 0°C, added with solutions of DIPEA (312.9 mg, 2.24 mmol) and pyridine sulfur trioxide (385.43 mg, 2.42 mmol) in DMSO (0.5 mL) dropwise sequentially, warmed to room temperature and stirred for 30 min. After the reaction was completed, the system was moved to an ice bath, quenched with 1 N HCl, diluted with water, and extracted three times with EA. The organic phases were combined, washed with saturated brine, dried over anhydrous sodium sulfate and concentrated to obtain a crude product, which was purified by silica gel column to obtain one of the single isomers **E4-a** (35 mg, 0.178 mmol, yield 29.7%). MS m/z = 197 $[M+H]^+$.

Similarly, referring to the synthesis method of **E4-a,** another intermediate **E4-b** of single configuration can be obtained by the same experimental operation. MS m/z = 197 $[M+H]^+$.

**Synthesis of Example 1**

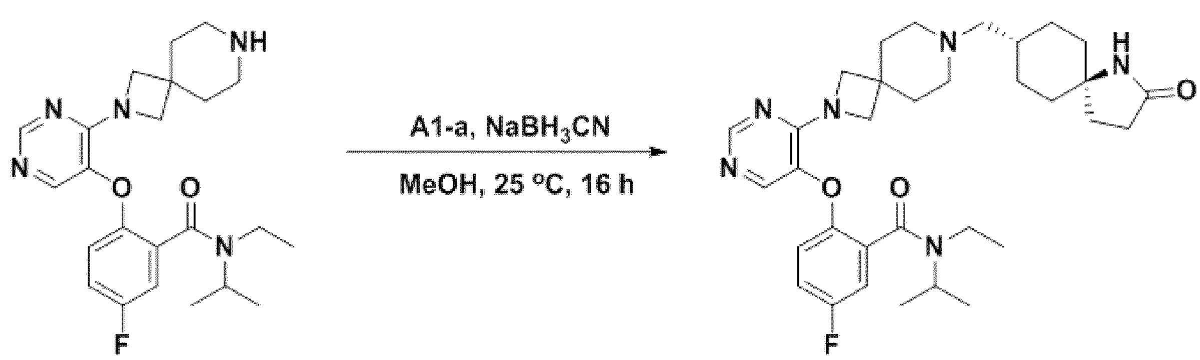


N1

Example 1

**N1** (268.92 mg, 629.03 μmol) was added to a solution of **A1-a** (95 mg, 524.19 μmol) in MeOH (10 mL), and the mixture was stirred at room temperature for 1 h. AcOH was added to adjust the pH to 4-5, and then $NaBH_3CN$ (49.54

mg, 786.29 μmol) was added. The mixture was stirred at room temperature for 16 h. After the reaction was completed, the mixture was separated and purified by Pre-HPLC (10 mM $NH_4HCO_3$ aqueous solution/acetonitrile system) to obtain **Example 1** compound (133.16 mg, 220.16 μmol, 42.00% yield, 98% purity) as a white solid. MS m/z = 593 $[M+H]^+$. NMR spectrum $^1H$ NMR (600 MHz, Methanol-d4) δ 8.23 (d, J = 9.6 Hz, 1H), 7.72 (d, J = 24.6 Hz, 1H), 7.23 - 7.14 (m, 2H), 6.99 (dd, J = 9.0, 4.2 Hz, 1H), 4.04 - 3.88 (m, 4H), 3.52 (dd, J = 13.7, 7.0 Hz, 1H), 3.36 (dd, J = 13.7, 7.0 Hz, 1H), 3.26 (q, J = 7.1 Hz, 1H), 2.35 (dd, J = 8.6, 7.5 Hz, 5H), 2.16 (d, J = 6.8 Hz, 2H), 1.96 (t, J = 8.1 Hz, 2H), 1.84 - 1.76 (m, 6H), 1.70 (d, J = 12.9 Hz, 2H), 1.54 (td, J = 13.3, 3.9 Hz, 3H), 1.31 (d, J = 6.5 Hz, 2H), 1.23 (t, J = 7.0 Hz, 3H), 1.18 (d, J = 6.7 Hz, 2H), 1.15 (d, J = 6.7 Hz, 2H), 1.13 - 1.05 (m, 3H).

**Synthesis of Example 5**

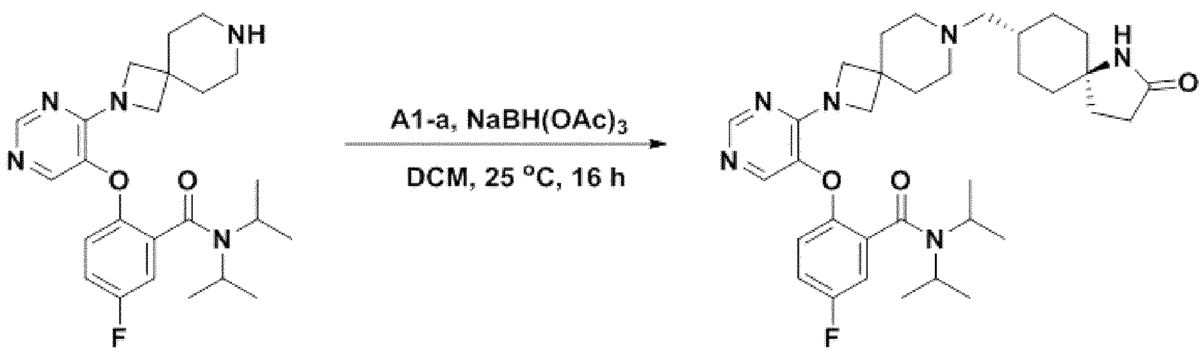


N1

Example 5

**N2** (3.95 g, 8.94 mmol) was added to a solution of **A1-a** (1.62 g, 8.94 mmol) in DCM (30 mL), and the mixture was stirred at room temperature for 1 h. AcOH was added to adjust the pH to 4-5, and then $NaBH(OAc)_3$ (5.68 g, 26.82 m mol) was added. The mixture was stirred at room temperature for 16 h. After the reaction was completed, the mixture was separated and purified by Pre-HPLC (10 mM $NH_4HCO_3$ aqueous solution/acetonitrile system) to obtain **Example 5** compound (2.14 g, 3.53 mmol, 39.46% yield, 98% purity) as a white solid. MS m/z = 607 $[M+H]^+$. NMR spectrum $^1H$ NMR (600 MHz, $CDCl_3$) δ 8.38 (s, 1H), 7.77 (s, 1H), 7.00 (d, *J* = 7.6 Hz, 2H), 6.87 - 6.70 (m, 1H), 5.78 (s, 1H), 3.97 (s, 2H), 3.88 (d, *J* = 9.2 Hz, 2H), 3.84 - 3.76 (m, 1H), 3.56 - 3.45 (m, 1H), 2.39 (t, *J* = 8.1 Hz, 3H), 2.28 (d, *J*= 15.3 Hz, 3H), 2.11 (d, *J* = 6.7 Hz, 2H), 1.94 (t, *J* = 8.1 Hz, 2H), 1.83 (s, 1H), 1.76 (s, 9H), 1.56 (s, 3H), 1.50 (d, *J* = 6.8 Hz, 3H), 1.45 (td, *J* = 12.8, 3.2 Hz, 3H), 1.15 *(d, J* = 6.6 Hz, 3H), 1.11 (d, *J* = 6.7 Hz, 3H), 1.04 (dd, *J* = 24.3, 11.4 Hz, 2H).

**Synthesis of Example 7**

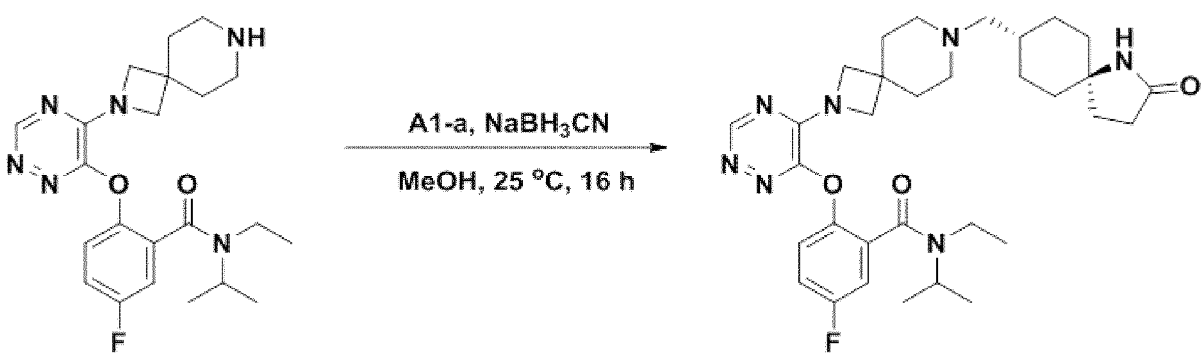


M1

Example 7

**M1** (40 mg, 93.35 μmol) was added to a solution of **Al-a** (16.92 mg, 93.35 μmol) in MeOH (5 mL), and the mixture was stirred at room temperature for 1 h. AcOH was added to adjust the pH to 4-5, and then $NaBH_3CN$ (8.82 mg, 140.02 μmol) was added. The mixture was stirred at room temperature for 16 h. After the reaction was completed,

the mixture was separated and purified by Pre-HPLC (10 mM $NH_4HCO_3$ aqueous solution/acetonitrile system) to obtain **Example** 7 compound (40.42 mg, 66.55 μmol, 71% yield, 97% purity) as a white solid. MS m/z = 594 $[M+H]^+$. NMR spectrum $^1H$ NMR (600 MHz, Methanol-d4) δ 8.38 (d, J = 1.8 Hz, 1H), 7.44 - 7.38 (m, 1H), 7.27 (tt, J = 8.5, 4.2 Hz, 1H), 7.21 (ddd, J = 14.9, 8.0, 3.1 Hz, 1H), 4.36 (d, J = 24.3 Hz, 2H), 3.98 - 3.86 (m, 2H), 3.81 (p, J = 6.6 Hz, 1H), 3.53 - 3.46 (m, 1H), 3.26 - 3.20 (m, 1H), 2.36 (t, J = 8.1 Hz, 5H), 2.18 (d, J = 6.8 Hz, 2H), 1.97 (t, J = 8.1 Hz, 2H), 1.87 (d, J = 5.2 Hz, 4H), 1.83 - 1.76 (m, 2H), 1.75 - 1.68 (m, 2H), 1.54 (td, J = 13.3, 3.8 Hz, 3H), 1.21 (d, J = 6.8 Hz, 2H), 1.15 (d, J = 7.0 Hz, 5H), 1.12 - 1.04 (m, 3H), 0.86 - 0.75 (m, 2H).

**Synthesis of Example 19**

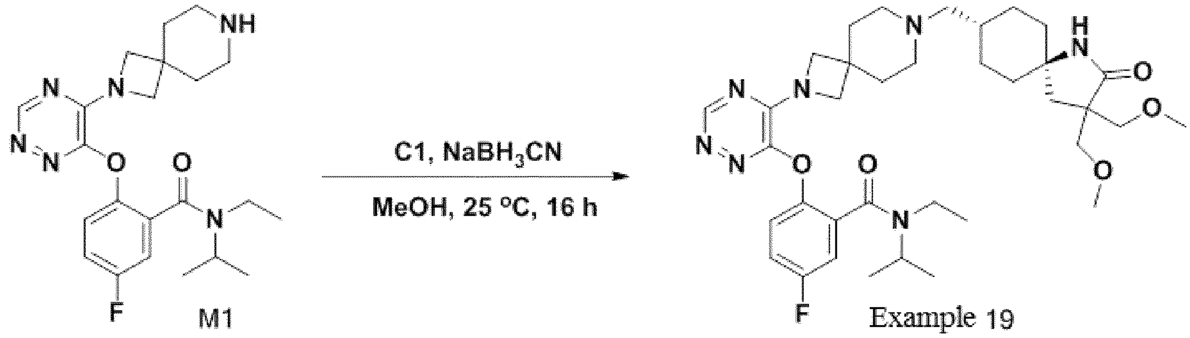


M1 Example 19

**M1** (26.2 mg, 61.26 μmol) was added to a solution of **C1** (15 mg, 55.59 μmol) in MeOH (3 mL), and the mixture was stirred at room temperature for 1 h. AcOH was then added to adjust the pH to 4-5, and $NaBH_3CN$ (38.3 mg, 610.1 μmol) was added. The mixture was stirred at room temperature for 16 h. After the reaction was completed, the mixture was separated and purified by Pre-HPLC (10 mM $NH_4HCO_3$ aqueous solution/acetonitrile system) to obtain **Example 20** compound (200 mg, 287.4 μmol, 70.66% yield, 98% purity) as a white solid. MS m/z = 696 $[M+H]^+$.

**Synthesis of Example 20**

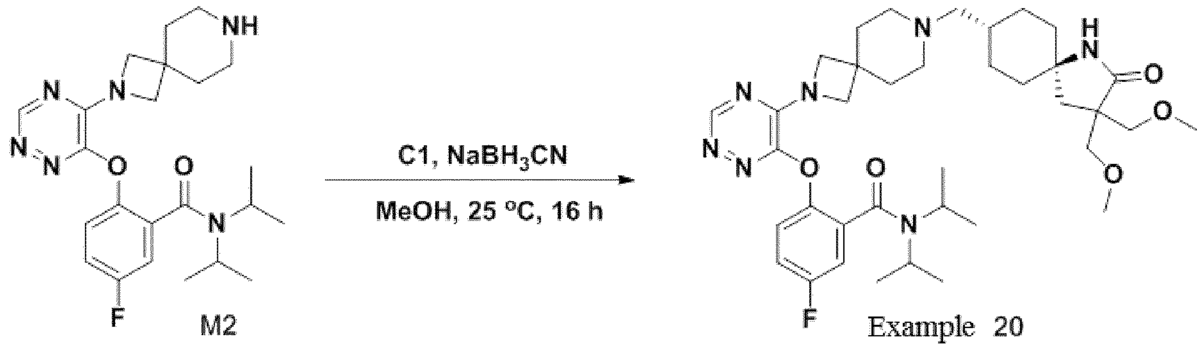


M2 Example 20

**M2** (180 mg, 406.8 μmol) was added to a solution of **C1** (98.6 mg, 366.1 μmol) in MeOH (5 mL), and the mixture was stirred at room temperature for 1 h. AcOH was then added to adjust the pH to 4-5, and $NaBH_3CN$ (38.3 mg, 610.1 μmol) was added. The mixture was stirred at room temperature for 16 h. After the reaction was completed, the mixture was separated and purified by Pre-HPLC (10 mM $NH_4HCO_3$ aqueous solution/acetonitrile system) to obtain **Example 20** compound (200 mg, 287.4 μmol, 70.66% yield, 98% purity) as a white solid. MS m/z = 696 $[M+H]^+$. NMR spectrum $^1H$ NMR (400 MHz, Chloroform-d) δ 8.46 (s, 1H), 7.26 - 7.23 (m, 1H), 7.10 (ddd, J = 9.1, 7.9, 3.0 Hz, 1H), 6.96 (dd, J = 7.8, 3.0 Hz, 1H), 5.75 (s, 1H), 4.47 (d, J = 10.3 Hz, 1H), 4.28 (d, J = 10.3 Hz, 1H), 3.94 - 3.83 (m, 2H), 3.79 (p, J = 6.6 Hz, 1H), 3.46 (d, J = 8.9 Hz, 2H), 3.43 - 3.36 (m, 1H), 3.33 (s, 7H), 3.30 (s, 1H), 2.32 (s, 3H), 2.13 - 2.02 (m, 4H), 1.90 - 1.64 (m, 9H), 1.50 (d, J = 6.8 Hz, 3H), 1.37 (dd, J = 12.9, 8.7 Hz, 6H), 1.16 - 0.95 (m, 5H), 0.72 (d, J = 6.6 Hz, 3H).

Synthesis of Example 23

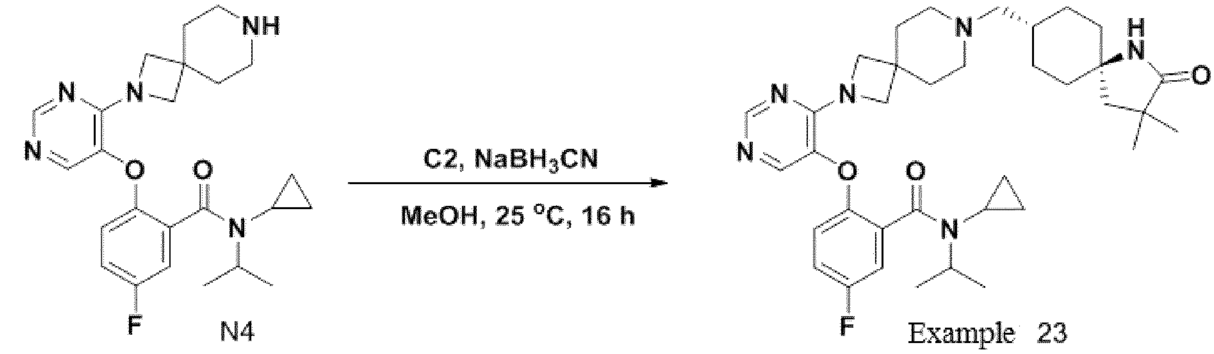


N4 Example 23

N4 (46 mg, 104.6 μmol) was added to a solution of **C2** (21.9 mg, 104.6 μmol) in MeOH (5 mL), and the mixture was stirred at room temperature for 1 h. Then AcOH was added to adjust the pH to 4-5, and $NaBH_3CN$ (9.85 mg, 156.9 μmol) was added. The mixture was stirred at room temperature for 16 h. After the reaction was completed, the mixture was separated and purified by Pre-HPLC (10 mM $NH_4HCO_3$ aqueous solution/acetonitrile system) to obtain **Example 23** (29 mg, 45.83 μmol, 43% yield, 98% purity) as a white solid. MS m/z = 633 $[M+H]^+$. NMR spectrum $^1H$ NMR (600 MHz, $CDCl_3$) δ 8.38 (s, 1H), 7.80 (s, 1H), 7.00 (s, 2H), 6.73 (d, $J$ = 5.1 Hz, 1H), 5.43 (s, 1H), 4.53 (s, 1H), 3.86 (s, 4H), 2.56 (s, 1H), 2.25 (s, 3H), 2.07 (d, $J$ = 4.8 Hz, 2H), 1.86 (s, 2H), 1.73 (s, 11H), 1.43 (s, 3H), 1.35 (d, $J$ = 6.4 Hz, 6H), 1.21 (s, 7H), 1.01 (d, $J$ = 11.6 Hz, 2H), 0.56 (s, 4H).

**Synthesis of Example 26**

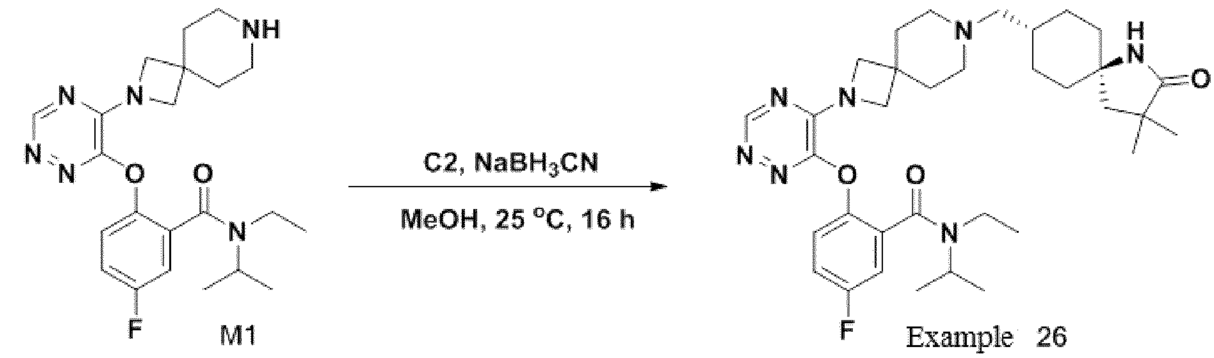


M1 Example 26

**M1** (43 mg, 100.34 μmol) was added to a solution of **C2** (21 mg, 100.34 μmol) in MeOH (3 mL), and the mixture was stirred at room temperature for 1 h. AcOH was then added to adjust the pH to 4-5, and $NaBH_3CN$ (9.46 mg, 150.51 μmol) was added. The mixture was stirred at room temperature for 16 h. After the reaction was completed, the mixture was separated and purified by Pre-HPLC (10 mM $NH_4HCO_3$ aqueous solution/acetonitrile system) to obtain **Example 26** (12.63 mg, 20.29 μmol, 20.22% yield, 99% purity) as a white solid. MS m/z = 622 $[M+H]^+$. NMR spectrum $^1H$ NMR (400 MHz, Methanol-$d_4$) δ 8.38 (s, 1H), 7.45 - 7.36 (m, 1H), 7.33 - 7.16 (m, 2H), 4.51 - 4.14 (m, 2H), 4.02 - 3.70 (m, 3H), 3.61 - 3.39 (m, 1H), 3.28 - 3.18 (m, 1H), 2.59 - 2.28 (m, 4H), 2.17 (d, $J$ = 6.8 Hz, 2H), 1.93 - 1.83 (m, 6H), 1.83 - 1.75 (m, 2H), 1.71 - 1.63 (m, 2H), 1.63 - 1.44 (m, 3H), 1.25 - 1.01 (m, 15H), 0.88 - 0.76 (m, 2H).

**Synthesis of Example 38-2**

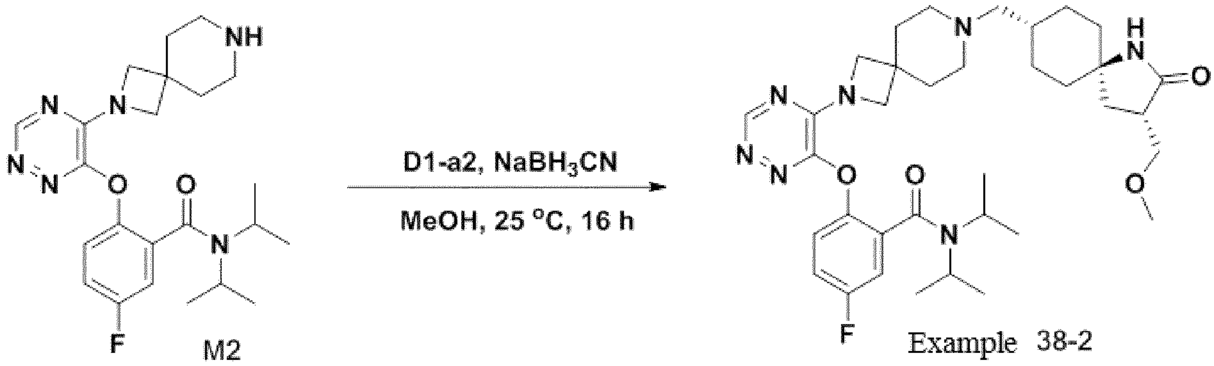


M2

Example 38-2

**M2** (66.38 mg, 150 μmol) was added to a solution of **D1-a2** (33.79 mg, 150 μmol) in MeOH (2 mL), and the mixture was stirred at room temperature for 1 h. AcOH was then added to adjust the pH to 4-5, and $NaBH_3CN$ (14.14 mg, 225 μmol) was added. The mixture was stirred at room temperature for 16 h. After the reaction was completed, the mixture was separated and purified by Pre-HPLC (10 mM $NH_4HCO_3$ aqueous solution/acetonitrile system) to obtain **Example 38-2** (42.76 mg, 63.63 μmol, 42.42% yield, 97% purity) as a white solid. MS m/z = 652 $[M+H]^+$. NMR spectrum $^1H$ NMR (400 MHz, Methanol-$d_4$) δ 8.38 (s, 1H), 7.39 (dd, *J* = 9.1, 4.5 Hz, 1H), 7.25 (ddd, *J* = 9.1, 8.1, 3.0 Hz, 1H), 7.16 (dd, *J* = 8.0, 3.0 Hz, 1H), 4.48 - 4.31 (m, 2H), 3.92 (q, *J* = 10.5 Hz, 2H), 3.75 (p, *J* = 6.6 Hz, 1H), 3.63 - 3.47 (m, 3H), 3.329 (s, 3H), 2.78 - 2.69 (m, 1H), 2.58 - 2.11 (m, 7H), 1.92 - 1.64 (m, 9H), 1.63 - 1.35 (m, 9H), 1.21 - 1.00 (m, 5H), 0.75 (d, *J* = 6.6 Hz, 3H).

**Synthesis of Example 41-1**

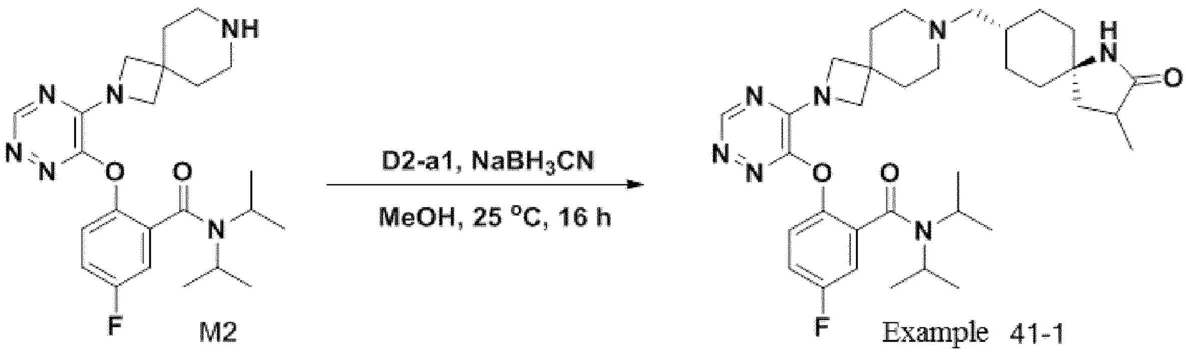


M2

Example 41-1

**M2** (44.40 mg, 100.34 μmol) was added to a solution **of D2-a1** (19.59 mg, 100.34 μmol) in MeOH (3 mL), and the mixture was stirred at room temperature for 1 h. AcOH was then added to adjust the pH to 4-5, and $NaBH_3CN$ (9.46 mg, 150.51 μmol) was added. The mixture was stirred at room temperature for 16 h. After the reaction was completed, the mixture was separated and purified by Pre-HPLC (10 mM $NH_4HCO_3$ aqueous solution/acetonitrile system) to obtain **Example 41-1** (25.06 mg, 40.26 μmol, 40.13% yield, 99% purity) as a white solid. MS m/z = 622 $[M+H]^+$. NMR spectrum $^1H$ NMR (400 MHz, Methanol-$d_4$) δ 8.38 (s, 1H), 7.39 (dd, *J* = 9.0, 4.5 Hz, 1H), 7.25 (ddd, *J* = 9.0, 8.0, 3.0 Hz, 1H), 7.16 (dd, *J* = 8.0, 3.0 Hz, 1H), 4.49 - 4.29 (m, 2H), 3.92 (q, *J* = 10.5 Hz, 2H), 3.75 (hept, *J* = 6.6 Hz, 1H), 3.54 (hept, *J* = 6.9 Hz, 1H), 2.65 - 2.22 (m, 6H), 2.17 (d, *J* = 6.9 Hz, 2H), 1.91-1.69 (m, 7H), 1.71-1.58 (m, 2H), 1.58 - -1.31 (m, 9H), 1.24-0.99 (m, 8H), 0.75 (d, *J* = 6.6 Hz, 3H).

**Synthesis of Example 41-2**

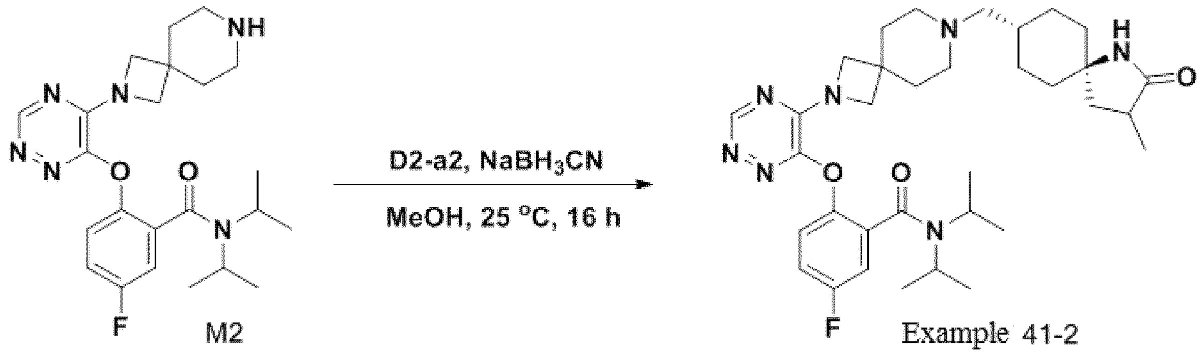


M2 Example 41-2

**M2** (44.40 mg, 100.34 μmol) was added to a solution of **D2-a2** (19.59 mg, 100.34 μmol) in MeOH (3 mL), and the mixture was stirred at room temperature for 1 h. AcOH was then added to adjust the pH to 4-5, and $NaBH_3CN$ (9.46 mg, 150.51 μmol) was added. The mixture was stirred at room temperature for 16 h. After the reaction was completed, the mixture was separated and purified by Pre-HPLC (10 mM $NH_4HCO_3$ aqueous solution/acetonitrile system) to obtain **Example 41-2** (20.57 mg, 33.05 μmol, 32.94% yield, 99% purity) as a white solid. MS m/z = 622 $[M+H]^+$. NMR spectrum $^1$H NMR (400 MHz, Methanol-$d_4$) δ 8.38 (s, 1H), 7.39 (dd, *J* = 9.0, 4.5 Hz, 1H), 7.25 (ddd, *J* = 9.0, 8.0, 3.0 Hz, 1H), 7.16 (dd, *J* = 8.0, 3.0 Hz, 1H), 4.49 - 4.29 (m, 2H), 3.92 (q, *J* = 10.5 Hz, 2H), 3.75 (hept, *J* = 6.6 Hz, 1H), 3.54 (hept, *J* = 6.9 Hz, 1H), 2.65 - 2.22 (m, 2H), 2.17 (d, *J* = 6.9 Hz, 2H), 1.91-1.69 (m, 7H), 1.63 (dd, *J* = 11.9, 3.4 Hz, 2H), 1.58 - 1.31 (m, 9H), 1.24 - 0.99 (m, 8H), 0.75 (d, *J* = 6.6 Hz, 3H).

Referring to the synthesis method of Example 1, the following example compounds were obtained by reacting raw material 1 and raw material 2:

| Raw material 1 | Raw material 2 | Example | Characterization data | Yield and/o r SFC retention time |
|---|---|---|---|---|
| N1 | A1-b | 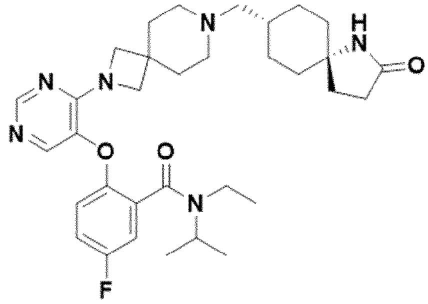 Example 2 | $^1$H NMR (600 MHz, Methanol-$d_4$) δ 8.52 (d, *J* = 7.3 Hz, 1H), 7.86 (s, 1H), 7.29 (tdd, *J* = 13.0, 6.5, 2.6 Hz, 3H), 4.24 (d, *J* = 69.8 Hz, 4H), 3.99 - 3.80 (m, 1H), 3.59 (d, *J* = 12.5 Hz, 2H), 3.51 - 3.38 (m, 2H), 3.01 (d, *J* = 7.0 Hz, 4H), 2.37 (t, *J* = 8.1 Hz, 2H), 2.26 (d, *J* = 14.0 Hz, 2H), 2.11 (s, 2H), 1.92 (q, *J* = 8.4 Hz, 3H), 1.77 (q, *J* = 9.6, 6.9 Hz, 4H), 1.58 (td, *J* = 14.3, 13.6, 4.1 Hz, 2H), 1.38 - 1.07 (m, 11H). MS m/z = 593 $[M+H]^+$ | 27.5% yield |

(continued)

| Raw material 1 | Raw material 2 | Example | Characterization data | Yield and/o r SFC retention time |
|---|---|---|---|---|
| N1 | B1-a | 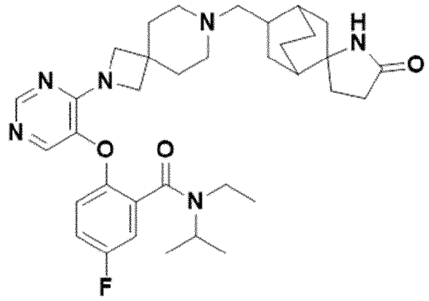 Example 3 | $^1$H NMR (600 MHz, Methanol-$d_4$) δ 8.26 - 8.21 (m, 1H), 7.77 - 7.69 (m, 1H), 7.24 - 7.14 (m, 2H), 7.05 - 6.95 (m, 1H), 4.10 - 3.83 (m, 5H), 3.52 (dq, *J* = 13.9, 7.0 Hz, 1H), 3.36 (dq, *J* = 14.0, 7.0 Hz, 1H), 2.52 - 2.12 (m, 9H), 1.93 - 1.71 (m, 9H), 1.68 - 1.46 (m, 6H), 1.37 - 1.06 (m, 10H). MS m/z = 619 [M+H]$^+$ | 28.1% yield (conta ining two isome rs) |
| | | **Example 3-1** | $^1$H NMR (400 MHz, Methanol-*d*4) δ 8.26 - 8.19 (m, 1H), 7.77 - 7.68 (m, 1H), 7.23 - 7.15 (m, 2H), 7.05 - 6.94 (m, 1H), 4.08 - 3.83 (m, 5H), 3.52 (dq, J = 14.1, 7.1 Hz, 1H), 3.42 - 3.32 (m, 1H), 2.48 - 2.14 (m, 10H), 1.92 - 1.72 (m, 11H), 1.68 - 1.46 (m, 6H), 1.34 - 1.05 (m, 12H). | P1 was resolv ed from **Exam ple 3;** SFC peak retenti on time: 2.807 min |
| | | **Example 3-2** | $^1$H NMR (400 MHz, Methanol-*d*4) δ 8.26 - 8.19 (m, 1H), 7.77 - 7.68 (m, 1H), 7.25 - 7.13 (m, 2H), 7.05 - 6.94 (m, 1H), 4.08 - 3.80 (m, 5H), 3.52 (dq, J = 14.1, 7.1 Hz, 1H), 3.37 (dt, J = 13.8, 7.0 Hz, 1H), 2.49 - 2.13 (m, 10H), 1.93 -1.71 (m, 11H), 1.68 -1.45 (m, 6H), 1.34 - 1.05 (m, 12H). | P2 was resolv ed from **Exam ple 3;** SFC peak retenti on time: 3.623 min |

(continued)

| Raw material 1 | Raw material 2 | Example | Characterization data | Yield and/o r SFC retention time |
|---|---|---|---|---|
| N1 | B1-b | 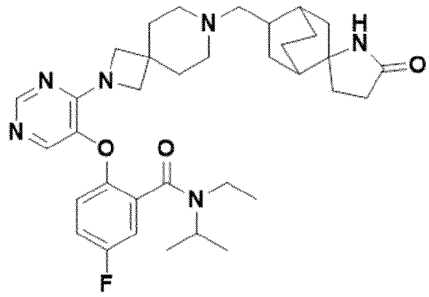 Example 4 | $^1$H NMR (600 MHz, Methanol-$d_4$) δ 8.26 - 8.19 (m, 1H), 7.77 - 7.69 (m, 1H), 7.23 - 7.15 (m, 2H), 7.05 - 6.94 (m, 1H), 4.08 - 3.83 (m, 5H), 3.52 (dq, *J* = 13.9, 7.0 Hz, 1H), 3.36 (dq, *J* = 13.9, 7.0 Hz, 1H), 2.54 - 2.15 (m, 9H), 1.96 - 1.62 (m, 11H), 1.56 - 1.43 (m, 4H), 1.38 - 1.05 (m, 10H). MS m/z = 619 $[M+H]^+$ | 29% yield (conta ining two isome rs) |
| | | **Example 4-1** | $^1$H NMR (400 MHz, Methanol-*d4*) δ 8.26 - 8.20 (m, 1H), 7.77 - 7.67 (m, 1H), 7.24 - 7.13 (m, 2H), 7.05 - 6.94 (m, 1H), 4.11 - 3.79 (m, 5H), 3.51 (dt, J = 14.4, 7.2 Hz, 1H), 3.37 (dt, J = 13.7, 7.0 Hz, 1H), 2.51 - 2.14 (m, 10H), 1.97 - 1.62 (m, 13H), 1.51 (dd, J = 13.9, 9.9 Hz, 5H), 1.39 - 1.04 (m, 12H). | P1 was resolv ed from Exam ple **4;** SFC peak retenti on time: 2.845 min |
| | | **Example 4-2** | $^1$H NMR (400 MHz, Methanol-*d4*) δ 8.26 - 8.19 (m, 1H), 7.77 - 7.67 (m, 1H), 7.24 - 7.11 (m, 2H), 7.05 - 6.94 (m, 1H), 4.08 - 3.82 (m, 5H), 3.52 (dq, J = 14.0, 7.0 Hz, 1H), 3.41 - 3.32 (m, 1H), 2.48 - 2.15 (m, 10H), 1.99 - 1.61 (m, 13H), 1.58 - 1.44 (m, 5H), 1.42 - 1.03 (m, 12H). | P2 was resolv ed from Exam ple **4;** SFC peak retenti on time: 3.643 min |

(continued)

| Raw material 1 | Raw material 2 | Example | Characterization data | Yield and/o r SFC retention time |
|---|---|---|---|---|
| N2 | A1-a | 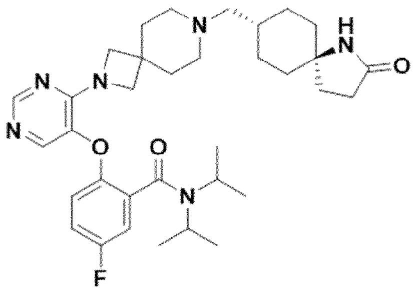 Example 5 | $^1$H NMR (600 MHz, $CDCl_3$) δ 8.38 (s, 1H), 7.77 (s, 1H), 7.00 (d, *J* = 7.6 Hz, 2H), 6.87 - 6.70 (m, 1H), 5.78 (s, 1H), 3.97 (s, 2H), 3.88 (d, *J* = 9.2 Hz, 2H), 3.84 - 3.76 (m, 1H), 3.56 - 3.45 (m, 1H), 2.39 (t, *J* = 8.1 Hz, 3H), 2.28 (d, *J* = 15.3 Hz, 3H), 2.11 (d, *J* = 6.7 Hz, 2H), 1.94 (t, *J* = 8.1 Hz, 2H), 1.83 (s, 1H), 1.76 (s, 9H), 1.56 (s, 3H), 1.50 (d, *J* = 6.8 Hz, 3H), 1.45 (td, *J* = 12.8, 3.2 Hz, 3H), 1.15 (d, *J* = 6.6 Hz, 3H), 1.11 (d, *J* = 6.7 Hz, 3H), 1.04 (dd, *J* = 24.3, 11.4 Hz, 2H). MS m/z = 607 $[M+H]^+$。 | 28.6% |
| N3 | A1-a | 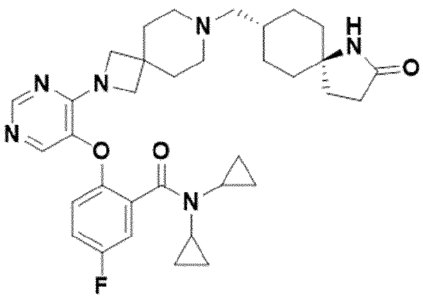 Example 6 | $^1$H NMR (400 MHz, Methanol-$d_4$) δ 8.24 (s, 1H), 7.69 (s, 1H), 7.25 - 7.15 (m, 2H), 6.95 (dd, *J* = 8.9, 4.2 Hz, 1H), 3.93 (s, 4H), 2.70 (s, 2H), 2.49 - 2.26 (m, 6H), 2.16 (d, *J* = 6.9 Hz, 2H), 1.96 (t, *J* = 8.1 Hz, 2H), 1.85 - 1.74 (m, 6H), 1.74 - 1.66 (m, 2H), 1.60 - 1.42 (m, 3H), 1.16 - 1.02 (m, 2H), 0.96 - 0.84 (m, 2H), 0.84 - 0.69 (m, 4H), 0.69 - 0.57 (m, 2H) MS m/z = 603 $[M+H]^+$ | 38.29 % |

(continued)

| Raw material 1 | Raw material 2 | Example | Characterization data | Yield and/o r SFC retention time |
|---|---|---|---|---|
| M1 | A1-a | 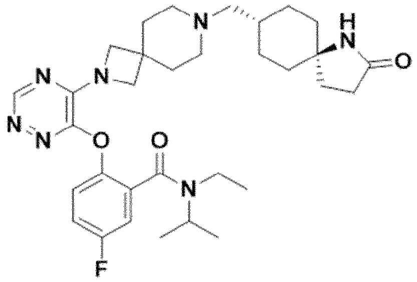 Example 7 | $^1$H NMR (600 MHz, Methanol-d4) δ 8.38 (d, J = 1.8 Hz, 1H), 7.44 - 7.38 (m, 1H), 7.27 (tt, J = 8.5, 4.2 Hz, 1H), 7.21 (ddd, J = 14.9, 8.0, 3.1 Hz, 1H), 4.36 (d, J = 24.3 Hz, 2H), 3.98 - 3.86 (m, 2H), 3.81 (p, J = 6.6 Hz, 1H), 3.53 - 3.46 (m, 1H), 3.26 - 3.20 (m, 1H), 2.36 (t, J = 8.1 Hz, 5H), 2.18 (d, J = 6.8 Hz, 2H), 1.97 (t, J = 8.1 Hz, 2H), 1.87 (d, J = 5.2 Hz, 4H), 1.83 - 1.76 (m, 2H), 1.75 - 1.68 (m, 2H), 1.54 (td, J = 13.3, 3.8 Hz, 3H), 1.21 (d, J = 6.8 Hz, 2H), 1.15 (d, J = 7.0 Hz, 5H), 1.12 - 1.04 (m, 3H), 0.86 - 0.75 (m, 2H).MS m/z = 594 [M+H]$^+$ | 71.29 % |
| M2 | A1-a | 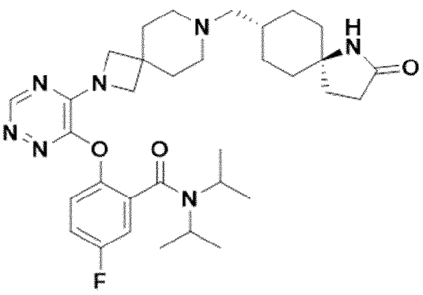 Example 8 | $^1$H NMR (400 MHz, Methanol-d4) δ 8.38 (s, 1H), 7.39 (dd, J = 9.1, 4.5 Hz, 1H), 7.25 (ddd, J = 9.1, 8.1, 3.1 Hz, 1H), 7.16 (dd, J = 8.0, 3.0 Hz, 1H), 4.50 - 4.28 (m, 2H), 3.92 (q, J = 10.5 Hz, 2H), 3.75 (p, J = 6.6 Hz, 1H), 3.55 (h, J = 7.1 Hz, 1H), 2.36 (dd, J = 8.7, 7.5 Hz, 6H), 2.17 (d, J = 6.9 Hz, 2H), 1.97 (t, J = 8.1 Hz, 2H), 1.91 - 1.75 (m, 6H), 1.71 (d, J = 12.9 Hz, 2H), 1.61 - 1.45 (m, 6H), 1.39 (d, J = 6.7 Hz, 3H), 1.20 - 1.01 (m, 5H), 0.75 (d, J = 6.6 Hz, 3H).MS m/z = 608 [M+H]$^+$ | 54% |

(continued)

| Raw material 1 | Raw material 2 | Example | Characterization data | Yield and/o r SFC retention time |
|---|---|---|---|---|
| M6 | A1-a | 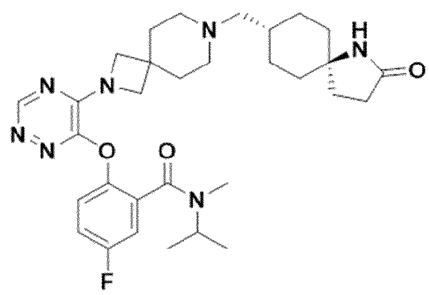 Example 9 | $^1$H NMR (600 MHz, $CDCl_3$) δ 8.45 (d, *J* = 15.3 Hz, 1H), 7.29 (dd, *J* = 9.0, 4.4 Hz, 1H), 7.23 (d, *J* = 4.4 Hz, 1H), 7.13 (s, 1H), 7.00 (d, *J* = 1.9 Hz, 1H), 5.56 (s, 1H), 4.91 - 4.58 (m, 1H), 4.52 - 4.15 (m, 2H), 3.87 (d, *J* = 6.6 Hz, 3H), 2.85 (s, 2H), 2.65 (s, 2H), 2.37 (s, 3H), 2.32 (s, 2H), 2.10 (d, *J* = 4.6 Hz, 2H), 1.93 (t, *J* = 8.0 Hz, 2H), 1.86 -1.70 (m, 8H), 1.65 (s, 1H), 1.44 (t, *J* = 11.4 Hz, 3H), 1.04 (dd, *J* = 35.3, 23.2 Hz, 7H), 0.80 (s, 2H) MS m/z = 580 $[M+H]^+$ | 18% |
| M7 | A1-a | 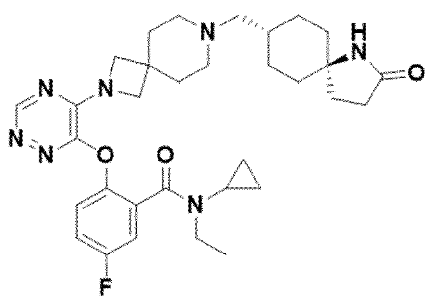 Example 10 | $^1$H NMR (400 MHz, Methanol-d4) δ 8.36 (s, 1H), 7.40 (dd, J = 9.0, 4.6 Hz, 1H), 7.28 (ddt, J = 17.1, 8.6, 4.4 Hz, 2H), 4.39 - 4.27 (m, 2H), 3.92 (s, 2H), 3.54 - 3.42 (m, 2H), 2.74 (td, J = 6.8, 3.5 Hz, 1H), 2.36 (dd, J = 8.7, 7.4 Hz, 6H), 2.18 (d, J = 6.9 Hz, 2H), 1.97 (t, J = 8.1 Hz, 2H), 1.92 - 1.65 (m, 9H), 1.54 (td, J = 13.1, 3.8 Hz, 3H), 1.11 (dt, J = 11.6, 4.6 Hz, 5H), 0.63 (dd, J = 26.7, 5.9 Hz, 3H). MS m/z = 592 $[M+H]^+$ | 53.58 % |

(continued)

| Raw material 1 | Raw material 2 | Example | Characterization data | Yield and/o r SFC retention time |
|---|---|---|---|---|
| M4 | A1-a | 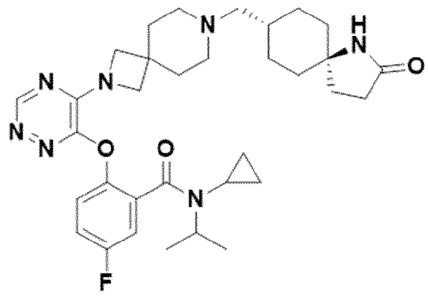<br>Example 11 | $^1$H NMR (600 MHz, Chloroform-*d*) δ 8.46 (s, 1H), 7.27 (s, 1H), 7.13 - 7.04 (m, 2H), 5.53 (s, 1H), 4.42 (s, 1H), 4.30 (s, 2H), 3.87 (s, 2H), 2.37 (t, *J* = 8.0 Hz, 4H), 2.10 (d, *J* = 7.0 Hz, 2H), 1.93 (t, *J* = 8.1 Hz, 2H), 1.86 - 1.70 (m, 9H), 1.64 (s, 2H), 1.43 (td, *J* = 13.1, 3.7 Hz, 3H), 1.24 (s, 6H), 1.03 (d, *J* = 12.7 Hz, 2H), 0.62 (d, *J* = 87.6 Hz, 4H).<br>MS m/z = 606 [M+H]$^+$ | 49.36 % |
| M3 | A1-a | 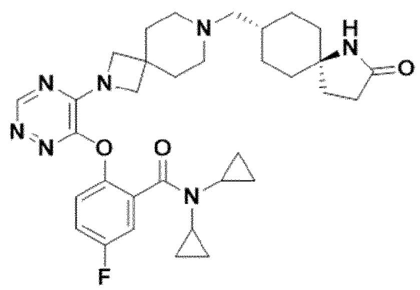<br>Example 12 | $^1$H NMR (400 MHz, Methanol-$d_4$) δ 8.38 (s, 1H), 7.38 (dd, *J* = 9.0, 4.5 Hz, 1H), 7.33 - 7.22 (m, 2H), 4.30 (s, 2H), 3.93 (s, 2H), 2.62 (s, 2H), 2.50 - 2.28 (m, 6H), 2.19 (d, *J* = 6.9 Hz, 2H), 1.97 (t, *J* = 8.1 Hz, 2H), 1.93 - 1.85 (m, 4H), 1.84 - 1.76 (m, 2H), 1.75 - 1.67 (m, 2H), 1.61 - 1.48 (m, 3H), 1.17 - 1.02 (m, 2H), 0.96 - 0.55 (m, 8H).<br>MS m/z = 604 [M+H]$^+$ | 29.41 % |

(continued)

| Raw material 1 | Raw material 2 | Example | Characterization data | Yield and/o r SFC retention time |
|---|---|---|---|---|
| N2 | B1-a | 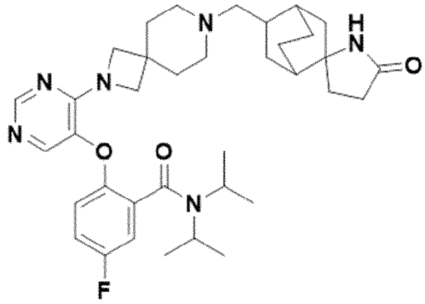<br>Example 13-1 | $^1$H NMR (600 MHz, $CDCl_3$) δ 8.39 (s, 1H), 7.80 (s, 1H), 7.03 - 6.94 (m, 2H), 6.76 (s, 1H), 3.92 (s, 3H), 3.78 (s, 1H), 3.50 (s, 2H), 2.39 (s, 2H), 2.32 - 2.24 (m, 2H), 2.13 (s, 2H), 1.78 (s, 7H), 1.62 (s, 10H), 1.53 (s, 6H), 1.47 (s, 6H), 1.25 (s, 12H), 1.14 (d, *J* = 6.5 Hz, 4H), 1.10 (s, 4H), 0.88 (s, 5H)<br>MS m/z = 633 $[M+H]^+$ | 55% (conta ining two isome rs) |
| | | **Example 13-1a** | $^1$H NMR (600 MHz, $CDCl_3$) δ 8.37 (s, 1H), 7.81 (d, *J* = 56.8 Hz, 1H), 6.97 (s, 2H), 6.76 (s, 1H), 5.82 (s, 1H), 3.91 (d, *J* = 37.8 Hz, 3H), 3.82 - 3.75 (m, 1H), 3.50 (s, 2H), 2.90 (s, 1H), 2.67 (s, 2H), 2.33 (s, 3H), 1.74 (s, 3H), 1.59 (s, 9H), 1.54 (d, *J* = 6.7 Hz, 5H), 1.48 (d, *J* = 6.6 Hz, 4H), 1.25 (s, 4H), 1.14 (d, *J* = 6.5 Hz, 5H), 1.11 (s, 3H), 0.91 - 0.82 (m, 3H).<br>MS m/z = 633 $[M+H]^+$ | P1 was resolv ed from Exam ple **13-1;** SFC peak retention time: 2.836 min |
| | | **Example 13-1b** | $^1$H NMR (600 MHz, $CDCl_3$) δ 8.37 (s, 1H), 7.81 (d, *J* = 56.8 Hz, 1H), 6.97 (s, 2H), 6.76 (s, 1H), 5.82 (s, 1H), 3.91 (d, *J* = 37.8 Hz, 3H), 3.82 - 3.75 (m, 1H), 3.50 (s, 2H), 2.90 (s, 1H), 2.67 (s, 2H), 2.33 (s, 3H), 1.74 (s, 3H), 1.59 (s, 9H), 1.54 (d, *J* = 6.7 Hz, 5H), 1.48 (d, *J* = 6.6 Hz, 4H), 1.25 (s, 4H), 1.14 (d, *J* = 6.5 Hz, 5H), 1.11 (s, 3H), 0.91 - 0.82 (m, 3H).<br>MS m/z = 633 $[M+H]^+$ | P2 was resolv ed from Exam ple **13-1;** SFC peak retenti on time: 3.922 min |

(continued)

| Raw material 1 | Raw material 2 | Example | Characterization data | Yield and/o r SFC retention time |
|---|---|---|---|---|
| N2 | B1-b | 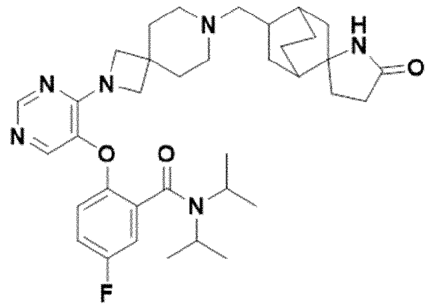 Example 13-2 | MS m/z = 633 [M+H]+ | 17% (conta ining two isome rs) |
| | | **Example 13-2a** | 1H NMR (600 MHz, $CDCl_3$) δ 8.38 (s, 1H), 7.78 (s, 1H), 6.99 (d, *J* = 7.8 Hz, 2H), 6.77 (s, 1H), 3.90 (s, 4H), 3.85 - 3.72 (m, 1H), 3.57 - 3.42 (m, 2H), 2.40 (dd, *J* = 16.8, 8.4 Hz, 2H), 2.30 (dd, *J* = 8.9, 4.9 Hz, 2H), 2.14 (d, *J* = 11.8 Hz, 2H), 1.77 (s, 5H), 1.63 (s, 7H), 1.54 (d, *J* = 6.7 Hz, 4H), 1.47 (d, *J* = 6.3 Hz, 5H), 1.25 (s, 7H), 1.14 (d, *J* = 6.6 Hz, 4H), 1.10 (d, *J* = 6.5 Hz, 3H), 0.90 - 0.81 (m, 4H). MS m/z = 633 [M+H]+ | P1 was resolv ed from Exam ple **13-2;** SFC peak retenti on time: 2.774 min |
| | | **Example 13-2b** | 1H NMR (600 MHz, $CDCl_3$) δ 8.37 (s, 1H), 7.81 (d, *J* = 56.8 Hz, 1H), 6.97 (s, 2H), 6.76 (s, 1H), 5.82 (s, 1H), 3.91 (d, *J* = 37.8 Hz, 3H), 3.82 - 3.75 (m, 1H), 3.50 (s, 2H), 2.90 (s, 1H), 2.67 (s, 2H), 2.33 (s, 3H), 1. 74 (s, 3H), 1. 59 (s, 9H), 1.54 (d, *J* = 6.7 Hz, 5H), 1.48 (d, *J* = 6.6 Hz, 4H), 1.25 (s, 4H), 1.14 (d, *J* = 6.5 Hz, 5H), 1.11 (s, 3H), 0.91 - 0.82 (m, 4H). MS m/z = 633 [M+H]+ | P1 was resolv ed from Exam ple **13-2;** SFC peak retenti on time: 3.953 min |

(continued)

| Raw material 1 | Raw material 2 | Example | Characterization data | Yield and/o r SFC retention time |
|---|---|---|---|---|
| | | 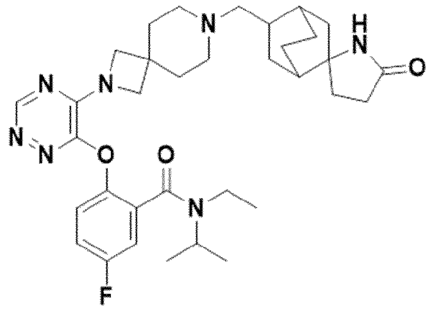 Example 14-1 | MS m/z = 620 $[M+H]^+$ | 33% (conta ining two isome rs) |
| M1 | B1-a | **Example 14-1a** | $^1$H NMR (400 MHz, Chloroform-d) δ 8.46 (d, J = 1.6 Hz, 1H), 7.25 (d, J = 4.5 Hz, 1H), 7.13 (ddt, J = 8.7, 7.1, 3.5 Hz, 1H), 7.02 (ddd, J = 13.5, 7.9, 3.0 Hz, 1H), 6.01 (s, 1H), 4.35 (d, J = 43.1 Hz, 2H), 3.88 (s, 3H), 3.58 - 3.41 (m, 1H), 3.19 - 3.05 (m, 1H), 2.41 - 2.28 (m, 5H), 2.22 (dt, J = 13.7, 6.7 Hz, 3H), 1.93 - 1.77 (m, 7H), 1.70 (d, J = 11.2 Hz, 5H), 1.56 (t, J = 8.9 Hz, 4H), 1.42 (t, J = 9.4 Hz, 1H), 1.14 (t, J = 6.9 Hz, 5H), 1.09 (d, J = 6.5 Hz, 2H), 1.04 (t, J = 7.1 Hz, 1H), 0.77 (d, J = 6.6 Hz, 2H). MS m/z = 620 $[M+H]^+$ | P1 was resolv ed from **14-1;** SFC peak retenti on time: 3.189 min |

(continued)

| Raw material 1 | Raw material 2 | Example | Characterization data | Yield and/o r SFC retention time |
|---|---|---|---|---|
| | | **Example 14-1b** | $^1$H NMR (400 MHz, Chloroform-d) δ 8.47 (s, 1H), 7.25 (d, J = 4.5 Hz, 1H), 7.18 - 7.08 (m, 1H), 7.02 (ddd, J = 13.5, 7.9, 3.0 Hz, 1H), 5.97 (s, 1H), 4.46 - 4.23 (m, 2H), 3.88 (s, 3H), 3.59 - 3.44 (m, 1H), 3.11 (dd, J = 12.9, 6.5 Hz, 1H), 2.33 (dd, J = 8.8, 6.9 Hz, 5H), 2.22 (dt, J = 13.8, 6.9 Hz, 3H), 1.90 - 1.75 (m, 7H), 1.73 - 1.62 (m, 5H), 1.56 (t, J = 8.0 Hz, 4H), 1.43 (dd, J = 21.5, 9.3 Hz, 1H), 1.14 (t, J = 6.8 Hz, 5H), 1.09 (d, J = 6.5 Hz, 2H), 1.04 (t, J = 7.1 Hz, 1H), 0.77 (d, J = 6.6 Hz, 2H). MS m/z = 620 [M+H]$^+$ | P2 was resolv ed from **14-1;** SFC peak retenti on time: 3.690 min |
| | | 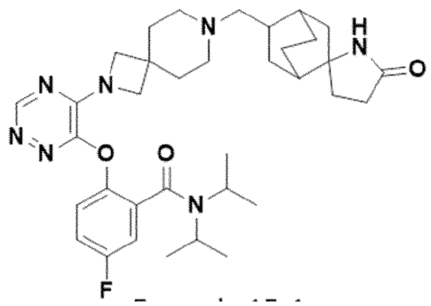 Example 15-1 | MS m/z = 634 [M+H]$^+$ | 61.8% (conta ining two isome rs) |

(continued)

| Raw material 1 | Raw material 2 | Example | Characterization data | Yield and/o r SFC retention time |
|---|---|---|---|---|
| M2 | B1-a | **Example 15-1a** | $^1$H NMR (400 MHz, Chloroform-d) δ 8.46 (s, 1H), 7.24 (d, J = 4.5 Hz, 1H), 7.11 (td, J = 8.6, 3.0 Hz, 1H), 6.96 (dd, J = 7.9, 3.0 Hz, 1H), 5.95 (s, 1H), 4.60 - 4.18 (m, 2H), 3.88 (s, 2H), 3.78 (q, J = 6.6 Hz, 1H), 3.39 (p, J = 6.8 Hz, 1H), 2.48 - 2.26 (m, 5H), 2.14 (m, 3H), 1.91 - 1.75 (m, 7H), 1.74 - 1.63 (m, 4H), 1.52 (dd, J = 16.7, 5.9 Hz, 7H), 1.38 (d, J = 6.8 Hz, 4H), 1.08 (d, J = 6.6 Hz, 4H), 0.71 (d, J = 6.5 Hz, 3H). MS m/z = 634 $[M+H]^+$ | P1 was resolv ed from **15-1;** SFC peak retenti on time: 2.611 min |
| | | **Example 15-1b** | $^1$H NMR (400 MHz, Chloroform-d) δ 8.46 (s, 1H), 7.26 - 7.23 (m, 1H), 7.11 (ddd, J = 9.0, 7.8, 3.1 Hz, 1H), 6.96 (dd, J = 7.9, 3.0 Hz, 1H), 5.99 (s, 1H), 4.61 - 4.15 (m, 2H), 3.88 (s, 2H), 3.79 (p, J = 6.6 Hz, 1H), 3.39 (p, J = 6.8 Hz, 1H), 2.33 (dd, J = 8.6, 6.7 Hz, 5H), 2.21 (d, J = 6.5 Hz, 3H), 1.92 - 1.74 (m, 7H), 1.68 (s, 4H), 1.49 (s, 7H), 1.38 (d, J = 6.8 Hz, 4H), 1.08 (d, J = 6.6 Hz, 4H), 0.71 (d, J = 6.5 Hz, 3H). MS m/z = 634 $[M+H]^+$ | P2 was resolv ed from **15-1;** SFC peak retenti on time: 2.993 min |

(continued)

| Raw material 1 | Raw material 2 | Example | Characterization data | Yield and/o r SFC retention time |
|---|---|---|---|---|
| M4 | B1-a | 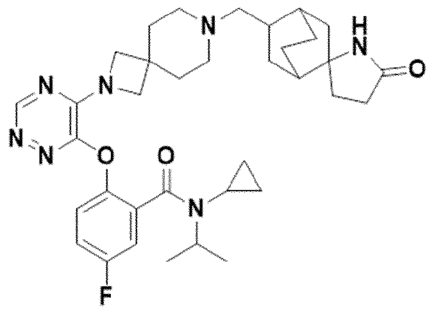<br>Example 16-1 | MS m/z = 632 [M+H]+ | 30% (conta ining two isome rs) |
| | | **Example 16-1a** | $^1$H NMR (400 MHz, Methanol-d4) δ 8.36 (s, 1H), 7.38(dd, J=9.1,4.5 Hz, 1H), 7.33 - 7.18 (m, 2H), 4.34 (s, 3H), 3.92 (s, 2H), 2.43 (s, 2H), 2.36 - 2.27 (m, 4H), 2.27 - 2.17 (m, 2H), 1.92 - 1.81 (m, 8H), 1.77 (td, J = 9.9, 5.2 Hz, 2H), 1.69 - 1.60 (m, 2H), 1.60 - 1.48 (m, 5H), 1.27 (d, J = 7.2 Hz, 7H), 0.71 (s, 2H), 0.57 (s, 2H).<br>MS m/z = 632 [M+H]+ | P1 was resolv ed from Exam ple **16-1;** SFC peak retenti on time: 4.798 min |
| | | **Example 16-1b** | $^1$H NMR (400 MHz, Methanol-d4) δ 8.36 (s, 1H), 7.38 (dd, J = 9.1, 4.5 Hz, 1H), 7.33 - 7.20 (m, 2H), 4.34 (s, 3H), 3.92 (s, 2H), 2.42 (s, 2H), 2.34 - 2.26 (m, 4H), 2.26 - 2.17 (m, 2H), 1.90 - 1.83 (m, 8H), 1.77 (td, J = 10.3, 9.8, 5.1 Hz, 2H), 1.64 (d, J = 17.1 Hz, 2H), 1.61 - 1.49 (m, 5H), 1.32 - 1.23 (m, 7H), 0.71 (s, 2H), 0.57 (s, 2H).<br>MS m/z = 632 [M+H]+ | P2 was resolv ed from Exam ple **16-1;** SFC peak retenti on time: 5.302 min |

(continued)

| Raw material 1 | Raw material 2 | Example | Characterization data | Yield and/o r SFC retention time |
|---|---|---|---|---|
| M4 | B1-b | 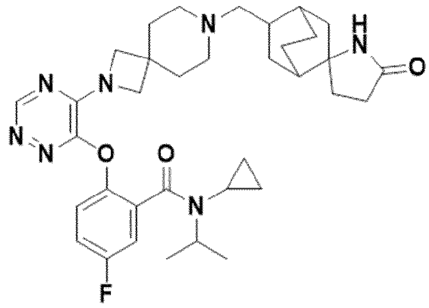<br>Example 16-2 | MS m/z = 632 $[M+H]^+$ | 38.7% (conta ining two isome rs) |
| | | **Example 16-2a** | $^1H$ NMR (400 MHz, Methanol-d4) δ 8.37 (s, 1H), 7.38 (dd, J = 9.1, 4.5 Hz, 1H), 7.34 - 7.21 (m, 2H), 4.36 (d, J = 31.1 Hz, 3H), 3.97 (s, 2H), 2.63 (s, 5H), 2.46 - 2.36 (m, 1H), 2.32 - 2.17 (m, 2H), 1.93 (d, J = 50.0 Hz, 8H), 1.77 (dt, J = 12.7, 3.9 Hz, 2H), 1.70 (d, J = 30.9 Hz, 2H), 1.61 - 1.43 (m, 5H), 1.27 (d, J = 8.4 Hz, 7H), 0.73 (s, 2H), 0.57 (s, 2H).<br>MS m/z = 632 $[M+H]^+$ | P1 was resolv ed from **16-2;** SFC peak retenti on time: 3.471 min |
| | | **Example 16-2b** | $^1H$ NMR (400 MHz, Methanol-d4) δ 8.36 (s, 1H), 7.39 (dd, J = 9.1, 4.5 Hz, 1H), 7.34 - 7.18 (m, 2H), 4.42 - 4.25 (m, 3H), 3.92 (s, 2H), 2.46 - 2.35 (m, 4H), 2.33 - 2.15 (m, 4H), 1.99 - 1.80 (m, 8H), 1.81 - 1.62 (m, 5H), 1.53 (t, J = 7.2 Hz, 4H), 1.28 (s, 7H), 0.71 (s, 2H), 0.57 (s, 2H).<br>MS m/z = 632 $[M+H]^+$ | P2 was resolv ed from **16-2;** SFC peak retenti on time: 4.697 min |

(continued)

| Raw material 1 | Raw material 2 | Example | Characterization data | Yield and/o r SFC retention time |
|---|---|---|---|---|
| N1 | C1 | 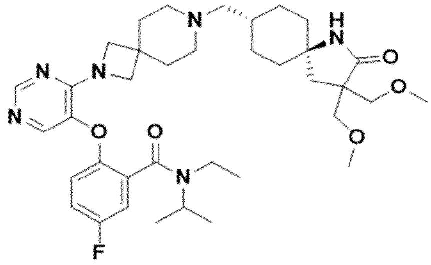<br>Example 17 | $^1$H NMR (400 MHz, Chloroform-d) δ 8.37 (d, J = 3.5 Hz, 1H), 7.77 (s, 1H), 7.02 (ddt, J = 7.5, 5.2, 2.8 Hz, 2H), 6.76 (dt, J = 9.8, 5.2 Hz, 1H), 5.45 (s, 1H), 4.06 - 3.77 (m, 5H), 3.54 - 3.42 (m, 3H), 3.33 (s, 9H), 2.27 (s, 3H), 2.06 (d, J = 8.4 Hz, 4H), 1.76 (d, J = 17.4 Hz, 8H), 1.67 (s, 3H), 1.42 - 1.32 (m, 3H), 1.25 (t, J = 7.0 Hz, 4H), 1.14 (dd, J = 6.7, 3.3 Hz, 5H).<br>MS m/z = 681 [M+H]$^+$ | 31.56 % |
| N2 | C1 | 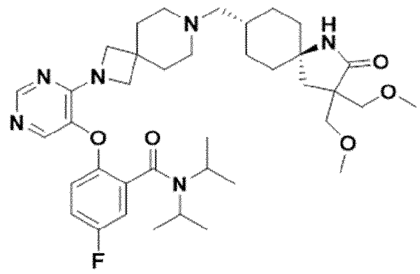<br>Example 18 | $^1$H NMR (400 MHz, Methanol-d4) δ 8.23 (s, 1H), 7.74 (s, 1H), 7.21 - 7.10 (m, 2H), 7.00 (dd, J = 9.0, 4.2 Hz, 1H), 4.11 - 3.89 (m, 4H), 3.83 (p, J = 6.6 Hz, 1H), 3.61 (h, J = 6.7 Hz, 1H), 3.39 (d, J = 8.8 Hz, 2H), 3.30 (s, 6H), 3.23 (d, J = 8.8 Hz, 2H), 2.38 (s, 4H), 2.17 (d, J = 6.9 Hz, 2H), 2.04 (s, 2H), 1.85 - 1.72 (m, 8H), 1.53 (d, J = 6.8 Hz, 3H), 1.47 (t, J = 7.9 Hz, 6H), 1.18 (d, J = 6.6 Hz, 3H), 1.08 (dd, J = 16.4, 9.3 Hz, 5H).<br>MS m/z = 695 [M+H]$^+$ | 22.53 % |

(continued)

| Raw material 1 | Raw material 2 | Example | Characterization data | Yield and/o r SFC retention time |
|---|---|---|---|---|
| M1 | C1 | 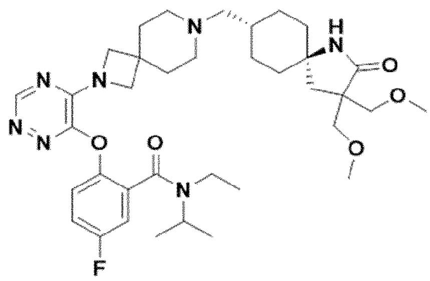<br>Example 19 | $^1$H NMR (600 MHz, Chloroform-d) δ 8.46 (d, J = 2.8 Hz, 1H), 7.25 (d, J = 4.4 Hz, 1H), 7.17 - 7.08 (m, 1H), 7.02 (ddd, J = 20.2, 7.8, 3.0 Hz, 1H), 5.42 (s, 1H), 4.33 (d, J = 66.0 Hz, 2H), 3.85 (dt, J = 13.2, 6.5 Hz, 3H), 3.46 (d, J = 8.9 Hz, 2H), 3.33 (s, 8H), 3.11 (dd, J = 13.3, 6.8 Hz, 1H), 2.32 (s, 3H), 2.08 (d, J = 18.2 Hz, 4H), 1.88 - 1.73 (m, 9H), 1.60 (s, 1H), 1.37 (ddd, J = 15.6, 12.5, 3.4 Hz, 3H), 1.18 - 1.01 (m, 9H), 0.77 (d, J = 6.6 Hz, 2H).<br>MS m/z = 682 $[M+H]^+$ | 28.16 % |
| M2 | C1 | 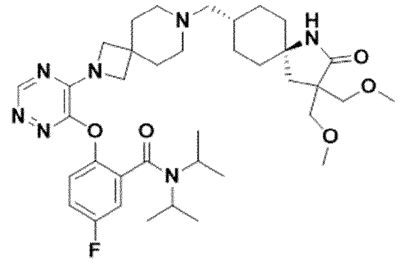<br>Example 20 | $^1$H NMR (400 MHz, Chloroform-d) δ 8.46 (s, 1H), 7.26 - 7.23 (m, 1H), 7.10 (ddd, J = 9.1, 7.9, 3.0 Hz, 1H), 6.96 (dd, J = 7.8, 3.0 Hz, 1H), 5.75 (s, 1H), 4.47 (d, J = 10.3 Hz, 1H), 4.28 (d, J = 10.3 Hz, 1H), 3.94 - 3.83 (m, 2H), 3.79 (p, J = 6.6 Hz, 1H), 3.46 (d, J = 8.9 Hz, 2H), 3.43 - 3.36 (m, 1H), 3.33 (s, 7H), 3.30 (s, 1H), 2.32 (s, 3H), 2.13 - 2.02 (m, 4H), 1.90 - 1.64 (m, 9H), 1.50 (d, J = 6.8 Hz, 3H), 1.37 (dd, J = 12.9, 8.7 Hz, 6H), 1.16 - 0.95 (m, 5H), 0.72 (d, J = 6.6 Hz, 3H).<br>MS m/z = 696 $[M+H]^+$ | 71.23% |

(continued)

| Raw material 1 | Raw material 2 | Example | Characterization data | Yield and/o r SFC retention time |
|---|---|---|---|---|
| N1 | C2 | 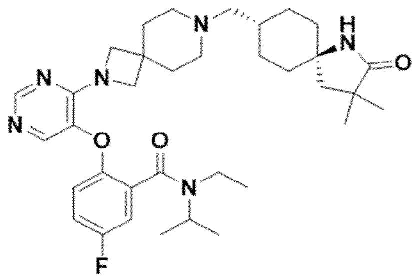 Example 21 | $^1$H NMR (400 MHz, Methanol-d$_4$) δ 8.26 - 8.19 (m, 1H), 7.77 - 7.67 (m, 1H), 7.25 - 7.13 (m, 2H), 7.06 - 6.94 (m, 1H), 4.09 - 3.82 (m, 5H), 3.52 (dq, *J* = 14.0, 7.0 Hz, 1H), 3.36 (dt, *J* = 14.1, 6.9 Hz, 1H), 2.35 (s, 4H), 2.15 (d, *J* = 6.8 Hz, 2H), 1.90 (s, 2H), 1.85 - 1.72 (m, 6H), 1.71 - 1.62 (m, 2H), 1.62 - 1.43 (m, 3H), 1.38 - 1.00 (m, 17H)<br>MS m/z = 621 [M+H]$^+$ | 38.76 % |
| N2 | C2 | 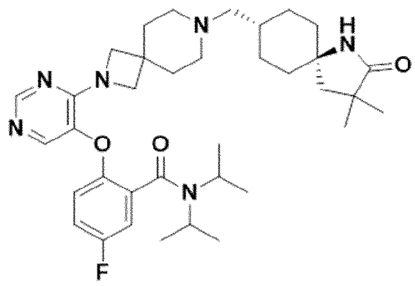 Example 22 | $^1$H NMR (400 MHz, Methanol-*d$_4$*) δ 8.23 (s, 1H), 7.74 (s, 1H), 7.23 - 7.09 (m, 2H), 6.99 (dd, *J* = 9.0, 4.2 Hz, 1H), 4.09 - 3.88 (m, 4H), 3.83 (p, *J* = 6.7 Hz, 1H), 3.62 (p, *J* = 6.8 Hz, 1H), 2.39 (s, 4H), 2.18 (d, *J* = 6.7 Hz, 2H), 1.90 (s, 2H), 1.87 - 1.73 (m, 6H), 1.71 - 1.62 (m, 2H), 1.62 - 1.42 (m, 9H), 1.22 - 1.14 (m, 9H), 1.16 -1.00 (m, 5H)<br>MS m/z = 635 [M+H]$^+$ | 28.51 % |
| N4 | C2 | 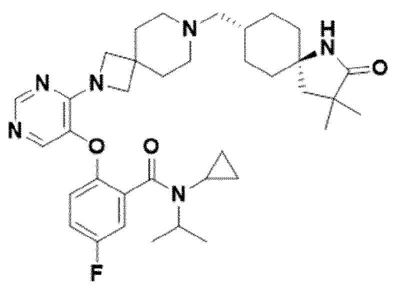 Example 23 | $^1$H NMR (600 MHz, $CDCl_3$) δ 8.38 (s, 1H), 7.80 (s, 1H), 7.00 (s, 2H), 6.73 (d, *J* = 5.1 Hz, 1H), 5.43 (s, 1H), 4.53 (s, 1H), 3.86 (s, 4H), 2.56 (s, 1H), 2.25 (s, 3H), 2.07 (d, *J* = 4.8 Hz, 2H), 1.86 (s, 2H), 1.73 (s, 11H), 1.43 (s, 3H), 1.35 (d, *J* = 6.4 Hz, 6H), 1.21 (s, 7H), 1.01 (d, *J* = 11.6 Hz, 2H), 0.56 (s, 4H)<br>MS m/z = 633 [M+H]$^+$ | 43% |

(continued)

| Raw material 1 | Raw material 2 | Example | Characterization data | Yield and/o r SFC retention time |
|---|---|---|---|---|
| N3 | C2 | 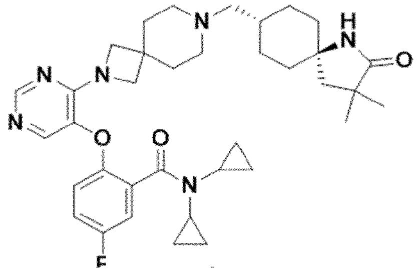<br>Example 24 | $^1$H NMR (400 MHz, Methanol-$d_4$) δ 8.24 (s, 1H), 7.69 (s, 1H), 7.26 - 7.15 (m, 2H), 6.96 (dd, $J$ = 8.9, 4.2 Hz, 1H), 3.93 (s, 4H), 2.70 (s, 2H), 2.35 (s, 4H), 2.15 (d, $J$ = 6.9 Hz, 2H), 1.90 (s, 2H), 1.85 - 1.74 (m, 6H), 1.71 - 1.62 (m, 2H), 1.61 - 1.43 (m, 3H), 1.18 (s, 6H), 1.08 (qd, $J$ = 13.4, 3.5 Hz, 2H), 0.94 - 0.87 (m, 2H), 0.83 - 0.70 (m, 4H), 0.70 - 0.60 (m, 2H)<br>MS m/z = 631 $[M+H]^+$ | 30.13 % |
| N5 | C2 | 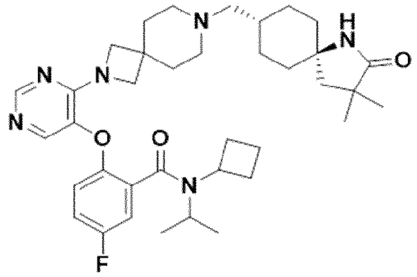<br>txampie 25 | $^1$H NMR (400 MHz, Chloroform-d) δ 8.35 (d, J = 9.5 Hz, 1H), 7.75 (d, J = 14.2 Hz, 1H), 6.98 (dd, J = 24.2, 7.9 Hz, 2H), 6.87 - 6.72 (m, 1H), 5.37 (s, 1H), 3.92 (d, J = 32.6 Hz, 5H), 3.23 - 3.02 (m, 1H), 2.28 (s, 3H), 2.13 - 2.05 (m, 3H), 1.86 (s, 3H), 1.75 (s, 7H), 1.67 (d, J = 15.8 Hz, 5H), 1.49 - 1.36 (m,6H), 1.22 (s, 7H), 1.05 (d, J = 37.9 Hz, 5H).<br>MS m/z = 647 $[M+H]^+$ | 53.41 % |

(continued)

| Raw material 1 | Raw material 2 | Example | Characterization data | Yield and/o r SFC retention time |
|---|---|---|---|---|
| M1 | C2 | 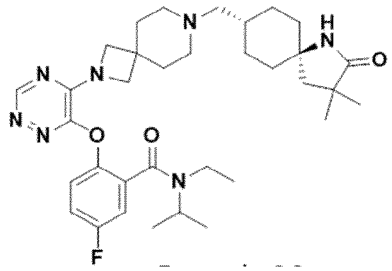 Example 26 | $^{1}$H NMR (400 MHz, Methanol-$d_4$) δ 8.38 (s, 1H), 7.45 - 7.36 (m, 1H), 7.33 - 7.16 (m, 2H), 4.51 - 4.14 (m, 2H), 4.02 - 3.70 (m, 3H), 3.61 - 3.39 (m, 1H), 3.28 - 3.18 (m, 1H), 2.59 - 2.28 (m, 4H), 2.17 (d, *J* = 6.8 Hz, 2H), 1.93 - 1.83 (m, 6H), 1.83 - 1.75 (m, 2H), 1.71 - 1.63 (m, 2H), 1.63 - 1.44 (m, 3H), 1.25 - 1.01 (m, 15H), 0.88 - 0.76 (m, 2H) MS m/z = 622 [M+H]$^+$ | 41.94 % |
| M2 | C2 | 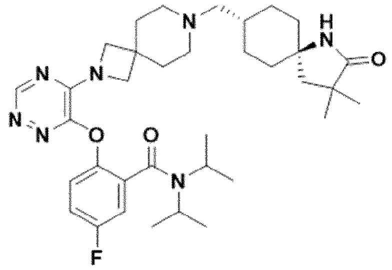 Example 27 | $^{1}$H NMR (400 MHz, Methanol-$d_4$) δ 8.38 (s, 1H), 7.39 (dd, *J* = 9.1, 4.5 Hz, 1H), 7.25 (ddd, *J* = 9.0, 8.1, 3.0 Hz, 1H), 7.16 (dd, *J* = 8.0, 3.0 Hz, 1H), 4.47 - 4.33 (m, 2H), 3.92 (q, *J* = 10.7 Hz, 2H), 3.75 (p, *J* = 6.6 Hz, 1H), 3.54 (p, *J* = 6.8 Hz, 1H), 2.54 - 2.29 (m, 4H), 2.20 (d, *J* = 6.8 Hz, 2H), 1.95 -1.74 (m, 8H), 1.72 - 1.63 (m, 2H), 1.63 - 1.43 (m, 6H), 1.39 (d, *J* = 6.7 Hz, 3H), 1.18 (s, 6H), 1.17 - 1.01 (m, 5H), 0.75 (d, *J* = 6.6 Hz, 3H) MS m/z = 636 [M+H]$^+$ | 62.42 % |

(continued)

| Raw material 1 | Raw material 2 | Example | Characterization data | Yield and/o r SFC retention time |
|---|---|---|---|---|
| **M6** | **C2** | 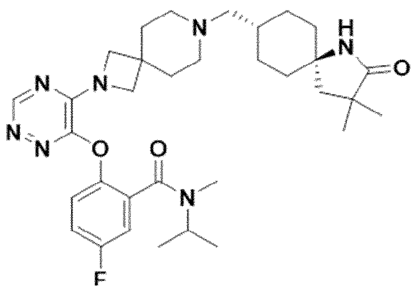 Example 28 | $^1$H NMR (400 MHz, Methanol-$d_4$) δ 8.40-8.33 (m, 1H), 7.41 (ddd, $J$ = 20.0, 9.1, 4.5 Hz, 1H), 7.28 (ddd, $J$ = 9.1, 8.0, 3.0 Hz, 1H), 7.21 (dd, $J$ = 8.0, 3.0 Hz, 1H), 4.65 (h, $J$ = 6.9 Hz, 1H), 4.4 (s, 1H), 4.36-4.27 (m, 1H), 3.95 - 3.90 (m, 2H), 2.91-2.67 (m, 3H), 2.61-2.29 (m, 4H), 2.21 (d, $J$ = 6.8 Hz, 2H), 1.94 - 1.85 (m, 6H), 1.84 - 1.74 (m, 2H), 1.73 - 1.63 (m, 2H), 1.63 - 1.46 (m, 3H), 1.23 - 0.94 (m, 12H), 0.93- 0.77 (m, 2H) MS m/z = 608 [M+H]$^+$ | 22.71 % |
| **M7** | **C2** | 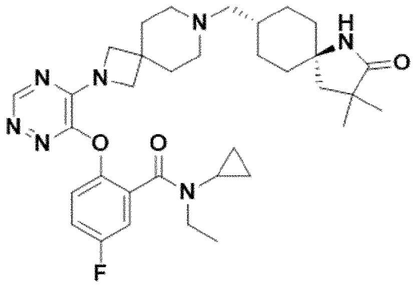 Example 29 | $^1$H NMR (400 MHz, Methanol-d4) δ 8.36 (s, 1H), 7.40 (dd, J = 9.0, 4.5 Hz, 1H), 7.34 - 7.19 (m, 2H), 4.31 (s, 2H), 3.92 (s, 2H), 3.48 (q, J = 7.3 Hz, 2H), 2.74 (tt, J = 7.1, 4.0 Hz, 1H), 2.42 (s, 4H), 2.18 (d, J = 6.9 Hz, 2H), 1.96 - 1.83 (m, 6H), 1.79 (dd, J = 14.1, 3.5 Hz, 2H), 1.67 (d, J = 12.8 Hz, 2H), 1.62 - 1.44 (m, 3H), 1.18 (s, 6H), 1.15 - 1.09 (m, 4H), 1.09 - 1.02 (m, 1H), 0.63 (dd, J = 26.5, 5.8 Hz, 4H). MS m/z = 620 [M+H]$^+$ | 54.14 % |

(continued)

| Raw material 1 | Raw material 2 | Example | Characterization data | Yield and/o r SFC retention time |
|---|---|---|---|---|
| M4 | C2 | 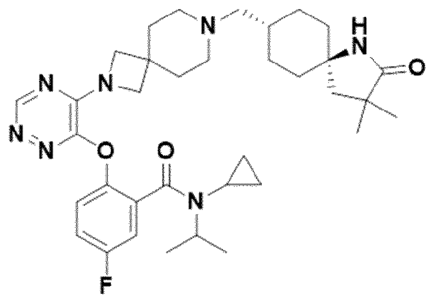 Example 30 | $^1$H NMR (400 MHz, $CDCl_3$) δ 8.46 (s, 1H), 7.27 (s, 1H), 7.10 (dd, *J* = 13.9, 5.7 Hz, 2H), 5.36 (s, 1H), 4.36 (d, *J* = 46.5 Hz, 3H), 3.87 (s, 2H), 2.32 (s, 4H), 2.09 (d, *J* = 6.9 Hz, 2H), 1.87 (s, 2H), 1.81 (s, 5H), 1.73 (s, 2H), 1.70 (s, 1H), 1.60 (s, 3H), 1.44 (t, *J* = 11.4 Hz, 3H), 1.22 (s, 8H), 1.09 - 0.96 (m, 2H), 0.86 (d, *J* = 11.5 Hz, 1H), 0.70 (s, 2H), 0.56 (s, 2H). MS m/z = 634 $[M+H]^+$ | 36% |
| M3 | C2 | 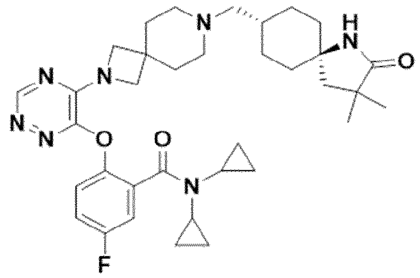 Example 31 | $^1$H NMR (400 MHz, Methanol-$d_4$) δ 8.38 (s, 1H), 7.38 (dd, *J* = 9.0, 4.5 Hz, 1H), 7.34 - 7.21 (m, 2H), 4.30 (s, 2H), 3.93 (s, 2H), 2.62 (s, 2H), 2.54 - 2.29 (m, 4H), 2.17 (d, *J* = 6.9 Hz, 2H), 1.95 - 1.84 (m, 6H), 1.84 - 1.74 (m, 2H), 1.72 - 1.62 (m, 2H), 1.63 - 1.43 (m, 3H), 1.18 (s, 6H), 1.09 (qd, *J* = 13.8, 3.7 Hz, 2H), 0.92 - 0.56 (m, 8H) MS m/z = 632 $[M+H]^+$ | 32.56 % |

(continued)

| Raw material 1 | Raw material 2 | Example | Characterization data | Yield and/o r SFC retention time |
|---|---|---|---|---|
| M5 | C2 | 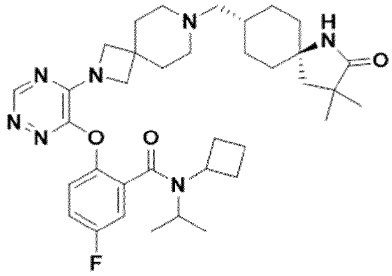 Example 32 | $^1$H NMR (400 MHz, Chloroform-d) δ 8.44 (s, 1H), 7.24 (s, 1H), 7.17 - 7.06 (m, 1H), 6.93 (dd, J = 44.6, 7.3 Hz, 1H), 5.42 (s, 1H), 4.60 - 4.03 (m, 3H), 3.87 (s, 2H), 3.71 (d, J = 8.6 Hz, 1H), 2.32 (s, 3H), 2.07 (d, J = 15.9 Hz, 3H), 1.87 (s, 3H), 1.82 (s,6H), 1.69 (t, J = 15.8 Hz, 6H), 1.54 - 1.36 (m, 6H), 1.22 (s, 9H), 1.03 (d, J = 12.8 Hz, 3H). MS m/z = 648 [M+H]$^+$ | 41% |
| M1 | C4 | 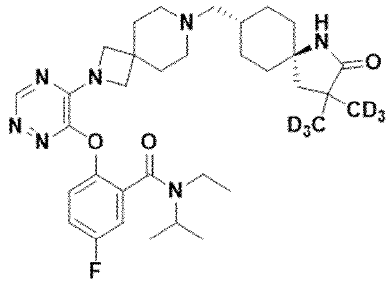 Example 33 | $^1$H NMR (400 MHz, Methanol-$d_4$) δ 8.38 (s, 1H), 7.45 - 7.36 (m, 1H), 7.31 - 7.23 (m, 1H), 7.24 - 7.16 (m, 1H), 4.44 - 4.28 (m, 2H), 3.99 - 3.86 (m, 2H), 3.81 (p, *J* = 6.6 Hz, 1H), 3.56 - 3.43 (m, 1H), 3.28 - 3.17 (m, 1H), 2.55 - 2.25 (m, 4H), 2.17 (d, *J* = 6.8 Hz, 2H), 1.91 - 1.83 (m, 6H), 1.83 - 1.75 (m, 2H), 1.71 - 1.63 (m, 2H), 1.63 - 1.43 (m, 3H), 1.24 - 1.02 (m, 9H), 0.81 (d, *J* = 6.6 Hz, 2H) MS m/z = 628 [M+H]$^+$ | 21.59 % |

(continued)

| Raw material 1 | Raw material 2 | Example | Characterization data | Yield and/o r SFC retention time |
|---|---|---|---|---|
| M2 | C4 | 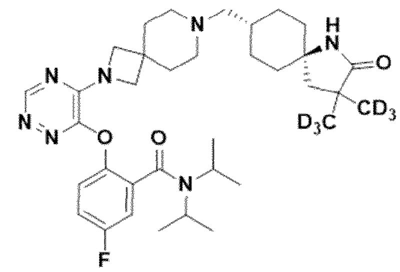<br>Example 34 | $^1$H NMR (400 MHz, Methanol-$d_4$) δ 8.38 (s, 1H), 7.39 (dd, $J$ = 9.1, 4.5 Hz, 1H), 7.25 (ddd, $J$ = 9.0, 8.0, 3.0 Hz, 1H), 7.16 (dd, $J$ = 8.0, 3.0 Hz, 1H), 4.48 - 4.31 (m, 2H), 3.92 (q, $J$ = 10.5 Hz, 2H), 3.75 (hept, $J$ = 6.6 Hz, 1H), 3.54 (hept, $J$ = 6.9 Hz, 1H), 2.60 - 2.23 (m, 4H), 2.17 (d, $J$ = 6.9 Hz, 2H), 1.93 - 1.74 (m, 8H), 1.71 - 1.62 (m, 2H), 1.62 - 1.46 (m, 6H), 1.39 (d, $J$ = 6.7 Hz, 3H), 1.18 - 1.01 (m, 5H), 0.74 (d, $J$ = 6.5 Hz, 3H)<br>MS m/z = 642 $[M+H]^+$ | 49.51 % |
| N1 | D1-a2 | 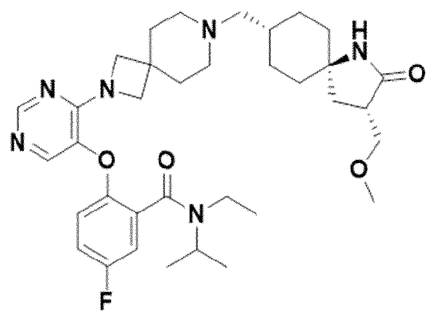<br>Example 35 | $^1$H NMR (400 MHz, Methanol-$d_4$) δ 8.24 (s, 1H), 7.74 (s, 1H), 7.24 - 7.14 (m, 2H), 7.00 (td, $J$ = 8.8, 4.2 Hz, 1H), 4.06 - 3.84 (m, 5H), 3.63 - 3.46 (m, 3H), 3.42 - 3.30 (m, 4H), 2.73 (tdd, $J$ = 9.0, 5.2, 3.6 Hz, 1H), 2.38 (s, 4H), 2.27 - 2.13 (m, 3H), 1.87 - 1.64 (m, 9H), 1.64 - 1.41 (m, 3H), 1.36 - 0.98 (m, 11H)<br>MS m/z = 637 $[M+H]^+$ | 31.70 % Single config uratio n; |

(continued)

| Raw material 1 | Raw material 2 | Example | Characterization data | Yield and/o r SFC retention time |
|---|---|---|---|---|
| N2 | D1-a2 | 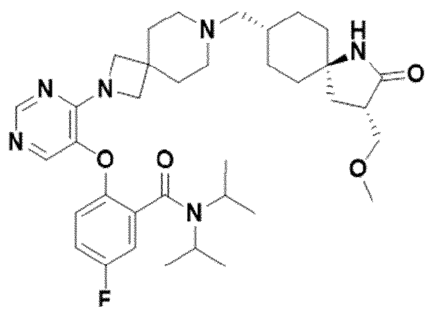<br>Example 36 | $^1$H NMR (400 MHz, Methanol-$d_4$) δ 8.26 (s, 1H), 7.78 (s, 1H), 7.22 - 7.11 (m, 2H), 6.98 (dd, $J$ = 8.9, 4.2 Hz, 1H), 4.03 (s, 2H), 3.97 - 3.90 (m, 2H), 3.83 (p, $J$ = 6.6 Hz, 1H), 3.67 - 3.55 (m, 2H), 3.54 - 3.46 (m, 1H), 3.33 (s, 3H), 2.74 (tdd, $J$ = 8.8, 5.2, 3.5 Hz, 1H), 2.60 (s, 4H), 2.39 (s, 2H), 2.23 (dd, $J$ = 13.0, 9.8 Hz, 1H), 1.95 - 1.65 (m, 9H), 1.65 - 1.42 (m, 9H), 1.21 - 1.04 (m, 8H)<br>MS m/z = 651 [M+H]$^+$ | 27.23 % Single config uratio n |
| M1 | D1-a1 | 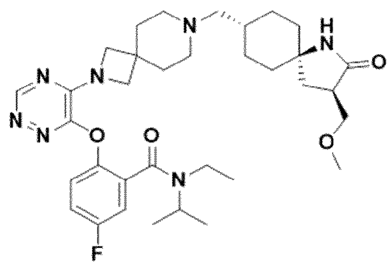<br>Example 37-1 | $^1$H NMR (400 MHz, Methanol-$d_4$) δ 8.38 (s, 1H), 7.45 - 7.36 (m, 1H), 7.33 - 7.16 (m, 2H), 4.43 - 4.30 (m, 2H), 4.00 - 3.87 (m, 2H), 3.81 (hept, $J$ = 6.9 Hz, 1H), 3.63 - 3.45 (m, 3H), 3.33 (s, 3H), 3.28 - 3.18 (m, 1H), 2.80 - 2.67 (m, 1H), 2.58 - 2.12 (m, 7H), 1.93 - 1.64 (m, 9H), 1.64 - 1.41 (m, 3H), 1.26 - 0.99 (m, 9H), 0.81 (d, $J$ = 6.6 Hz, 2H)<br>MS m/z = 638 [M+H]$^+$ | 23.94 % |

(continued)

| Raw material 1 | Raw material 2 | Example | Characterization data | Yield and/o r SFC retention time |
|---|---|---|---|---|
| M1 | D1-a2 | 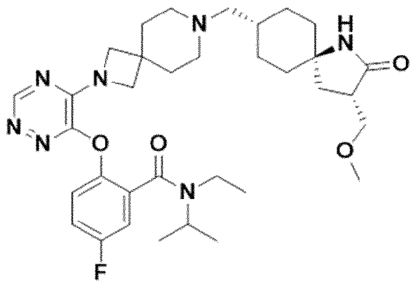 Example 37-2 | $^1$H NMR (400 MHz, Methanol-$d_4$) δ 8.38 (s, 1H), 7.45 - 7.36 (m, 1H), 7.33 - 7.16 (m, 2H), 4.43 - 4.30 (m, 2H), 4.00 - 3.87 (m, 2H), 3.81 (hept, *J* = 6.9 Hz, 1H), 3.63 - 3.45 (m, 3H), 3.33 (s, 3H), 3.28 - 3.18 (m, 1H), 2.80 - 2.67 (m, 1H), 2.58 - 2.12 (m, 7H), 1.93 - 1.64 (m, 9H), 1.64 - 1.41 (m, 3H), 1.26 - 0.99 (m, 9H), 0.81 (d, *J* = 6.6 Hz, 2H) MS m/z = 638 [M+H]$^+$ | 43.39 % |
| M2 | D1-a1 | 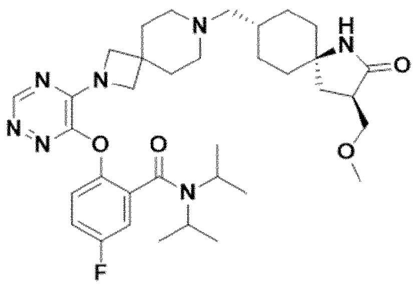 Example 38-1 | $^1$H NMR (400 MHz, Methanol-$d_4$) δ 8.38 (s, 1H), 7.39 (dd, *J* = 9.1, 4.5 Hz, 1H), 7.25 (ddd, *J* = 9.1, 8.1, 3.0 Hz, 1H), 7.16 (dd, *J* = 8.0, 3.0 Hz, 1H), 4.48 - 4.31 (m, 2H), 3.92 (q, *J* = 10.5 Hz, 2H), 3.75 (p, *J* = 6.6 Hz, 1H), 3.63 - 3.47 (m, 3H), 2.78 - 2.69 (m, 1H), 2.58 - 2.11 (m, 7H), 1.92 - 1.64 (m, 9H), 1.63 - 1.35 (m, 9H), 1.21 - 1.00 (m, 5H), 0.75 (d, *J* = 6.6 Hz, 3H) MS m/z = 652 [M+H]$^+$ | 39.65 % |

(continued)

| Raw material 1 | Raw material 2 | Example | Characterization data | Yield and/o r SFC retention time |
|---|---|---|---|---|
| M2 | D1-a2 | 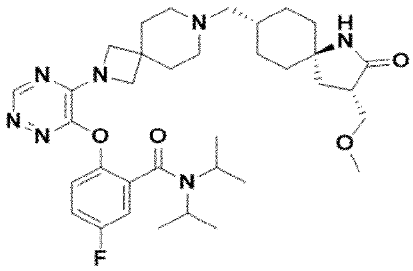 Example 38-2 | $^1$H NMR (400 MHz, Methanol-$d_4$) δ 8.38 (s, 1H), 7.39 (dd, $J$ = 9.1, 4.5 Hz, 1H), 7.25 (ddd, $J$ = 9.1, 8.1, 3.0 Hz, 1H), 7.16 (dd, $J$ = 8.0, 3.0 Hz, 1H), 4.48 - 4.31 (m, 2H), 3.92 (q, $J$ = 10.5 Hz, 2H), 3.75 (p, $J$ = 6.6 Hz, 1H), 3.63 - 3.47 (m, 3H), 3.329 (s, 3H), 2.78 - 2.69 (m, 1H), 2.58 - 2.11 (m, 7H), 1.92 - 1.64 (m, 9H), 1.63 - 1.35 (m, 9H), 1.21 -1.00 (m, 5H), 0.75 (d, $J$ = 6.6 Hz, 3H). MS m/z = 652 $[M+H]^+$ | 42.42 % |
| N2 | D2-a2 | 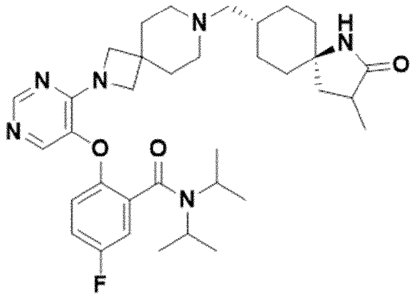 Example 39 | $^1$H NMR (400 MHz, Chloroform-d) δ 8.37 (s, 1H), 7.76 (s, 1H), 6.98 (dd, J = 7.7, 2.6 Hz, 2H), 6.81 - 6.73 (m, 1H), 5.41 (s, 1H), 3.96 (s, 2H), 3.87 (d, J = 9.2 Hz, 2H), 3.78 (h, J = 6.6 Hz, 1H), 3.49 (p, J = 6.8 Hz, 1H), 2.55 (ddt, J = 16.4, 9.2, 7.2 Hz, 1H), 2.44 - 2.19 (m, 5H), 2.09 (s, 2H), 1.75 (s, 8H), 1.67 (s, 4H), 1.54 (d, J = 6.8 Hz, 4H), 1.48 (d, J = 6.8 Hz, 4H), 1.20 (d, J = 7.1 Hz, 3H), 1.13 (d, J = 6.6 Hz, 3H), 1.09 (d, J = 6.7 Hz, 3H). MS m/z = 621 $[M+H]^+$ | 10.5% (conta ining two isome rs) |

(continued)

| Raw material 1 | Raw material 2 | Example | Characterization data | Yield and/o r SFC retention time |
|---|---|---|---|---|
| **M1** | **D2-a1** | 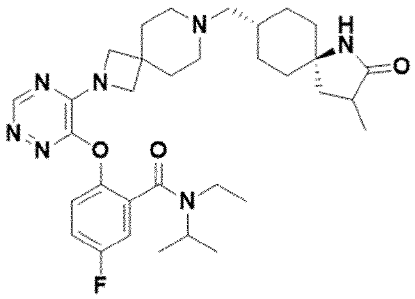 Example 40-1 | $^1$H NMR (400 MHz, Methanol-$d_4$) δ 8.38 (s, 1H), 7.45 - 7.36 (m, 1H), 7.33 - 7.16 (m, 2H), 4.47 - 4.28 (m, 2H), 4.01 - 3.86 (m, 2H), 3.81 (p, $J$ = 6.6 Hz, 1H), 3.57 - 3.41 (m, 1H), 3.28 - 3.17 (m, 1H), 2.64 - 2.23 (m, 6H), 2.17 (d, $J$ = 6.9 Hz, 2H), 1.93 - 1.69 (m, 7H), 1.71 - 1.57 (m, 2H), 1.58 - 1.36 (m, 3H), 1.24 - 1.01 (m, 12H), 0.81 (d, $J$ = 6.6 Hz, 2H) MS m/z = 608 $[M+H]^+$ | 23.15 % |
| **M1** | **D2-a2** | 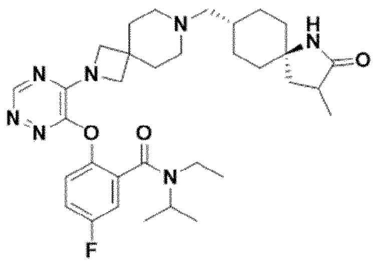 Example 40-2 | $^1$H NMR (400 MHz, Methanol-$d_4$) δ 8.38 (s, 1H), 7.45 - 7.36 (m, 1H), 7.33 - 7.16 (m, 2H), 4.47 - 4.28 (m, 2H), 4.01 - 3.86 (m, 2H), 3.81 (p, $J$ = 6.6 Hz, 1H), 3.57 - 3.41 (m, 1H), 3.28 - 3.17 (m, 1H), 2.64 - 2.23 (m, 6H), 2.17 (d, $J$ = 6.9 Hz, 2H), 1.93 - 1.69 (m, 7H), 1.71 - 1.57 (m, 2H), 1.58 - 1.36 (m, 3H), 1.24 - 1.01 (m, 12H), 0.81 (d, $J$ = 6.6 Hz, 2H) MS m/z = 608 $[M+H]^+$ | 34.72 % |

(continued)

| Raw material 1 | Raw material 2 | Example | Characterization data | Yield and/o r SFC retention time |
|---|---|---|---|---|
| **M2** | **D2-a1** | 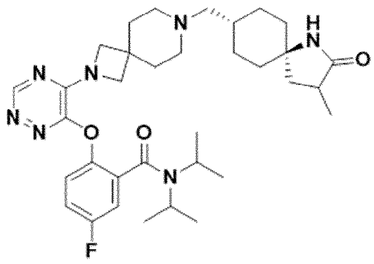<br>Example 41-1 | $^1$H NMR (400 MHz, Methanol-$d_4$) δ 8.38 (s, 1H), 7.39 (dd, $J$ = 9.0, 4.5 Hz, 1H), 7.25 (ddd, $J$ = 9.0, 8.0, 3.0 Hz, 1H), 7.16 (dd, $J$ = 8.0, 3.0 Hz, 1H), 4.49 - 4.29 (m, 2H), 3.92 (q, $J$ = 10.5 Hz, 2H), 3.75 (hept, $J$ = 6.6 Hz, 1H), 3.54 (hept, $J$ = 6.9 Hz, 1H), 2.65 - 2.22 (m, 6H), 2.17 (d, $J$ = 6.9 Hz, 2H), 1.91 - 1.69 (m, 7H), 1.71 - 1.58 (m, 2H), 1.58 - 1.31 (m, 9H), 1.24 - 0.99 (m, 8H), 0.75 (d, $J$ = 6.6 Hz, 3H)<br>MS m/z = 622 $[M+H]^+$ | 40.13 % |
| M2 | D2-a2 | 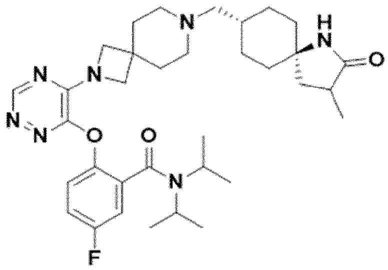<br>Example 41-2 | $^1$H NMR (400 MHz, Methanol-$d_4$) δ 8.38 (s, 1H), 7.39 (dd, $J$ = 9.0, 4.5 Hz, 1H), 7.25 (ddd, $J$ = 9.0, 8.0, 3.0 Hz, 1H), 7.16 (dd, $J$ = 8.0, 3.0 Hz, 1H), 4.49 - 4.29 (m, 2H), 3.92 (q, $J$ = 10.5 Hz, 2H), 3.75 (hept, $J$ = 6.6 Hz, 1H), 3.54 (hept, $J$ = 6.9 Hz, 1H), 2.65 - 2.22 (m, 2H), 2.17 (d, $J$ = 6.9 Hz, 2H), 1.91 - 1.69 (m, 7H), 1.63 (dd, $J$ = 11.9, 3.4 Hz, 2H), 1.58 - 1.31 (m, 9H), 1.24 - 0.99 (m, 8H), 0.75 (d, $J$ = 6.6 Hz, 3H)<br>MS m/z = 622 $[M+H]^+$ | 32.94 % |

(continued)

| Raw material 1 | Raw material 2 | Example | Characterization data | Yield and/o r SFC retention time |
|---|---|---|---|---|
| M1 | D3-a1 | 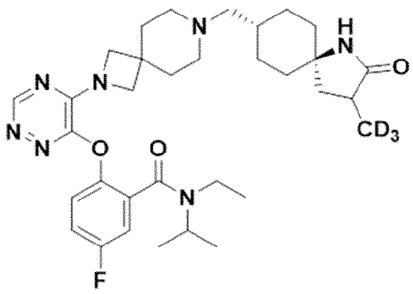 Example 42-1 | 1H NMR (400 MHz, Methanol-d4) δ 8.38 (d, J = 1.2 Hz, 1H), 7.40 (dt, J = 9.3, 4.7 Hz, 1H), 7.32 - 7.24 (m, 1H), 7.20 (dd, J = 8.0, 3.0 Hz, 1H), 4.36 (d, J = 13.0 Hz, 2H), 4.02 - 3.74 (m, 3H), 3.57 - 3.43 (m, 1H), 3.27 - 3.06 (m, 1H), 2.61 - 2.26 (m, 6H), 2.18 (d, J = 6.9 Hz, 2H), 1.87 (t, J = 5.4 Hz, 4H), 1.83 - 1.70 (m, 3H), 1.63 (dd, J = 11.9, 3.5 Hz, 2H), 1.55 - 1.37 (m, 3H), 1.25 - 1.01 (m, 9H), 0.81 (d, J = 6.6 Hz, 2H). MS m/z = 611 $[M+H]^+$ | 34.75 % |
| M1 | D3-a2 | 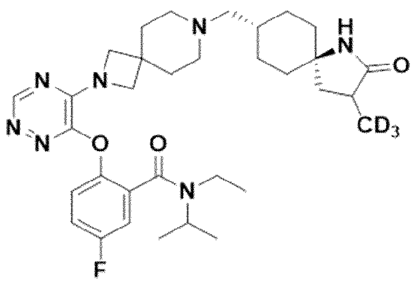 Example 42-2 | $^1$H NMR (400 MHz, Methanol-d4) δ 8.38 (d, J = 1.2 Hz, 1H), 7.40 (dt, J = 9.3, 4.7 Hz, 1H), 7.32 - 7.24 (m, 1H), 7.20 (dd, J = 8.0, 3.0 Hz, 1H), 4.36 (d, J = 13.0 Hz, 2H), 4.02 - 3.74 (m, 3H), 3.57 - 3.43 (m, 1H), 3.27 - 3.06 (m, 1H), 2.61 - 2.26 (m, 6H), 2.18 (d, J = 6.9 Hz, 2H), 1.87 (t, J = 5.4 Hz, 4H), 1.83 - 1.70 (m, 3H), 1.63 (dd, J = 11.9, 3.5 Hz, 2H), 1.55 - 1.37 (m, 3H), 1.25 - 1.01 (m, 9H), 0.81 (d, J = 6.6 Hz, 2H). MS m/z = 611 $[M+H]^+$ | 34.75 % |

(continued)

| Raw material 1 | Raw material 2 | Example | Characterization data | Yield and/o r SFC retention time |
|---|---|---|---|---|
| M2 | D3-a1 | 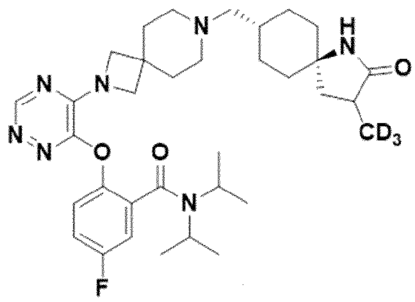 Example 43-1 | $^1$H NMR (400 MHz, Methanol-d4) δ 8.38 (s, 1H), 7.39 (dd, J = 9.1, 4.5 Hz, 1H), 7.25 (ddd, J = 9.0, 8.1, 3.0 Hz, 1H), 7.16 (dd, J = 8.0, 3.0 Hz, 1H), 4.49 - 4.30 (m, 2H), 3.92 (q, J = 10.5 Hz, 2H), 3.76 (h, J = 6.6 Hz, 1H), 3.55 (h, J = 6.8 Hz, 1H), 2.55 (t, J = 9.1 Hz, 1H), 2.40 (dd, J = 12.7, 9.1 Hz, 2H), 2.18 (d, J = 6.8 Hz, 2H), 1.86 (d, J = 5.6 Hz, 8H), 1.63 (dd, J = 11.9, 3.4 Hz, 2H), 1.50 (d, J = 6.8 Hz, 5H), 1.44 - 1.35 (m, 5H), 1.20 - 0.99 (m, 6H), 0.75 (d, J = 6.6 Hz, 3H). MS m/z = 625 [M+H]$^+$ | 47.6% |
| M2 | D3-a2 | 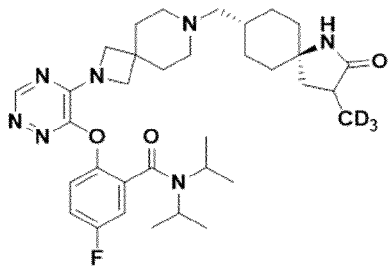 Example 43-2 | MS m/z = 625 [M+H]$^+$ | 58.7% |
| | | 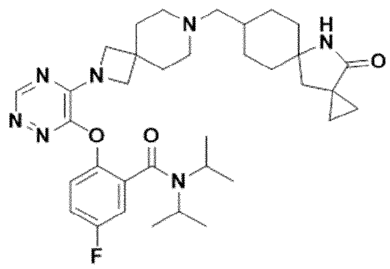 Example 44 | MS m/z = 634 [M+H]$^+$ | 44% (conta ining two isome rs) |

(continued)

| Raw material 1 | Raw material 2 | Example | Characterization data | Yield and/o r SFC retention time |
|---|---|---|---|---|
| M2 | C3 | **Example 44-1** | $^1$H NMR (400 MHz, MeOD) δ 8.39 (s, 1H), 7.41 (dd, *J* = 9.1, 4.5 Hz, 1H), 7.32 - 7.23 (m, 1H), 7.17 (dd, *J* = 8.0, 3.0 Hz, 1H), 4.46 (d, *J* = 10.3 Hz, 1H), 4.36 (d, *J* = 10.2 Hz, 1H), 3.93 (q, *J* = 10.2 Hz, 2H), 3.82 - 3.72 (m, 1H), 3.63 - 3.48 (m, 1H), 2.43 (d, *J* = 31.3 Hz, 4H), 2.24 (d, *J* = 6.9 Hz, 2H), 1.99 (s, 2H), 1.88 (d, *J* = 3.8 Hz, 4H), 1.85 - 1.71 (m, 5H), 1.60 (d, *J* = 3.6 Hz, 1H), 1.57 (d, *J* = 3.2 Hz, 1H), 1.52 (d, *J* = 6.8 Hz, 4H), 1.41 (d, *J* = 6.7 Hz, 3H), 1.33 -1.23 (m, 2H), 1.16 (d, *J* = 6.6 Hz, 3H), 1.01 (d, *J* = 2.8 Hz, 2H), 0.79 - 0.73 (m, 5H).<br>MS m/z = 634 $[M+H]^+$ | P1 was resolv ed from Exam ple **44;** SFC peak retenti on time: 1.507 min |

(continued)

| Raw material 1 | Raw material 2 | Example | Characterization data | Yield and/o r SFC retention time |
|---|---|---|---|---|
| | | **Example 44-2** | $^1$H NMR (400 MHz, MeOD) δ 8.39 (s, 1H), 7.41 (dd, *J* = 9.1, 4.5 Hz, 1H), 7.32 - 7.23 (m, 1H), 7.18 (dd, *J* = 8.0, 3.0 Hz, 1H), 4.59 (s, 1H), 4.46 (d, *J* = 10.2 Hz, 1H), 4.37 (d, *J* = 9.8 Hz, 1H), 4.02 - 3.86 (m, 2H), 3.77 (dt, *J* = 13.1, 6.6 Hz, 1H), 3.55 (dt, *J* = 13.5, 6.7 Hz, 1H), 2.49 (s, 4H), 2.21 (d, *J* = 6.5 Hz, 2H), 2.05 (s, 2H), 1.89 (s, 4H), 1.80 (t, *J* = 12.4 Hz, 4H), 1.63 (dd, *J* = 13.5, 4.0 Hz, 3H), 1.52 (d, *J* = 6.7 Hz, 3H), 1.40 (d, *J* = 6.7 Hz, 3H), 1.16 (d, *J* = 6.6 Hz, 3H), 1.10 (d, *J* = 12.5 Hz, 2H), 1.02 (d, *J* = 2.8 Hz, 2H), 0.77 (dd, *J* = 6.8, 4.5 Hz, 5H).<br>MS m/z = 634 [M+H]+ | P2 was resolv ed from Exam ple **44;** SFC peak retenti on time: 5.826 min |
| **M1** | **C7** | 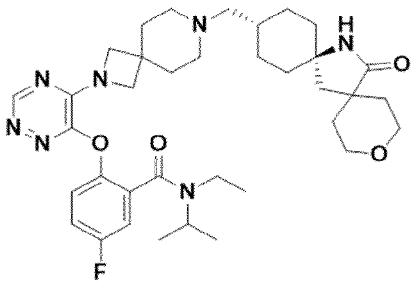<br>Example 45 | $^1$H NMR (400 MHz, Chloroform-d) δ 8.40 (s, 1H), 7.18 (d, *J* = 4.4 Hz, 1H), 7.10 - 7.02 (m, 1H), 6.99 - 6.91 (m, 1H), 5.53 (s, 1H), 4.41 - 4.18 (m, 2H), 3.95 - 3.75 (m, 5H), 3.44 (td, *J* = 11.1, 2.2 Hz, 3H), 3.12 - 2.98 (m, 1H), 2.26 (s, 3H), 2.12 - 1.95 (m, 5H), 1.88 (s, 2H), 1.60 (d, *J* = 12.6 Hz, 3H), 1.41 (t, *J* = 12.6 Hz, 3H), 1.33 - 1.24 (m, 3H), 1.21 - 1.18 (m, 1H), 1.12 - 0.94 (m, 9H), 0.70 (d, *J* = 6.6 Hz, 2H)<br>MS m/z = 664 [M+H]+ | 3.98% |

(continued)

| Raw material 1 | Raw material 2 | Example | Characterization data | Yield and/o r SFC retention time |
|---|---|---|---|---|
| M1 | E4-a | 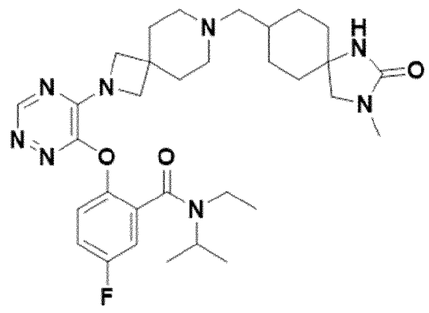 Example 46-1 | $^1$H NMR (400 MHz, Chloroform-d) δ 8.39 (d, *J* = 1.6 Hz, 1H), 7.18 (d, *J* = 4.5 Hz, 1H), 7.09 -7.02 (m, 1H), 6.94 (dd, *J* = 7.8, 3.1 Hz, 1H), 4.38 - 4.18 (m, 2H), 4.14 (s, 1H), 3.85 - 3.74 (m, 3H), 3.44 (s, 1H), 3.12 (s, 2H), 3.08 - 3.00 (m, 1H), 2.71 (s, 3H), 2.26 (s, 4H), 2.03 (s, 2H), 1.62 (s, 6H), 1.33 (td, *J* = 11.6, 9.9, 2.6 Hz, 3H), 1.22 - 1.16 (m, 1H), 1.12-0.83 (m, 10H), 0.70 (d, *J* = 6.6 Hz, 2H) MS m/z = 609 [M+H]$^+$ | 25.94 % SFC peak retenti on time: 4.809 min |
| M1 | E4-b | 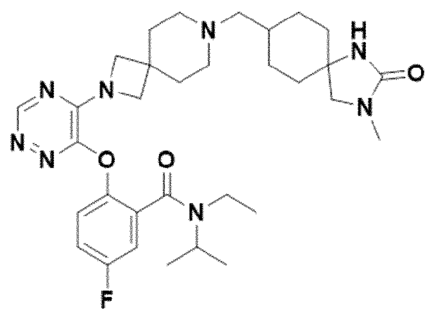 Example 46-2 | MS m/z = 609 [M+H]$^+$ | 21.2% SFC peak retenti on time: 3.754 min |
| M2 | E4-a | 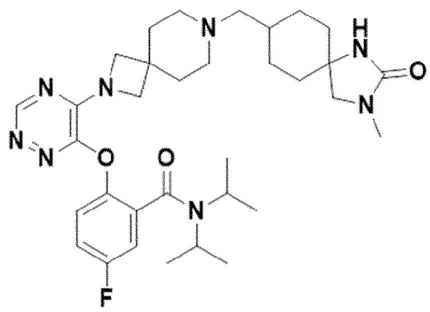 Example 47-1 | MS m/z = 623 [M+H]$^+$ | 3.34% SFC peak retenti on time: 4.517 min |

(continued)

| Raw material 1 | Raw material 2 | Example | Characterization data | Yield and/o r SFC retention time |
|---|---|---|---|---|
| M2 | E4-b | 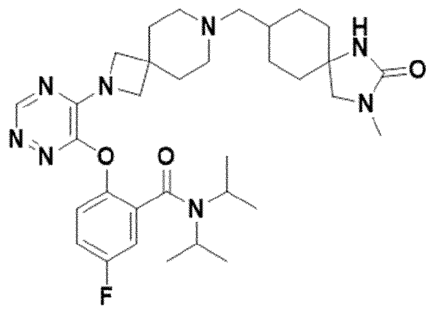 Example 47-1 | MS m/z = 623 $[M+H]^+$ | 19.46 % SFC peak retenti on time: 3.618 min |
| M2 | E2-a | 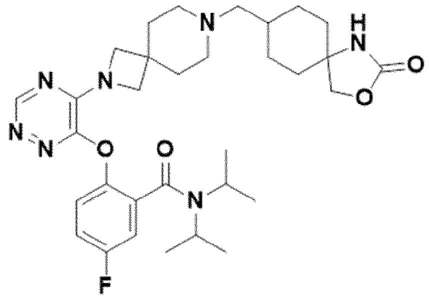 Example 49 | $^1$H NMR (400 MHz, Chloroform-d) δ 8.46 (s, 1H), 7.26 - 7.23 (m, 1H), 7.11 (ddd, J = 9.1, 7.9, 3.1 Hz, 1H), 6.96 (dd, J = 7.8, 3.0 Hz, 1H), 5.20 (s, 1H), 4.48 (s, 1H), 4.29 (d, J = 10.4 Hz, 1H), 4.14 (s, 2H), 3.88 (s, 2H), 3.79 (p, J = 6.6 Hz, 1H), 3.39 (p, J = 6.8 Hz, 1H), 2.32 (s, 3H), 2.09 (s, 2H), 1.96 - 1.74 (m, 7H), 1.67 (s, 2H), 1.44 (s, 6H), 1.38 (d, J = 6.7 Hz, 3H), 1.08 (d, J = 6.6 Hz, 3H), 0.91 (d, J = 14.2 Hz, 2H), 0.71 (d, J = 6.5 Hz, 3H). MS m/z = 610 $[M+H]^+$ | 50.74 % |

(continued)

| Raw material 1 | Raw material 2 | Example | Characterization data | Yield and/o r SFC retention time |
|---|---|---|---|---|
| N1 | E3-a | 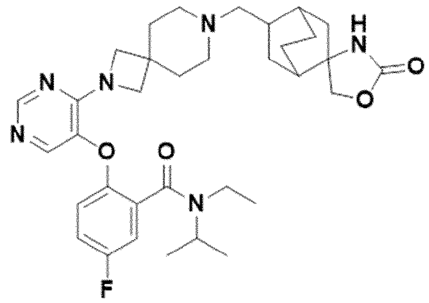<br>Example 50-1 | $^1$H NMR (600 MHz, Chloroform-*d*) δ 8.32 (s, 1H), 7.72 (s, 1H), 6.99 - 6.90 (m, 2H), 6.67 (dd, *J* = 10.0, 4.4 Hz, 1H), 5.89 (s, 1H), 4.30 (d, *J* = 8.5 Hz, 1H), 4.02 - 3.68 (m, 6H), 3.47 - 3.10 (m, 2H), 2.48 - 1.97 (m, 5H), 1. 79 -1.62 (m, 11H), 1.56 -1.35 (m, 4H), 1.18 (t, *J* = 7.0 Hz, 4H), 1.04 (dt, *J* = 34.9, 6.7 Hz, 6H)<br>MS m/z = 621 [M+H]$^+$ | 36.7% (conta ining two isome rs) |
| | | **Example 50-1a** | MS m/z = 621 [M+H]$^+$ | P1 was resolv ed from **Exam ple** 50-1; SFC peak retenti on time: 2.286 min |
| | | **Example 50-1b** | MS m/z = 621 [M+H]$^+$ | P2 was resolv ed from **Exam ple 50-1;** SFC peak retenti on time: 3.908 min |

(continued)

| Raw material 1 | Raw material 2 | Example | Characterization data | Yield and/o r SFC retention time |
|---|---|---|---|---|
| N1 | E3-b | 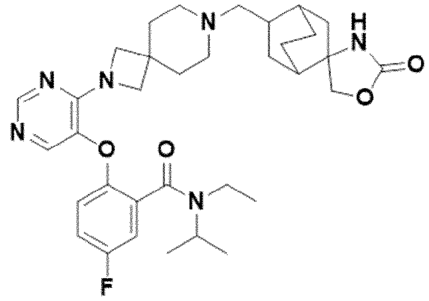 Example 50-2 | $^1$H NMR (600 MHz, Chloroform-d) δ 8.31 (q, *J* = 6.1 Hz, 1H), 7.72 (d, *J* = 19.8 Hz, 1H), 6.95 (qd, *J* = 6.3, 5.8, 2.7 Hz, 2H), 6.69 (dd, *J* = 9.9, 4.3 Hz, 1H), 4.28 (dd, *J* = 11.7, 8.5 Hz, 1H), 4.01 - 3.74 (m, 6H), 3.52 - 3.40 (m, 1H), 3.29 - 3.09 (m, 1H), 2.27 (d, *J* = 72.6 Hz, 5H), 1.75 (d, *J* = 34.3 Hz, 7H), 1.62 (dd, *J* = 13.6, 8.2 Hz, 3H), 1.55 - 1.41 (m, 3H), 1.32 - 1.16 (m, 6H), 1.13 - 1.00 (m, 6H). MS m/z = 621 [M+H]$^+$ | 36% |
| | | **Example 50-2a** | MS m/z = 621 [M+H]$^+$ | P1 was resolv ed from **Exam ple 50-2;** SFC peak retenti on time: 2.925 min |
| | | **Example 50-2b** | MS m/z = 621 [M+H]$^+$ | P2 was resolv ed from **Exam ple 50-2;** SFC peak retenti on time: 3.975 min |

(continued)

| Raw material 1 | Raw material 2 | Example | Characterization data | Yield and/o r SFC retention time |
|---|---|---|---|---|
| N2 | E3-a | 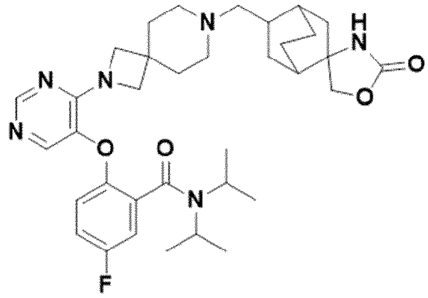<br>Example 51-1 | $^1$H NMR (600 MHz, Chloroform-d) δ 8.37 (s, 1H), 7.77 (s, 1H), 6.99 (t, J = 8.0 Hz, 2H), 6.78 - 6.74 (m, 1H), 4.37 (d, J = 8.5 Hz, 1H), 4.07 - 3.92 (m, 3H), 3.87 (d, J = 9.2 Hz, 2H), 3.79 (p, J = 6.6 Hz, 1H), 3.49 (p, J = 6.8 Hz, 1H), 2.30 (s, 2H), 2.10 (s, 2H), 1.66 (s, 15H), 1.54 (d, J = 6.8 Hz, 4H), 1.48 (d, J = 6.7 Hz, 4H), 1.13 (d, J = 6.7 Hz, 3H), 1.10 (d, J = 6.7 Hz, 3H) MS m/z = 635 [M+H]$^+$ | 15% |
| | | **Example 51-1a** | MS m/z = 635 [M+H]$^+$ | P1 was resolv ed from **Exam ple 51-1;** SFC peak retenti on time: 2.989 min |
| | | **Example 51-1b** | MS m/z = 635 [M+H]$^+$ | P2 was resolv ed from **Exam ple 51-1;** SFC peak retenti on time: 4.302 min |

(continued)

| Raw material 1 | Raw material 2 | Example | Characterization data | Yield and/o r SFC retention time |
|---|---|---|---|---|
| N1 | E3-b | 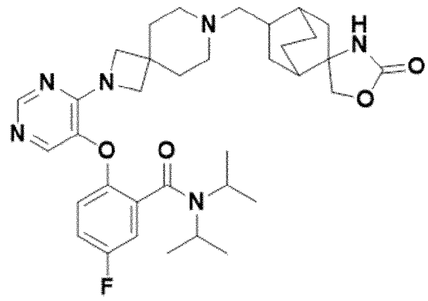<br>Example 51-2 | $^1$H NMR (600 MHz, Chloroform-d) δ 8.37 (s, 1H), 7.77 (s, 1H), 6.98 (d, J = 7.9 Hz, 2H), 6.77 (dd, J = 8.8, 4.1 Hz, 1H), 4.34 (d, J = 8.5 Hz, 1H), 4.06 - 3.92 (m, 3H), 3.89 (d, J = 9.1 Hz, 2H), 3.78 (p, J = 6.6 Hz, 1H), 3.49 (p, J = 6.8 Hz, 1H), 2.38 (s, 4H), 1.91 - 1.67 (m, 12H), 1.65 -1.57 (m, 2H), 1.54 (d, J = 7.0 Hz, 4H), 1.48 (d, J = 6.8 Hz, 4H), 1.36 (ddd, J = 14.6, 7.5, 3.5 Hz, 1H), 1.13 (d, J = 6.7 Hz, 3H), 1.10 (d, J = 6.7 Hz, 3H)<br>MS m/z = 635 [M+H]+ | 14.5% |
| | | **Example 51-2a** | MS m/z = 635 [M+H]+ | P1 was resolv ed from **Example 51-2;** SFC peak retenti on time: 2.896 min |
| | | **Example 51-2b** | MS m/z = 635 [M+H]+ | P2 was resolv ed from **Exam ple 51-2;** SFC peak retenti on time: 4.215 min |

(continued)

| Raw material 1 | Raw material 2 | Example | Characterization data | Yield and/o r SFC retention time |
|---|---|---|---|---|
| M1 | E3-a | 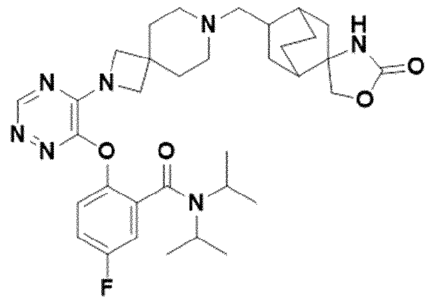 Example 52-1 | $^1$H NMR (600 MHz, Chloroform-*d*) δ 8.46 (s, 1H), 7.26 - 7.24 (m, 1H), 7.11 (td, *J* = 8.6, 3.1 Hz, 1H), 6.96 (dd, *J* = 7.8, 3.1 Hz, 1H), 4.48 (s, 1H), 4.37 (d, *J* = 8.5 Hz, 1H), 4.29 (s, 1H), 4.02 (d, *J* = 8.5 Hz, 1H), 3.92 - 3.84 (m, 2H), 3.79 (p, *J* = 6.6 Hz, 1H), 3.39 (p, *J* = 6.7 Hz, 1H), 2.34 (s, 4H), 2.12 (s, 3H), 1.83 - 1.60 (m, 14H), 1.50 (d, *J* = 6.8 Hz, 3H), 1.38 (d, *J* = 6.7 Hz, 3H), 1.08 (d, *J* = 6.7 Hz, 3H), 0.71 (d, *J* = 6.6 Hz, 3H). MS m/z = 636 [M+H]$^+$ | 15.3% |
| | | **Example 52-1a** | MS m/z = 636 [M+H]$^+$ | P1 was resolv ed from **Exam ple 52-1;** SFC peak retenti on time: 2.735 min |
| | | **Example 52-1b** | MS m/z = 636 [M+H]$^+$ | P2 was resolv ed from **Exam ple 52-1;** SFC peak retenti on time: 3.833 min |

(continued)

| Raw material 1 | Raw material 2 | Example | Characterization data | Yield and/o r SFC retention time |
|---|---|---|---|---|
| M2 | E3-b | 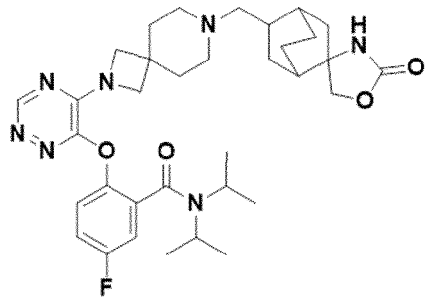 Example 52-2 | $^1$H NMR (600 MHz, Chloroform-d) δ 8.46 (s, 1H), 7.25 (d, J = 4.3 Hz, 1H), 7.11 (dt, J = 11.5, 5.8 Hz, 1H), 6.96 (dd, J = 7.8, 3.1 Hz, 1H), 4.50 (s, 1H), 4.33 (d, J = 8.7 Hz, 2H), 4.04 (dd, J = 16.0, 8.4 Hz, 1H), 3.93 - 3.86 (m, 2H), 3.79 (p, J = 6.7 Hz, 1H), 3.39 (p, J = 6.9 Hz, 1H), 2.23 (s, 7H), 1.92 - 1.65 (m, 14H), 1.50 (d, J = 6.7 Hz, 3H), 1.38 (d, J = 6.9 Hz, 3H), 1.08 (d, J = 6.6 Hz, 3H), 0.72 (d, J = 6.5 Hz, 3H). MS m/z = 636 [M+H]$^+$ | 14.8% |
| | | **Example 52-2a** | MS m/z = 636 [M+H]$^+$ | P1 was resolv ed from **Exam ple 52-2;** SFC peak retenti on time: 2.402 min |
| | | **Example 52-2b** | MS m/z = 636 [M+H]$^+$ | P2 was resolv ed from **Exam ple 52-2;** SFC peak retenti on time: 3.942 min |

**Synthesis of Example 54**

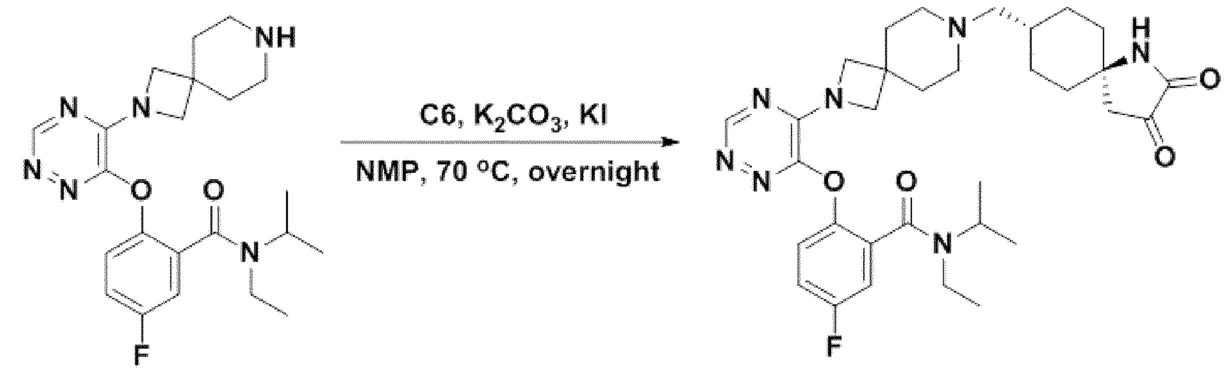


M1

Example 54

**C6** (132 mg, 0.284 mmol, TF) was dissolved in NMP (3 mL), added with KI (94.79 mg, 0.571 mmol), and stirred at room temperature for 0.5 h. Then **M1** (177 mg, 0.38 mmol, Cl) and $K_2CO_3$ (525 mg, 3.81 mmol) were added. After replacement with nitrogen, the mixture was heated to 70°C and stirred overnight. After the reaction was completed, the mixture was diluted with water and extracted three times with EA. The organic phases were combined, washed with saturated brine, dried over anhydrous sodium sulfate and concentrated to obtain a crude product, which was purified by pre-HPLC (10 mM $NH_4HCO_3$ aqueous solution/acetonitrile system) to obtain **Example 54** (1.55 mg, 0.002 mmol, 5.9%). $^1$H NMR (400 MHz, Chloroform-d) δ 8.40 (d, J = 1.5 Hz, 1H), 7.21 (d, J = 4.5 Hz, 0H), 7.18 (s, 0H), 7.06 (ddd, J = 7.9, 6.4, 3.1 Hz, 1H), 6.95 (ddd, J = 13.5, 7.9, 3.0 Hz, 1H), 5.91 (d, J = 1.9 Hz, 1H), 5.84 (s, 1H), 4.46 - 4.21 (m, 2H), 3.92 - 3.74 (m, 3H), 3.48 (d, J = 6.1 Hz, 3H), 3.09 (d, J = 54.9 Hz, 5H), 1.89 - 1.82 (m, 4H), 1.77 (d, J = 14.0 Hz, 2H), 1.62 (dd, J = 12.8, 3.6 Hz, 2H), 1.58 - 1.55 (m, 2H), 1.50 (d, J = 18.5 Hz, 2H), 1.26 - 1.13 (m, 3H), 1.11 - 0.94 (m, 7H), 0.70 (d, J = 6.6 Hz, 2H). MS m/z = 608 $[M+H]^+$.

**Synthesis of Example 56**

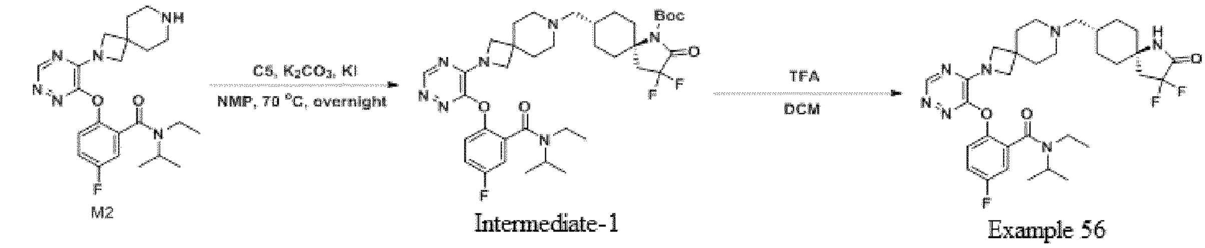


M2

Intermediate-1

Example 56

**C5** (45 mg, 95.3 μmol) was dissolved in NMP (1 mL), added with KI (19.39 mg, 116.8 μmol), and stirred at room temperature for 0.5 h. Then **M2** (44 mg, 77.87 μmol) and $K_2CO_3$ (59.51 mg, 431.22 μmol) were added. After replacement with nitrogen, the mixture was heated to 70°C and stirred overnight. After the reaction was completed, the mixture was diluted with water and extracted three times with EA. The organic phases were combined, washed with saturated brine, dried over anhydrous sodium sulfate and concentrated to obtain a crude product, which was purified by pre-HPLC (10 mM $NH_4HCO_3$ aqueous solution/acetonitrile system) to obtain **Intermediate-1** (15 mg, 20.55 μmol, yield 26.39%).

**Intermediate-1** (15 mg, 20.55 μmol) was dissolved in DCM (0.5 mL), added with TFA (0.5 mL) dropwise, and stirred at room temperature for 1 h. After the reaction was completed, the mixture was concentrated to obtain a crude product which was purified by pre-HPLC to obtain **Example 56** (1.16 mg, 1.84 μmol, yield 8.9%). MS m/z = 630 $[M+H]^+$.

Referring to the synthesis method of Example **56,** the following example compounds were obtained:

| Raw material 1 | Raw material 2 | Example | Characterization data | Remark |
|---|---|---|---|---|
| **M2** | **C5** | 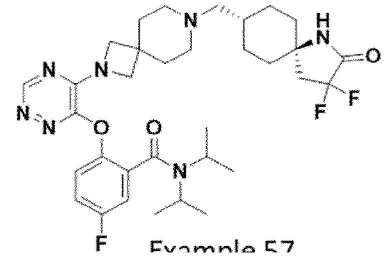 Example 57 | MS m/z = 644 $[M+H]^+$ | 6.9% |
| **M1** | **D4-a1** | 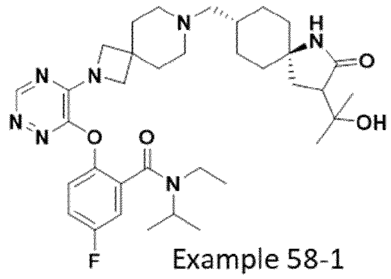 Example 58-1 | MS m/z = 652 $[M+H]^+$ | 92% |
| | | **Example 58-1a** | $^1H$ NMR (400 MHz, $CDCl_3$) δ 8.45 (s, 1H), 7.27 (s, 1H), 7.24 (d, *J* = 4.5 Hz, 1H), 7.12 (d, *J* = 3.1 Hz, 1H), 7.00 (dd, *J* = 7.8, 2.9 Hz, 1H), 5.84 (s, 1H), 4.84 (s, 1H), 4.46 - 4.18 (m, 2H), 3.86 (s, 3H), 3.51 (s, 1H), 3.12 (s, 1H), 2.68 (t, *J* = 10.0 Hz, 1H), 2.32 (s, 3H), 2.21 (dd, *J* = 12.7, 9.7 Hz, 2H), 2.11 (s, 2H), 1.82 (s, 7H), 1.69 (s, 6H), 1.40 (dd, *J* = 20.2, 7.8 Hz, 3H), 1.20 (d, *J* = 9.9 Hz, 6H), 1.14 (t, *J* = 6.9 Hz, 4H), 1.08 (d, *J* = 5.0 Hz, 3H), 0.76 (d, *J* = 5.7 Hz, 2H). MS m/z = 652 $[M+H]^+$ | P1 was resolved from **Example 58-1;** SFC peak retention time: 2.200 min |
| | | **Example 58-1b** | $^1H$ NMR (400 MHz, $CDCl_3$) δ 8.46 (s, 1H), 7.27 (d, *J* = 2.2 Hz, 1H), 7.24 (d, *J* = 4.5 Hz, 1H), 7.17 - 7.09 (m, 1H), 7.00 (dd, *J* = 7.8, 3.0 Hz, 1H), 5.73 (s, 1H), 4.82 (s, 1H), 4.51 - 4.20 (m, 2H), 3.86 (s, 3H), 3.52 (d, *J* = 6.3 Hz, 1H), 3.11 (d, *J* = 6.3 Hz, 1H), 2.68 (t, *J* = 10.0 Hz, 1H), 2.32 (s, 3H), 2.26 - 2.17 (m, 2H), 2.11 (s, 2H), 1.82 (s, 5H), 1.64 (s, 8H), 1.46 - 1.36 (m, 3H), 1.20 (d, *J* = 9.9 Hz, 7H), 1.13 (d, *J* = 7.0 Hz, 4H), 1.08 (d, *J* = 5.8 Hz, 2H), 0.76 (d, *J* = 5.9 Hz, 2H). MS m/z = 652 $[M+H]^+$ | P2 was resolved from **Example 58-1;** SFC peak retention time: 2.995 min |

(continued)

| Raw material 1 | Raw material 2 | Example | Characterization data | Remark |
|---|---|---|---|---|
| M2 | D4-a1 | 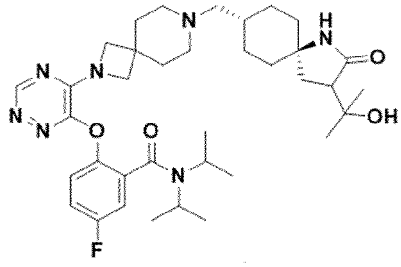<br>Example 59-1 | MS m/z = 666 [M+H]+ | 96% |
| | | **Example 59-1a** | $^1$H NMR (400 MHz, $CDCl_3$) δ 8.45 (s, 1H), 7.24 (d, *J* = 4.5 Hz, 1H), 7.13 - 7.06 (m, 1H), 6.95 (dd, *J* = 7.8, 3.0 Hz, 1H), 5.76 (s, 1H), 4.83 (s, 1H), 4.48 (d, *J* = 8.9 Hz, 1H), 4.28 (d, *J* = 9.9 Hz, 1H), 3.87 (s, 2H), 3.78 (dd, *J* = 13.1, 6.5 Hz, 1H), 3.43 - 3.33 (m, 1H), 2.68 (t, *J* = 10.0 Hz, 1H), 2.33 (s, 3H), 2.22 (dd, *J* = 12.8, 9.6 Hz, 2H), 2.12 (s, 2H), 1.82 (s, 7H), 1.67 (s, 6H), 1.50 (d, *J* = 6.7 Hz, 4H), 1.38 (d, *J* = 6.3 Hz, 4H), 1.20 (d, *J* = 9.7 Hz, 6H), 1.08 (d, *J* = 6.5 Hz, 4H), 0.71 (d, *J* = 6.5 Hz, 3H).<br>MS m/z = 666 [M+H]+ | P1 was resolved from **Example 59-1;** SFC peak retention time: 3.001 min |
| | | **Example 59-1b** | $^1$H NMR (400 MHz, $CDCl_3$) δ 8.45 (s, 1H), 7.23 (s, 1H), 7.15 - 7.05 (m, 1H), 6.95 (dd, *J* = 7.8, 2.9 Hz, 1H), 5.77 (s, 1H), 4.83 (s, 1H), 4.48 (d, *J* = 9.9 Hz, 1H), 4.28 (d, *J* = 10.0 Hz, 1H), 3.87 (s, 2H), 3.78 (dt, *J* = 13.2, 6.6 Hz, 1H), 3.44 - 3.33 (m, 1H), 2.68 (t, *J* = 10.0 Hz, 1H), 2.32 (s, 3H), 2.22 (dd, *J* = 12.8, 9.6 Hz, 1H), 2.11 (d, *J* = 5.2 Hz, 2H), 1.81 (s, 5H), 1.66 (s, 6H), 1.50 (d, *J* = 6.7 Hz, 4H), 1.38 (d, *J* = 6.2 Hz, 4H), 1.20 (d, *J* = 9.9 Hz, 6H), 1.08 (d, *J* = 6.4 Hz, 4H), 0.71 (d, *J* = 6.5 Hz, 3H).<br>MS m/z = 666 [M+H]+ | P2 was resolved from **Example 59-1;** SFC peak retention time: 3.860 min |

(continued)

| Raw material 1 | Raw material 2 | Example | Characterization data | Remark |
|---|---|---|---|---|
| M1 | D5-a | 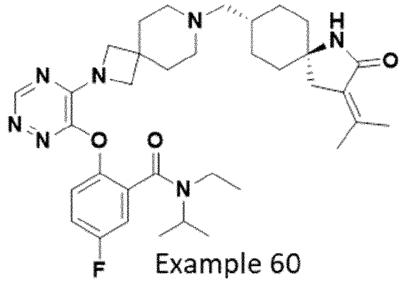 Example 60 | $^1$H NMR (400 MHz, $CDCl_3$) δ 8.46 (s, 1H), 7.27 (s, 1H), 7.25 (d, *J* = 4.6 Hz, 1H), 7.16 - 7.08 (m, 1H), 7.00 (dd, *J* = 7.9, 3.0 Hz, 1H), 5.44 (s, 1H), 4.34 (dd, *J* = 23.1, 16.7 Hz, 2H), 3.86 (d, *J* = 6.9 Hz, 3H), 3.51 (s, 1H), 3.10 (s, 1H), 2.50 (s, 2H), 2.32 (s, 3H), 2.24 (s, 3H), 2.11 (s, 2H), 1.79 (d, *J* = 22.4 Hz, 8H), 1.66 (s, 7H), 1.39 (t, *J* = 11.5 Hz, 3H), 1.09 (ddd, *J* = 23.9, 14.0, 7.0 Hz, 9H), 0.76 (d, *J* = 6.0 Hz, 2H). MS m/z = 634 $[M+H]^+$ | 85% |
| M2 | D5-a | 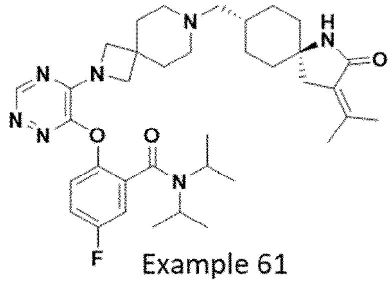 Example 61 | $^1$H NMR (400 MHz, $CDCl_3$) δ 8.45 (s, 1H), 7.26 - 7.22 (m, 1H), 7.13 - 7.06 (m, 1H), 6.95 (dd, *J* = 7.8, 3.0 Hz, 1H), 5.48 (s, 1H), 4.47 (d, *J* = 10.1 Hz, 1H), 4.28 (d, *J* = 10.2 Hz, 1H), 3.97 - 3.71 (m, 3H), 3.49 - 3.31 (m, 1H), 2.50 (s, 2H), 2.28 (d, *J* = 33.5 Hz, 7H), 2.11 (d, *J* = 5.2 Hz, 2H), 1.76 (s, 10H), 1.67 (d, *J* = 12.9 Hz, 2H), 1.50 (d, *J* = 6.7 Hz, 4H), 1.38 (d, *J* = 6.5 Hz, 6H), 1.07 (d, *J* = 6.6 Hz, 4H), 0.71 (d, *J* = 6.5 Hz, 3H). MS m/z = 648 $[M+H]^+$ | 87% |

**Synthesis of Example 62**

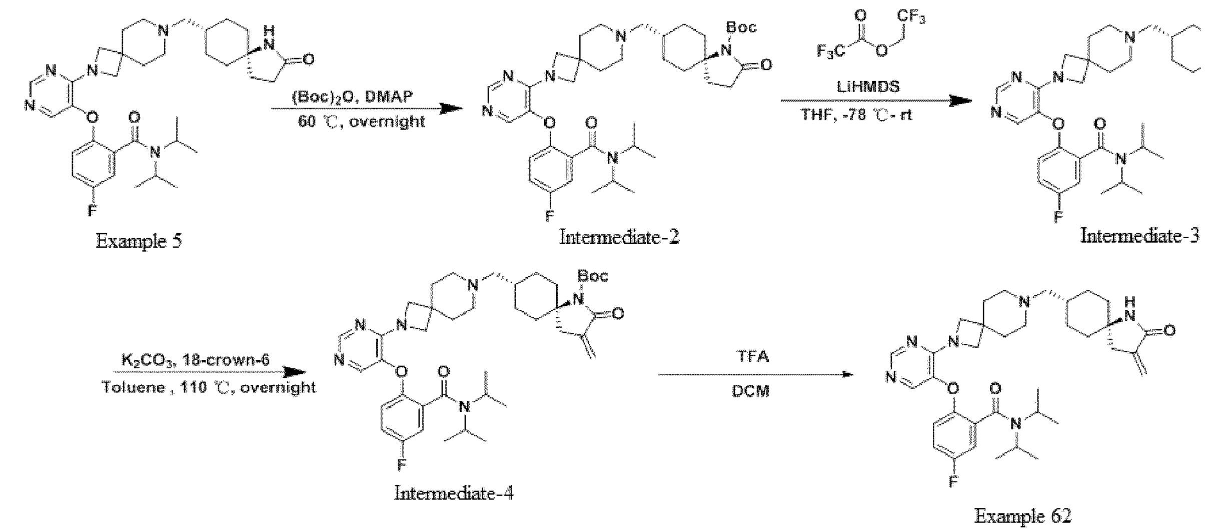

Example 5
Intermediate-2
Intermediate-3
Intermediate-4
Example 62

**Example 5** (230 mg, 0.38 mmol), $(Boc)_2O$ (124 mg, 0.57 mmol), DMAP (9 mg, 0.076 mmol) and TEA (78 mg, 0.76 mmol) were dissolved in DCM (3 mL) and reacted at 60°C overnight under nitrogen protection. After the reaction was completed, the mixture was directly concentrated to obtain a crude product which was purified by silica gel column (PE/EA= 5:1-3:1, v/v) to obtain **Intermediate-2** (190 mg, 0.236 mmol, yield 62.1%). MS m/z = 707 $[M+H]^+$.

**Intermediate-2** (95 mg, 134.39 μmol) was dissolved in THF (2 mL), cooled to -78°C, slowly added with LiHMDS (0.2 mL, 268.78 μmol) dropwise, and stirred at this temperature for 1 h. Then ethyl trifluoroacetate (81.86 mg, 416.62 μmol) was added, warmed to room temperature and stirred overnight. After the reaction was completed, the system was moved to an ice bath, quenched with saturated aqueous ammonium chloride, and extracted three times with EA. The organic phases were combined, washed with saturated brine, dried over anhydrous sodium sulfate and concentrated to obtain a crude product, which was purified by silica gel column to obtain the crude **Intermediate-3.** MS m/z = 803 $[M+H]^+$.

**Intermediate-3** (60 mg, 74.73 μmol) and paraformaldehyde (78.53 mg, 2.62 mmol) were dissolved in toluene (2 mL), then added with potassium carbonate (32.02 mg, 231.66 μmol) and 1,8-crown ether-6 (5.93 mg, 22.42 μmol), heated to 110°C and stirred overnight. After the reaction was completed, the mixture was diluted with water and extracted three times with EA. The organic phases were combined, washed with saturated brine, dried over anhydrous sodium sulfate and concentrated to obtain a crude product, which was purified by silica gel column (PE/EA= 1:1, v/v) to obtain **Intermediate-4** (20 mg, 27.82 μmol, yield 37.23%). MS m/z = 719 $[M+H]^+$.

**Intermediate-4** (4 mg, 5.48 μmol) was dissolved in DCM (0.5 mL), added with TFA(0.5 mL) dropwise, and stirred at room temperature for 1 h. After the reaction was completed, the mixture was concentrated to obtain a crude product which was purified by pre-HPLC to obtain **Example 62** (1.16 mg, 1.84 μmol, yield 33.58%). MS m/z = 619 $[M+H]^+$.

**Synthesis of Example 63**

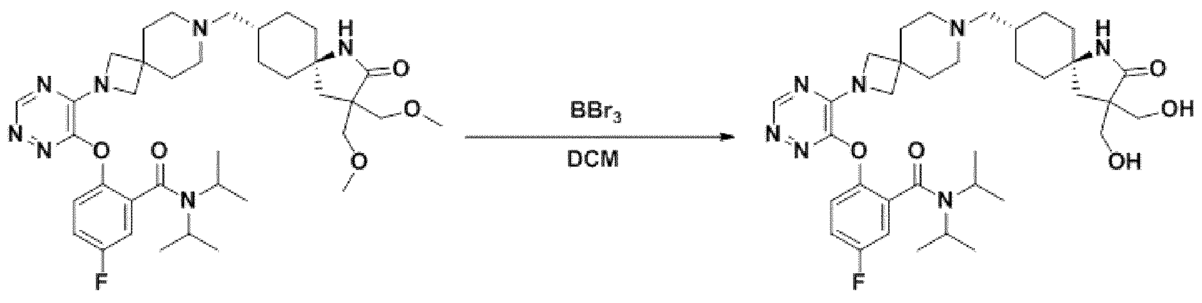


Example 20 Example 63

**Example 20** (70 mg, 100 μmol) was dissolved in anhydrous DCM (1 mL), cooled to - 70°C, and $BBr_3$ solution (0.6 mL, 600 μmol) was slowly added dropwise. After the addition was completed, the mixture was naturally heated to room temperature and stirred overnight. After the reaction was completed, the mixture was concentrated to obtain a crude product which was purified by pre-HPLC (10 mM $NH_4HCO_3$ aqueous solution/acetonitrile system) to obtain **Example 63** (10 mg, 14.9 μmol, yield 14.9%). $^1H$ NMR (400 MHz, Chloroform-*d*) δ 8.45 (s, 1H), 7.24 (d, *J* = 4.5 Hz, 1H), 7.10 (ddd, *J* = 9.0, 7.8, 3.0 Hz, 1H), 6.95 (dd, *J* = 7.8, 3.0 Hz, 1H), 5.75 (s, 1H), 4.47 (d, *J* = 10.4 Hz, 1H), 4.28 (d, *J* = 10.3 Hz, 1H), 3.87 (s, 2H), 3.81 - 3.68 (m, 5H), 3.39 (p, *J* = 6.7 Hz, 1H), 2.33 (s, 3H), 2.10 (d, *J* = 5.6 Hz, 2H), 1.85 - 1.74 (m, 11H), 1.57 - 1.31 (m, 10H), 1.08 (d, *J* = 6.5 Hz, 4H), 0.71 (d, *J* = 6.4 Hz, 3H).

**Synthesis of Example 64 and Example 65**

Referring to the synthesis method of Example 1, the following example compounds were obtained by reacting raw material 1 and raw material 2:

| Raw mat eria l 1 | Raw mate rial 2 | Example | Characterization data | Yield and/o r SFC retent ion time |
|---|---|---|---|---|
| N6 | A1-b | 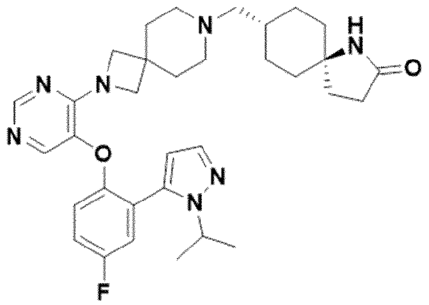 Example 64 | $^1$H NMR (600 MHz, Methanol-d4) δ 8.20 (s, 1H), 7.69 (s, 1H), 7.57 (d, J = 1.8 Hz, 1H), 7.28 (ddd, J = 9.0, 7.8, 3.1 Hz, 1H), 7.22 (dd, J = 8.4, 3.1 Hz, 1H), 7.08 (dd, J = 9.1, 4.5 Hz, 1H), 6.27 (d, J = 1.9 Hz, 1H), 4.41 (p, J = 6.6 Hz, 1H), 3.77 (s, 4H), 2.35 (dd, J = 8.6, 7.5 Hz, 5H), 2.16 (d, J = 6.9 Hz, 2H), 1.96 (t, J = 8.1 Hz, 2H), 1.82 - 1.67 (m, 9H), 1.53 (qd, J = 12.3, 11.5, 5.7 Hz, 3H), 1.39 (d, J = 6.6 Hz, 6H), 1.14 - 1.04 (m, 2H). MS m/z = 636 $[M+H]^+$ | 20% |
| M8 | A1-b | 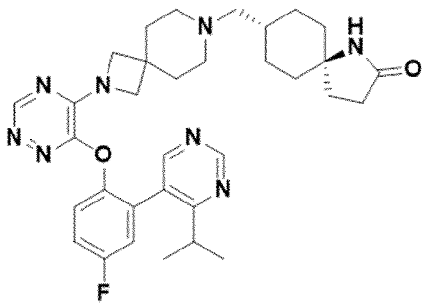 Example 65 | $^1$H NMR (400 MHz, Methanol-$d_4$) δ 9.12 (s, 1H), 8.59 (s, 1H), 8.38 (s, 1H), 7.45 - 7.40 (m, 1H), 7.38 (d, *J* = 8.9 Hz, 1H), 7.34 (dd, *J* = 6.1, 2.8 Hz, 1H), 4.43 (s, 2H), 3.95 (s, 2H), 3.14 - 3.02 (m, 1H), 2.54 - 2.31 (m, 6H), 2.19 (d, *J* = 6.9 Hz, 2H), 1.97 (t, *J* = 8.1 Hz, 2H), 1.90 (t, *J* = 5.4 Hz, 4H), 1.85 - 1.76 (m, 2H), 1.71 (d, *J* = 12.8 Hz, 2H), 1.54 (td, *J* = 13.0, 3.8 Hz, 3H), 1.23 (d, *J* = 6.7 Hz, 6H), 1.17 - 1.03 (m, 2H). MS m/z = 601 $[M+H]^+$ | 46% |

The SFC chiral resolution peak retention times and conditions of some of the example compounds in the above table are shown in the table below

| Example | SFC chiral resolution peak retention times and conditions |
|---|---|
| **Example 3-1** | P1 was resolved from **Example 3;** SFC peak retention time: 2.807 min Chiral column model: CHIRALPAKAS; Specification: 3μm,150mm*3mm; Mobile phase A-$CO_2$, Mobile phase B-0.1%DEA/ethanol, A/B=70/30: Flow rate: 1mL/min: Column temperature: 40°C |
| **Example 3-2** | P2 was resolved from **Example 3;** SFC peak retention time: 3.623 min Chiral column model: CHIRALPAK AS; Specification: 3μm,150mm*3mm; Mobile phase A-$CO_2$, Mobile phase B-0.1%DEA/ethanol, A/B=70/30: Flow rate: 1mL/min: Column temperature: 40°C |

(continued)

| Example | SFC chiral resolution peak retention times and conditions |
|---|---|
| **Example 4-1** | P1 was resolved from Example **4;** SFC peak retention time: 2.845 min Chiral column model: CHIRALPAK AS; Specification: 3μm,150mm*3mm; Mobile phase A-$CO_2$, Mobile phase B-0.1%DEA/ethanol, A/B=70/30: Flow rate: 1mL/min: Column temperature: 40°C |
| **Example 4-2** | P2 was resolved from Example **4;** SFC peak retention time: 3.643 min Chiral column model: CHIRALPAK AS; Specification: 3μm,150mm*3mm; Mobile phase A-$CO_2$, Mobile phase B-0.1%DEA/ethanol, A/B=70/30: Flow rate: 1mL/min: Column temperature: 40°C |
| **Example 13-1a** | P1 was resolved from Example **13-1;** SFC peak retention time: 2.836 min Chiral column model: CHIRALPAK AS; Specification: 3μm,150mm*3mm; Mobile phase A-$CO_2$, Mobile phase B-0.1%DEA/ethanol, A/B=72/28: Flow rate: 1mL/min: Column temperature: 40°C |
| **Example 13-1b** | P2 was resolved from Example **13-1;** SFC peak retention time: 3.922 min Chiral column model: CHIRALPAK AS; Specification: 3μm,150mm*3mm; Mobile phase A-$CO_2$, Mobile phase B-0.1%DEA/ethanol, A/B=72/28: Flow rate: 1mL/min: Column temperature: 40°C |
| **Example 13-2a** | P1 was resolved from Example **13-2;** SFC peak retention time: 2.774 min Chiral column model: CHIRALPAK AS; Specification: 3μm,150mm*3mm; Mobile phase A-$CO_2$, Mobile phase B-0.1%DEA/ethanol, A/B=72/28: Flow rate: 1mL/min: Column temperature: 40°C |
| **Example 13-2b** | P1 was resolved from Example **13-2;** SFC peak retention time: 3.953 min Chiral column model: CHIRALPAK AS; Specification: 3μm,150mm*3mm; Mobile phase A-$CO_2$, Mobile phase B-0.1%DEA/ethanol, A/B=72/28: Flow rate: 1mL/min: Column temperature: 40°C |
| **Example 14-1a** | P1 was resolved from **14-1;** SFC peak retention time: 3.189 min Chiral column model: CHIRALPAK AD; Specification: 3μm,150mm*3mm; Mobile phase A-$CO_2$, Mobile phase B-0.1%DEA/ethanol, A/B=65/35: Flow rate: 1mL/min: Column temperature: 40°C |
| **Example 14-1b** | P2 was resolved from **14-1;** SFC peak retention time: 3.690 min Chiral column model: CHIRALPAK AD; Specification: 3μm,150mm*3mm; Mobile phase A-$CO_2$, Mobile phase B-0.1%DEA/ethanol, A/B=65/35: Flow rate: 1mL/min: Column temperature: 40°C |
| **Example 15-1a** | P1 was resolved from **15-1;** SFC peak retention time: 2.611 min Chiral column model: CHIRALPAK AD; Specification: 3μm,150mm*3mm; Mobile phase A-$CO_2$, Mobile phase B-0.1%DEA/ethanol, A/B=68/32: Flow rate: 1mL/min: Column temperature: 40°C |

(continued)

| Example | SFC chiral resolution peak retention times and conditions |
|---|---|
| **Example 15-1b** | P2 was resolved from **15-1;**<br>SFC peak retention time: 2.993 min<br>Chiral column model: CHIRALPAK AD; Specification: 3μm,150mm*3mm;<br>Mobile phase A-$CO_2$, Mobile phase B-0.1%DEA/ethanol, A/B=68/32: Flow rate: 1mL/min: Column temperature: 40°C |
| **Example 16-1a** | P1 was resolved from Example **16-1;**<br>SFC peak retention time: 4.798 min<br>Chiral column model: CHIRALPAK AD; Specification: 3μm,150mm*3mm;<br>Mobile phase A-$CO_2$, Mobile phase B-0.1%DEA/ethanol, A/B=60/40: Flow rate: 1mL/min: Column temperature: 40°C |
| **Example 16-1b** | P2 was resolved from Example **16-1**;SFC peak retention time: 5.302 min<br>Chiral column model: CHIRALPAK AD; Specification: 3μm,150mm*3mm;<br>Mobile phase A-$CO_2$, Mobile phase B-0.1%DEA/ethanol, A/B=60/40: Flow rate: 1mL/min: Column temperature: 40°C |
| **Example 16-2a** | P1 was resolved from **16-2;** SFC peak retention time: 3.471 min<br>Chiral column model: CHIRALPAK AD; Specification: 3μm,150mm*3mm;<br>Mobile phase A-$CO_2$, Mobile phase B-0.1%DEA/ethanol, A/B=60/40: Flow rate: 1mL/min: Column temperature: 40°C |
| **Example 16-2b** | P2 was resolved from **16-2**;SFC peak retention time: 4.697 min<br>Chiral column model: CHIRALPAK AD; Specification: 3μm,150mm*3mm;<br>Mobile phase A-$CO_2$, Mobile phase B-0.1%DEA/ethanol, A/B=60/40: Flow rate: 1mL/min: Column temperature: 40°C |
| **Example 44-1** | P1 was resolved from Example **44;**<br>SFC peak retention time: 1.507 min<br>Chiral column model: CHIRALPAK AS; Specification: 3μm,150mm*3mm;<br>Mobile phase A-$CO_2$, Mobile phase B-0.1%DEA/ethanol, A/B=75/25: Flow rate: 1mL/min: Column temperature: 40°C |
| **Example 44-2** | P2 was resolved from Example **44;**<br>SFC peak retention time: 5.826 min<br>Chiral column model: CHIRALPAK AS; Specification: 3μm,150mm*3mm;<br>Mobile phase A-$CO_2$, Mobile phase B-0.1%DEA/ethanol, A/B=75/25: Flow rate: 1mL/min: Column temperature: 40°C |
| **Example 46-1** | SFC peak retention time: 4.809 min<br>Chiral column model: CHIRALCEL OD; Specification: 3μm,150mm*3mm;<br>Mobile phase A-$CO_2$, Mobile phase B-0.1%DEA/ethanol, A/B=78/22: Flow rate: 1mL/min: Column temperature: 40°C |
| **Example 46-2** | SFC peak retention time: 3.754 min<br>Chiral column model: CHIRALCEL OD; Specification: 3μm,150mm*3mm;<br>Mobile phase A-$CO_2$, Mobile phase B-0.1%DEA/ethanol, A/B=78/22: Flow rate: 1mL/min: Column temperature: 40°C |
| **Example 47-1** | SFC peak retention time: 4.517 min<br>Chiral column model: CHIRALCEL OD; Specification: 3μm,150mm*3mm;<br>Mobile phase A-$CO_2$, Mobile phase B-0.1%DEA/ethanol, A/B=78/22: Flow rate: 1mL/min: Column temperature: 40°C |

(continued)

| Example | SFC chiral resolution peak retention times and conditions |
|---|---|
| **Example 47-2** | SFC peak retention time: 3.618 min<br>Chiral column model: CHIRALCEL OD; Specification: 3μm,150mm*3mm;<br>Mobile phase A-$CO_2$, Mobile phase B-0.1%DEA/ethanol, A/B=78/22: Flow rate: 1mL/min: Column temperature: 40°C |
| **Example 50-1a** | P1 was resolved from **Example 50-1;**<br>SFC peak retention time: 2.286 min<br>Chiral column model: CHIRALPAK AS; Specification: 3μm,150mm*3mm;<br>Mobile phase A-$CO_2$, Mobile phase B-0.1%DEA/ethanol, A/B=72/28: Flow rate: 1mL/min: Column temperature: 40°C |
| **Example 50-1b** | P2 was resolved from **Example 50-1;**<br>SFC peak retention time: 3.908 min<br>Chiral column model: CHIRALPAK AS; Specification: 3μm,150mm*3mm;<br>Mobile phase A-$CO_2$, Mobile phase B-0.1%DEA/ethanol, A/B=72/28: Flow rate: 1mL/min: Column temperature: 40°C |
| **Example 50-2a** | P1 was resolved from **Example 50-2;**<br>SFC peak retention time: 2.925 min<br>Chiral column model: CHIRALPAK AS; Specification: 3μm,150mm*3mm;<br>Mobile phase A-$CO_2$, Mobile phase B-0.1%DEA/ethanol, A/B=72/28: Flow rate: 1mL/min: Column temperature: 40°C |
| **Example 50-2b** | P2 was resolved from **Example 50-2;**<br>SFC peak retention time: 3.975 min<br>Chiral column model: CHIRALPAK AS; Specification: 3μm,150mm*3mm;<br>Mobile phase A-$CO_2$, Mobile phase B-0.1%DEA/ethanol, A/B=72/28: Flow rate: 1mL/min: Column temperature: 40°C |
| **Example 51-1a** | P1 was resolved from **Example 51-1;**<br>SFC peak retention time: 2.989 min<br>Chiral column model: CHIRALPAK AS; Specification: 3μm,150mm*3mm;<br>Mobile phase A-$CO_2$, Mobile phase B-0.1%DEA/ethanol, A/B=75/25: Flow rate: 1mL/min: Column temperature: 40°C |
| **Example 51-1b** | P2 was resolved from **Example 51-1;**<br>SFC peak retention time: 4.302 min<br>Chiral column model: CHIRALPAK AS; Specification: 3μm,150mm*3mm;<br>Mobile phase A-$CO_2$, Mobile phase B-0.1%DEA/ethanol, A/B=75/25: Flow rate: 1mL/min: Column temperature: 40°C |
| **Example 51-2a** | P1 was resolved from **Example 51-2;**<br>SFC peak retention time: 2.896 min<br>Chiral column model: CHIRALPAK AS; Specification: 3μm,150mm*3mm;<br>Mobile phase A-$CO_2$, Mobile phase B-0.1%DEA/ethanol, A/B=75/25: Flow rate: 1mL/min: Column temperature: 40°C |
| **Example 51-2b** | P2 was resolved from **Example 51-2;**<br>SFC peak retention time: 4.215 min<br>Chiral column model: CHIRALPAK AS; Specification: 3μm,150mm*3mm;<br>Mobile phase A-$CO_2$, Mobile phase B-0.1%DEA/ethanol, A/B=75/25: Flow rate: 1mL/min: Column temperature: 40°C |

(continued)

| Example | SFC chiral resolution peak retention times and conditions |
|---|---|
| **Example 52-1a** | P1 was resolved from **Example 52-1;**<br>SFC peak retention time: 2.735min<br>Chiral column model: CHIRALPAK AS; Specification: 3μm,150mm*3mm;<br>Mobile phase A-$CO_2$, Mobile phase B-0.1%DEA/ethanol, A/B=72/28: Flow rate: 1mL/min: Column temperature: 40°C |
| **Example 52-1b** | P2 was resolved from **Example 52-1;**<br>SFC peak retention time: 3.833min<br>Chiral column model: CHIRALPAK AS; Specification: 3μm,150mm*3mm;<br>Mobile phase A-$CO_2$, Mobile phase B-0.1%DEA/ethanol, A/B=72/28: Flow rate: 1mL/min: Column temperature: 40°C |
| **Example 52-2a** | P1 was resolved from **Example 52-2;**<br>SFC peak retention time: 2.402min<br>Chiral column model: CHIRALPAK AS; Specification: 3μm,150mm*3mm;<br>Mobile phase A-$CO_2$, Mobile phase B-0.1%DEA/ethanol, A/B=72/28: Flow rate: 1mL/min: Column temperature: 40°C |
| **Example 52-2b** | P2 was resolved from **Example 52-2;**<br>SFC peak retention time: 3.942min<br>Chiral column model: CHIRALPAK AS; Specification: 3μm,150mm*3mm;<br>Mobile phase A-$CO_2$, Mobile phase B-0.1%DEA/ethanol, A/B=72/28: Flow rate: 1mL/min: Column temperature: 40°C |
| **Example 58-1a** | P1 was resolved from **Example 58-1;**<br>SFC peak retention time: 2.200 min<br>Chiral column model: CHIRALPAK AD; Specification: 3μm,150mm*3mm;<br>Mobile phase A-$CO_2$, Mobile phase B-0.1%DEA/isopropyl alcohol, A/B=65/35: Flow rate: 1mL/min: Column temperature: 40°C |
| **Example 58-1b** | P2 was resolved from **Example 58-1;**<br>SFC peak retention time: 2.995 min<br>Chiral column model: CHIRALPAK AD; Specification: 3μm,150mm*3mm;<br>Mobile phase A-$CO_2$, Mobile phase B-0.1%DEA/isopropyl alcohol, A/B=65/35: Flow rate: 1mL/min: Column temperature: 40°C |
| **Example 59-1a** | P1 was resolved from **Example 59-1;**<br>SFC peak retention time: 3.001 min<br>Chiral column model: CHIRALPAK AD; Specification: 3μm,150mm*3mm;<br>Mobile phase A-$CO_2$, Mobile phaseB-isopropyl alcohol, A/B=72/28: Flow rate: 1mL/min: Column temperature: 40°C |
| **Example 59-1b** | P2 was resolved from **Example 59-1;**<br>SFC peak retention time: 3.860 min<br>Chiral column model: CHIRALPAK AD; Specification: 3μm,150mm*3mm;<br>Mobile phase A-$CO_2$, Mobile phaseB-isopropyl alcohol, A/B=72/28: Flow rate: 1mL/min: Column temperature: 40°C |

In order to illustrate the absolute configuration of the compounds of the present disclosure, crystals of intermediates **A1-a** (FIG. 1), **AI-b** (FIG. 2), **7-5a1** (FIG. 3), **7-5a2** (FIG. 4) and **Example 19** (FIG. 5) were cultured and obtained. The instrument parameters are as follows: detection instrument: Bruker D8 Venture; instrument model: D8 Venture; light source: gallium target; X -ray: Ga (λ = 1.34 Å). The technical effects of the present disclosure are illustrated by test examples below:

**Test Example 1: Menin-MLL interaction inhibitory activity assay**

The inhibition of Menin/MLL-4-43 peptide interaction by small molecule inhibitors was quantitatively detected by fluorescence polarization competition assay. The experiment was performed in a 384-well plate (Corning, Cat# 3575) using a reaction buffer consisting of 50 mM Tris, pH 7.5, 50 mM NaCl, and 1 mM DTT. A 40 μL reaction system included 10 μL of 8 nM Menin recombinant protein and 10 μL of the test compound at different concentrations. The compound was pre-incubated with Menin protein for 15 min, and then 20 μL of 10 nM FITC-MI,L4-43-peptide was added. After incubation on a shaker at 25°C for 60 min, the fluorescence polarization signal (FP 485 520 520) was read using BMG PHERAStar detection. The experimental data was analyzed and processed by GraphPad Prism 6 software to obtain the $IC_{50}$ value. The control compound 1 was prepared by referring to the method of Example 64 in WO2017214367.

**Table 1. Menin-MLL interaction inhibitory activity assay**

| Example No. | $IC_{50}$ (nM) | Example No. | $IC_{50}$ (nM) |
|---|---|---|---|
| Control compound 1 | 156.1 | Example 38-1 | 16.3 |
| Example 1 | 36.3 | Example 38-2 | 2.8 |
| Example 3 | 24 | Example 39 | 23.8 |
| Example 3-1 | 29 | Example 40-1 | 14.8 |
| Example 3-2 | 113 | Example 40-2 | 34.9 |
| Example 4 | 29 | Example 41-1 | 10.8 |
| Example 4-1 | 12.1 | Example 41-2 | 22.4 |
| Example 4-2 | 60.7 | Example 42-1 | 15.3 |
| Example 5 | 34 | Example 42-2 | 44.5 |
| Example 7 | 62.1 | Example 43-1 | 12.4 |
| Example 8 | 67.2 | Example 43-2 | 48.7 |
| Example 11 | 166.9 | Example 44-1 | 48.8 |
| Example 13-1 | 21.3 | Example 44-2 | 7.9 |
| Example 13-2 | 24.9 | Example 45 | 2.5 |
| Example 13-1a | 176.6 | Example 46-1 | 35.3 |
| Example 13-1b | 33.4 | Example 47-1 | 20.2 |
| Example 13-2a | 16.5 | Example 49 | 42 |
| Example 13-2b | 142.4 | Example 50-1 | 36 |
| Example 14-1 | 102.9 | Example 50-2 | 39.9 |
| Example 14-1a | 49.2 | Example 50-1b | 21.8 |
| Example 15-1 | 90.9 | Example 50-2a | 21.1 |
| Example 15-1a | 34.7 | Example 50-2b | 61.1 |
| Example 15-1b | 141.6 | Example 51-1 | 35.3 |
| Example 16-2 | 163.1 | Example 51-2 | 47.6 |
| Example 17 | 5.0 | Example 51-1b | 46.7 |
| Example 18 | 6.3 | Example 51-2a | 70.7 |
| Example 19 | 3.3 | Example 51-2b | 141.2 |
| Example 20 | 6.0 | Example 52-1 | 134.7 |
| Example 21 | 9.8 | Example 52-2 | 93.9 |
| Example 22 | 16.6 | Example 52-1b | 38.7 |

(continued)

| Example No. | $IC_{50}$ (nM) | Example No. | $IC_{50}$ (nM) |
|---|---|---|---|
| Example 23 | 12 | Example 52-2a | 81.9 |
| Example 25 | 31.2 | Example 52-2b | 198.1 |
| Example 26 | 7.4 | Example 58-1 | 2.9 |
| Example 27 | 16.3 | Example 58-1a | 5.4 |
| Example 28 | 133.9 | Example 58-1b | 5.9 |
| Example 30 | 33 | Example 59-1 | 2.5 |
| Example 32 | 39.3 | Example 59-la | 7.8 |
| Example 33 | 8.4 | Example 59-1b | 10.3 |
| Example 34 | 6.4 | Example 60 | 28.4 |
| Example 35 | 3.6 | Example 61 | 25.5 |
| Example 36 | 4.4 | Example 62 | 19.9 |
| Example 37-1 | 12.9 | Example 63 | 8.3 |
| Example 37-2 | 3.3 | Example 64 | 33.2 |

**Test Example 2: Test of the inhibitory ability of Menin-MLL interaction inhibitors on cell proliferation**

The inhibitory ability of the compounds of the present disclosure on the proliferation of tumor cell lines (including cell lines containing MLL fusion protein such as MV4-11, MOLM-13, THP-1 and NOMO-1, control cell lines without MLL fusion protein such as HL-60, K562 and MOLM-16, and cell lines containing NPM1 mutation such as OCI-AML3) was evaluated by using the cell viability analysis method. The cells were plated in 96-well plates at a certain concentration (e.g., 5,000-20,000 cells/well), and then an equal volume of culture medium containing 2 times the final concentration of the test compound (the final concentration ranging from 1 nM to 10 μM) was added. The plates were placed in an incubator and cultured at 37°C and 5% $CO_2$ for 72-168 h. Before the test, an equal volume of CellTiter-Glo® Luminescent reagent was added, and the plate was incubated at room temperature for 10 min before detection using an ELISA reader (BMG LABTECH). The data were analyzed using GraphPad Prism software to obtain the $IC_{50}$ value and compound fitting curve. The control compound 1 was prepared by referring to the method of Example 64 in WO2017214367.

**Table 2. Test of the inhibitory ability of Menin-MLL interaction inhibitors on cell proliferation**

| No. | MV4-11 ($GI_{50}$/nM), 72 h | MOLM-13 ($GI_{50}$/nM), 168 h | OCI-AML3 ($GI_{50}$/nM), 168 h |
|---|---|---|---|
| Control compound 1 | 82.7 | | |
| Example 1 | 76.2 | 580.2 | 277.1 |
| Example 3 | 94.7 | | |
| Example 3-1 | 323.6 | 47.4 | 66.5 |
| Example 3-2 | 86.1 | | |
| Example 4 | 61 | | |
| Example 4-1 | 55 | | |
| Example 5 | 47.8 | 46.5 | 241.5 |
| Example 7 | 137 | 389.3 | 323 |
| Example 8 | 196.9 | 96.4 | 141.6 |
| Example 13-1 | 82.6 | | |
| Example 13-2 | 49.7 | | |
| Example 13-1b | 55.2 | 63.3 | 31.2 |

(continued)

| No. | MV4-11 ($GI_{50}$/nM), 72 h | MOLM-13 ($GI_{50}$/nM), 168 h | OCI-AML3 ($GI_{50}$/nM), 168 h |
|---|---|---|---|
| Example 13-2a | 25.2 | | |
| Example 13-2b | 152.9 | | |
| Example 14-1a | 86.1 | | |
| Example 15-1a | 56.6 | 154.2 | 82.5 |
| Example 16-1 | 129.9 | | |
| Example 17 | 13.8 | 7.9 | 14.2 |
| Example 18 | 12.2 | 12.8 | 21.2 |
| Example 19 | 13.2 | 33.1 | 28.5 |
| Example 20 | 14.2 | 36.1 | 35.2 |
| Example 21 | 13.1 | | 39.8 |
| Example 22 | 11.3 | | 24 |
| Example 23 | 20 | 157.3 | 77.9 |
| Example 25 | 6.9 | 86.3 | 64.3 |
| Example 26 | 19.2 | 162.1 | 94 |
| Example 27 | 13 | | 32.1 |
| Example 30 | 35.2 | 197.1 | 52 |
| Example 32 | 17.8 | 179.1 | 63.6 |
| Example 33 | 20.5 | | |
| Example 34 | 12.6 | | |
| Example 35 | 11.7 | | |
| Example 36 | 13.1 | | |
| Example 37-1 | 88.3 | 353.8 | 182.6 |
| Example 37-2 | 15.2 | 46.5 | 40.9 |
| Example 38-1 | 53.8 | 194.4 | 115.1 |
| Example 38-2 | 13.1 | 23.3 | 21.8 |
| Example 39 | 20 | | |
| Example 40-1 | 41.2 | 121.5 | 198.9 |
| Example 40-2 | 82.8 | | |
| Example 41-1 | 25.5 | 52.7 | 64.1 |
| Example 42-1 | 45.4 | 215.6 | 110.9 |
| Example 43-1 | 27.8 | 132.6 | 92 |
| Example 43-2 | 90 | 137.4 | 80.6 |
| Example 44-1 | 16.7 | 111.2 | 60.5 |
| Example 44-2 | 9.7 | 30.4 | 26.4 |
| Example 45 | 12.7 | 54.1 | 48.4 |
| Example 46-1 | 100.3 | 329.4 | 140.1 |
| Example 47-1 | 118.2 | | |
| Example 49 | 51.3 | 109.8 | 108.1 |

(continued)

| No. | MV4-11 ($GI_{50}$/nM), 72 h | MOLM-13 ($GI_{50}$/nM), 168 h | OCI-AML3 ($GI_{50}$/nM), 168 h |
|---|---|---|---|
| Example 50-1 | 74.4 | | |
| Example 50-2 | 69.1 | | |
| Example 50-1b | 26.4 | 88.9 | 61.7 |
| Example 50-2a | 20.5 | 125.3 | 56.4 |
| Example 50-2b | 26.4 | 93.3 | 119.3 |
| Example 51-1 | 21.1 | 101.1 | 68.5 |
| Example 51-2 | 18.8 | 93.3 | 35.3 |
| Example 51-1b | 16.1 | | 20.3 |
| Example 51-2a | 11.7 | | 31.5 |
| Example 52-1b | 11.9 | | 40 |
| Example 52-2a | 43.6 | | 47.9 |
| Example 52-2b | | | 50.6 |
| Example 58-1 | 17.2 | 88.9 | 42.4 |
| Example 58-1a | 17.7 | 54.4 | 22.9 |
| Example 58-1b | 18 | 56.9 | 30.2 |
| Example 59-1 | 15.3 | 55.9 | 34.7 |
| Example 59-1a | 9.6 | 36.2 | 18.5 |
| Example 59-1b | 9.5 | 34.3 | 20.1 |
| Example 60 | 35 | 175.2 | 92.4 |
| Example 61 | 23 | 107.5 | 72.1 |
| Example 62 | 34.8 | | |
| Example 64 | 82.7 | | |

**Test Example 3: Test of stability of compounds in liver microsome**

Test purpose: To determine the stability of some of the compounds of the present disclosure in mouse, dog and human liver microsomes by LC-MS/MS method.

Test materials: The test drugs were in-house prepared example compounds of the present disclosure; the positive reference compound SNDX-5613 was prepared by the method of Example 253 in patent WO2017214367; the control compound 1 was prepared by the method of Example 64 in WO2017214367; liver microsomes were purchased from CORNING.

Test method:

The total volume of each incubation system was approximately 45 μL, and the medium was 100 mM phosphate buffer (PBS, pH 7.4), including liver microsomal protein at a final concentration of 0.5 mg/mL, 1.00 μM compound and 2.00 mM NADPH, and the organic phase content was <1%. Incubation was performed in a 37°C incubator, and 135 μL of ice-cold acetonitrile was added after 0, 5, 15, 30 and 60 min of reaction to terminate the reaction. The positive control group was incubated with 1.00 μM Ketanserin, 0.5 mg/mL liver microsomal protein and 2.00 mM NADPH in a 37°C incubator for 0, 5, 15, 30 and 60 min respectively, and then 135 μL of ice-cold acetonitrile was added to terminate the reaction. The 96-well plates were shaken at 600 rpm for 10 min and then centrifuged at 4700 rpm at 4°C for 15 min. 80 μL of supernatant was mixed with 320 μL of pure water, and the remaining amount of the compound was detected by LC-MS/MS method. The *in vitro* half life (T1/2) and intrinsic clearance (CLint) were calculated according to the following formula:

$$T_{1/2} = 0.693/k;$$

Intrinsic clearance (Clint) = (0.693/$T_{1/2}$) × (1/liver microsome concentration) × conversion factor. k is the linear regression slope of the remaining percentage of the ln compound-incubation time. The conversion factor is as follows:

| Species | Microsomal protein per gram of liver (mg/g) | Liver weight per kg body weight (g/kg) | Conversion factor* | Liver blood flow rate (ml/min/kg) |
|---|---|---|---|---|
| Mouse | 45 | 87.5 | 3937.5 | 90 |
| Dog | 77.9 | 32 | 2492.8 | 30.9 |
| Human | 48.8 | 25.7 | 1254.2 | 20.7 |

The specific test results are shown in Table 3.

**Table 3.** Stability of test compounds in mouse, human and dog liver microsomes

| Example No. | Half life $T_{1/2}$ (min) | | |
|---|---|---|---|
| | Mouse | Human | Dog |
| **SNDX-5613** | 70.71 | 11.99 | 63 |
| **Control compound 1** | 3.75 | 4.77 | |
| **Example-7** | >120 | 77.87 | 60.26 |
| **Example-8** | 66.63 | 47.14 | 93.65 |
| **Example-14-1a** | >120 | 57.27 | |
| **Example-15-1a** | 91.18 | 36.86 | >120 |
| **Example-17** | >120 | 31.33 | >120 |
| **Example-19** | >120 | 80.93 | >120 |
| **Example-20** | 110.93 | 74.92 | >120 |
| **Example-23** | 48.46 | 36.28 | |
| **Example-26** | 79.66 | 37.06 | 62.43 |
| **Example-37-1** | >120 | >120 | >120 |
| **Example-37-2** | >120 | 65.25 | 90.16 |
| **Example-38-1** | >120 | 87.97 | >120 |
| **Example-38-2** | 65.01 | 62.70 | 98.15 |
| **Example-42-1** | >120 | 45.41 | 79.86 |
| **Example-43-1** | 70.03 | 34.89 | 35.42 |
| **Example-45** | >120 | 37.07 | 91.94 |
| **Example-46-1** | >120 | 78.54 | >120 |
| **Example-47-1** | >120 | 46.8 | 77.00 |
| **Example-58-1a** | >120 | 23.73 | 69.32 |
| **Example-58-1b** | >120 | 35.58 | 68.37 |
| **Example-59-1b** | 98.96 | 28.73 | 70.16 |

Compared with the positive compound SNDX-5613, some example compounds of the present disclosure have

better metabolic stability to liver microsomes, and especially have significant advantages in the stability in human liver microsomes.

## Test Example 4. *In vivo* pharmacokinetic study

Test purpose: To determine the pharmacokinetic properties of some of the example compounds of the present disclosure in mice, rats and dogs after single intravenous injection (i.v.) and oral gavage (i.g.) administration by LC-MS/MS method.

Research methods: An appropriate amount of compound was weighed and prepared into a transparent clear solution of a certain concentration using 0.9% sodium chloride injection and 1.5 equivalents of 1M HCl aqueous solution based on the molar number of the compound. After SPF male ICR mice, SPF male SD rats, and male beagle dogs were fasted overnight, the test compound solution was given by intravenous injection and oral gavage at the corresponding dose. Anticoagulated whole blood of the test animals was collected at 5 min, 15 min, 30 min, 1 h, 2 h, 4 h, 8 h, and 24 h after administration, and plasma was separated. Plasma samples at each time point were detected by LC-MS/MS, and the plasma concentration of the compound was determined by the standard curve correction method. Phoenix WinNonlin 5.2 non-compartmental model was used to calculate the pharmacokinetic parameters of terminal elimination half-life (t1/2), blood peak concentration (Cmax), area under the concentration-time curve (AUC), clearance (CL), and bioavailability (F%), which was directly obtained from the serum concentration results. Blood drug concentration and pharmacokinetic parameters are expressed as mean ± standard deviation (X ± SD). The specific test protocols for each species are as follows.

### Pharmacokinetics test in mouse

Test animals: SPF male ICR mice, weighing 25-30 g, 12 mice/compound, purchased from Chengdu Dossy Experimental Animal Co., Ltd.

Test design: On the day of the test, ICR mice were randomly divided into groups according to body weight, fasted but with free water for 12-14 h one day before administration, and fed 2 h after administration.

Preparation of test compound solution: The concentration of the test compound for oral gavage was 1 mg/mL. An appropriate amount of compound was weighed and prepared into a 1 mg/mL transparent clear solution using 0.9% sodium chloride injection and 1.5 equivalents of 1M HCl aqueous solution. The concentration of the test compound for intravenous injection was 0.2 mg/mL. The 1 mg/mL transparent clear solution was diluted to a concentration of 0.2 mg/mL using 0.9% sodium chloride injection. The volume of 1 M HCl solution added was calculated as: volume of 1 M HCl solution added (mL) = weighed amount of compound (mg)/molecular weight × 1.5 × 1000. The amount added during preparation must not exceed the calculated value.

The test substance was administered as follows: intravenous injection: 1 mg/kg of dose, 5 mL/kg of volume, 6 mice; oral gavage: 10 mg/kg of dose, 10 mL/kg of volume, 6 mice.

Sample collection: Blood (40-50 μl) was collected in anticoagulant tubes sprayed with EDTA-K2 through the orbital venous plexus at 5 min, 15 min, 30 min, 1 h, 2 h, 4 h, 8 h, and 24 h after administration, and centrifuged at 10,000 rpm for 20 min within 1 h (the blood was saved on wet ice before and after centrifugation). The supernatant, which was plasma, was collected and frozen in a refrigerator at -20°C or below for LC-MS/MS analysis of Full PK of male ICR mice (2 mice per group, 4 blood collection time points per mouse, crossover).

Table 4. Pharmacokinetic parameters[a] of compounds in mice

| Example No. | Main PK parameters for oral administration, p.o. (10 mg/kg) | | | |
|---|---|---|---|---|
| | $T_{1/2}$ (h) | $C_{max}$ (ng/mL) | $AUC_{last}$ (hr*ng/mL) | F, % |
| **SNDX-5613** | 1.53 | 1296 | 2483 | 88 |
| **Example-1** | 1.51 | 2475 | 5294 | 79 |
| **Example-5** | 1.37 | 2621 | 7696 | 84 |
| **Example-5** | 1.91 | 2535 | 8285 | 72 |
| **Example-13-1b** | 3.84 | 2014 | 7766 | 72 |
| **Example-15-1a** | 1.17 | 3026 | 6409 | 60 |
| **Example-17** | 1.12 | 2125 | 3217 | 56 |
| **Example-18** | 1.34 | 1962 | 4040 | 75 |

(continued)

| Example No. | Main PK parameters for oral administration, p.o. (10 mg/kg) | | | |
|---|---|---|---|---|
| | $T_{1/2}$ (h) | $C_{max}$ (ng/mL) | $AUC_{last}$ (hr*ng/mL) | F, % |
| **Example-20** | 1.88 | 919 | 2178 | 84 |
| **Example-23** | 2.52 | 1513 | 2652 | 119 |
| **Example-38-1** | 1.06 | 2264 | 5058 | 67 |
| **Example-38-2** | 0.83 | 824 | 1976 | 55 |
| **Example-49** | 1.10 | 1346 | 2320 | 77 |
| Note: [a] 1mg/kg for intravenous injection | | | | |

Compared with the positive compound SNDX-5613, some example compounds of the present disclosure have superior pharmacokinetic properties and significant advantages especially in terms of exposure amount and oral bioavailability.

**Pharmacokinetics test in rats**

Test animals: SPF male SD rats, weighing 180-220 g, 6 rats/compound, purchased from Chengdu Dossy Experimental Animal Co., Ltd.

Test design: On the day of the test, SD rats were randomly divided into groups according to body weight, fasted but with free water for 12-14 h one day before administration, and fed 2 h after administration.

Preparation of test compound solution: The concentration of the test compound for oral gavage was 1 mg/mL. An appropriate amount of compound was weighed and prepared into a 1 mg/mL transparent clear solution using 0.9% sodium chloride injection and 1.5 equivalents of 1M HCl aqueous solution. The concentration of the test compound for intravenous injection was 0.5 mg/mL. The 1 mg/mL transparent clear solution was diluted to a concentration of 0.5 mg/mL using 0.9% sodium chloride injection. The volume of 1 M HCl solution added was calculated as: volume of 1 M HCl solution added (mL) = weighed amount of compound (mg)/molecular weight $\times$ 1.5 $\times$ 1000. The amount added during preparation must not exceed the calculated value.

The test substance was administered as follows: intravenous injection: 1 mg/kg of dose, 2 mL/kg of volume, 3 rats; oral gavage: 10 mg/kg of dose, 10 mL/kg of volume, 3 rats.

Sample collection: Blood (40-50 μl) was collected in anticoagulant tubes sprayed with EDTA-K2 through the orbital venous plexus at 5 min, 15 min, 30 min, 1 h, 2 h, 4 h, 8 h, and 24 h after administration, and centrifuged at 10,000 rpm for 20 min within 1 h (the blood was saved on wet ice before and after centrifugation). The supernatant, which was plasma, was collected and froze in a refrigerator at -20°C or below for LC-MS/MS analysis of Full PK of male SD rats (1 rat per group, 8 blood collection time points per rat).

Some of the example compounds of the present disclosure have excellent pharmacokinetic properties in rats, especially in terms of exposure amount and oral bioavailability.

**Pharmacokinetics test in Beagle dogs**

Test animals: Male beagle dogs, weighing 8-10 kg, 6 dogs/compound, purchased from Beijing Marshall Biotechnology Co., Ltd.

Test design: On the day of the test, beagle dogs were randomly divided into groups according to their body weight, fasted but with free water for 12-14 h one day before administration, and fed 4 h after administration.

Preparation of test compound solution: The concentration of the test compound for oral gavage was 5 mg/mL. An appropriate amount of compound was weighed and prepared into a 1 mg/mL transparent clear solution using 0.9% sodium chloride injection and 1.5 equivalents of 1M HCl aqueous solution. The concentration of the test compound for intravenous injection was 1 mg/mL. The 5 mg/mL transparent clear solution was diluted to a concentration of 1 mg/mL using 0.9% sodium chloride injection. The volume of 1 M HCl solution added was calculated as: volume of 1 M HCl solution added (mL) = weighed amount of compound (mg)/molecular weight $\times$ 1.5 $\times$ 1000. The amount added during preparation must not exceed the calculated value.

The test substance was administered as follows: intravenous injection: 1 mg/kg of dose, 1 mL/kg of volume, 3 dogs; oral gavage: 5 mg/kg of dose, 5 mL/kg of volume, 3 dogs.

Sample collection: Blood (500 μl) was collected in anticoagulant tubes sprayed with EDTA-K2 through the

orbital venous plexus at 5 min, 15 min, 30 min, 1 h, 2 h, 4 h, 8 h, and 24 h after administration, and centrifuged within 1 hour (3000 g, 4°C, 5 min). The supernatant, which was plasma, was collected and frozen in a refrigerator at -20°C or below for LC-MS/MS analysis.

Some of the example compounds of the present disclosure have excellent oral bioavailability in beagle dogs.

**Test Example 5: CYP450 enzyme inhibition test**

In this test, human CYP enzyme recombinant protein was incubated with different concentrations (1 and 10 μM) of test compounds and corresponding probe drugs, and the changes in CYP enzyme activity were measured to evaluate the inhibitory ability of the test compounds on each CYP subtype.

The test results show that some of the example compounds of the present disclosure have basically no inhibitory effect on CYP3A4, CYP2C9, CYP2C8, CYP2C19, CYP2D6, and CYP1A2 at a concentration of 10 μM, with an IC50 greater than 10 μM.

**Test Example 6: hERG potassium channel effect test**

The hERG inhibition test was performed on some of the example compounds of the present disclosure using the manual electrophysiological patch clamp method. The cell line was derived from HEK293 cells that overexpressed hERG potassium ion channels. The specific experimental protocol was developed by PharmaCore Labs with reference to literature published in peer-reviewed journals and was performed by PharmaCore Labs in accordance with its standard experimental operating procedures. The highest test concentration was 30 μM. The results are shown in Table 5.

Table 5. Effects of compounds on hERG potassium channels

| No. | hERG $IC_{50}$ (μM) | Average inhibition rate of hERG current at the highest compound tested concentration (%) |
|---|---|---|
| **Example-5** | >30 | 33.09%$^{@30\mu M}$ |
| **Example-19** | >30 | 12.81%$^{@30\mu M}$ |
| **Example-20** | >30 | 13.22%$^{@30\mu M}$ |
| **Example-37-1** | >30 | 13.18%$^{@30\mu M}$ |
| **Example-38-1** | >30 | 10.33%$^{@30\mu M}$ |
| **Example-41-1** | >30 | 13.18%$^{@30\mu M}$ |

The results show that some of the example compounds of the present disclosure have almost no obvious blocking effect on hERG potassium ion channels within the test concentration range, which is significantly better than the reported $IC_{50}$ value of the positive reference compound SNDX-5613 of 5-15 μM.

**Test Example 10. Study on the anti-tumor pharmacodynamics of the example compounds on the BALB/c nude mouse model with subcutaneous transplantation of MV-4-11 cells**

Eight-week-old female BALB/c nude mice were purchased from Zhejiang Vital River Laboratory Animal Technology Co., Ltd. MV-4-11 cells were purchased from Nanjing Cobioer Biotechnology Co., Ltd., and cultured in the culture medium of IMDM plus 10% FBS serum at 37°C and 5% $CO_2$. MV-4-11 cells were cultured *in vitro* (suspension cells), and cells in the logarithmic growth phase were collected. The cells were gently rinsed twice with PBS, and the cell pellet was gently blown with PBS to resuspend the cells to prepare a single cell suspension. After counting, the final cell concentration was adjusted to $1\times10^7$ cells/100 μL, added with completely liquefied Matrigel matrix gel at a ratio of 1:1, and mixed evenly. Each mouse was inoculated with $1\times10^7$ cells in the right axilla, and the inoculation volume was 100 μL/mouse. Tumor growth was observed regularly. On the 8th day after inoculation, when the tumor grew to an average of 131 $mm^3$, the mice were randomly divided and administered according to tumor size and mouse weight (n=8). The example compound was administered by gavage at 15 mg/kg and 30 mg/kg twice a day for 32 consecutive days. A clear solution of the example compound was prepared by mixing with an appropriate amount of 0.9% sodium chloride injection by vortex, and then adding with 1.5 equivalents of 1N HCl hydrochloric acid based on the molar number of the compound to dissolve. During the experiment, animal activity was observed once a day, each animal was weighed once before administration, and the long and short diameters of the tumor were measured with a vernier caliper twice a week. After

32 days of administration, the tumor volume was measured to evaluate the antitumor efficacy of the example compound. At the end of the experiment, all surviving experimental animals were sacrificed. The calculation formula for tumor volume is: $V = 0.5 (a \times b^2)$, where a and b represent the long and short diameters of the tumor, respectively.

The results show that some of the example compounds of the present disclosure show good *in vivo* efficacy at doses of 15 mg/kg and 30 mg/kg.

In summary, the compounds provided by the present disclosure have excellent inhibitory activity on Menin-MLL protein-protein interaction and cell proliferation, and have more significant advantages in safety and bioavailability. These compounds may become new clinical drugs for the treatment of cancer or other diseases mediated by Menin-MLL interactions.

## Claims

1. A compound represented by Formula I, or a deuterated compound, a stereoisomer or a pharmaceutically acceptable salt thereof:

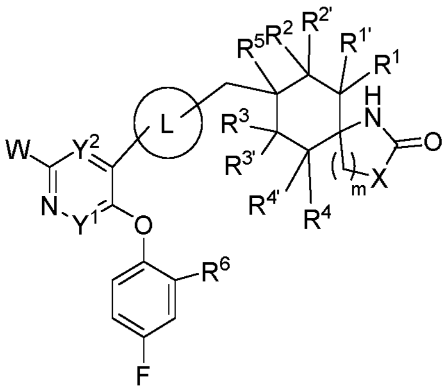

Formula I

wherein,

ring L is

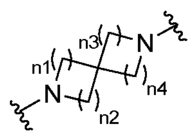

;

wherein n1, n2, n3, n4 are independently 1 or 2;
$Y^1$ and $Y^2$ are independently CH or N;
m is selected from the group consisting of 1, 2 and 3;
W is selected from the group consisting of hydrogen, halogen, cyano, nitro, $-C_{1-6}$ alkyl, $-C_{2-6}$ alkenyl, $-C_{2-6}$ alkynyl, halogen-substituted $-C_{1-6}$ alkyl, halogen-substituted $-C_{2-6}$ alkenyl, halogen-substituted $-C_{2-6}$ alkynyl, $-C_{0-4}$ alkylene-$OR^{W1}$, and $-C_{0-4}$ alkylene-$NR^{W1}R^{W2}$;
$R^{W1}$ and $R^{W2}$ are independently selected from the group consisting of hydrogen, $-C_{1-6}$ alkyl, - $C_{2-6}$ alkenyl, $-C_{2-6}$ alkynyl, 3- to 10-membered carbocyclyl and 4- to 10-membered heterocycloalkyl;
X is selected from the group consisting of $CR^aR^b$, $NR^a$, O and S;
$R^a$ and $R^b$ are independently selected from the group consisting of hydrogen, halogen, cyano, nitro, $-C_{1-6}$ alkyl, deuterium-substituted $-C_{1-6}$ alkyl, $-C_{2-6}$ alkenyl, $-C_{2-6}$ alkynyl, halogen-substituted $-C_{1-6}$ alkyl, halogen-substituted $-C_{2-6}$ alkenyl, halogen-substituted $-C_{2-6}$ alkynyl, $-C_{0-4}$ alkylene-$OR^{A1}$, $-C_{0-4}$ alkylene-$NR^{A1}R^{A2}$, $-C_{0-4}$ alkylene-$NR^{A1}C(O)R^{A2}$, $-C_{0-4}$ alkylene-$C(O)NR^{A1}R^{A2}$, $-C_{0-4}$ alkylene-$C(O)R^{A1}$, $-C_{0-4}$ alkylene-$S(O)_2NR^{A1}R^{A2}$, $-C_{0-4}$ alkylene-$NR^{A1}S(O)_2R^{A2}$, $-C_{0-4}$ alkylene-(3-to 10-membered carbocyclyl), $-C_{0-4}$ alkylene-(4- to 10-membered heterocycloalkyl), $-C_{0-4}$ alkylene-(6- to 10-membered aromatic ring) and $-C_{0-4}$ alkylene-(5- to 10-membered aromatic heterocycle); wherein the alkyl, alkenyl, alkynyl, alkylene, carbocyclyl, heterocycloalkyl, aromatic

ring, or aromatic heterocycle is further optionally substituted by one, two, three or four independent $R^{B1}$;
alternatively, $R^a$, $R^b$ and the atom connecting therewith together form

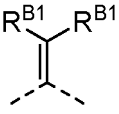 , 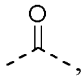, 3- to 10-membered carbocyclyl or 4- to 10-membered heterocycloalkyl; wherein the carbocyclyl or heterocycloalkyl is further optionally substituted by one, two, three or four independent $R^{B1}$;

$R^{A1}$ and $R^{A2}$ are independently selected from the group consisting of hydrogen, $-C_{1-6}$ alkyl, $- C_{2-6}$ alkenyl, $-C_{2-6}$ alkynyl, $-C_{0-4}$ alkylene-$C(O)R^{B1}$, $-C_{0-4}$ alkylene-(3- to 10-membered carbocyclyl), $-C_{0-4}$ alkylene-(4- to 10-membered heterocycloalkyl), $-C_{0-4}$ alkylene-(6- to 10-membered aromatic ring) and $-C_{0-4}$ alkylene-(5- to 10-membered aromatic heterocycle); wherein the alkyl, alkenyl, alkynyl, alkylene, carbocyclyl, heterocycloalkyl, aromatic ring, or aromatic heterocycle is further optionally substituted by one, two, three or four independent $R^{B1}$;

each $R^{B1}$ is independently selected from the group consisting of hydrogen, deuterium, halogen, cyano, nitro, $-C_{1-6}$ alkyl, $-C_{2-6}$ alkenyl, $-C_{2-6}$ alkynyl and $-C_{0-4}$ alkylene-$OR^{C1}$;

$R^{C1}$ is independently selected from the group consisting of hydrogen, $-C_{1-6}$ alkyl, $-C_{2-6}$ alkenyl, $-C_{2-6}$ alkynyl, halogen-substituted $-C_{1-6}$ alkyl, halogen-substituted $-C_{2-6}$ alkenyl and halogen-substituted $-C_{2-6}$ alkynyl;

$R^1$, $R^2$, $R^3$, $R^4$, $R^5$, $R^{1'}$, $R^{2'}$, $R^{3'}$ and $R^{4'}$ are independently selected from the group consisting of hydrogen, halogen, cyano, nitro, $-C_{1-6}$ alkyl, $-C_{2-6}$ alkenyl, $-C_{2-6}$ alkynyl, halogen-substituted $- C_{1-6}$ alkyl, halogen-substituted $-C_{2-6}$ alkenyl, halogen-substituted $-C_{2-6}$ alkynyl, $-C_{0-4}$ alkylene-$OR^{D1}$, $-C_{0-4}$ alkylene-$NR^{D1}R^{D2}$, $-C_{0-4}$ alkylene-(3- to 10-membered carbocyclyl), $-C_{0-4}$ alkylene-(4- to 10-membered heterocycloalkyl), $-C_{0-4}$ alkylene-(6- to 10-membered aromatic ring) and $-C_{0-4}$ alkylene-(5- to 10-membered aromatic heterocycle); wherein the alkyl, alkenyl, alkynyl, alkylene, carbocyclyl, heterocycloalkyl, aromatic ring, or aromatic heterocycle is further optionally substituted by one, two, three or four independent $R^{D3}$;

alternatively, $R^1$ and $R^{1'}$, $R^2$ and $R^{2'}$, $R^3$ and $R^{3'}$, or $R^4$ and $R^{4'}$ connected to the identical atom are independently interconnected to form 3- to 10-membered carbocyclyl, 4- to 10-membered heterocycloalkyl,

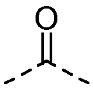 , 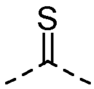 , 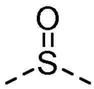 , or 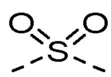 ;

wherein the carbocyclyl or heterocycloalkyl is further optionally substituted by one, two, three or four independent $R^{D3}$;

alternatively, two non-adjacent groups or three groups of $R^1$, $R^2$, $R^3$, $R^4$ and $R^5$ are interconnected, and together with the ring where the atom connecting therewith is located, form 7-to 12-membered bridged cycloalkyl or 7- to 12-membered bridged heterocycloalkyl; wherein the bridged cycloalkyl or bridged heterocycloalkyl is further optionally substituted by one, two, three, or four independent $R^{D3}$;

$R^{D1}$ and $R^{D2}$ are independently selected from the group consisting of hydrogen, $-C_{1-6}$ alkyl, $- C_{2-6}$ alkenyl, $-C_{2-6}$ alkynyl, $-C_{0-4}$ alkylene-(3- to 10-membered carbocyclyl), $-C_{0-4}$ alkylene-(4- to 10-membered heterocycloalkyl), $-C_{0-4}$ alkylene-(6- to 10-membered aromatic ring) and $-C_{0-4}$ alkylene-(5- to 10-membered aromatic heterocycle); wherein the alkyl, alkenyl, alkynyl, alkylene, carbocyclyl, heterocycloalkyl, aromatic ring, or aromatic heterocycle is further optionally substituted by one, two, three or four independent $R^{D4}$;

each $R^{D4}$ is independently selected from the group consisting of hydrogen, $-C_{1-6}$ alkyl, $-C_{2-6}$ alkenyl, $-C_{2-6}$ alkynyl, halogen-substituted $-C_{1-6}$ alkyl, halogen-substituted $-C_{2-6}$ alkenyl and halogen-substituted $-C_{2-6}$ alkynyl;

each $R^{D3}$ is independently selected from the group consisting of hydrogen, halogen, cyano, nitro, oxo, $-C_{1-6}$ alkyl, $-C_{2-6}$ alkenyl, $-C_{2-6}$ alkynyl, halogen-substituted $-C_{1-6}$ alkyl, halogen-substituted $-C_{2-6}$ alkenyl, halogen-substituted $-C_{2-6}$ alkynyl, $-C_{0-4}$ alkylene-$OR^{d1}$, $-C_{0-4}$ alkylene-$OC(O)R^{d1}$, $-C_{0-4}$ alkylene-$SR^{d1}$, $-C_{0-4}$ alkylene-$S(O)_2R^{d1}$, $-C_{0-4}$ alkylene-$S(O)R^{d1}$, $-C_{0-4}$ alkylene$S(O)_2NR^{d1}R^{a2}$, $-C_{0-4}$ alkylene-$S(O)NR^{d1}R^{d2}$, $-C_{0-4}$ alkylene-$S(O)(NH)R^{d1}$, $-C_{0-4}$ alkylene$S(O)(NH)NR^{d1}R^{d2}$, $-C_{0-4}$ alkylene-$C(O)R^{d1}$, $-C_{0-4}$ alkylene-$C(O)OR^{d1}$, $-C_{0-4}$ alkylene-$C(O)NR^{d1}R^{d2}$, $-C_{0-4}$ alkylene-$NR^{d1}R^{d2}$, $-C_{0-4}$ alkylene-$NR^{d1}C(O)R^{d2}$, $-C_{0-4}$ alkylene-$NR^{d1}S(O)_2R^{d2}$, $-C_{0-4}$ alkylene-$NR^{d1}S(O)R^{d2}$, $-C_{0-4}$ alkylene-$P(O)R^{d1}R^{d2}$, $-C_{0-4}$ alkylene-$P(O)(OR^{d1})R^{d2}$, $-C_{0-4}$ alkylene-$P(O)(OR^{d1})(OR^{d2})$, $-C_{0-4}$ alkylene-(3- to 10-membered carbocyclyl), $-C_{0-4}$ alkylene-(4- to 10-membered heterocycloalkyl), $-C_{0-4}$ alkylene-(6- to 10-membered aromatic ring) and $-C_{0-4}$ alkylene-(5- to 10-membered aromatic heterocycle);

$R^{d1}$ and $R^{d2}$ are independently selected from the group consisting of hydrogen, $-C_{1-6}$ alkyl, $- C_{2-6}$ alkenyl, $-C_{2-6}$

alkynyl, halogen-substituted -$C_{1-6}$ alkyl, halogen-substituted -$C_{2-6}$ alkenyl and halogen-substituted -$C_{2-6}$ alkynyl; $R^6$ is selected from the group consisting of hydrogen, -$C_{1-6}$ alkyl, -$C_{2-6}$ alkenyl, -$C_{2-6}$ alkynyl, -$C_{0-4}$ alkylene-C(O)$R^{E1}$, -$C_{0-4}$ alkylene-C(O)O$R^{E1}$, -$C_{0-4}$ alkylene-C(O)N$R^{E1}R^{E2}$, -$C_{0-4}$ alkylene-N$R^{E1}$C(O)$R^{E2}$, -$C_{0-4}$ alkylene-N$R^{E1}$S(O)$_2R^{E2}$, -$C_{0-4}$ alkylene-N$R^{E1}$S(O)$R^{E2}$, -$C_{0-4}$ alkylene-(5- to 10-membered aromatic ring), -$C_{0-4}$ alkylene-(5- to 10-membered heteroaromatic ring), -$C_{0-4}$ alkylene-(3- to 10-membered carbocyclyl), -$C_{0-4}$ alkylene-(4- to 10-membered heterocycloalkyl), - $C_{0-4}$ alkylene-S(O)$R^{E1}$, -$C_{0-4}$ alkylene-S(O)$_2R^{E1}$, -$C_{0-4}$ alkylene-S(O)$_2$N$R^{E1}R^{E2}$, -$C_{0-4}$ alkyleneS(O)(NH)$R^{E1}$, -$C_{0-4}$ alkylene-S(O)(NH)N$R^{E1}R^{E2}$, -$C_{0-4}$ alkylene-O$R^{E1}$, -$C_{0-4}$ alkylene-OC(O)$R^{E1}$, - $C_{0-4}$ alkylene-S$R^{E1}$, -$C_{0-4}$ alkylene-P(O)$R^{E1}R^{E2}$, -$C_{0-4}$ alkylene-P(O)(O$R^{E1}$)$R^{E2}$ and -$C_{0-4}$ alkylene-P(O)(O$R^{E1}$)(O$R^{E2}$); wherein the alkyl, alkylene, alkenyl, alkynyl, carbocyclyl, heterocycloalkyl, aryl or heteroaryl is further optionally substituted by one, two, three or four independent $R^{E5}$;

$R^{E1}$ and $R^{E2}$ are each independently selected from the group consisting of hydrogen, -$C_{1-6}$ alkyl, -$C_{0-4}$ alkylene-(3- to 10-membered carbocyclyl), -$C_{0-4}$ alkylene-(4- to 10-membered heterocycloalkyl), -$C_{0-4}$ alkylene-O$R^{E3}$, -$C_{0-4}$ alkylene-(5- to 10-membered aromatic ring), -$C_{0-4}$ alkylene-(5- to 10-membered heteroaromatic ring), -$C_{0-4}$ alkylene-S(O)$R^{E3}$, -$C_{0-4}$ alkyleneS(O)$_2R^{E3}$, -$C_{0-4}$ alkylene-S(O)$_2$N$R^{E3}R^{E4}$, -$C_{0-4}$ alkylene-S(O)(NH)$R^{E3}$, -$C_{0-4}$ alkyleneS(O)(NH)N$R^{E3}R^{E4}$, -$C_{0-4}$ alkylene-OC(O)$R^{E3}$ and -$C_{0-4}$ alkylene-S$R^{E3}$; wherein the alkyl, alkylene, carbocyclyl, heterocycloalkyl, aryl or heteroaryl is further optionally substituted by one, two, three or four independent $R^{E5}$;

alternatively, $R^{E1}$ and $R^{E2}$ are interconnected to form 4- to 10-membered heterocycloalkyl or 4- to 10-membered bridged heterocycloalkyl; wherein the heterocycloalkyl or bridged heterocycloalkyl is further optionally substituted by one, two, three or four independent $R^{E5}$;

$R^{E3}$ and $R^{E4}$ are independently selected from the group consisting of hydrogen, -$C_{1-6}$ alkyl, - $C_{2-6}$ alkenyl, -$C_{2-6}$ alkynyl, halogen-substituted -$C_{1-6}$ alkyl, halogen-substituted -$C_{2-6}$ alkenyl and halogen-substituted -$C_{2-6}$ alkynyl;

alternatively, $R^{E3}$, $R^{E4}$ and the nitrogen atom connecting therewith together form 4- to 10-membered heterocycloalkyl or 4- to 10-membered bridged heterocycloalkyl; wherein the heterocycloalkyl or bridged heterocycloalkyl is further optionally substituted by one, two, three or four independent $R^{E5}$;

alternatively, $R^{E3}$ and $R^{E4}$ are interconnected to form 4- to 10-membered heterocycloalkyl or 4- to 10-membered bridged heterocycloalkyl; wherein the heterocycloalkyl or bridged heterocycloalkyl is further optionally substituted by one, two, three or four independent $R^{E5}$;

each $R^{E5}$ is independently selected from the group consisting of hydrogen, halogen, cyano, nitro, oxo, -$C_{1-6}$ alkyl, -$C_{2-6}$ alkenyl, -$C_{2-6}$ alkynyl, halogen-substituted -$C_{1-6}$ alkyl, halogen-substituted -$C_{2-6}$ alkenyl, halogen-substituted -$C_{2-6}$ alkynyl, -O($C_{1-6}$ alkyl), -$NH_2$, -$C_{0-4}$ alkylene-(3-to 10-membered carbocyclyl), -$C_{0-4}$ alkylene-(4-to 10-membered heterocycloalkyl), -$C_{0-4}$ alkylene-(5- to 10-membered aromatic ring) and -$C_{0-4}$ alkylene-(5- to 10-membered heteroaromatic ring); wherein the carbocyclyl, heterocycloalkyl, aryl or heteroaryl is further optionally substituted by one, two, three or four independent $R^{E6}$; and

$R^{E6}$ is independently selected from the group consisting of hydrogen, halogen, cyano, nitro, - $C_{1-6}$ alkyl, -$C_{2-6}$ alkenyl, -$C_{2-6}$ alkynyl, halogen-substituted -$C_{1-6}$ alkyl, halogen-substituted -$C_{2-6}$ alkenyl and halogen-substituted -$C_{2-6}$ alkynyl.

**2.** The compound according to claim 1, wherein,

X is $CR^aR^b$ or $NR^a$;

$R^a$ and $R^b$ are independently selected from the group consisting of hydrogen, halogen, cyano, nitro, -$C_{1-6}$ alkyl, deuterium-substituted -$C_{1-6}$ alkyl, -$C_{2-6}$ alkenyl, -$C_{2-6}$ alkynyl, halogen-substituted -$C_{1-6}$ alkyl, halogen-substituted -$C_{2-6}$ alkenyl, halogen-substituted -$C_{2-6}$ alkynyl, -$C_{0-2}$ alkylene-O$R^{A1}$, -$C_{0-2}$ alkylene-N$R^{A1}R^{A2}$, -$C_{0-2}$ alkylene-N$R^{A1}$C(O)$R^{A2}$, -$C_{0-4}$ alkylene-C(O)N$R^{A1}R^{A2}$, -$C_{0-4}$ alkylene-C(O)$R^{A1}$, -$C_{0-4}$ alkylene-S(O)$_2$N$R^{A1}R^{A2}$, -$C_{0-4}$ alkylene-N$R^{A1}$S(O)$_2R^{A2}$, -$C_{0-2}$ alkylene-(3-to 10-membered carbocyclyl), -$C_{0-2}$ alkylene-(4- to 10-membered heterocycloalkyl), -$C_{0-2}$ alkylene-(6- to 10-membered aromatic ring) and -$C_{0-2}$ alkylene-(5- to 10-membered aromatic heterocycle); wherein the alkyl, alkenyl, alkynyl, alkylene, carbocyclyl, heterocycloalkyl, aromatic ring or aromatic heterocycle is further optionally substituted by one, two, three or four independent $R^{B1}$;

alternatively, $R^a$, $R^b$ and the atom connecting therewith together form

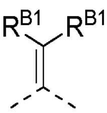 , 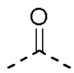 ,

3- to 6-membered carbocyclyl or 4- to 6-membered heterocycloalkyl; wherein the carbocyclyl or heterocycloalkyl is further optionally substituted by one, two, three or four independent $R^{B1}$;

$R^{A1}$ and $R^{A2}$ are independently selected from the group consisting of hydrogen, $-C_{1-6}$ alkyl, $-C_{2-6}$ alkenyl, $-C_{2-6}$ alkynyl, $-C_{1-2}$ alkylene-C(O)$R^{B1}$, $-C_{1-2}$ alkylene-(3- to 10-membered carbocyclyl), $-C_{1-2}$ alkylene-(4- to 10-membered heterocycloalkyl), $-C_{1-2}$ alkylene-(6- to 10-membered aromatic ring) and $-C_{1-2}$ alkylene-(5- to 10-membered aromatic heterocycle); wherein the alkyl, alkenyl, alkynyl, alkylene, carbocyclyl, heterocycloalkyl, aromatic ring or aromatic heterocycle is further optionally substituted by one, two, three or four independent $R^{B1}$;
$Y^1$ and $Y^2$ are independently CH or N;
m is 1 or 2; and
W is selected from the group consisting of hydrogen, methyl, trifluoromethyl, methoxy and methylamino.

**3.** The compound according to claim 2, wherein,

X is $NR^a$; and
$R^a$ is selected from the group consisting of hydrogen, methyl, ethyl, isopropyl, cyclopropyl,

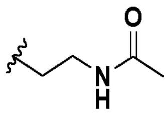, 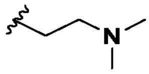, 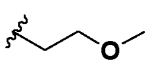, 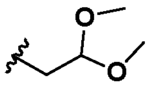, 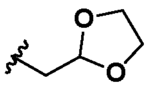, 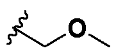, 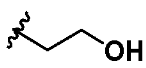,

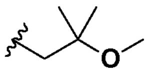 and 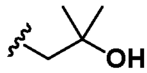.

**4.** The compound according to claim 2, wherein,

X is $CR^aR^b$;
$R^a$ and $R^b$ are independently selected from the group consisting of hydrogen, fluorine, methyl,

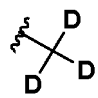,

ethyl, isopropyl,

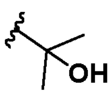, 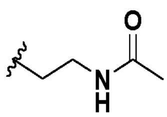, 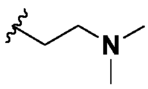, 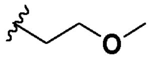, 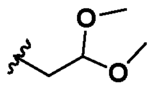,

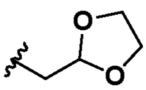, 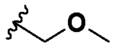, 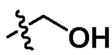, 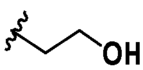, 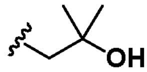 and 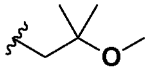;

alternatively, $R^a$, $R^b$ and the atom connecting therewith together form

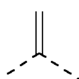, 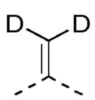, 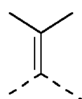,

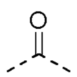, 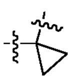, 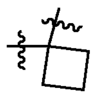, 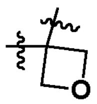, 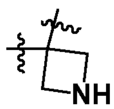, 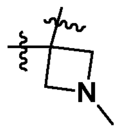, 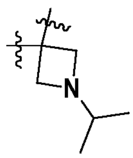, 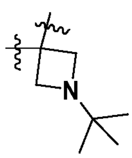, 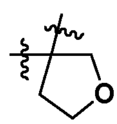

or

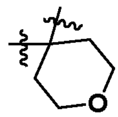.

5. The compound according to any one of claims 1 to 4, wherein, the ring L is selected from the group consisting of

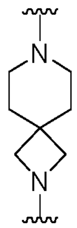, 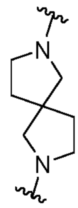, 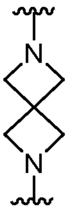, 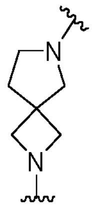, 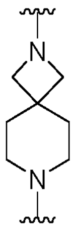

and

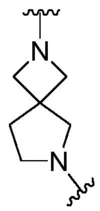.

6. The compound according to any one of claims 2 to 5, wherein, $R^1$, $R^2$, $R^3$, $R^4$, $R^5$, $R^{1'}$, $R^{2'}$, $R^{3'}$ and $R^{4'}$ are each independently selected from the group consisting of hydrogen, halogen, cyano, nitro, $-C_{1-6}$ alkyl, $-C_{2-6}$ alkenyl, $-C_{2-6}$ alkynyl, halogen-substituted $-C_{1-6}$ alkyl, halogen-substituted $-C_{2-6}$ alkenyl and halogen-substituted $-C_{2-6}$ alkynyl.

7. The compound according to any one of claims 2 to 5, wherein,

$R^{1'}$, $R^{2'}$, $R^{3'}$ and $R^{4'}$ are independently selected from the group consisting of hydrogen, halogen, cyano, nitro, oxo, $-C_{1-6}$ alkyl, $-C_{2-6}$ alkenyl, $-C_{2-6}$ alkynyl, halogen-substituted $-C_{1-6}$ alkyl, halogen-substituted $-C_{2-6}$ alkenyl, halogen-substituted $-C_{2-6}$ alkynyl, $-C_{0-4}$ alkylene-$OR^{D1}$ and $-C_{0-4}$ alkylene-$NR^{D1}R^{D2}$;
$R^{D1}$ and $R^{D2}$ are independently selected from the group consisting of hydrogen, $-C_{1-6}$ alkyl, - $C_{2-6}$ alkenyl and $-C_{2-6}$ alkynyl;
two non-adjacent groups of $R^1$, $R^2$, $R^3$, $R^4$ and $R^5$ are interconnected, and together with the ring where the atom connecting therewith is located form 7- to 12-membered bridged cycloalkyl or 7- to 12-membered bridged heterocycloalkyl;
wherein two non-adjacent groups of $R^1$, $R^2$, $R^3$, $R^4$ and $R^5$ are interconnected into a connecting chain selected from the group consisting of -O-, $-(CR^{D3}R^{D3})_q-$, $-(CR^{D3}R^{D3})_n-O-(CR^{D3}R^{D3})_n-$, - $(CR^{D3}R^{D3})_n-S-(CR^{D3}R^{D3})_n-$, $-(CR^{D3}R^{D3})_n-N(R^{D3})-(CR^{D3}R^{D3})_n-$, $-O-(CR^{D3}R^{D3})_n-O-$, $-O-(CR^{D3}R^{D3})_n-S-$, $-O-(CR^{D3}R^{D3})_n-N(R^{D3})-$, $-S-(CR^{D3}R^{D3})_n-O-$, $-S-(CR^{D3}R^{D3})_n-S-$, $-S-(CR^{D3}R^{D3})_n-N(R^{D3})-$, $-N(R^{D3})-(CR^{D3}R^{D3})_n-N(R^{D3})-$, $-N(R^{D3})-(CR^{D3}R^{D3})_n-O-$ and $-N(R^{D3})-(CR^{D3}R^{D3})_n-S-$;
each n is independently selected from the group consisting of 0, 1, 2 and 3;
each q is independently selected from the group consisting of 1, 2 and 3; and
each $R^{D3}$ is independently selected from the group consisting of hydrogen, halogen, cyano, nitro, oxo, $-C_{1-6}$ alkyl, $-C_{2-6}$ alkenyl, $-C_{2-6}$ alkynyl, halogen-substituted $-C_{1-6}$ alkyl, halogen-substituted $-C_{2-6}$ alkenyl and halogen-substituted $-C_{2-6}$ alkynyl; alternatively, two $R^{D3}$ together form

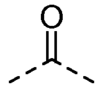, 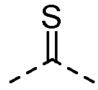, 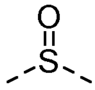 or 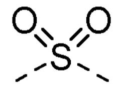.

**8.** The compound according to claim 7, wherein,

two groups of $R^1$, $R^2$, $R^3$, $R^4$ and $R^5$ are interconnected, and together with the ring where the atom connecting therewith is located, form

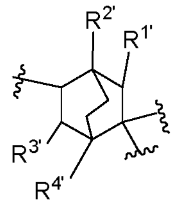, 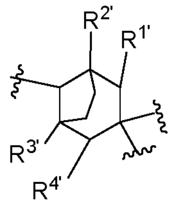, 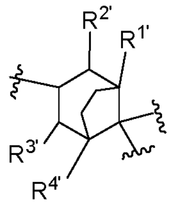,

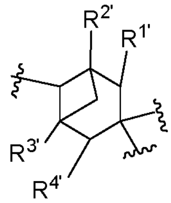 or 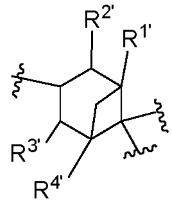;

wherein, $R^{1'}$, $R^{2'}$, $R^{3'}$ and $R^{4'}$ are independently hydrogen or $-C_{1-6}$ alkyl.

**9.** The compound according to any one of claims 2 to 5, wherein,

$R^{1'}$, $R^{2'}$, $R^{3'}$ and $R^{4'}$ are independently selected from the group consisting of hydrogen, halogen, cyano, nitro, oxo, $-C_{1-6}$ alkyl, $-C_{2-6}$ alkenyl, $-C_{2-6}$ alkynyl, halogen-substituted $-C_{1-6}$ alkyl, halogen-substituted $-C_{2-6}$ alkenyl, halogen-substituted $-C_{2-6}$ alkynyl, $-C_{0-4}$ alkylene-$OR^{D1}$ and $-C_{0-4}$ alkylene-$NR^{D1}R^{D2}$;
$R^{D1}$ and $R^{D2}$ are independently selected from the group consisting of hydrogen, $-C_{1-6}$ alkyl, - $C_{2-6}$ alkenyl and $-C_{2-6}$ alkynyl;
three groups of $R^1$, $R^2$, $R^3$, $R^4$ and $R^5$ are interconnected, and together with the ring where the atom connecting therewith is located, form 7- to 12-membered bridged cycloalkyl or 7- to 12-membered bridged heterocycloalkyl; wherein two groups of $R^1$, $R^2$, $R^3$, $R^4$ and $R^5$ are interconnected into a connecting chain selected from the group consisting of -O-, $-(CR^{D3}R^{D3})_q-$, $-(CR^{D3}R^{D3})_n-O-(CR^{D3}R^{D3})_n-$, - $(CR^{D3}R^{D3})_n-S-(CR^{D3}R^{D3})_n-$, $-(CR^{D3}R^{D3})_n-N(R^{D3})-(CR^{D3}R^{D3})_n-$, $-O-(CR^{D3}R^{D3})_n-O-$, $-O-(CR^{D3}R^{D3})_n-S-$, $-O-(CR^{D3}R^{D3})_n-N(R^{D3})-$, $-S-(CR^{D3}R^{D3})_n-O-$, $-S-(CR^{D3}R^{D3})_n-S-$, $-S-(CR^{D3}R^{D3})_n-N(R^{D3})-$, $-N(R^{D3})-(CR^{D3}R^{D3})_n-N(R^{D3})-$, $-N(R^{D3})-(CR^{D3}R^{D3})_n-O-$ and $-N(R^{D3})-(CR^{D3}R^{D3})_n-S-$; and the other group is connected to the carbon atom or nitrogen atom on the connecting chain formed by the two groups;
each n is independently selected from the group consisting of 0, 1, 2 and 3;
each q is independently selected from the group consisting of 1, 2 and 3; and
each $R^{D3}$ is independently selected from the group consisting of hydrogen, halogen, cyano, nitro, oxo, $-C_{1-6}$ alkyl, $-C_{2-6}$ alkenyl, $-C_{2-6}$ alkynyl, halogen-substituted $-C_{1-6}$ alkyl, halogen-substituted $-C_{2-6}$ alkenyl and halogen-substituted $-C_{2-6}$ alkynyl; alternatively, two $R^{D3}$ together form

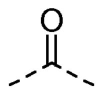, 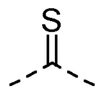, 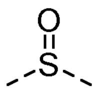 or 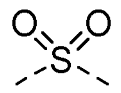.

**10.** The compound according to claim 9, wherein,
$R^1$, $R^4$ and $R^5$ are interconnected, and together with the ring where the atom connecting therewith is located, form

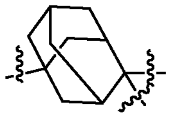
.

**11.** The compound according to any one of claims 1 to 10, wherein, $R^6$ is selected from the group consisting of $-C(O)NR^{E1}R^{E2}$, $-NR^{E1}C(O)R^{E2}$, $-NR^{E1}S(O)_2R^{E2}$, - $NR^{E1}S(O)R^{E2}$, -5- to 10-membered aromatic ring, -5- to 10-membered heteroaromatic ring, -3- to 10-membered carbocyclyl, -4- to 10-membered heterocycloalkyl, $-S(O)R^{E1}$, $-S(O)_2R^{E1}$, - $S(O)_2NR^{E1}R^{E2}$, $-S(O)(NH)R^{E1}$, $-S(O)(NH)NR^{E1}R^{E2}$, $-OR^{E1}$, $-OC(O)R^{E1}$ and $-SR^{E1}$; wherein the alkyl, alkylene, alkenyl, alkynyl, carbocyclyl, heterocycloalkyl, aryl or heteroaryl is further optionally substituted by one, two, three or four independent $R^{E5}$.

**12.** The compound according to claim 11, wherein $R^6$ is selected from the group consisting of

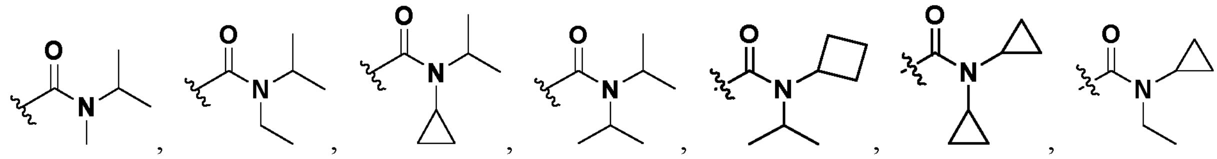
, , , , , , ,

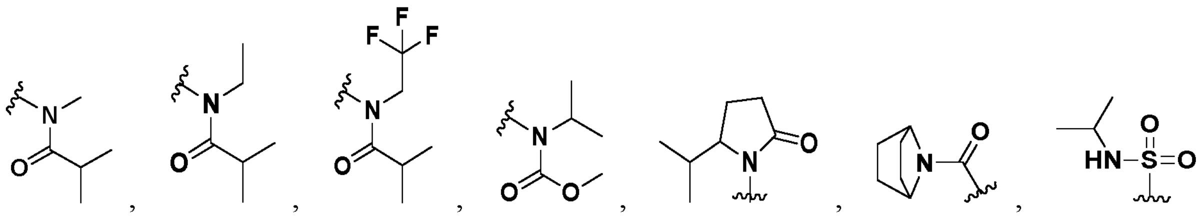
, , , , , , ,

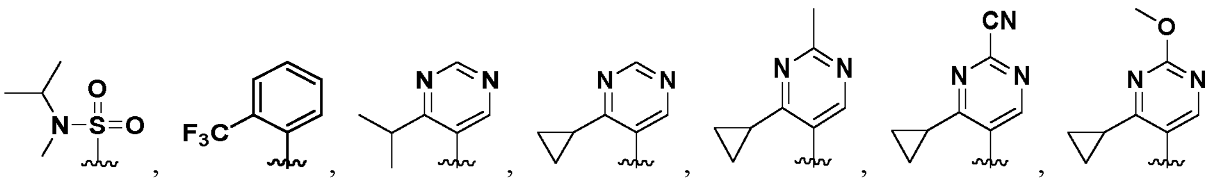
, , , , , , ,

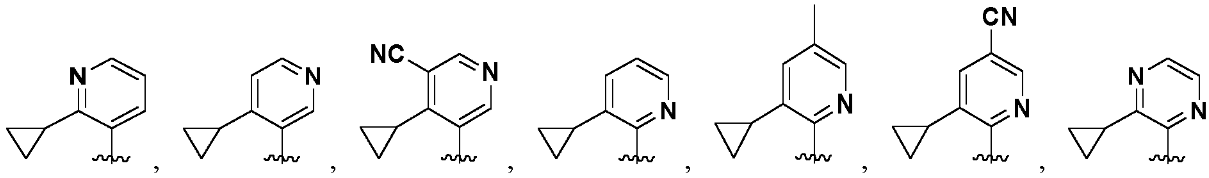
, , , , , , ,

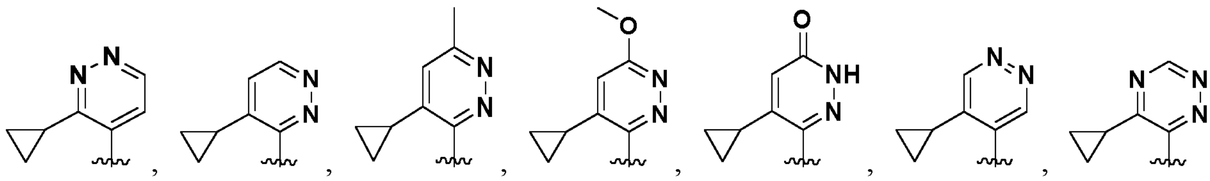
, , , , , , ,

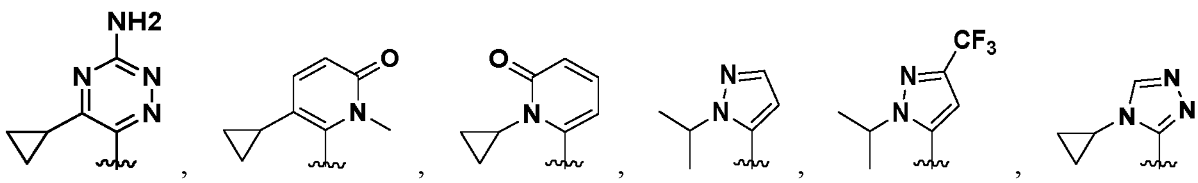
, , , , , ,

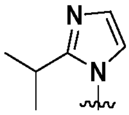, 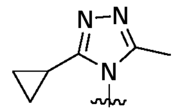, 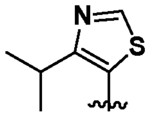, 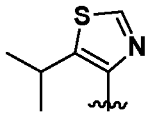, 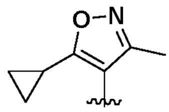 and 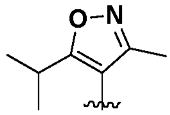.

**13.** The compound according to claim 1, wherein the compound is represented by Formula II:

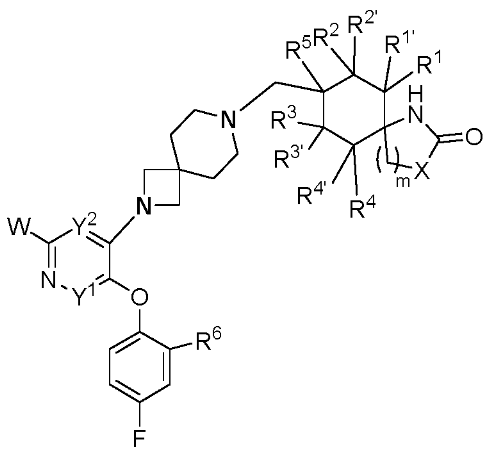

Formula II

wherein, $Y^1$, $Y^2$, W, X, $R^6$, m, $R^1$, $R^2$, $R^3$, $R^4$, $R^5$, $R^{1'}$, $R^{2'}$, $R^{3'}$ and $R^{4'}$ are as defined in claim 1.

**14.** The compound according to claim 1, wherein the compound is represented by Formula IIa or Formula IIb:

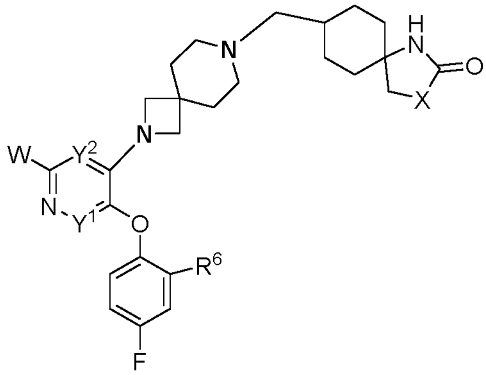

Formula IIa

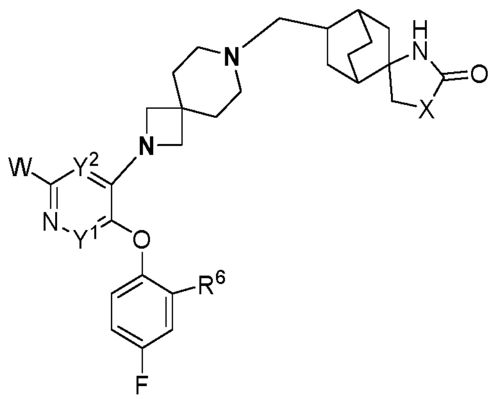

Formula IIb

wherein, $Y^1$, $Y^2$, W, X and $R^6$ are as defined in claim 1.

**15.** The compound according to claim 14, wherein the compound is represented by Formula IIIa or Formula IIIb:

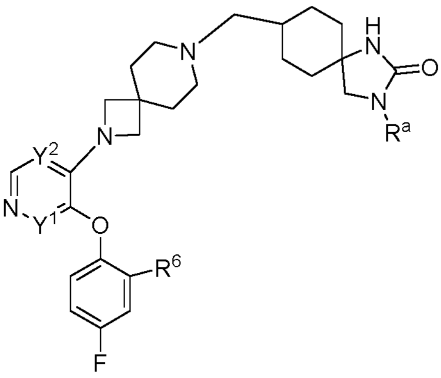

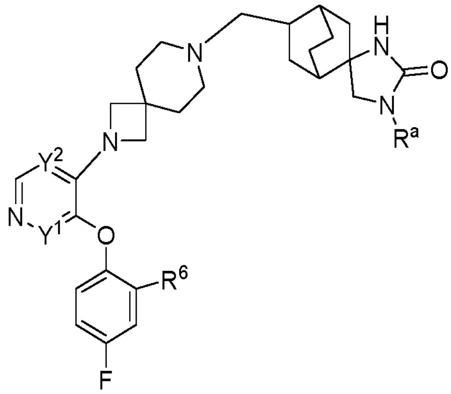

Formula IIIa Formula IIIb

wherein,

$Y^1$ and $Y^2$ are independently CH or N;

$R^a$ is selected from the group consisting of hydrogen, methyl, ethyl, isopropyl, cyclopropyl,

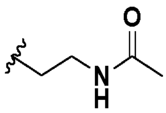, 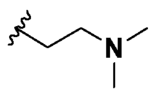, 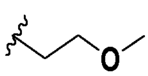, 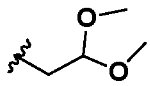, 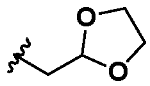, 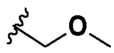, 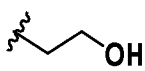,

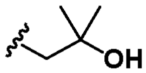 and 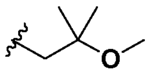;

$R^6$ is selected from the group consisting of

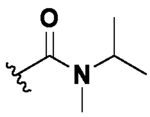, 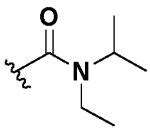, 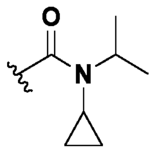,

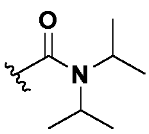, 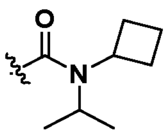, 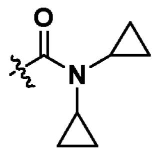, 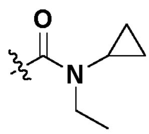, 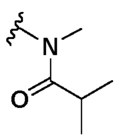, 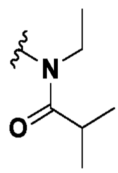, 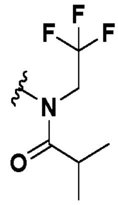,

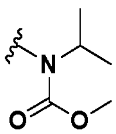, 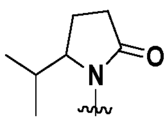, 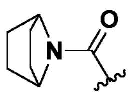, 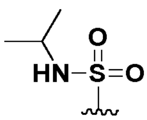, 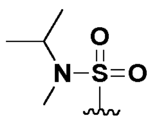, 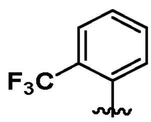, 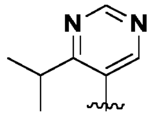,

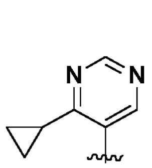, 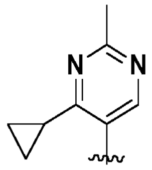, 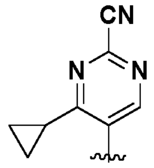, 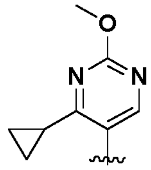, 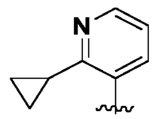, 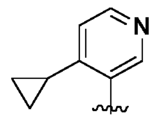, 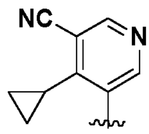,

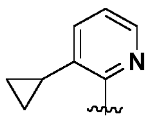, 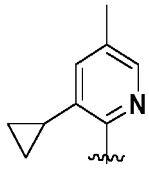, 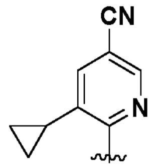, 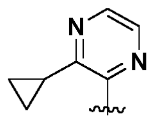, 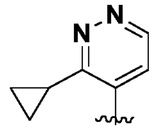, 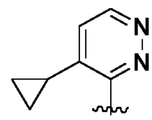, 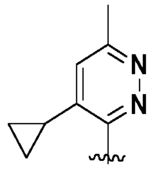,

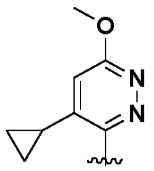, 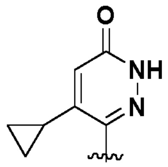, 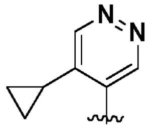, 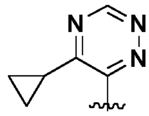, 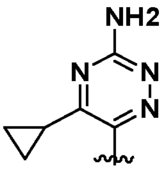, 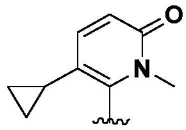,

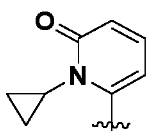, 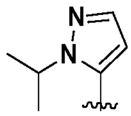, 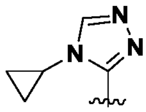, 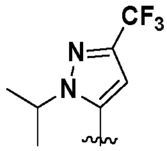, 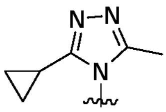, 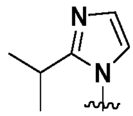, 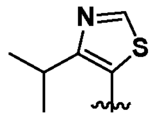,

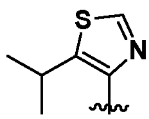, 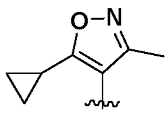 and 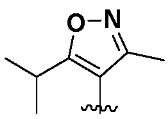.

**16.** The compound according to claim 14, wherein the compound is represented by Formula IVa or Formula IVb:

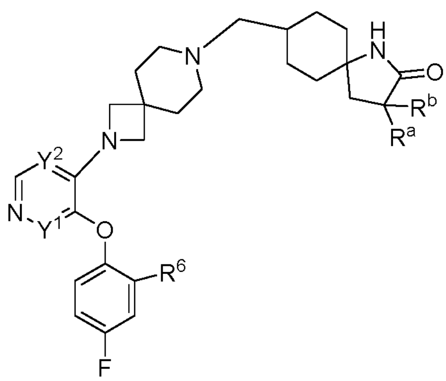

Formula IVa

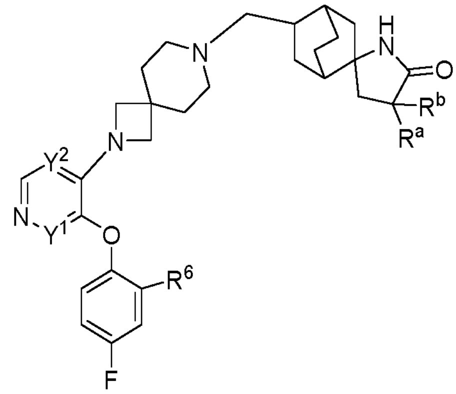

Formula IVb

wherein,

$Y^1$ and $Y^2$ are independently CH or N;

$R^a$ and $R^b$ are independently selected from the group consisting of hydrogen, fluorine, methyl,

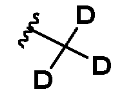,

ethyl, isopropyl,

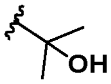, 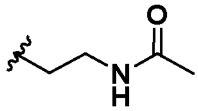, 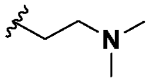, 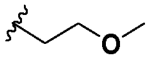, 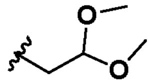,

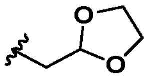, 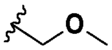, 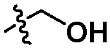, 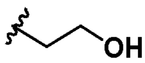, 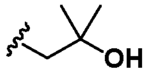 and 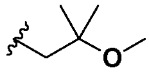;

alternatively, $R^a$, $R^b$ and the atom connecting therewith together form

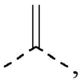, 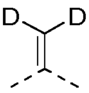, 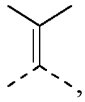,

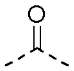, 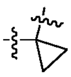, 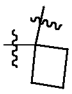, 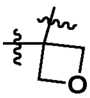, 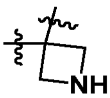, 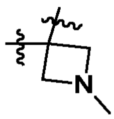, 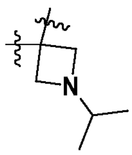, 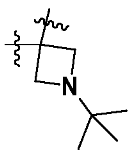, 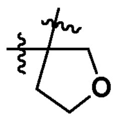

or

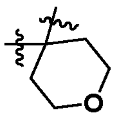;

and
$R^6$ is selected from the group consisting of

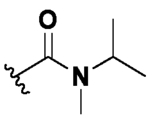, 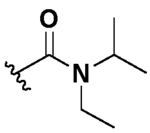, 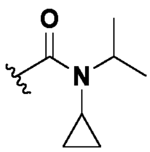,

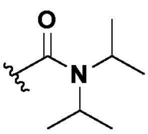, 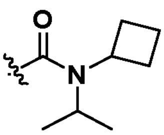, 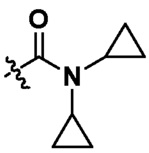, 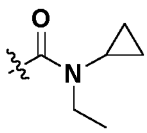, 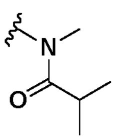, 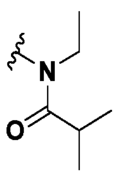, 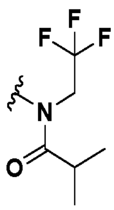,

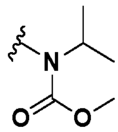, 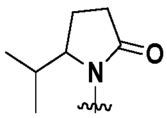, 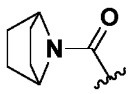, 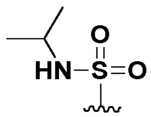, 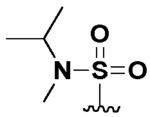, 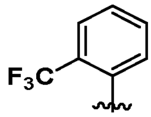, 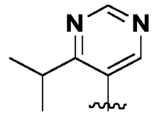,

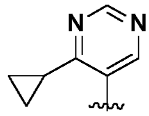, 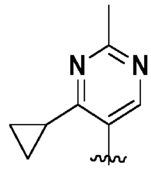, 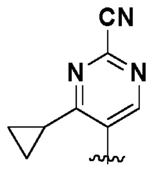, 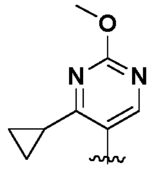, 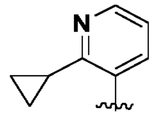, 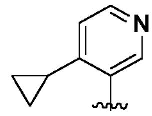, 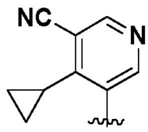,

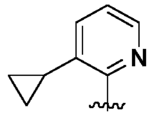, 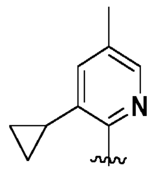, 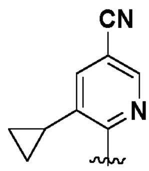, 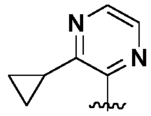, 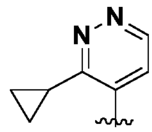, 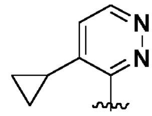, 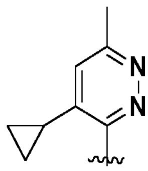,

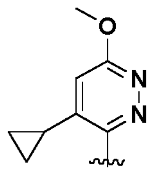, 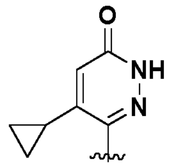, 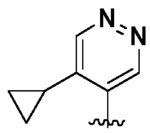, 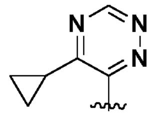, 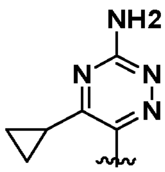, 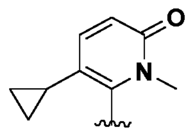,

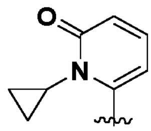, 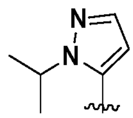, 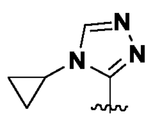, 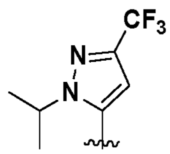, 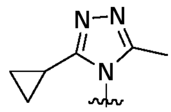, 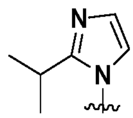, 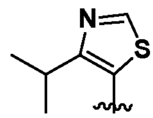,

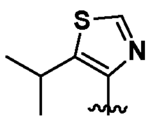, 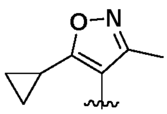 and 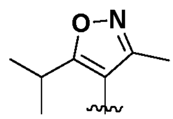.

**17.** The compound according to claim 16, wherein the compound is represented by Formula Va:

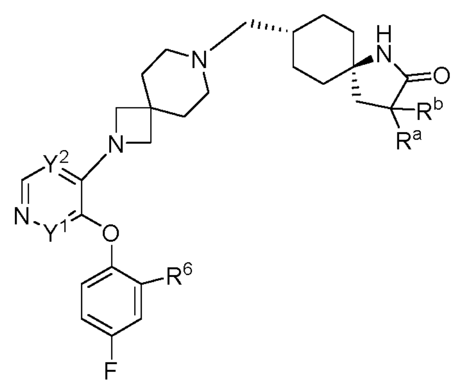

Formula Va

wherein,

$Y^1$ and $Y^2$ are independently CH or N;

$R^a$ and $R^b$ are independently selected from the group consisting of hydrogen, fluorine, methyl,

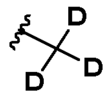,

ethyl, isopropyl,

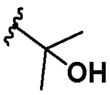, 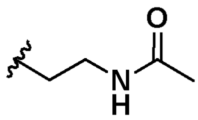, 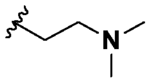, 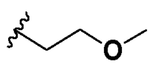, 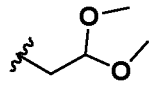,

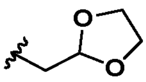, 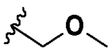, 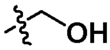, 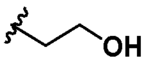, 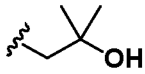 and 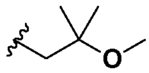;

alternatively, $R^a$, $R^b$ and the atom connecting therewith together form

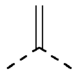, 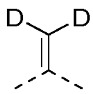, 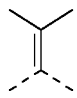,

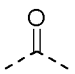, 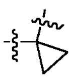, 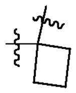, 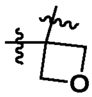, 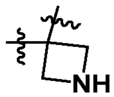, 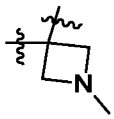, 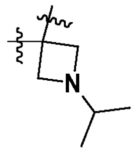, 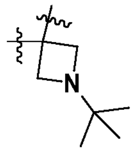, 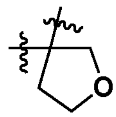

or

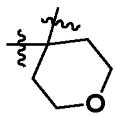;

$R^6$ is selected from the group consisting of

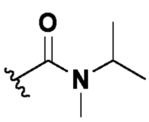, 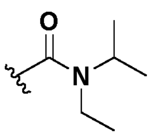, 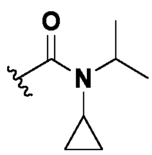,

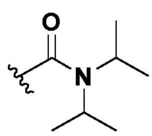, 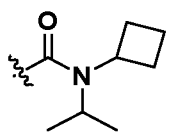, 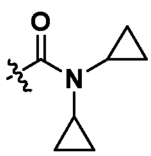, 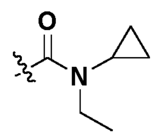, 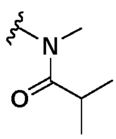, 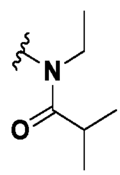, 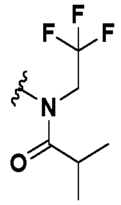,

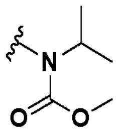, 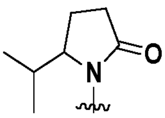, 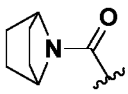, 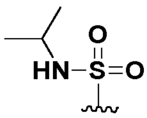, 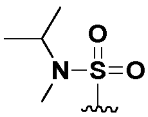, 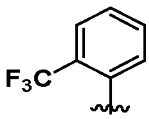, 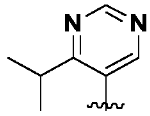,

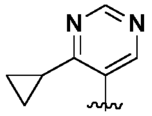, 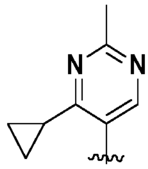, 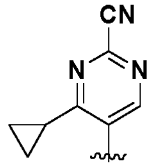, 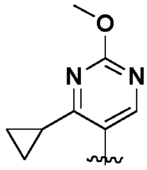, 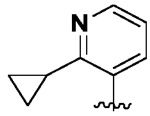, 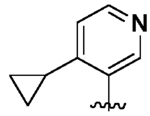, 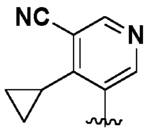,

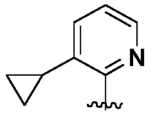, 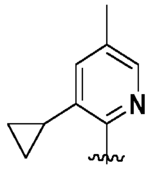, 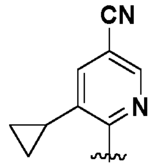, 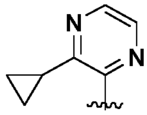, 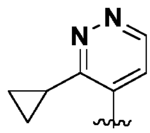, 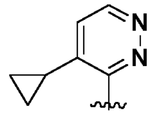, 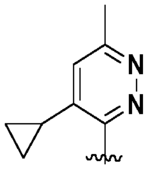,

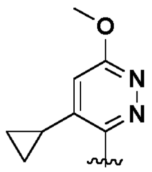, 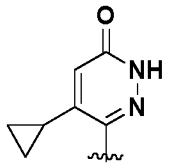, 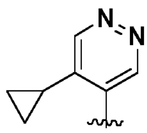, 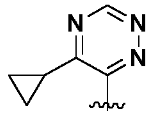, 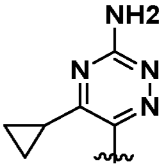, 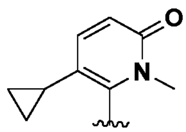,

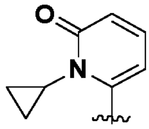, 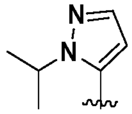, 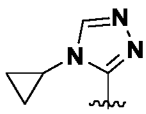, 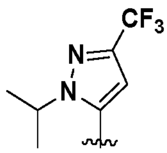, 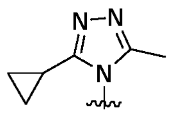, 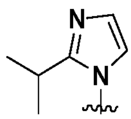, 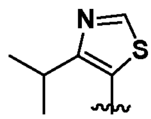,

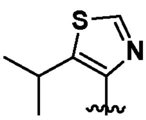, 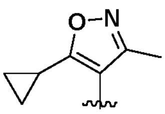 and 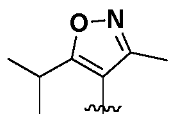.

18. The compound according to any one of claims 1 to 17, wherein the compound is selected from the group consisting of:

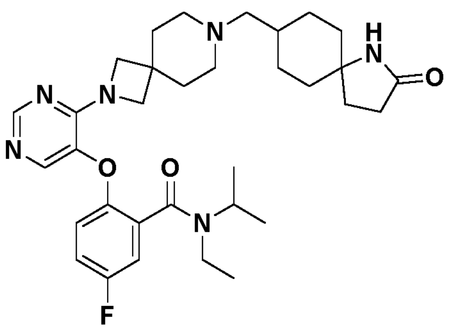, 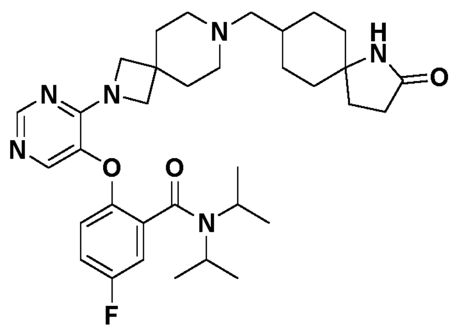,

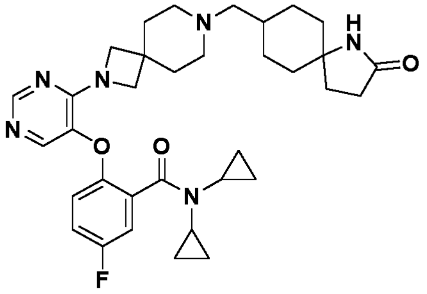

,

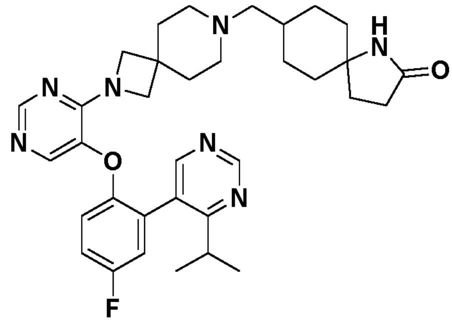

,

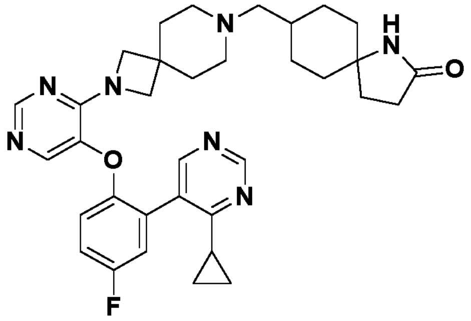

,

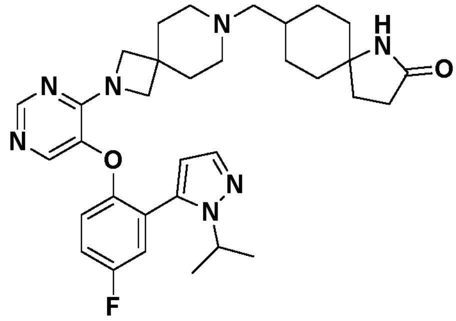

,

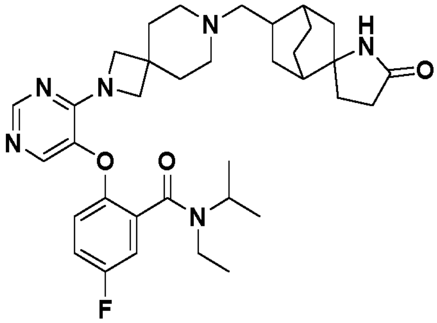

,

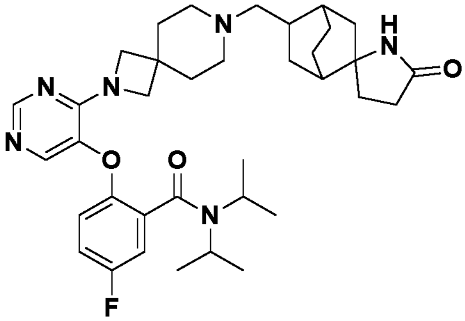

,

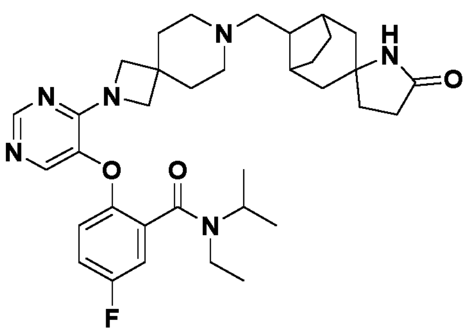

,

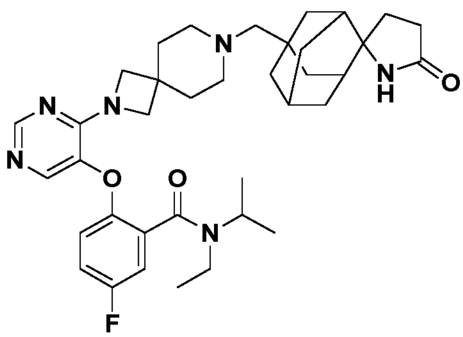

,

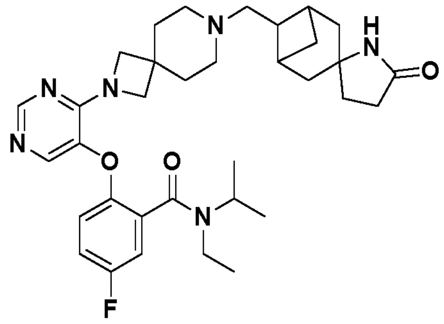
,
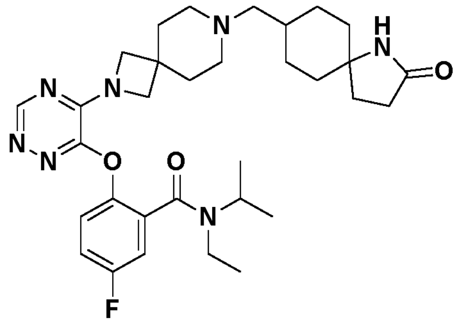
,
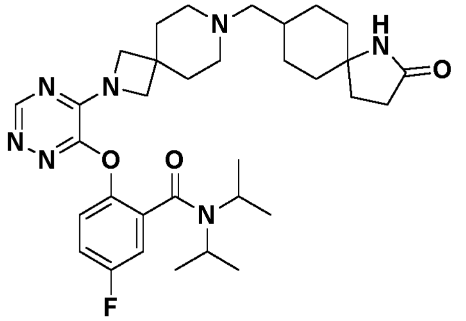
,
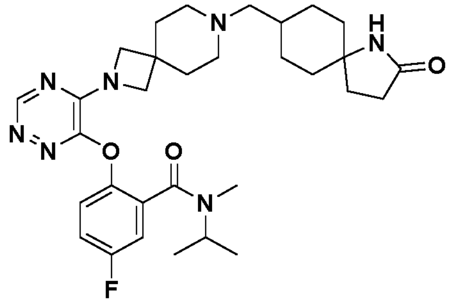
,
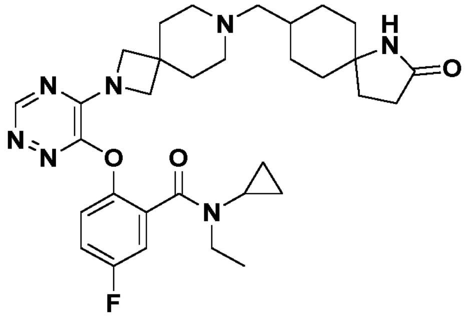
,
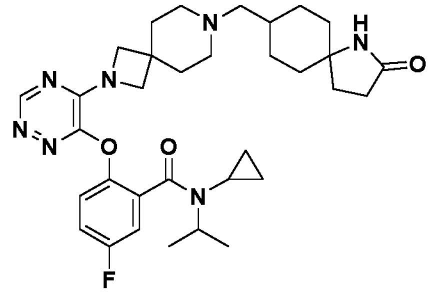
,
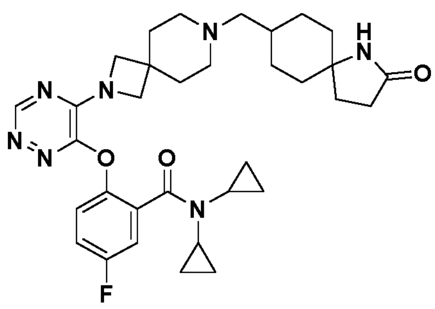
,
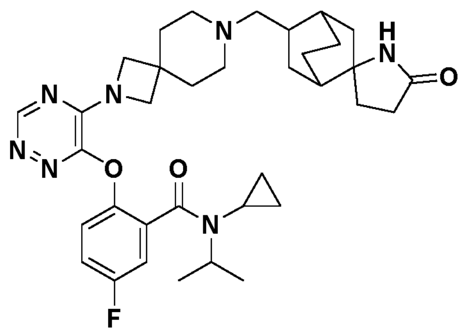
,

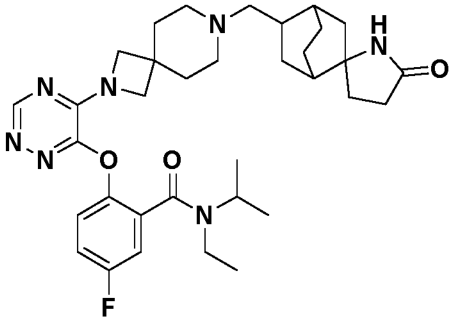
,

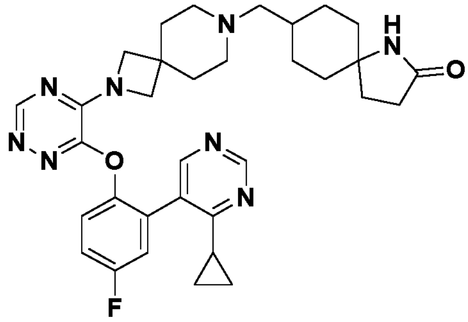
,

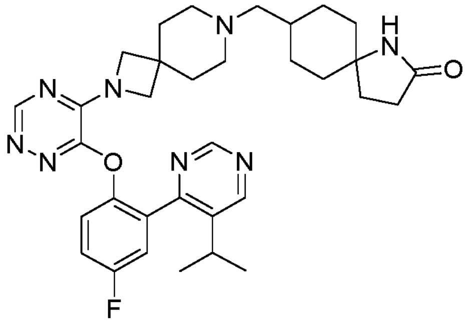
,

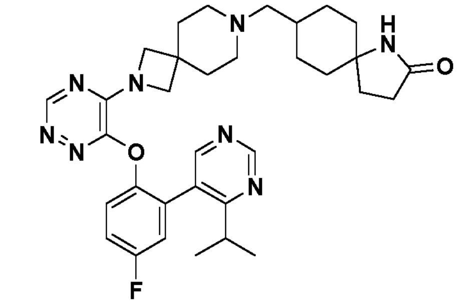
,

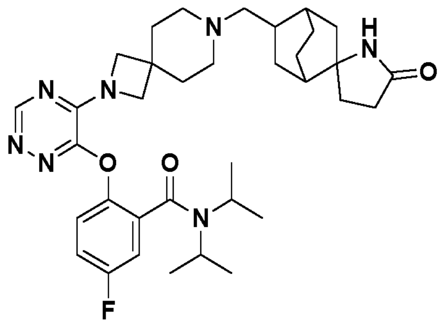
,

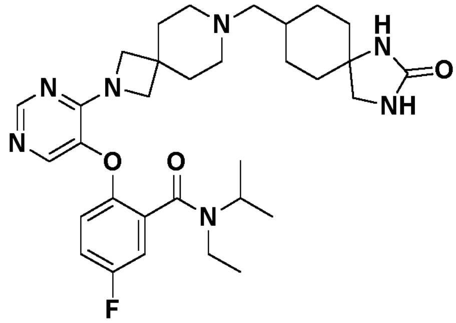
,

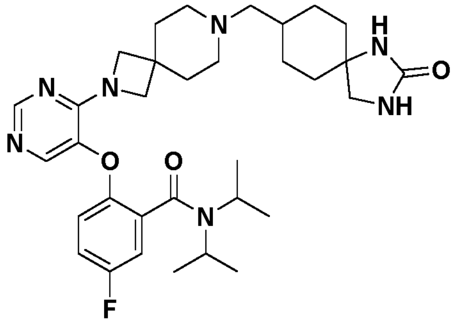
,

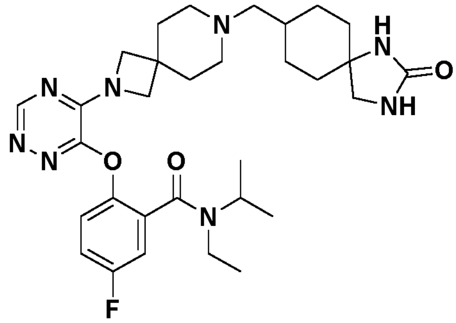
,

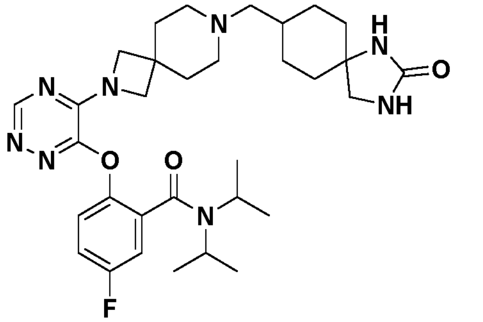

,

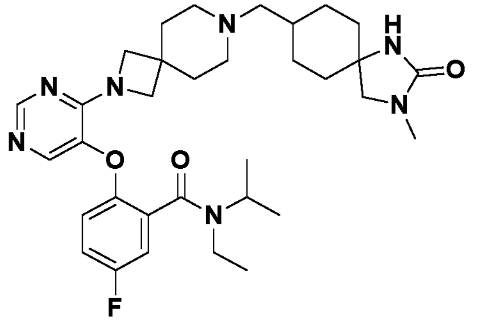

,

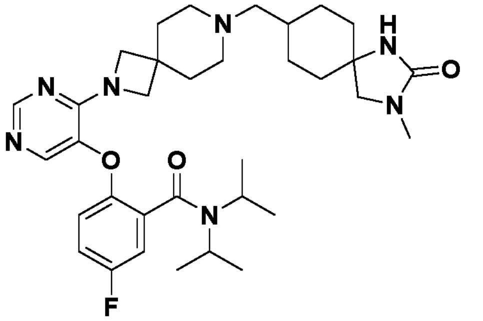

,

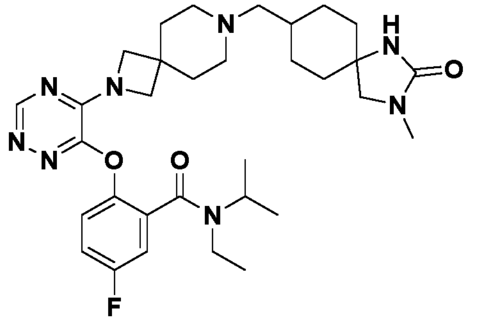

,

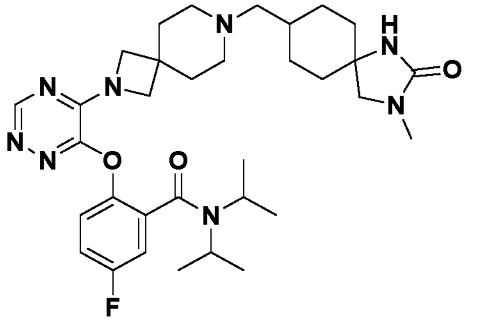

,

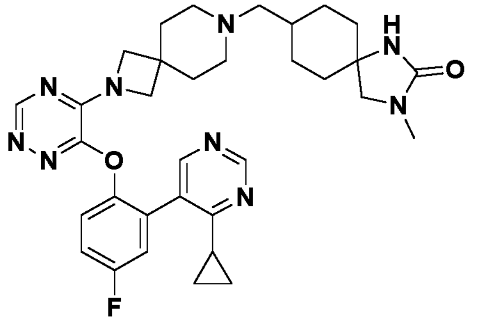

,

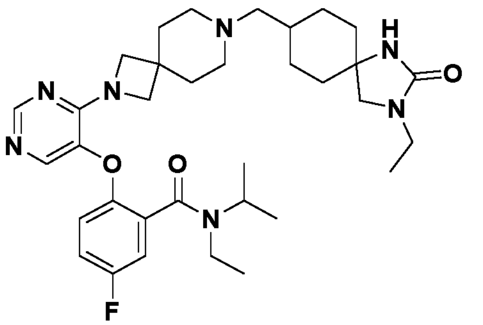

,

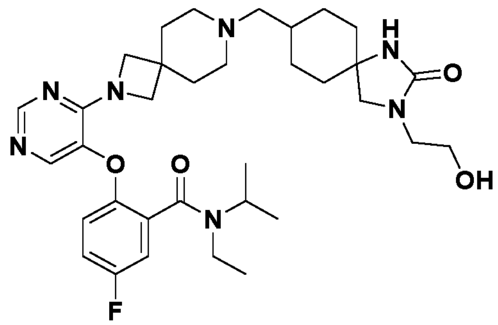

,

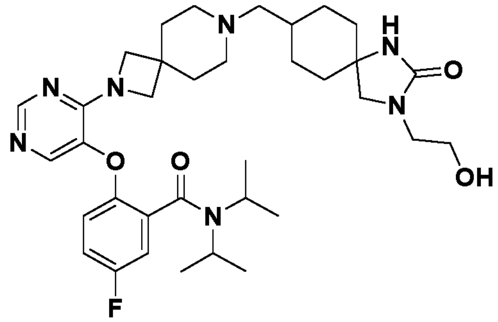

,

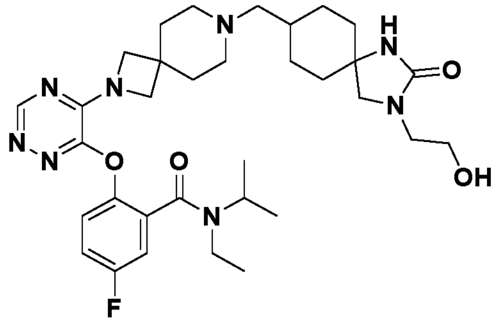

,

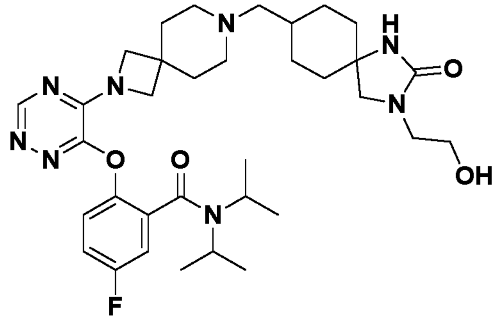

,

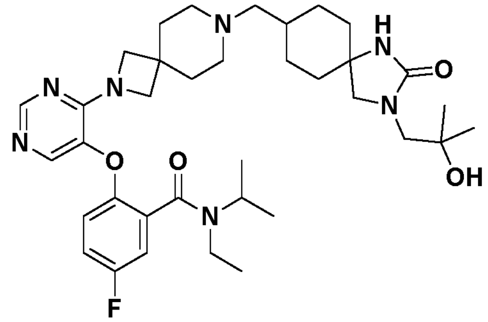

,

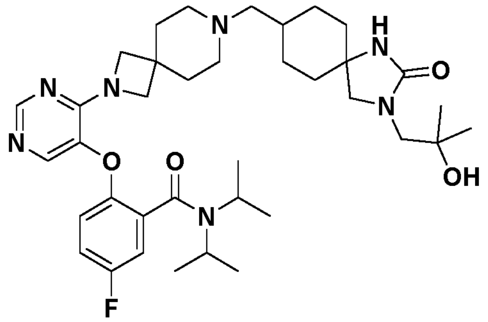

,

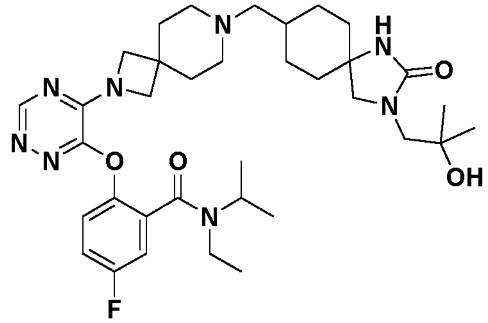

,

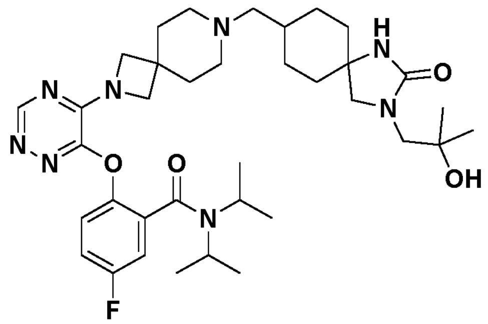

,

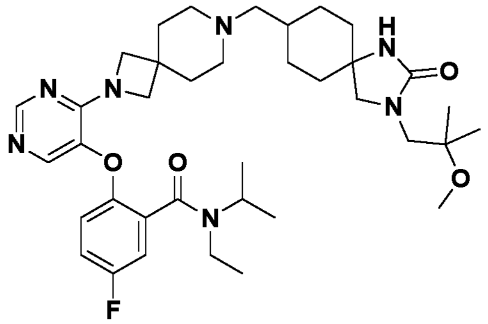

,

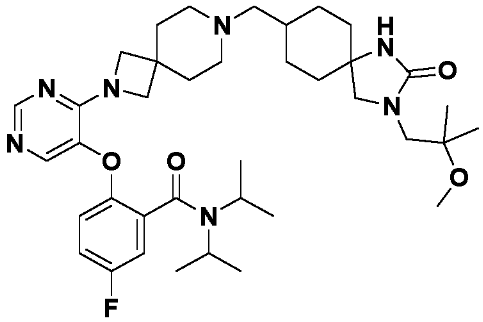

,

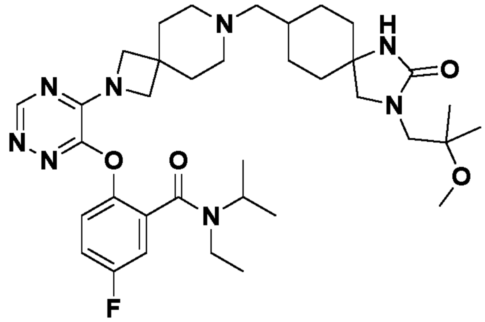

,

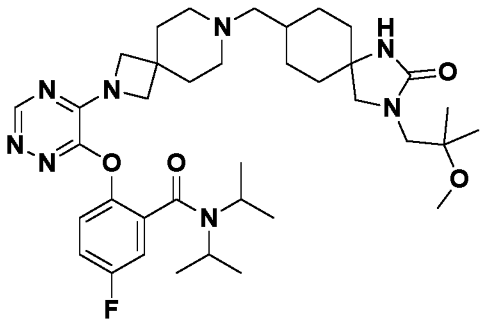

,

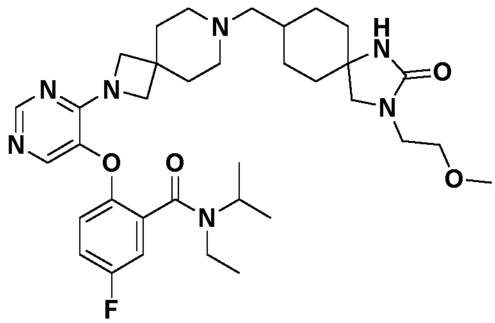

,

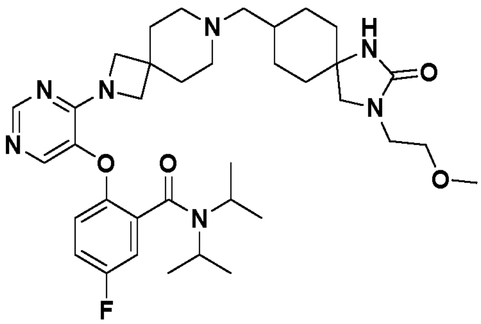

,

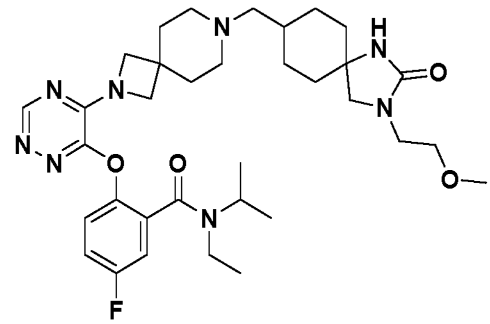

,

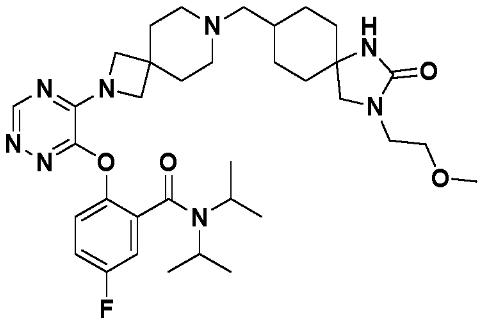

,

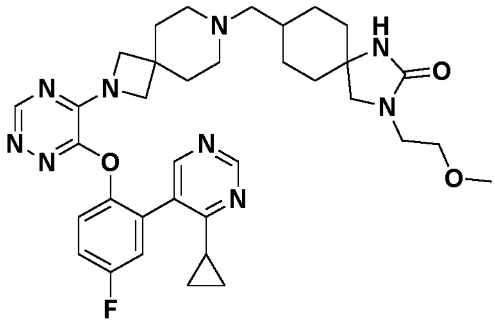

,

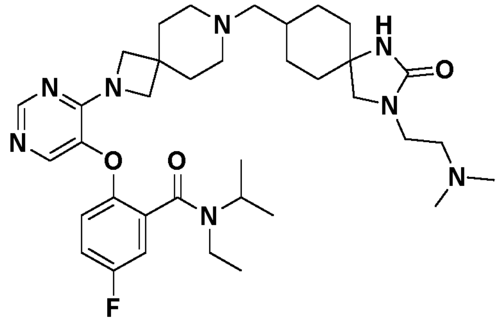
,
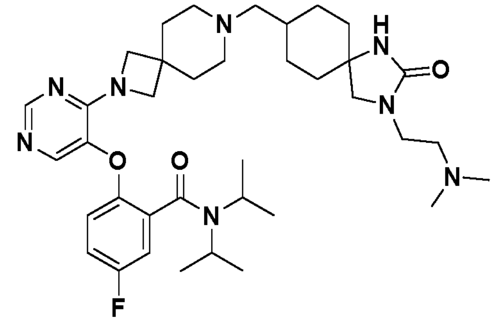
,
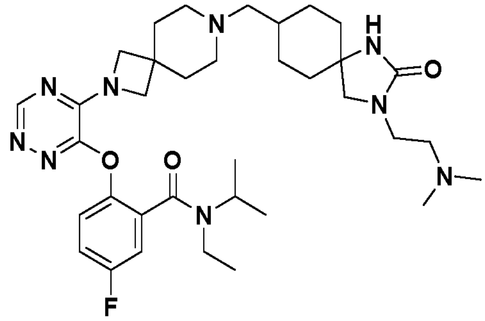
,
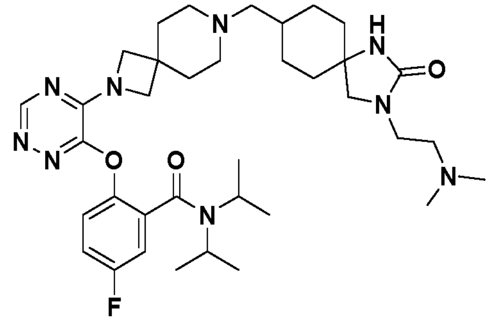
,
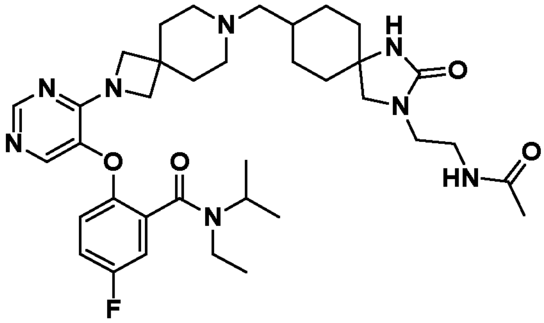
,
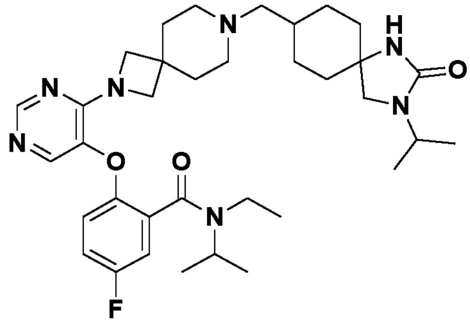
,
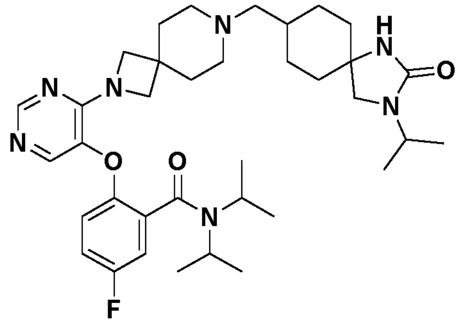
,
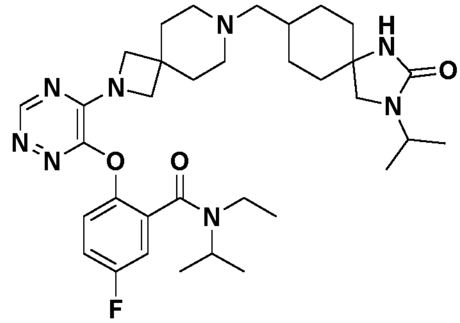
,

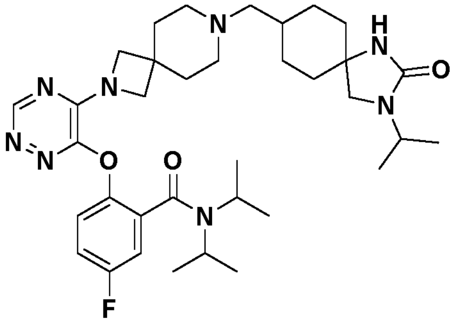

,

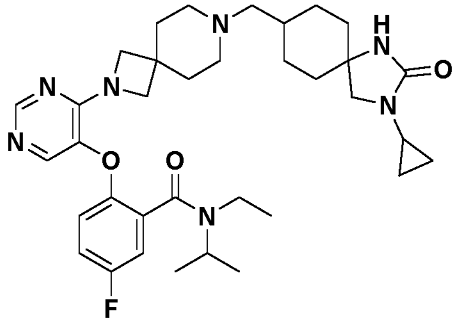

,

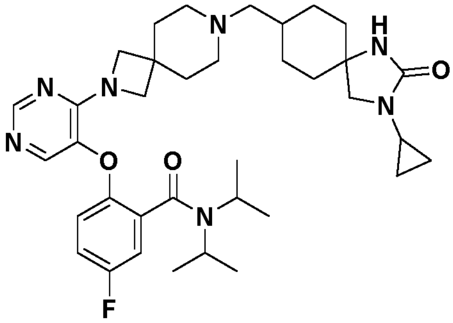

,

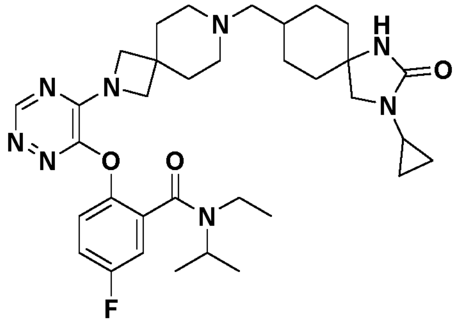

,

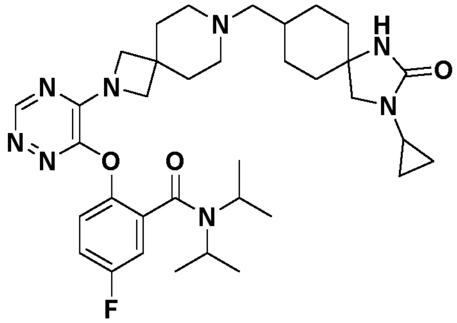

,

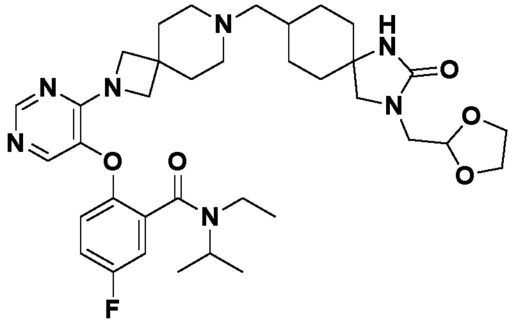

,

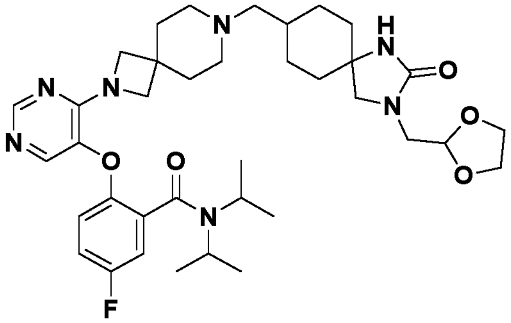

,

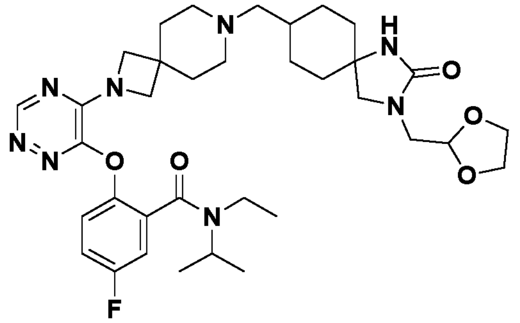

,

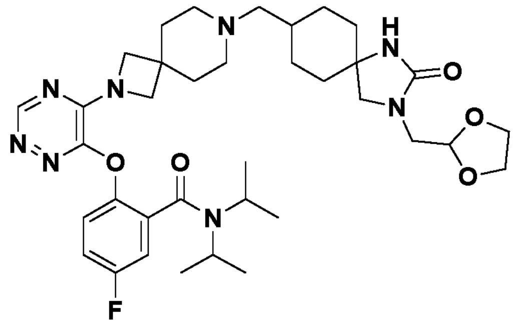

,

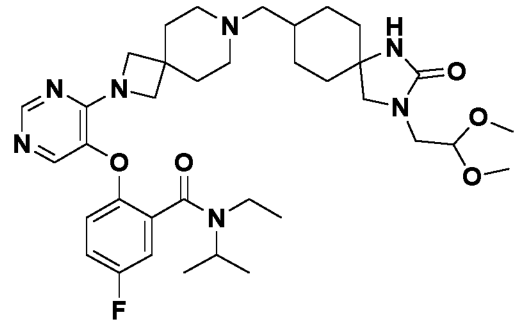

,

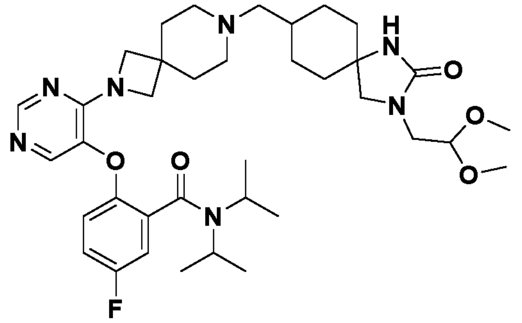

,

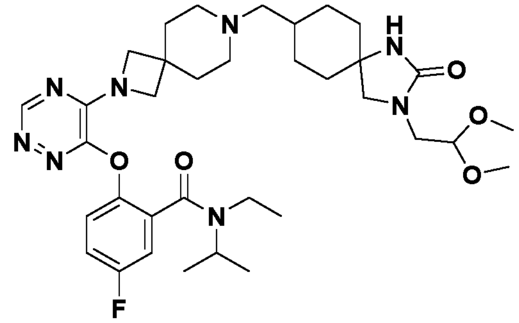

,

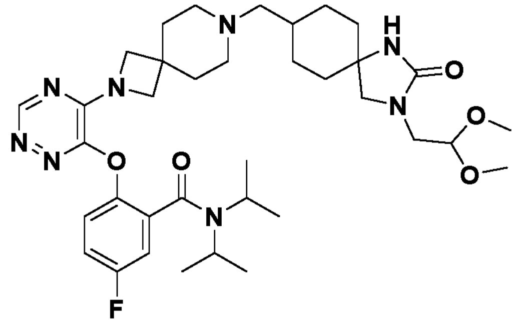

,

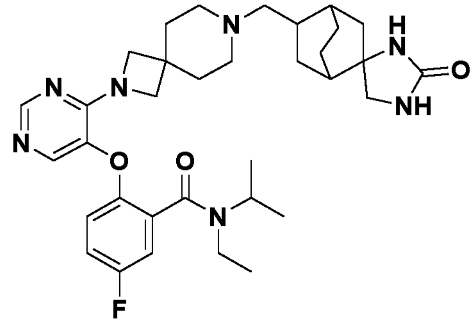

,

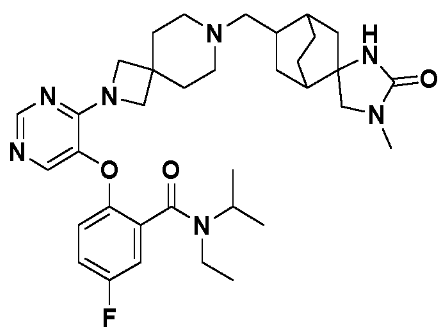

,

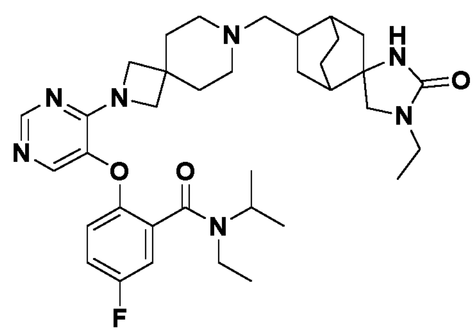

,

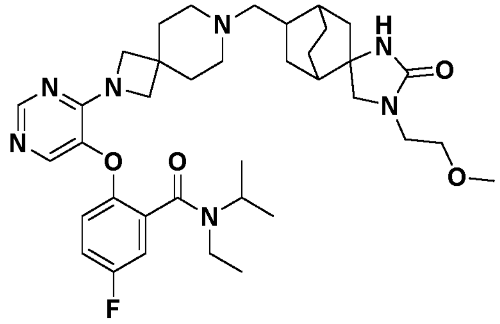

,

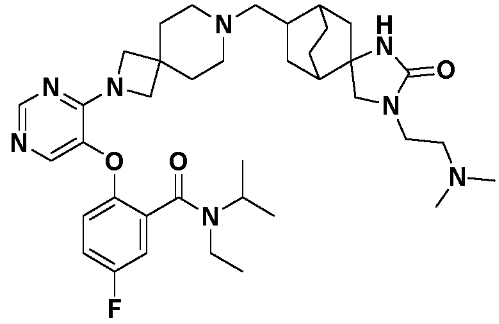

,

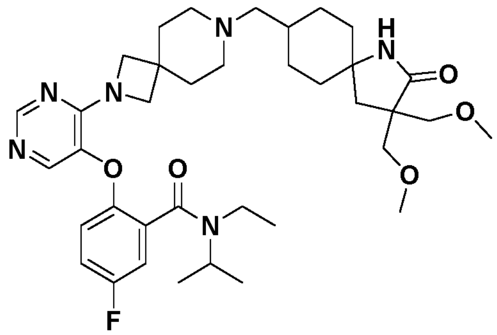

,

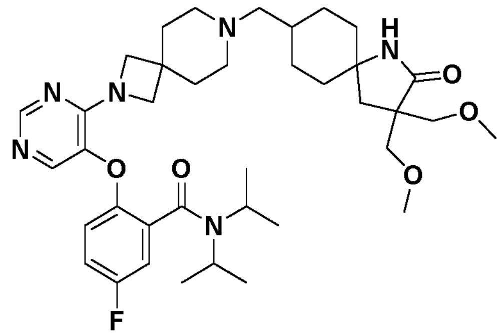

,

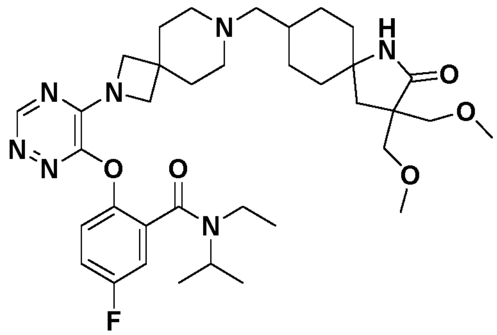

,

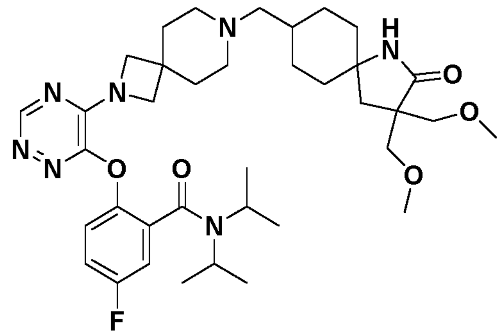

,

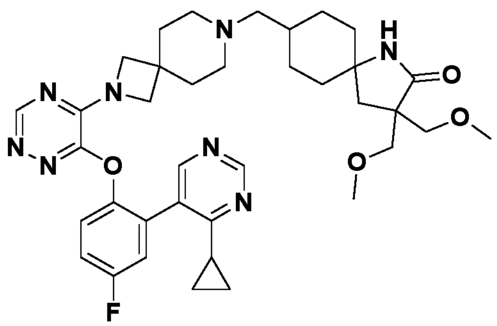

,

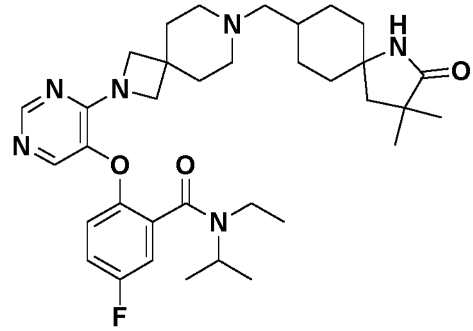

,

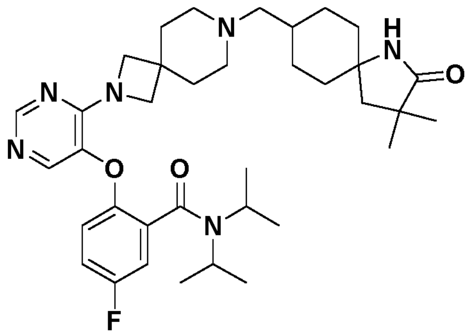

,

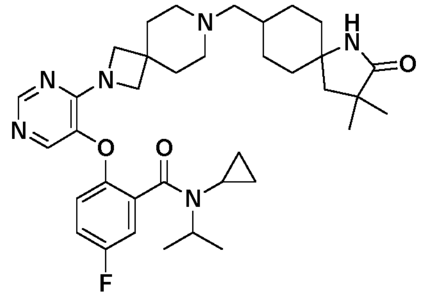

,

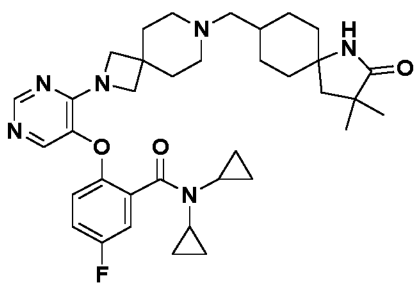

,

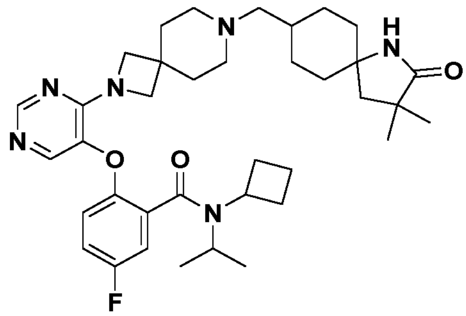

,

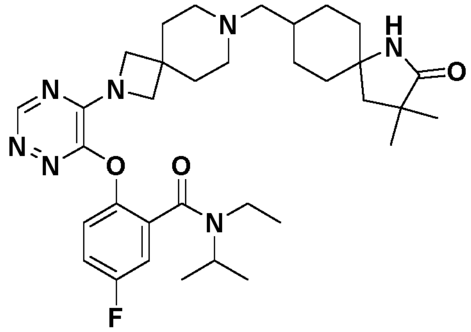

,

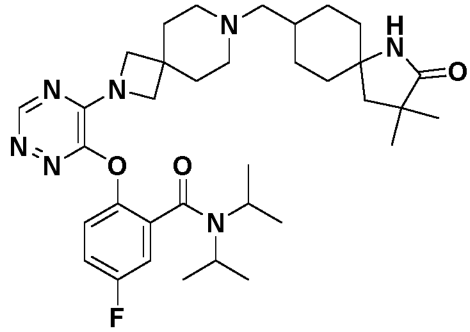

,

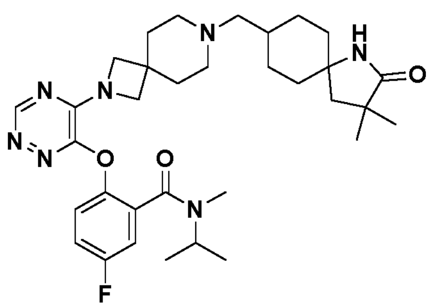

,

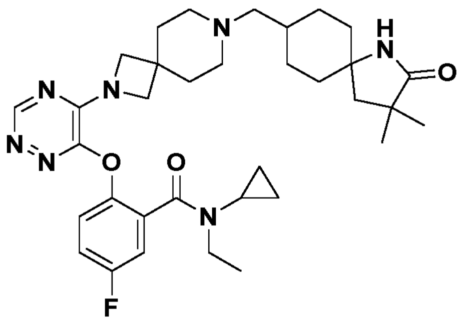

,

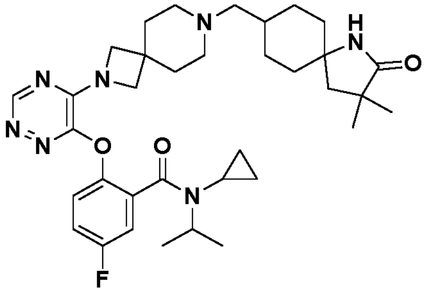
,

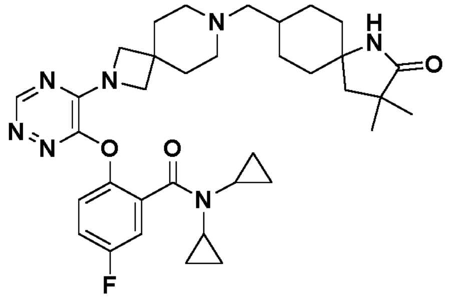
,

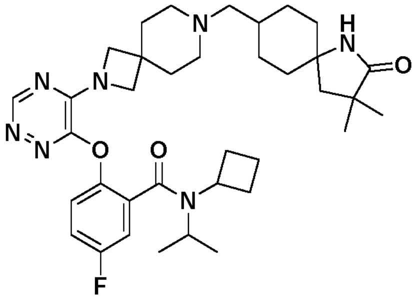
,

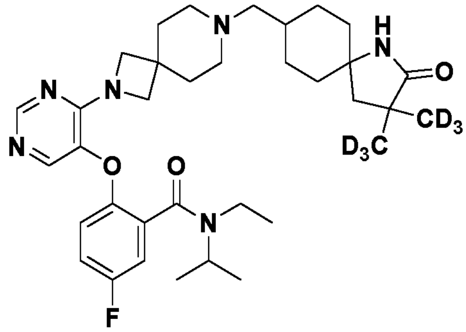
,

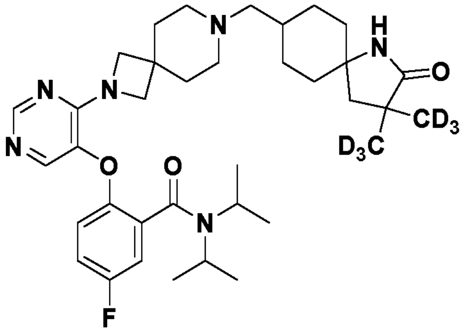
,

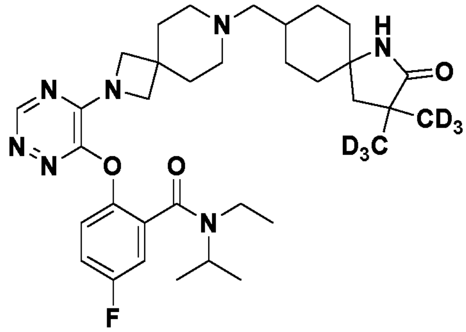
,

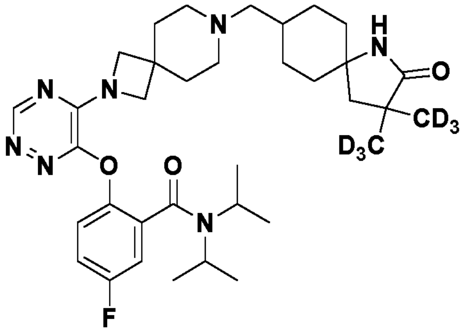
.

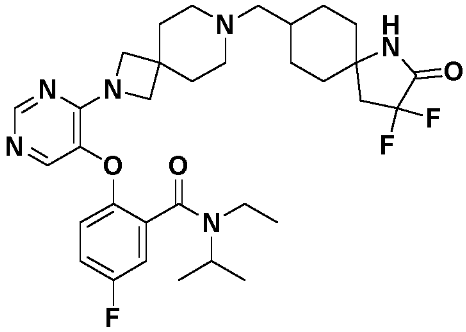
.

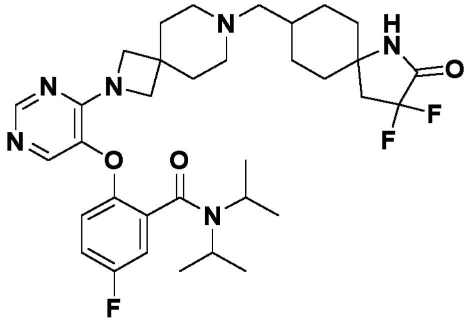

,

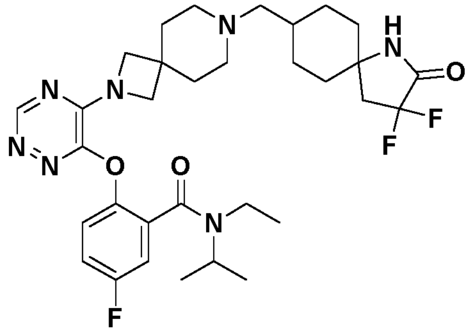

,

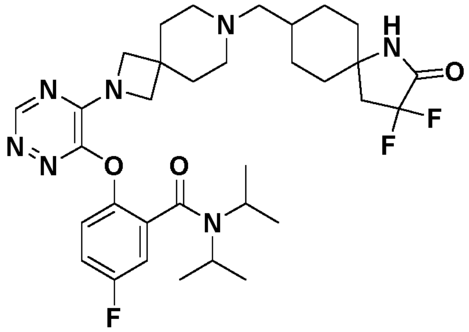

,

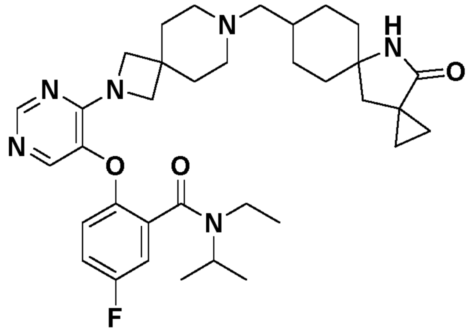

,

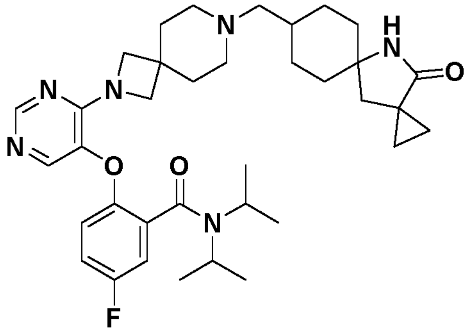

,

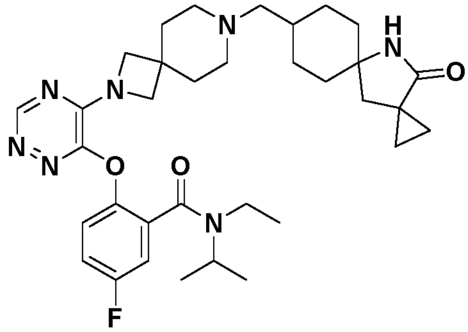

,

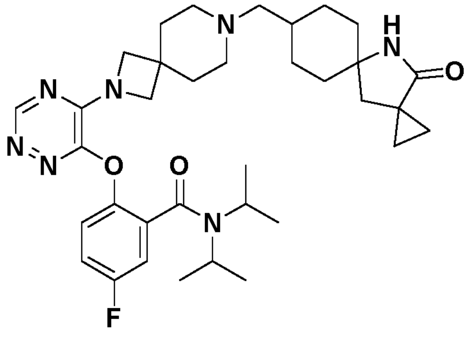

,

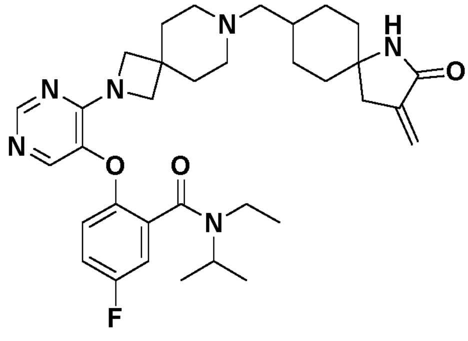

.

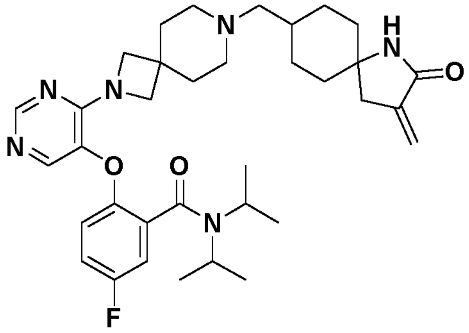

,

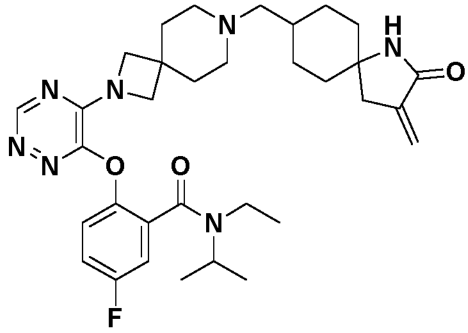

,

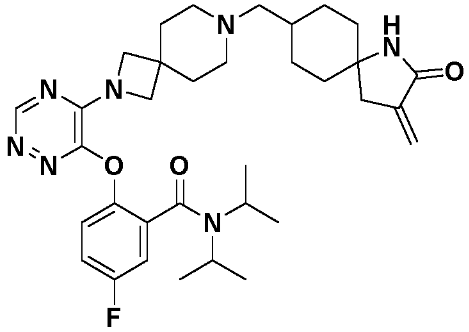

,

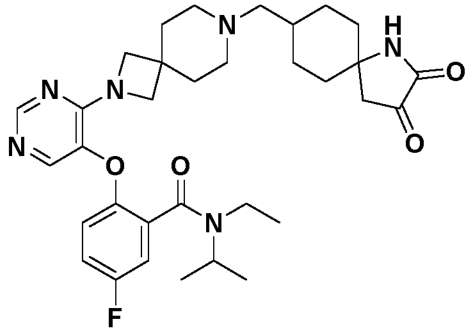

,

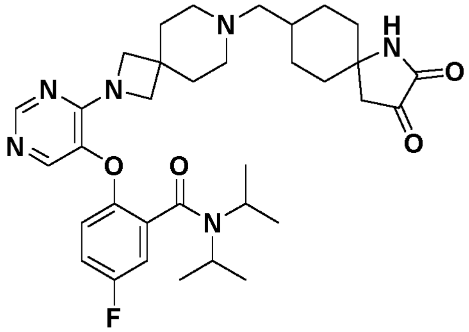

,

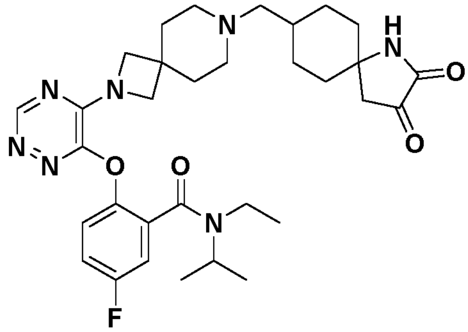

,

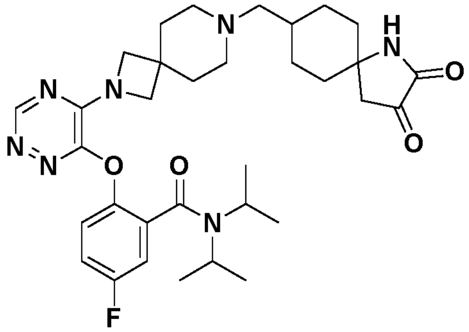

,

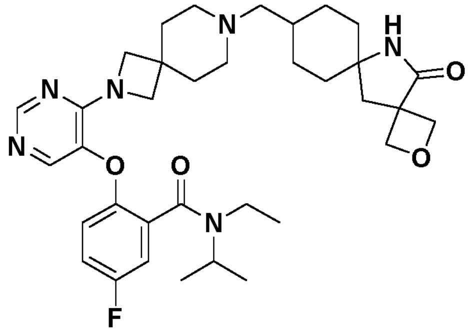

,

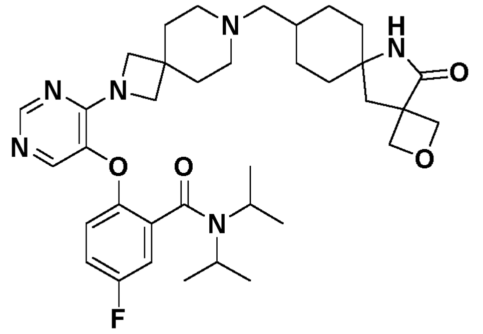
,
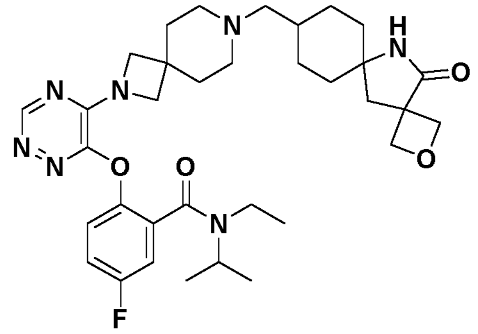
,

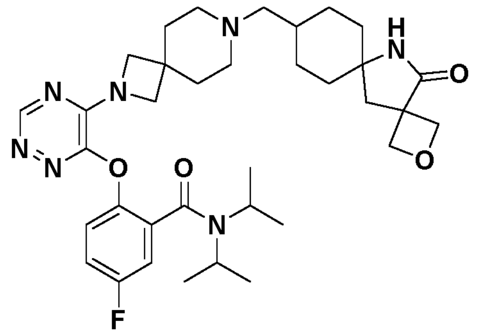
,
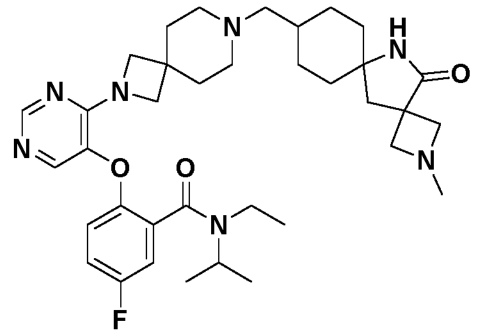
,

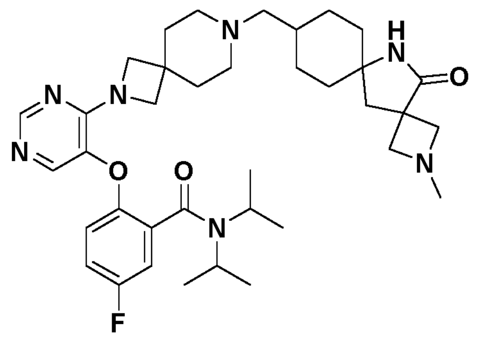
,
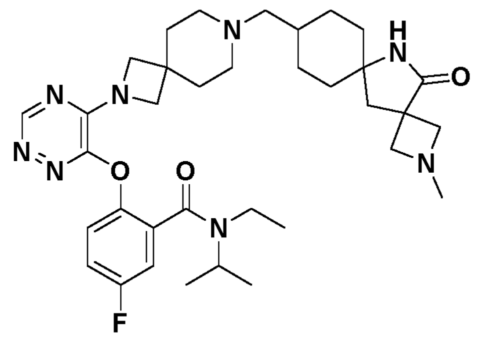
,

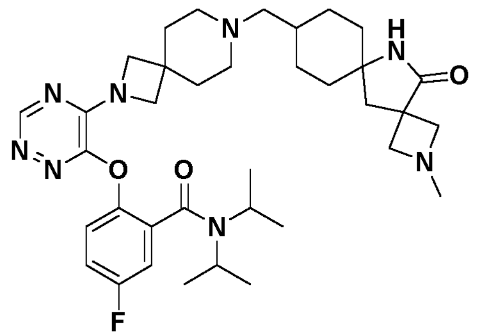
,
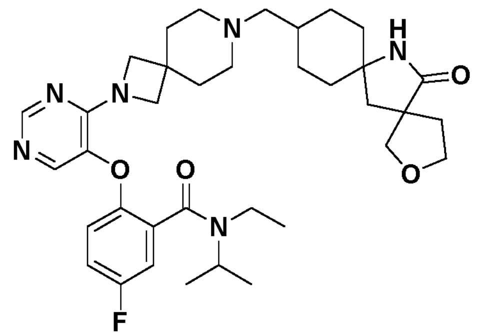
,

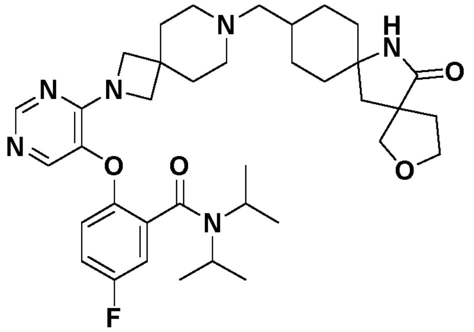

,

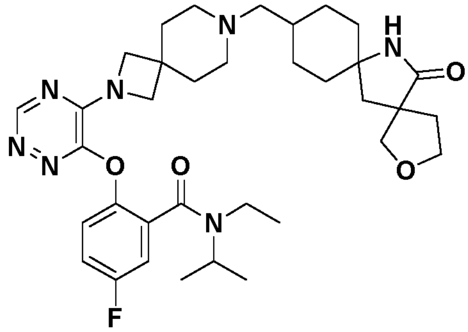

,

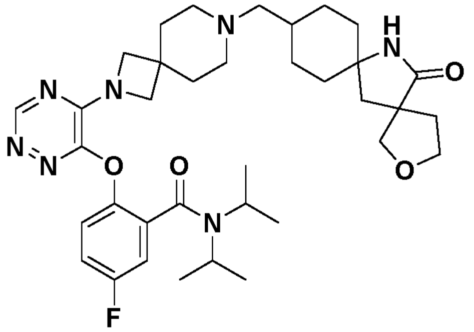

,

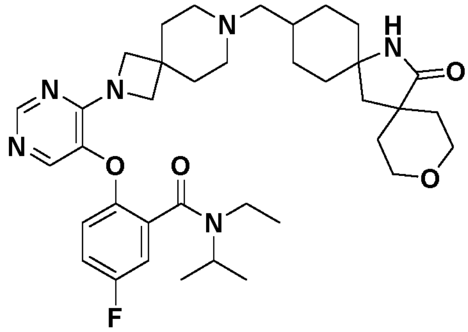

,

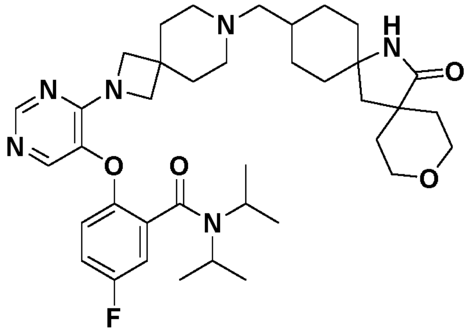

,

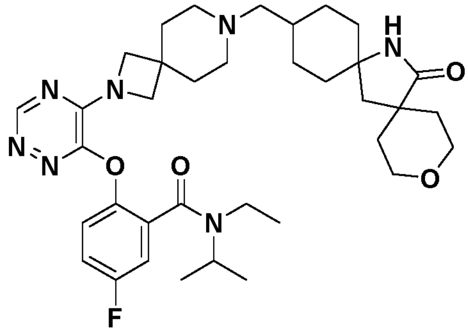

,

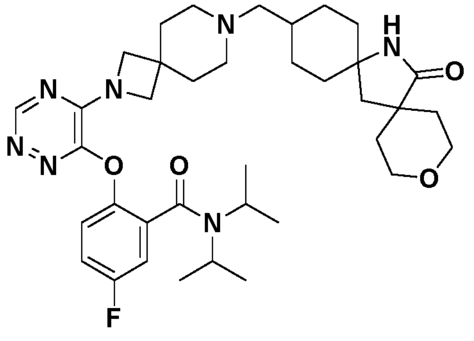

.

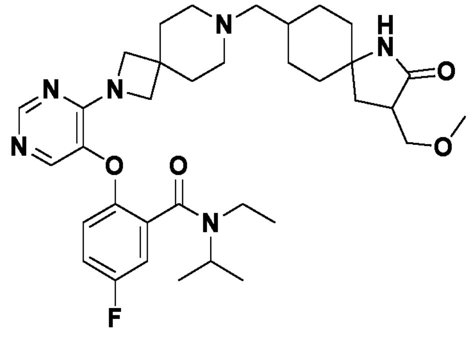

.

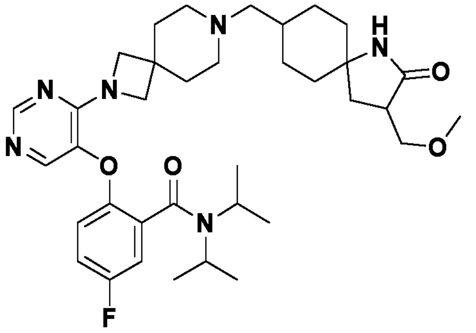

,

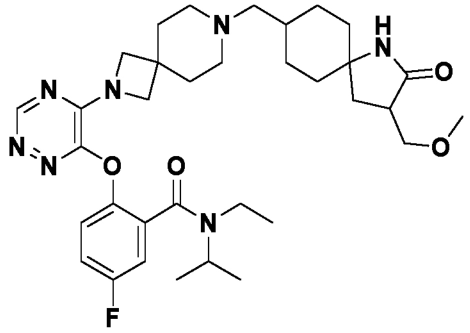

,

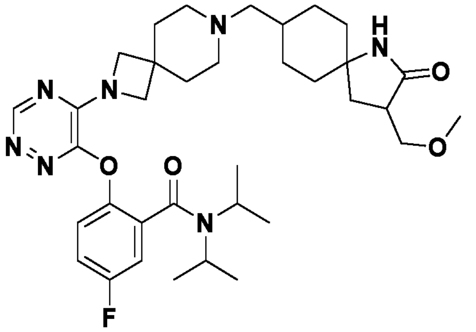

,

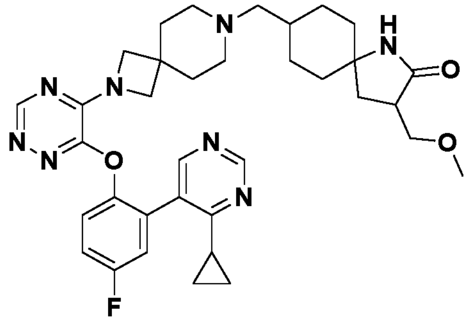

,

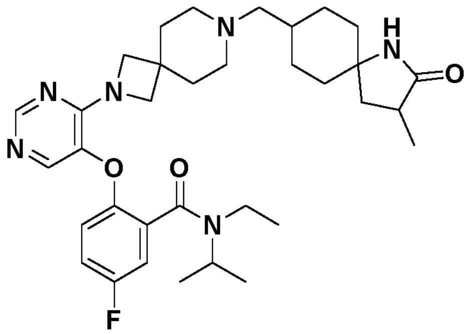

,

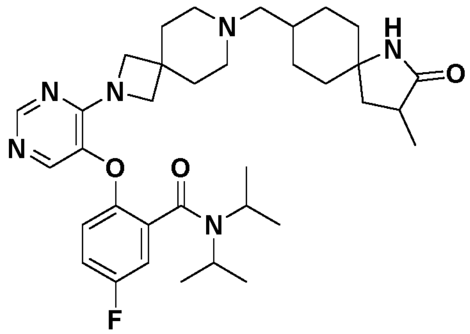

,

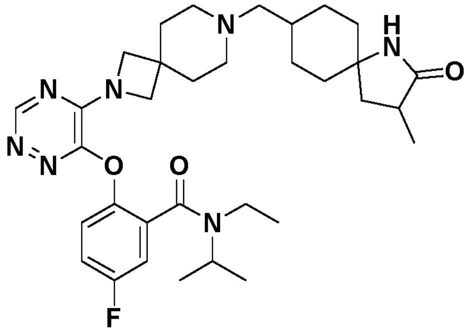

,

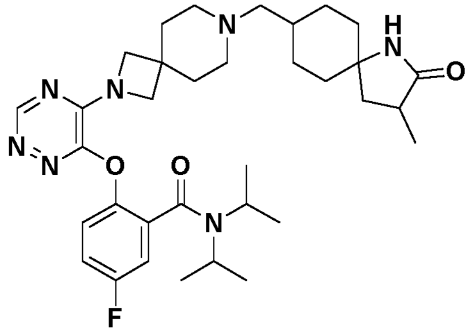

.

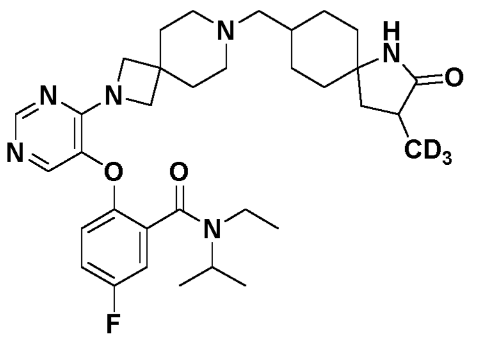
,
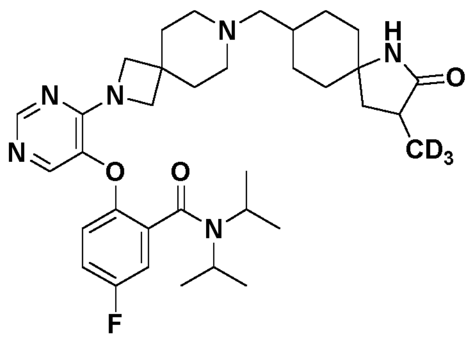
,
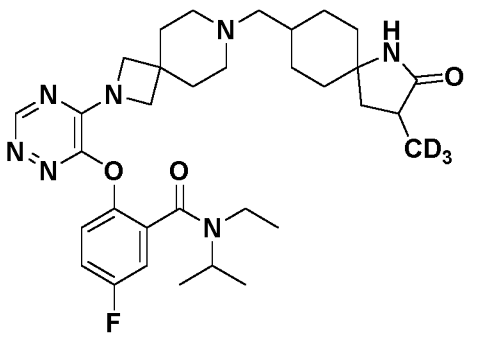
,
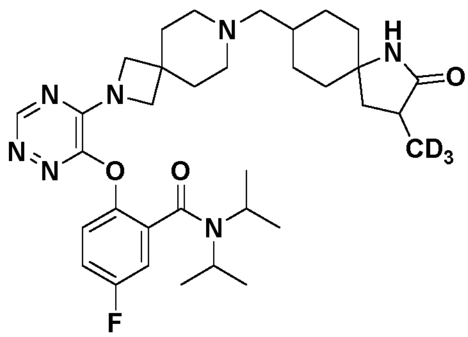
,
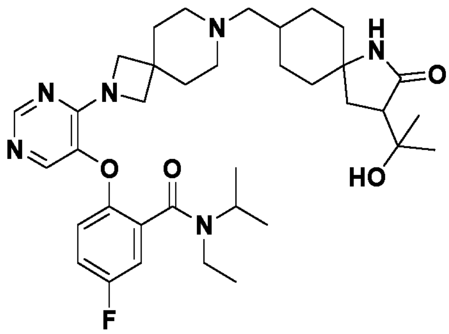
,
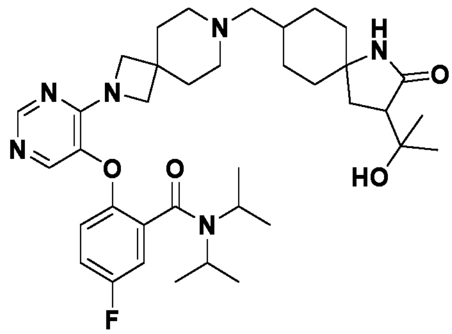
,
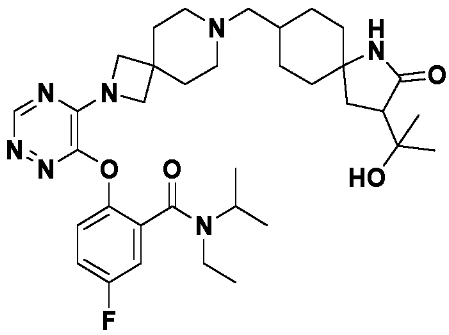
,
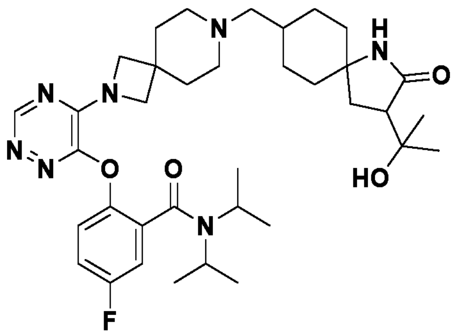
,

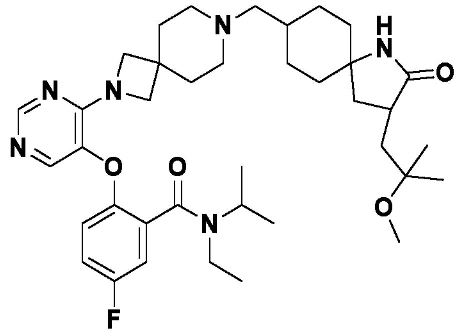

,

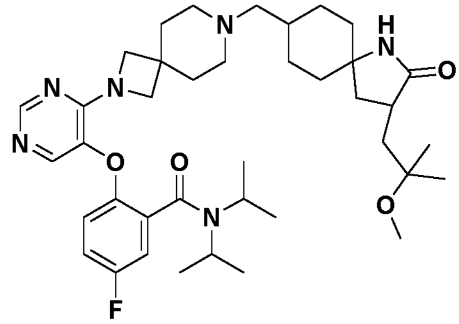

,

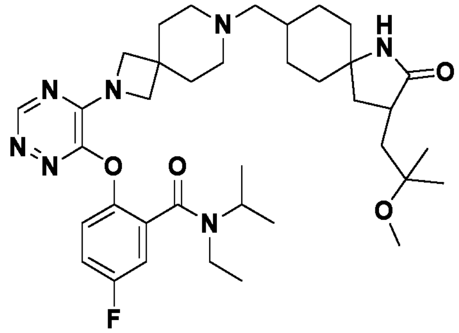

,

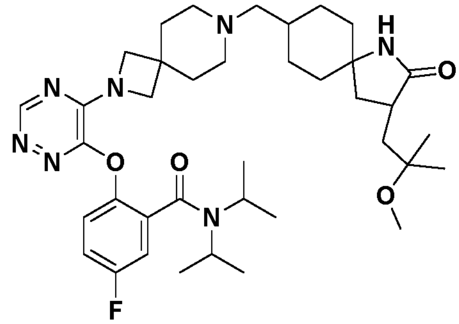

,

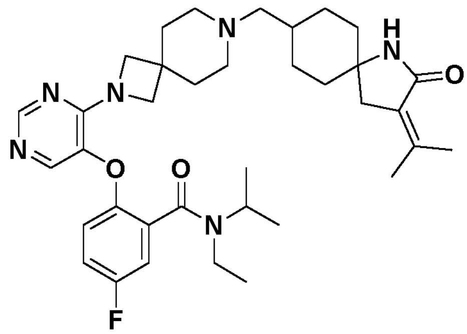

,

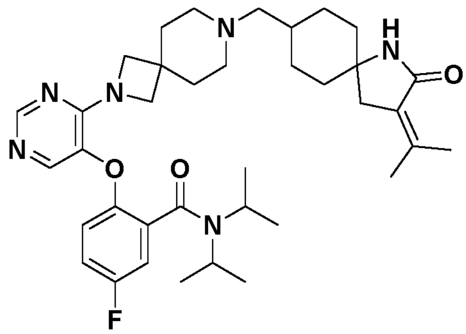

,

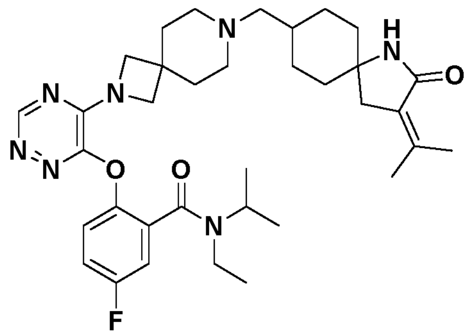

,

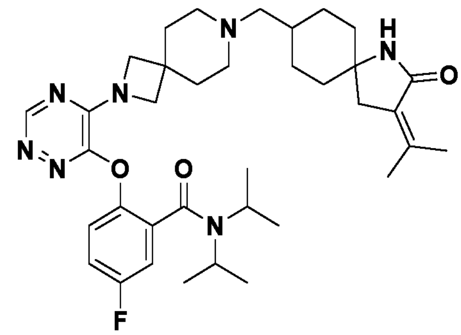

,

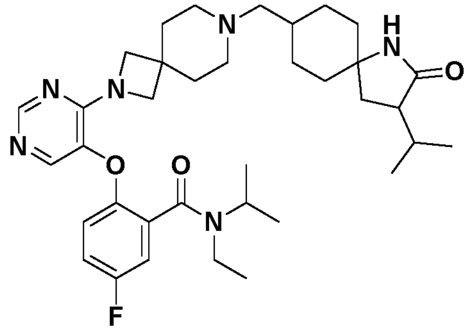

,

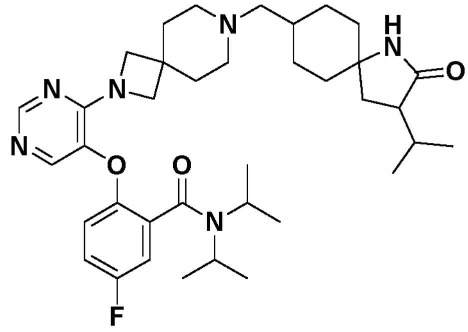

,

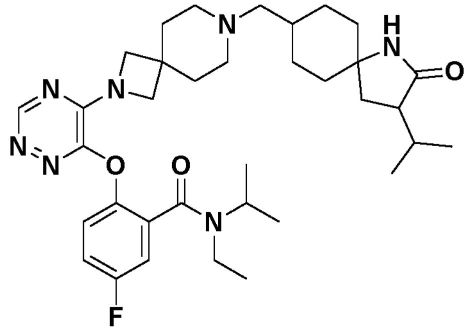

,

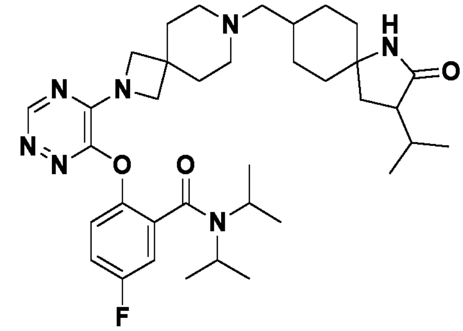

,

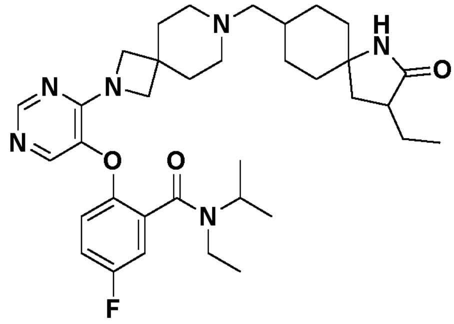

,

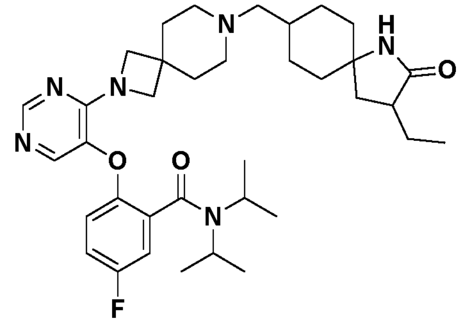

,

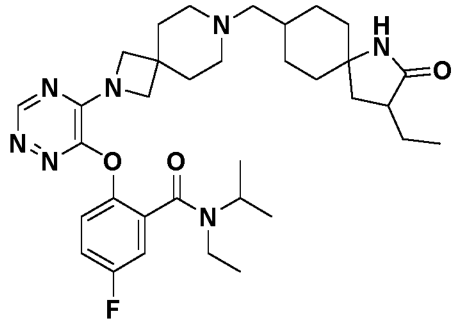

,

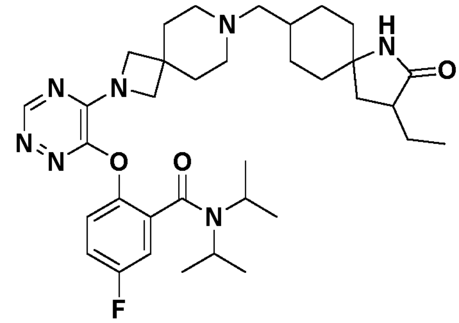

,

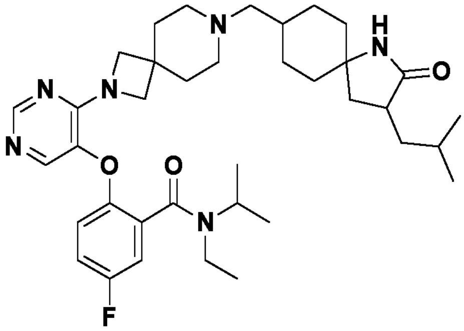
,

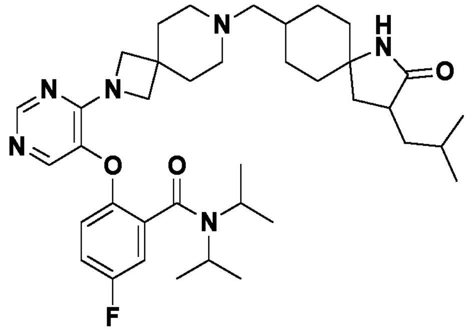
,

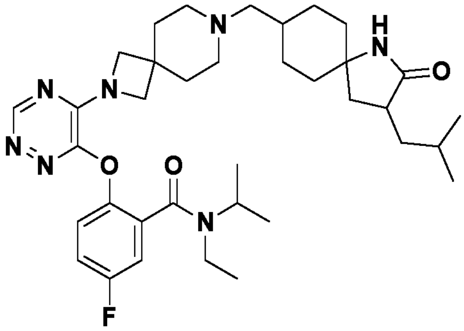
,

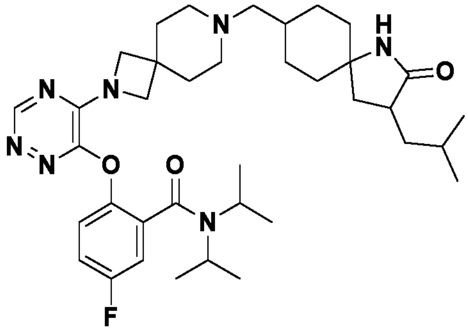
,

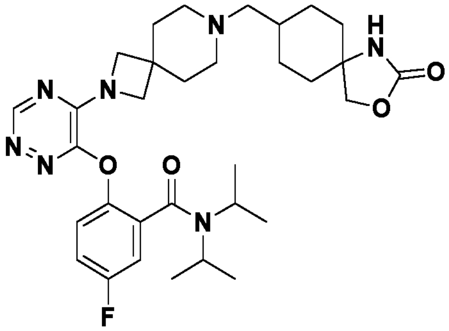
,

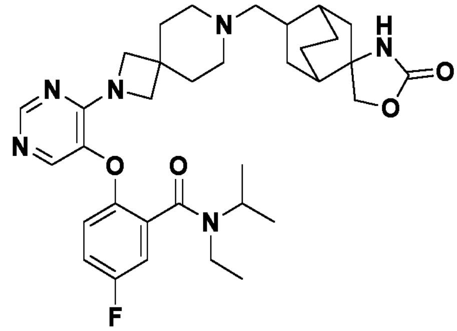
,

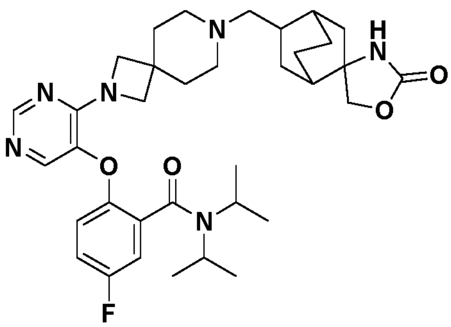
,

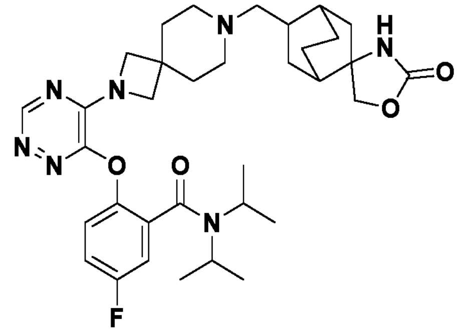
,

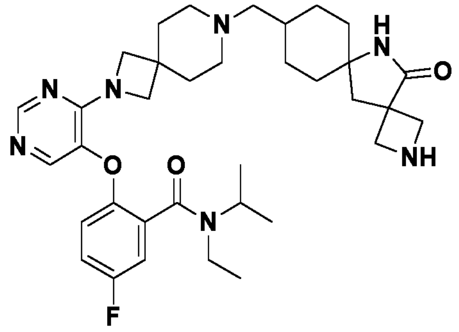

,

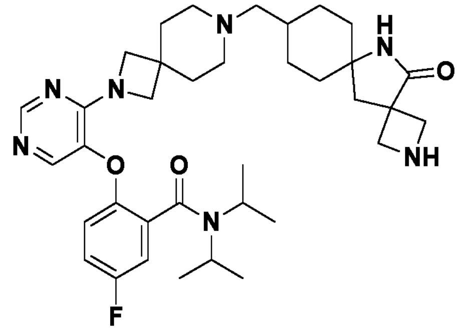

,

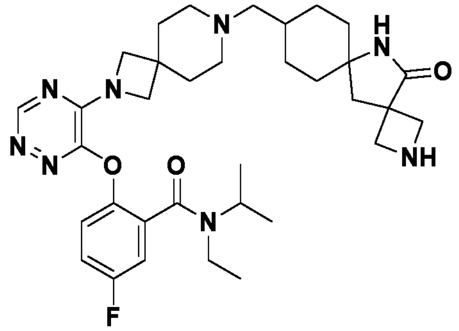

,

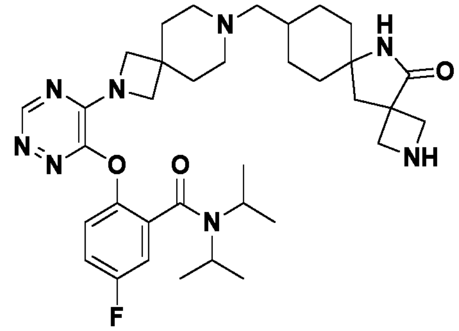

,

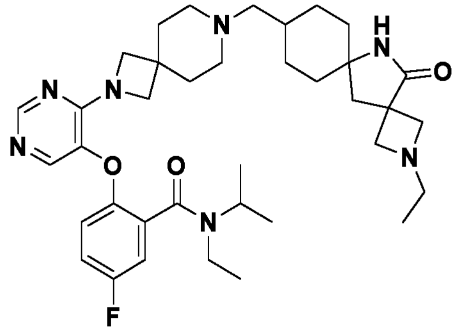

,

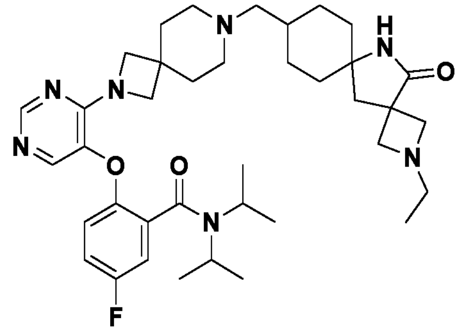

,

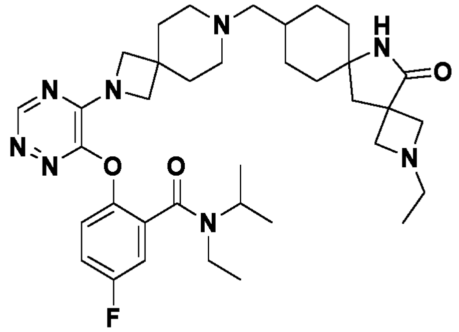

.

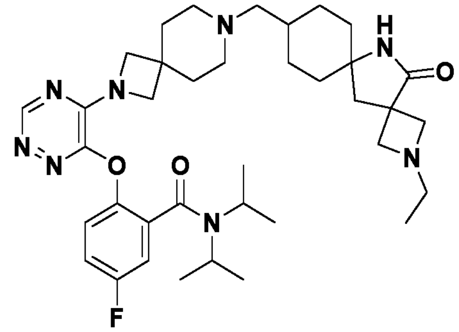

.

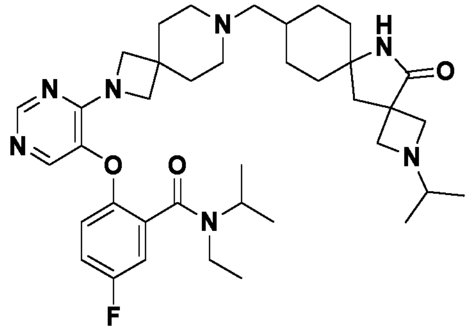

,

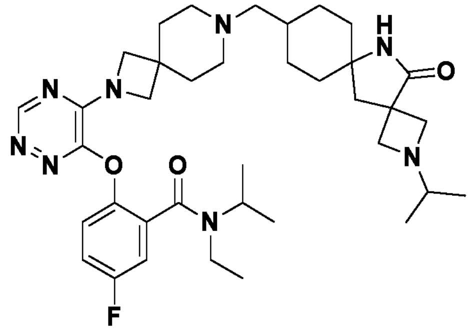

,

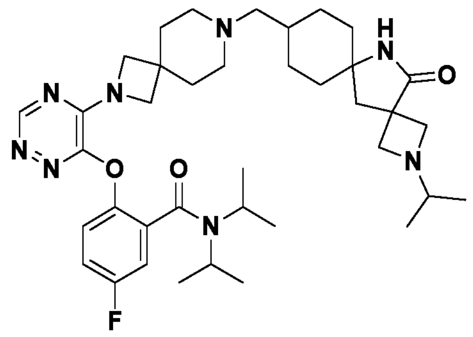

,

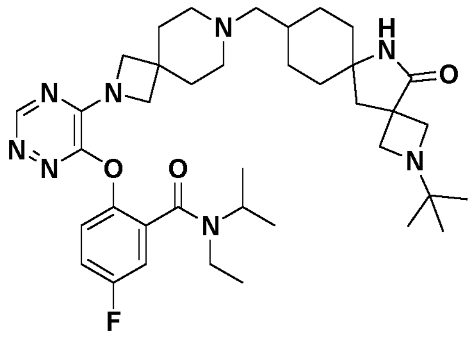

,

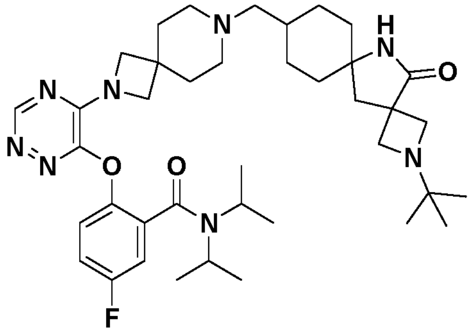

and

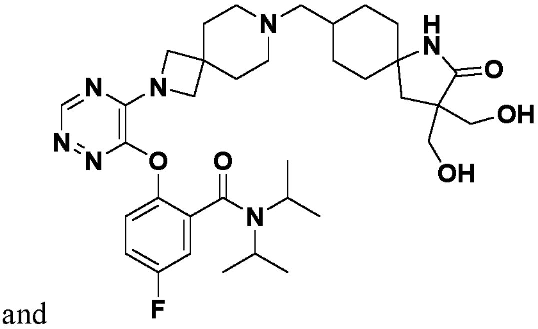

.

**19.** The compound according to any one of claims 1 to 17, wherein the compound is selected from the group consisting of:

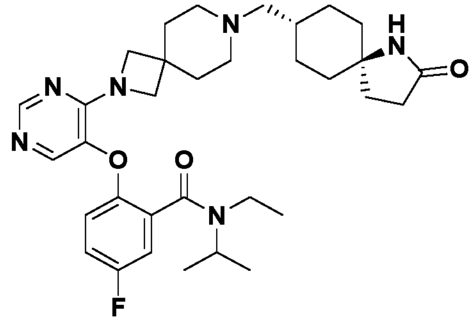

,

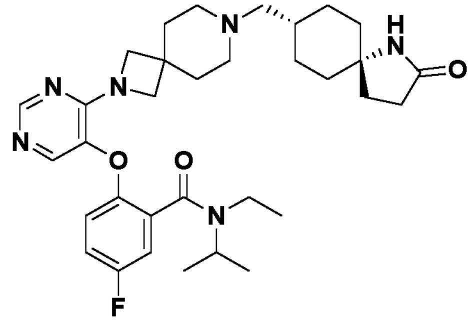

,

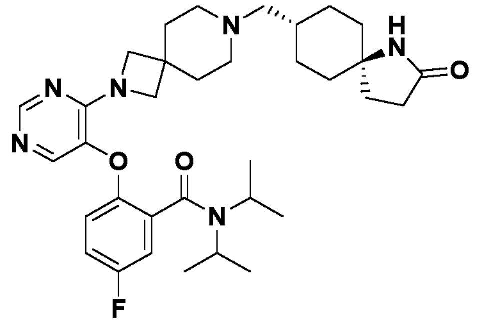
,

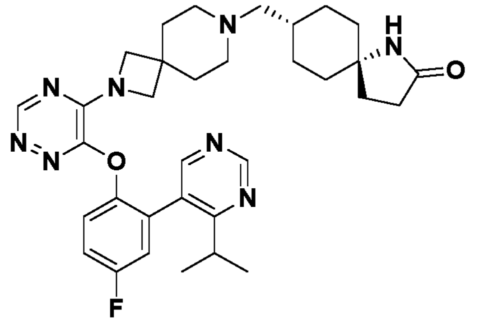
,

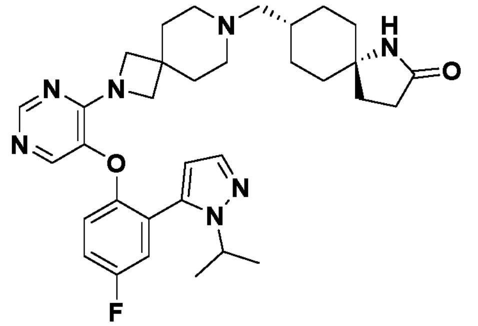
,

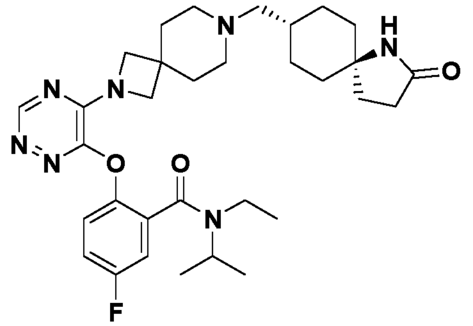
,

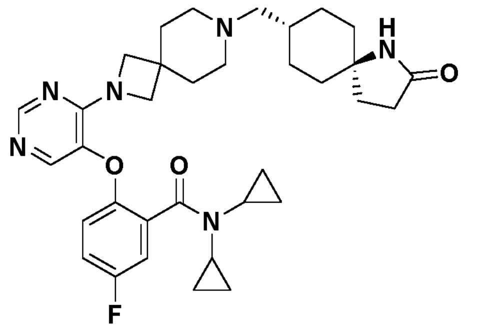
,

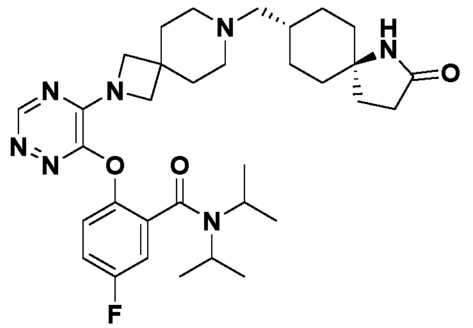
,

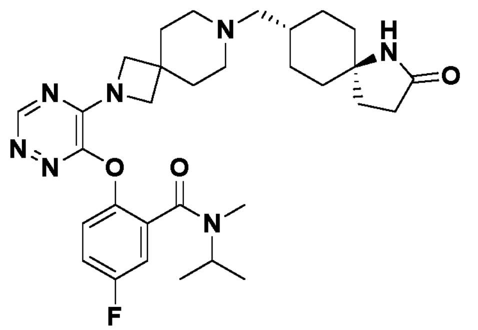
,

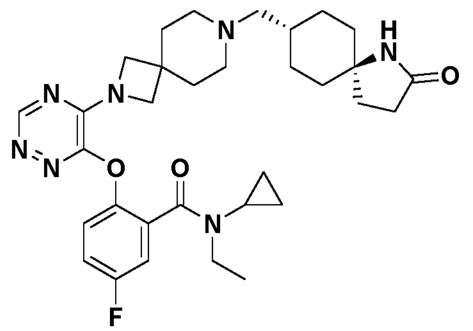
,

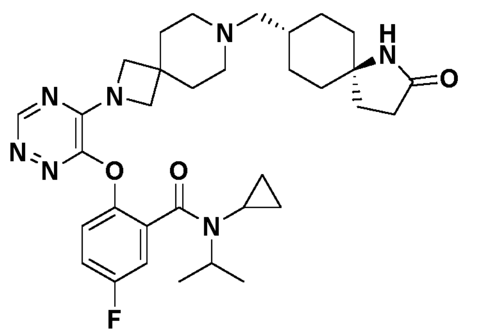
,
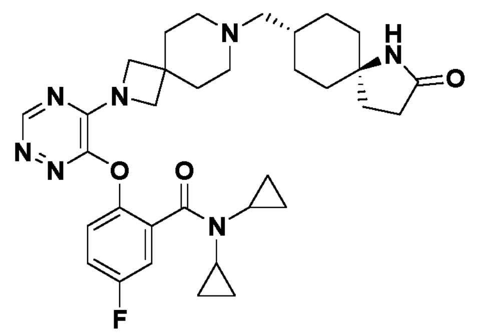
,

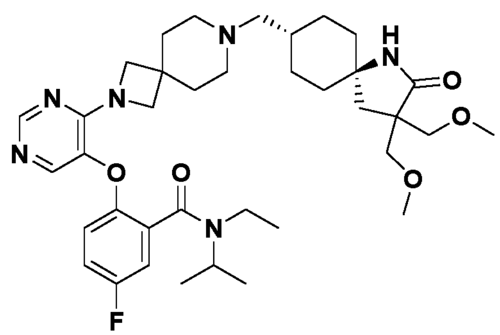
,
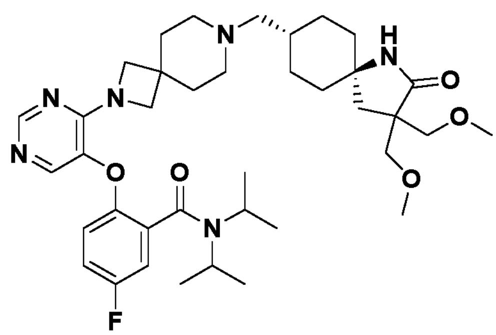
,

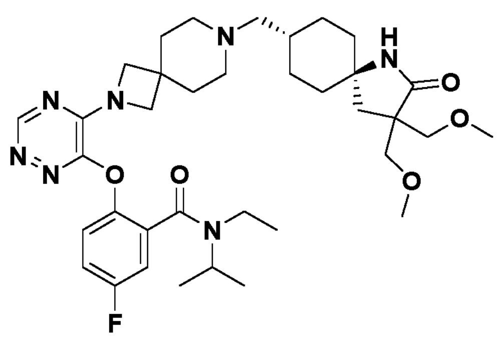
,
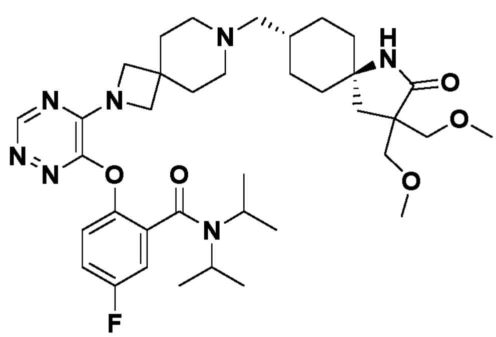
,

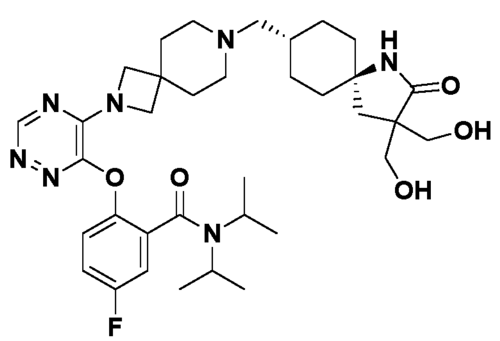
,
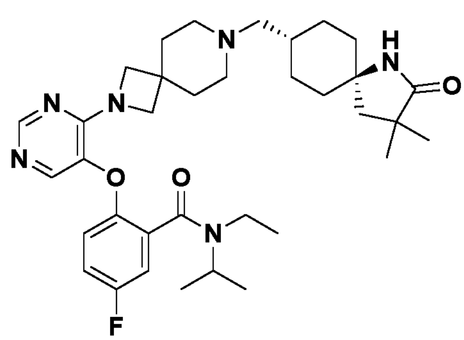
,

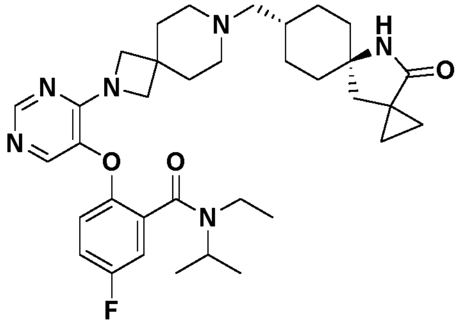
,
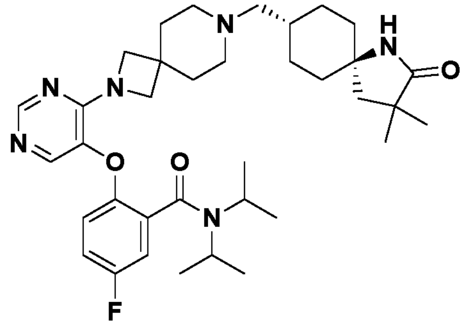
,
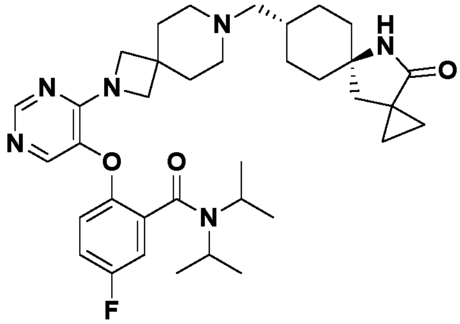
,
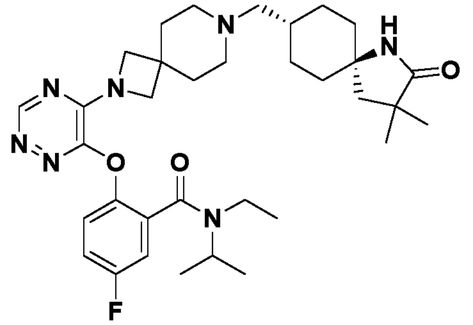
,
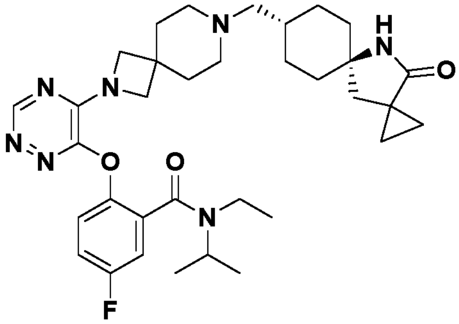
,
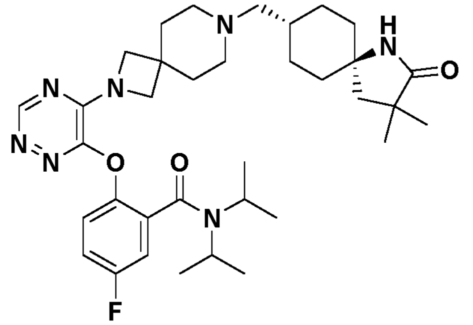
,
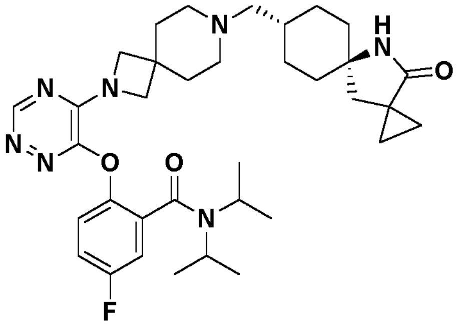
,
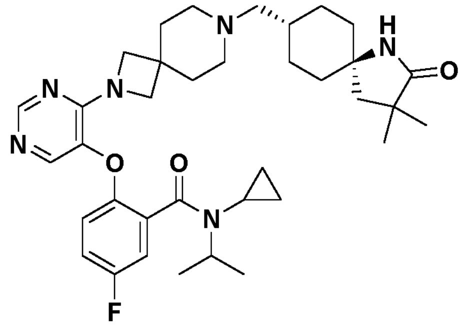
,

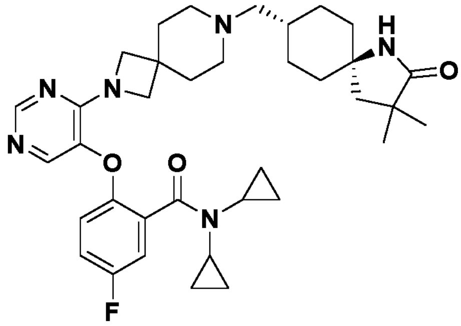,

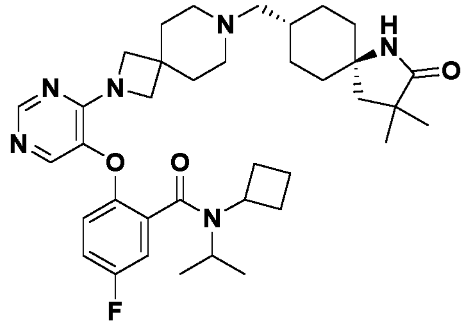,

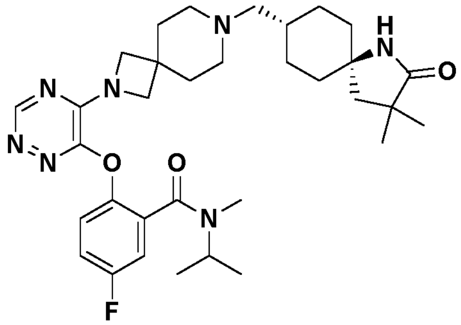,

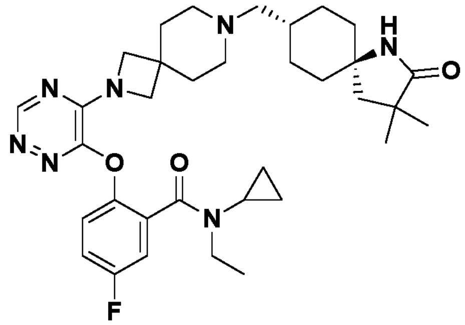,

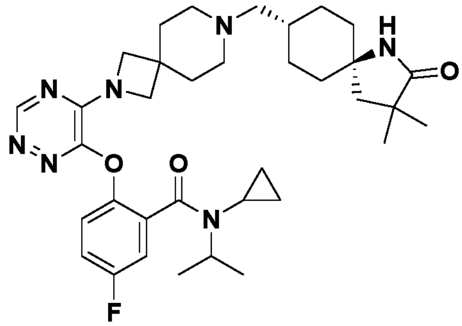,

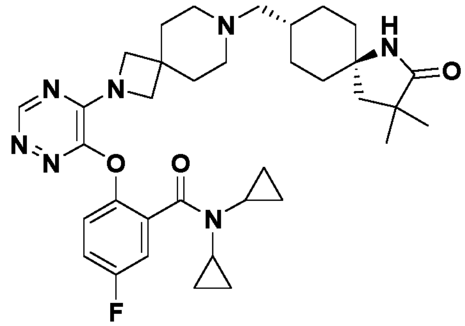,

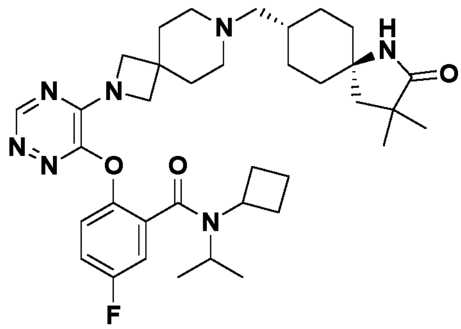,

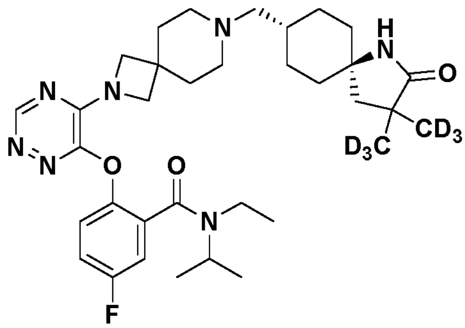,

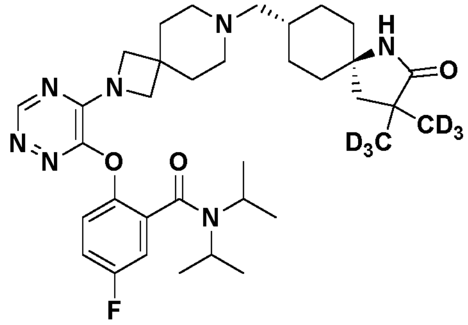

,

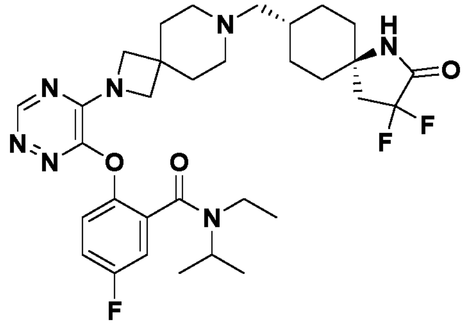

,

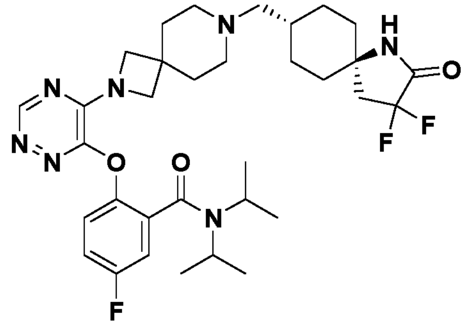

,

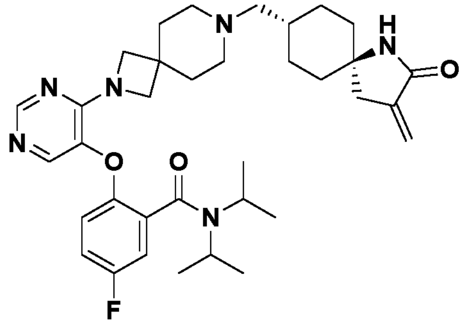

,

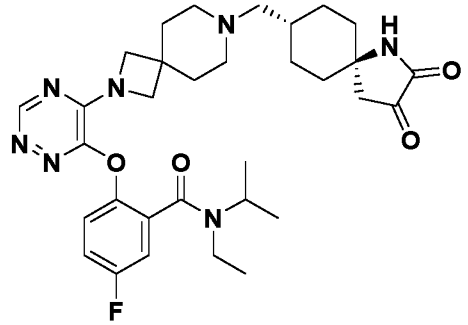

,

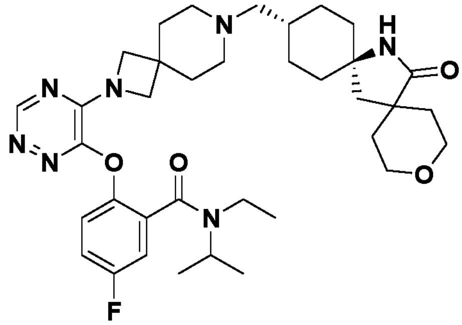

,

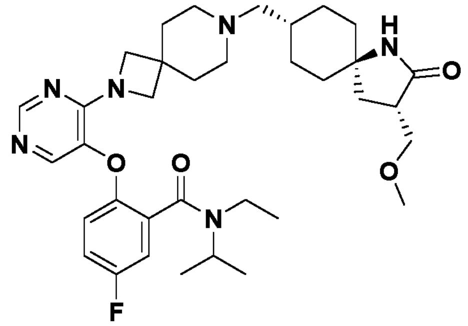

,

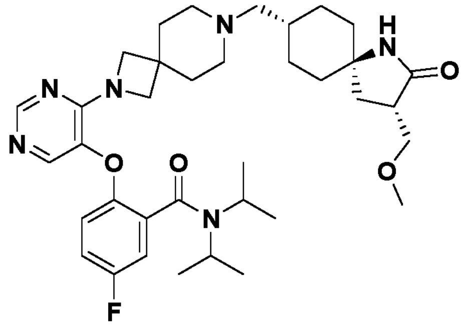

,

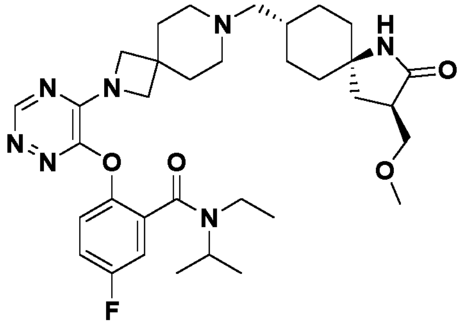

,

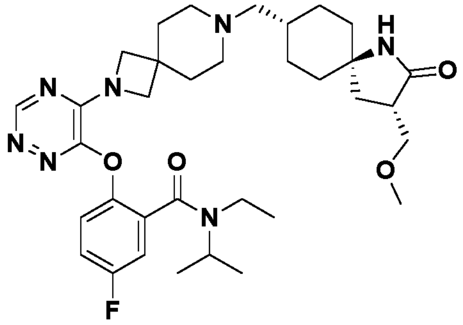

,

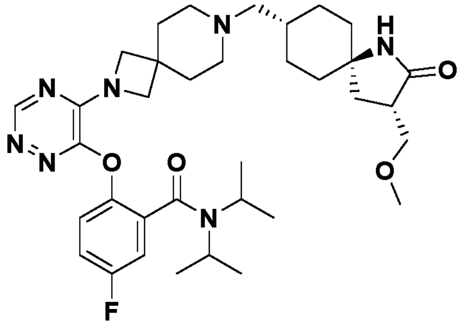

,

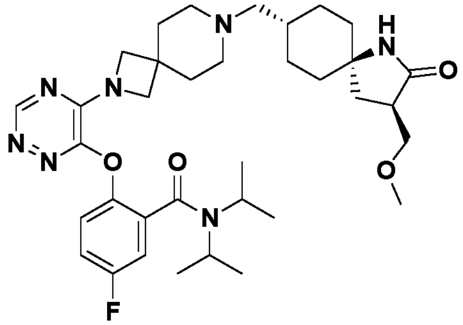

,

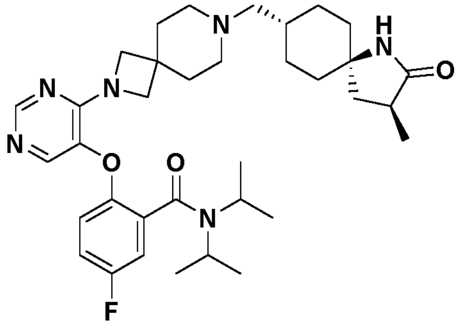

,

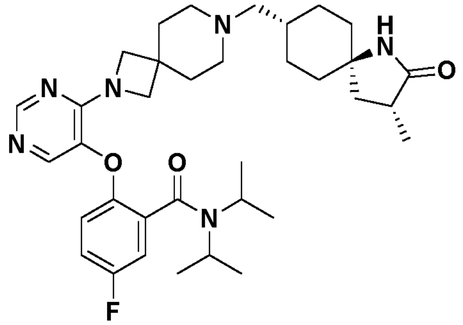

,

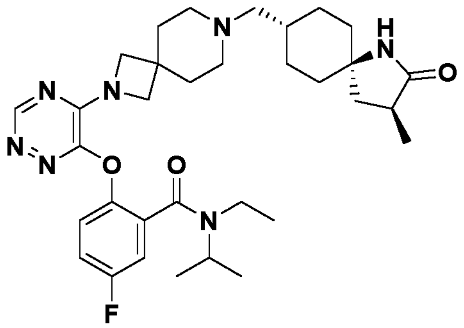

,

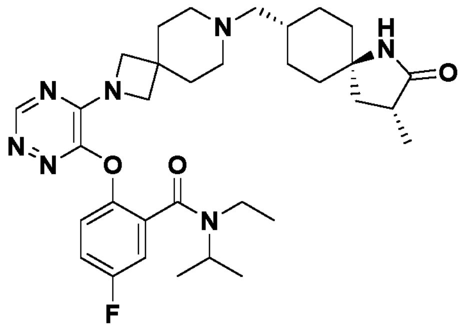

,

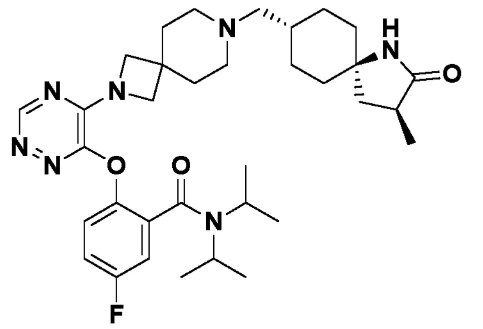

,

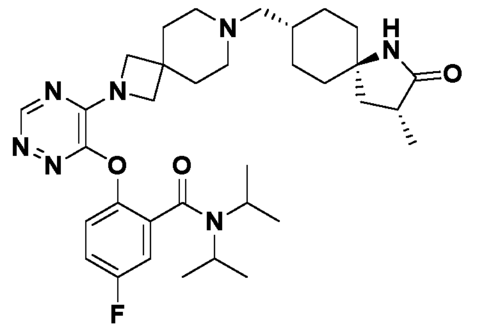

,

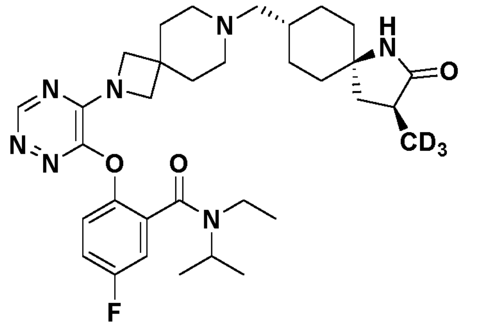

,

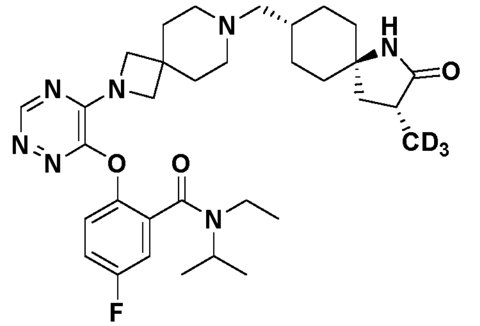

,

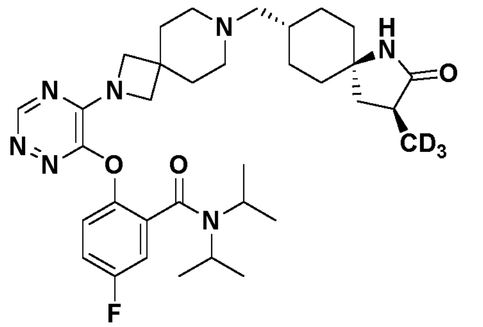

,

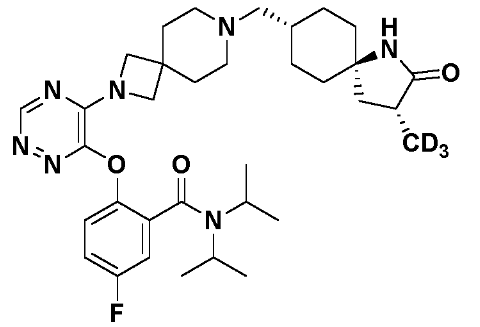

,

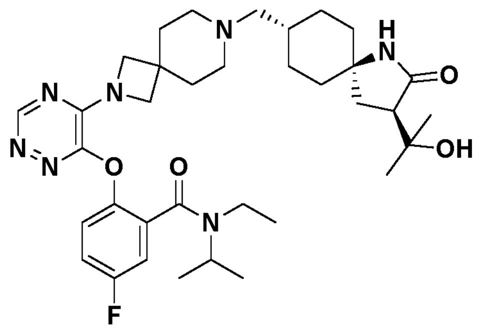

,

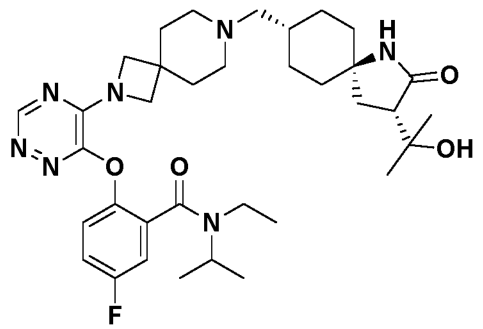

,

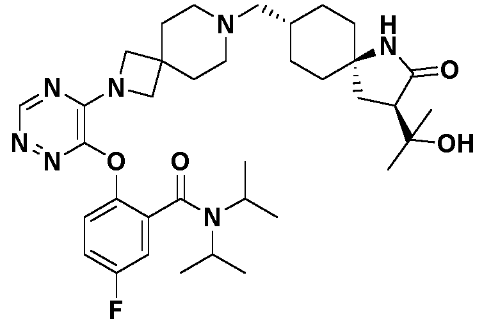
,
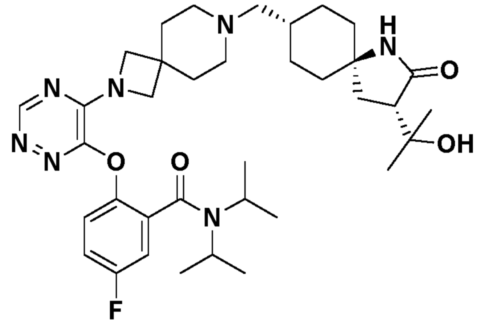
,

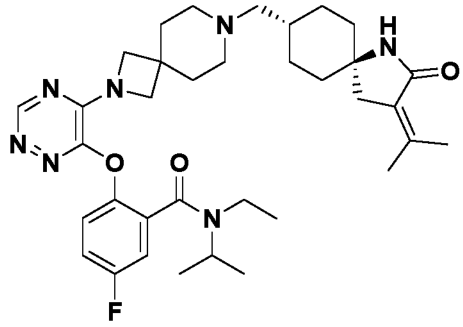
,
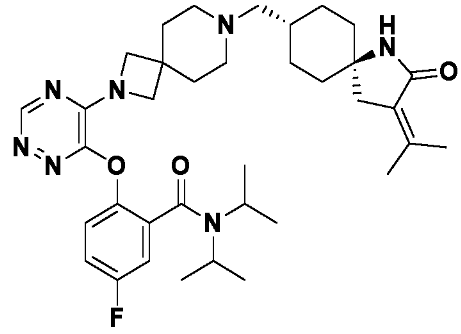
,

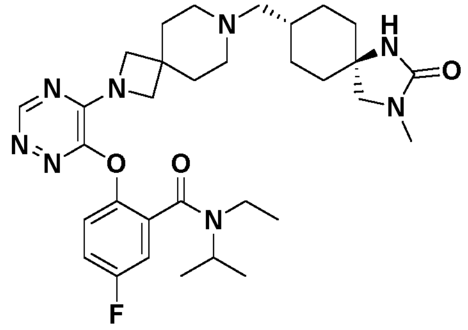
,
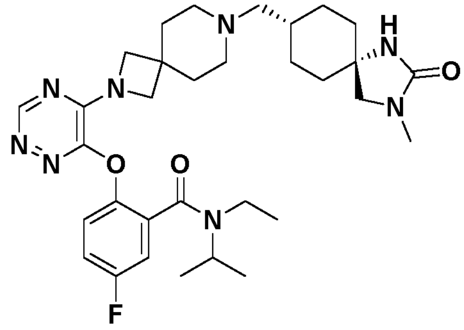
,

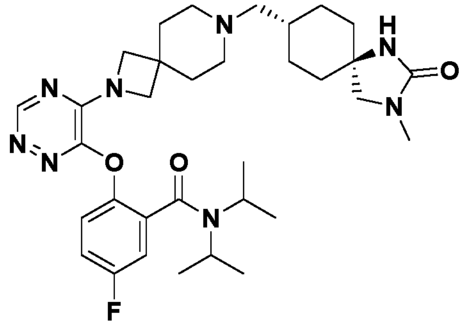
,
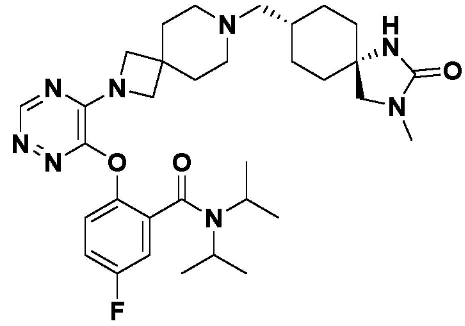
,

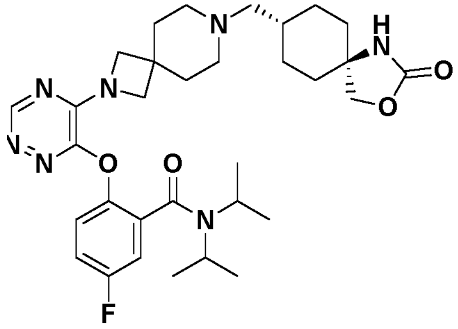

and

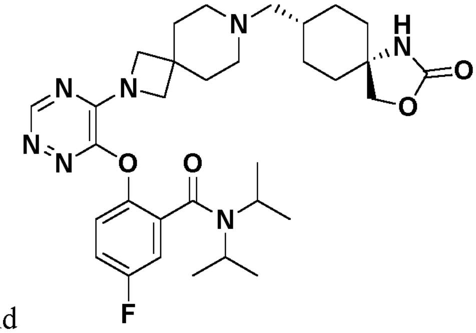

.

**20.** Use of the compound according to any one of claims 1 to 19, or the deuterated compound, stereoisomer thereof or pharmaceutically acceptable salt thereof in the manufacture of a medicament for treating a disease associated with abnormal Menin activity.

**21.** Use of the compound according to any one of claims 1 to 19, or the deuterated compound, stereoisomer thereof or pharmaceutically acceptable salt thereof in the manufacture of a medicament for treating cancer.

**22.** A pharmaceutical composition comprising a preparation prepared from the compound according to any one of claims 1 to 19, or the deuterated compound, stereoisomer thereof or pharmaceutically acceptable salt thereof.

**23.** The pharmaceutical composition according to claim 22, further comprising a pharmaceutically acceptable carrier, excipient or vehicle.

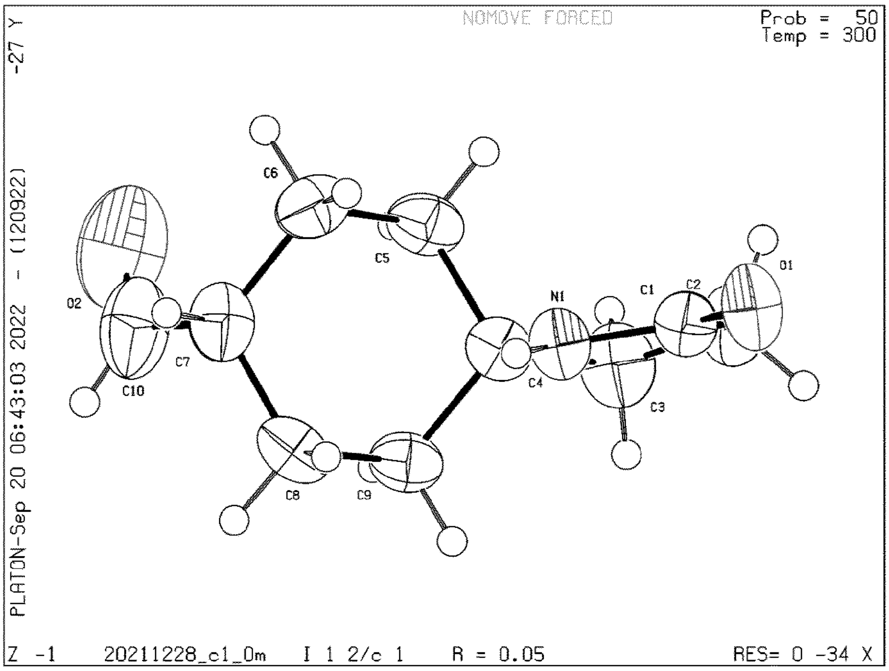
NOMOVE FORCED
Prob = 50
Temp = 300
-27 Y
PLATON-Sep 20 06:43:03 2022 - (120922)
Z -1 20211228_c1_0m I 1 2/c 1 R = 0.05 RES= 0 -34 X

FIG. 1

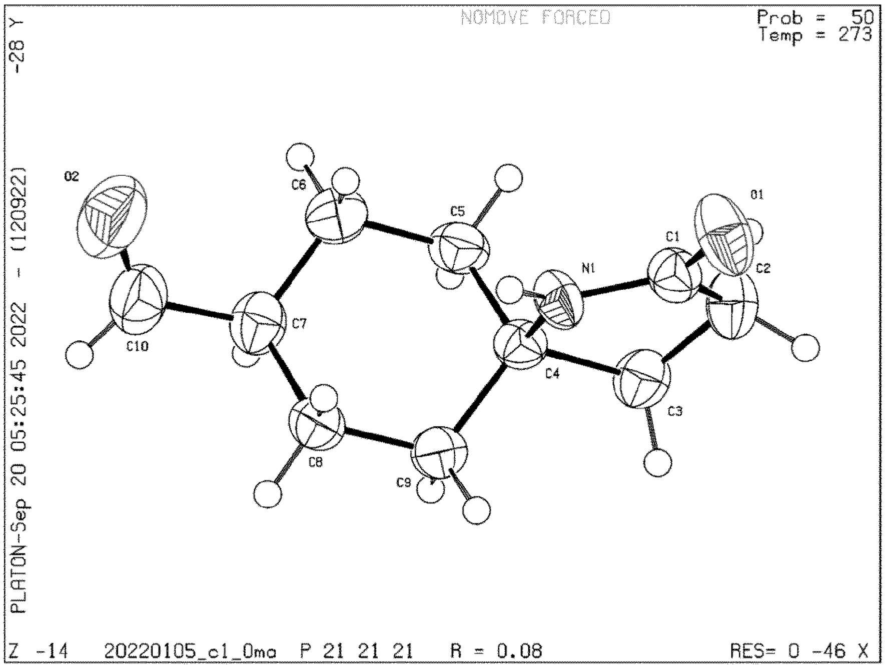
NOMOVE FORCED
Prob = 50
Temp = 273
-28 Y
PLATON-Sep 20 05:25:45 2022 - (120922)
Z -14 20220105_c1_0ma P 21 21 21 R = 0.08 RES= 0 -46 X

FIG. 2

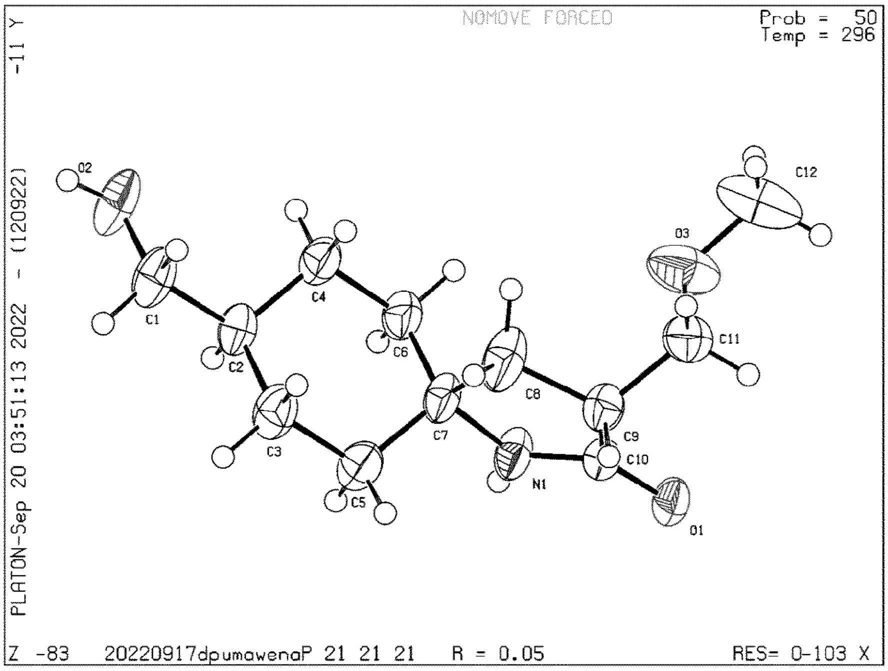
NOMOVE FORCED
Prob = 50
Temp = 296
-11 Y
PLATON-Sep 20 03:51:13 2022 - (120922)
Z -83 20220917dpumawenaP 21 21 21 R = 0.05
RES= 0-103 X

FIG. 3

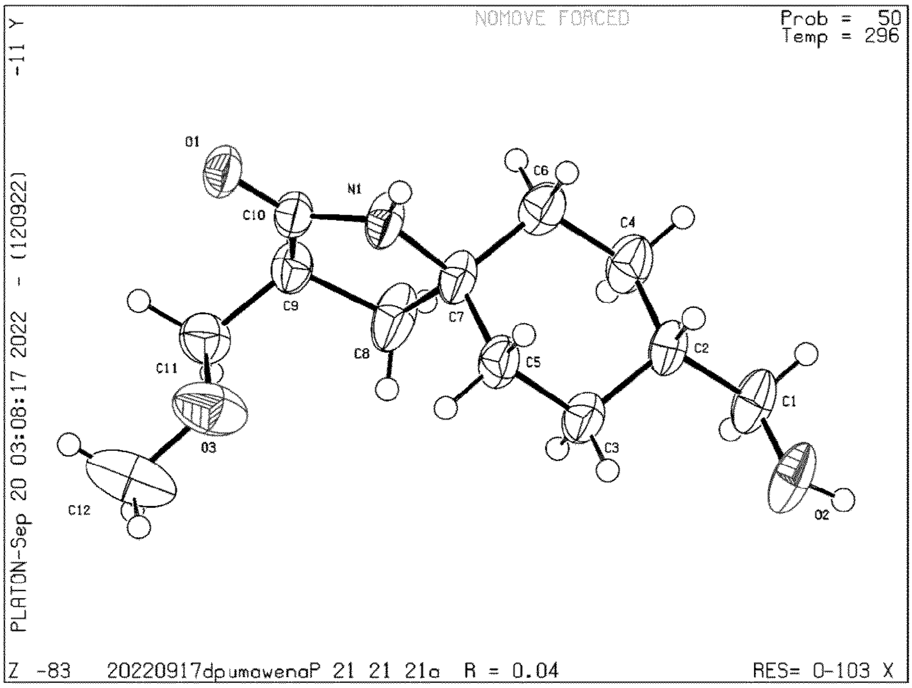
NOMOVE FORCED
Prob = 50
Temp = 296
-11 Y
PLATON-Sep 20 03:08:17 2022 - (120922)
Z -83 20220917dpumawenaP 21 21 21a R = 0.04
RES= 0-103 X

FIG. 4

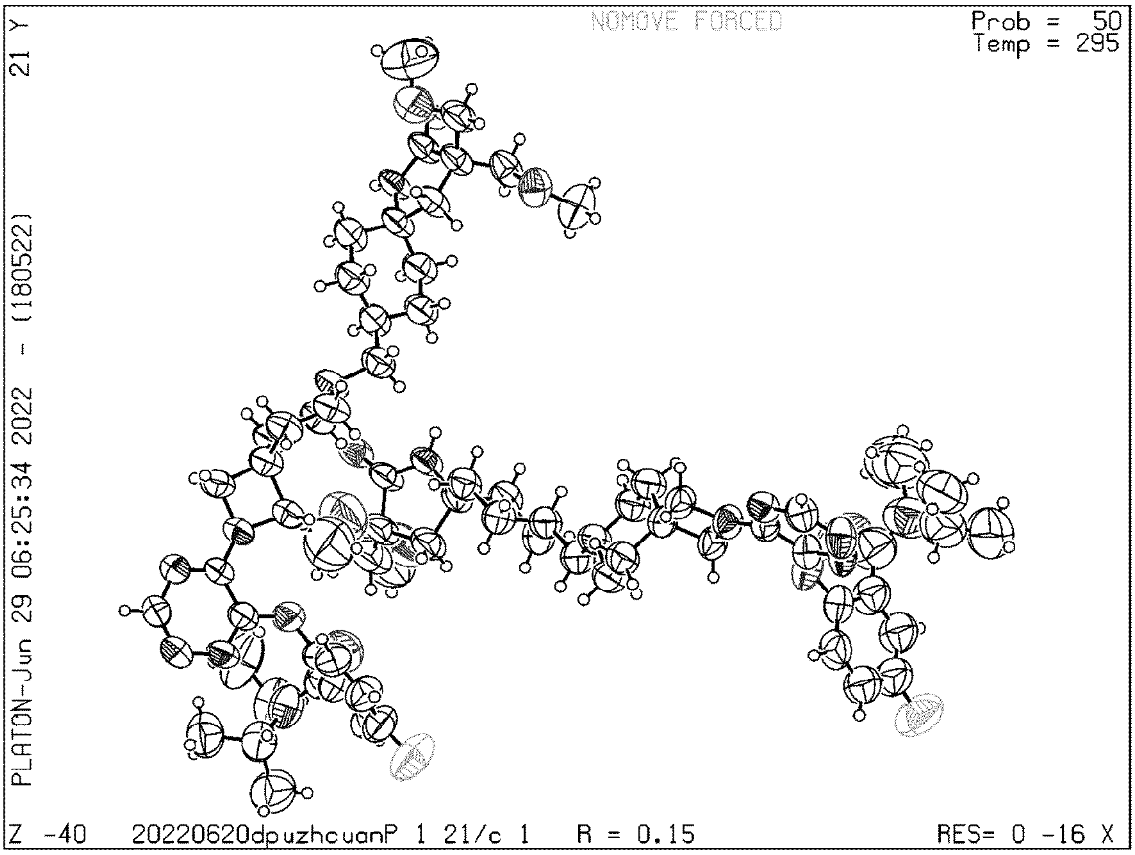
NOMOVE FORCED
Prob = 50
Temp = 295
21 Y
PLATON-Jun 29 06:25:34 2022 - (180522)
Z -40 20220620dpuzhcuanP 1 21/c 1 R = 0.15 RES= 0 -16 X

FIG. 5

## INTERNATIONAL SEARCH REPORT

International application No.
**PCT/CN2022/143908**

**A. CLASSIFICATION OF SUBJECT MATTER**

C07D 405/14(2006.01)i;A61K 31/407(2006.01)i;A61P 35/00(2006.01)i

According to International Patent Classification (IPC) or to both national classification and IPC

**B. FIELDS SEARCHED**

Minimum documentation searched (classification system followed by classification symbols)

C07D

Documentation searched other than minimum documentation to the extent that such documents are included in the fields searched

Electronic data base consulted during the international search (name of data base and, where practicable, search terms used)

CNTXT, WPABS, WPABSC, ENTXT, ENTXTC, DWPI, VEN, CJFD, REGISTRY, CAPLUS: MARPAT依据式I及实施例进行了亚结构检索, substructure search conducted by MARPAT according to formula I and embodiments, SNDX-5613, WO2017214367, menin, MLL

**C. DOCUMENTS CONSIDERED TO BE RELEVANT**

| Category* | Citation of document, with indication, where appropriate, of the relevant passages | Relevant to claim No. |
|---|---|---|
| A | WO 2014164543 A1 (THE REGENTS OF THE UNIVERSITY OF MICHIGAN) 09 October 2014 (2014-10-09)<br>embodiments | 1-23 |
| A | WO 2015191701 A1 (THE TRUSTEES OF THE UNIVERSITY OF PENNSYLVANIA) 17 December 2015 (2015-12-17)<br>embodiments | 1-23 |
| A | WO 2016040330 A1 (THE REGENTS OF THE UNIVERSITY OF MICHIGAN; KURA ONCOLOGY, INC.) 17 March 2016 (2016-03-17)<br>embodiments | 1-23 |
| A | WO 2017214367 A1 (VITAE PHARMACEUTICALS INC.) 14 December 2017 (2017-12-14)<br>embodiments | 1-23 |
| A | WO 2018175746 A1 (KURA ONCOLOGY, INC.) 27 September 2018 (2018-09-27)<br>embodiments | 1-23 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **08 February 2023** | **20 February 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| Facsimile No. **(86-10)62019451** | Telephone No. |

Form PCT/ISA/210 (second sheet) (July 2022)

**INTERNATIONAL SEARCH REPORT**
**Information on patent family members**

International application No.
**PCT/CN2022/143908**

| Patent document cited in search report | | | Publication date (day/month/year) | Patent family member(s) | | | Publication date (day/month/year) |
|---|---|---|---|---|---|---|---|
| WO | 2014164543 | A1 | 09 October 2014 | None | | | |
| WO | 2015191701 | A1 | 17 December 2015 | None | | | |
| WO | 2016040330 | A1 | 17 March 2016 | US | 2017247391 | A1 | 31 August 2017 |
| | | | | US | 10246464 | B2 | 02 April 2019 |
| WO | 2017214367 | A1 | 14 December 2017 | None | | | |
| WO | 2018175746 | A1 | 27 September 2018 | None | | | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2017214367 A